# EUROPEAN PATENT SPECIFICATION

(11) **EP 0 906 313 B1**
(45) Date of publication and mention of the grant of the patent: **03.03.2004**
(21) Application number: 97920438.5
(22) Date of filing: 21.04.1997
(51) Int. Cl.: C07D 477/14, C07D 519/00, A61K 31/41

(54) **CARBAPENEM ANTIBACTERIAL COMPOUNDS, COMPOSITIONS CONTAINING SUCH COMPOUNDS AND METHODS OF TREATMENT**
ANTIBAKTERIELLE CARBAPENEM-VERBINDUNGEN, SIE ENTHALTENDE ZUSAMMENSETZUNGEN UNDBEHANDLUNGSVERFAHREN
COMPOSES ANTIBACTERIENS A BASE DE CARBAPENEM, COMPOSITIONS CONTENANT DE TELS COMPOSES ET METHODES DE TRAITEMENT

(30) Priority: 24.04.1996 US 16184 P; 10.05.1996 GB 9609741
(43) Date of publication of application: 07.04.1999
(73) Proprietor: MERCK & CO., INC., Rahway, New Jersey 07065 (US)
(72) Inventor: WILKENING, Robert, R., Rahway, NJ 07065 (US); RATCLIFFE, Ronald, W., Rahway, NJ 07065 (US); BLIZZARD, Timothy, A., Rahway, NJ 07065 (US)
(74) Representative: Hiscock, Ian James
(86) International application number: PCT/US1997/006626
(87) International publication number: WO 1997/040048

(56) References cited:
- EP-A- 0 429 341
- WO-A-91/16323
- WO-A-95/21841

## Description

### BACKGROUND OF THE INVENTION

The present invention relates to carbapenem antibacterial agents in which the carbapenem nucleus is substituted at the 2-position with a naphthosultam linked through a CH₂ group. The naphthosultam is further substituted with various substituent groups including at least one cationic group.

The carbapenems of the present invention are useful against gram positive microorganisms, especially methicillin resistant Staphylococcus aureus (MRSA), methicillin resistant Staphylococcus epidermidis (MRSE), and methicillin resistant coagulase negative Staphylococci (MRCNS). The antibacterial compounds of the present invention thus comprise an important contribution to therapy for treating infections caused by these difficult to control pathogens. There is an increasing need for agents effective against such pathogens (MRSA/MRCNS) which are at the same time relatively free from undesirable side effects.

### SUMMARY OF THE INVENTION

The compounds of the invention are represented by formula I: 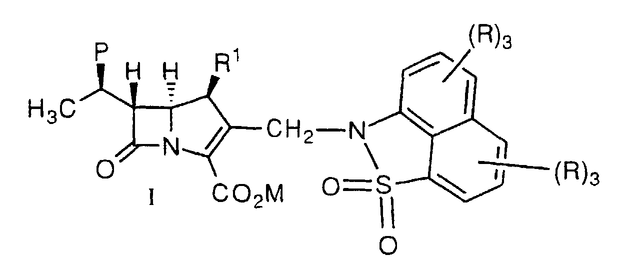 or a pharmaceutically acceptable salt thereof, wherein:
R¹ represents H or methyl;
CO₂M represents a carboxylic acid, a carboxylate anion, a pharmaceutically acceptable ester group or a carboxylic acid protected by a protecting group;
P represents hydrogen, hydroxyl, F or hydroxyl protected by a hydroxyl-protecting group;
each R is independently selected from: -R*; -Q; hydrogen; halo; -CN; -NO₂; -NR^{a}R^{b}; -OR^{c}; -SR^{c}; -C(O)NR^{a}R^{b}; -C(O)OR^{h}; -S(O)R^{c}; -SO₂R^{c}; -SO₂NR^{a}R^{b}; -NR^{a}SO₂R^{b}; -C(O)R^{a}; -OC(O)R^{a}; -OC(O)NR^{a}R^{b}; -NR^{a}C(O)NR^{b}R^{c}; -NR^{a}CO₂R^{h}; -OCO₂R^{h}; - NR^{a}C(O)R^{b}; -C₁₋₆ straight- or branched-chain alkyl, unsubstituted or substituted with one to four R^{d} groups; and -C₃₋₇ cycloalkyl, unsubstituted or substituted with one to four R^{d} groups;
   with the proviso that at least one R is present which contains at least one positive charge;
each R^{a}, R^{b} and R^{c} independently represents hydrogen, -R*, -C₁₋₆ straight- or branched-chain alkyl, unsubstituted or substituted with one to four R^{d} groups, or -C₃₋₇ cycloalkyl, unsubstituted or substituted with one to four R^{d} groups;
   or R^{a} and R^{b} taken together with any intervening atoms represent a 4-6 membered saturated ring optionally interrupted by one or more of O, S, NR^{c}, with R^{c} as defined above, or -C(O)-, said ring being unsubstituted or substituted with one to four Rⁱ groups;
   or R^{b} and R^{c} taken together with any intervening atoms represent a 4-6 membered saturated ring optionally interrupted by one to three of 0, S, NR^{a}, with R^{a} as defined above, or -C(O)-, said ring being unsubstituted or substituted with one to four Rⁱ groups;
each R^{d} independently represents halo; -CN; -NO₂; -NR^{e}R^{f}; -OR^{g}; -SR^{g}; -CONR^{e}R^{f}; -COOR^{g}; -SOR^{g}; -SO₂R^{g}; -SO₂NR^{e}R^{f}; - NR^{e}SO₂R^{f}; -COR^{e}; -NR^{e} COR^{f}; -OCOR^{e}; -OCONR^{e}R^{f}; -NR^{e}CONR^{f}R^{g}; - NR^{e}CO₂R^{h}; -OCO₂R^{h}; -C(NR^{e})]NR^{f}R^{g}; -NR^{e}C(NH)NR^{f}R^{g}; - NR^{e}C(NR^{f})R^{g}; -R* or -Q;
R^{e}, R^{f} and R^{g} represent hydrogen; -R*; -C₁₋₆ straight- or branched-chain alkyl unsubstituted or substituted with one to four Rⁱ groups;
   or R^{e} and R^{f} taken together with any intervening atoms represent a 4-6 membered saturated ring optionally interrupted by one to three of O, S, -C(O)- or NR^{g} with R^{g} as defined above, said ring being unsubstituted or substituted with one to four Rⁱ groups;
each Rⁱ independently represents halo; -CN; -NO₂; phenyl; -NHSO₂R^{h}; -OR^{h}, -SR^{h}; -N(R^{h})₂; -N⁺(R^{h})₃; -C(O)N(R^{h})₂; -SO₂N(R^{h})₂; heteroaryl; heteroarylium; -CO₂R^{h}; -C(O)R^{h}; -OCOR^{h}; -NHCOR^{h}; guanidinyl; carbamimidoyl or ureido;
each R^{h} independently represents hydrogen, a -C₁₋₆ straight or branched-chain alkyl group, a -C₃-C₆ cycloalkyl group or phenyl, or when two R^{h} groups are present, said R^{h} groups may be taken in combination and represent a 4-6 membered saturated ring, optionally interrupted by one or two of O, S, SO₂, -C(O)-, NH and NCH₃;
Q is selected from the group consisting of: 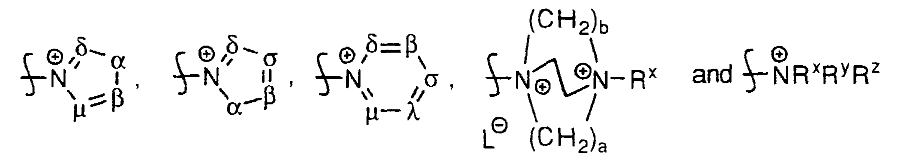 wherein:
   a and b are 1, 2 or 3;
   L⁻ is a pharmaceutically acceptable counterion;
   α represents 0, S or NR^{s};
   β, δ, λ, µ and σ represent CR^{t}, N or N⁺R^{s}, provided that no more than one of β, δ, λ, µ and σ is N⁺R^{s};
R* is selected from the group consisting of: 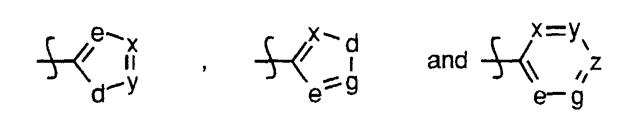 wherein:
   d represents O, S or NR^{k};
   e, g, x, y and z represent CR^{m}, N or N⁺R^{k}, provided that no more than one of e, g, x, y and z in any given structure represents N⁺R^{k};
R^{k} represents hydrogen; -C₁₋₆ straight- or branched-chain alkyl, unsubstituted or substituted with one to four Rⁱ groups; or -(CH₂)ₙQ where n = 1, 2 or 3 and Q is as previously defined;
each R^{m} independently represents a member selected from the group consisting of: hydrogen; halo; -CN; -NO₂; -NRⁿR^{o}; -ORⁿ; -SRⁿ; -CONRⁿR^{o}; -COOR^{h}; -SORⁿ; -SO₂Rⁿ; -SO₂NRⁿR^{o}; -NRⁿSO₂R^{o}; -CORⁿ; -NRⁿCOR^{o}; -OCORⁿ; -OCONRⁿR^{o}; -NRⁿCO₂R^{h}; - NRⁿCONR^{o}R^{h}; -OCO₂R^{h}; -CNRⁿNR^{o}R^{h}; -NRⁿCNHNR^{o}R^{h}; - NRⁿC(NR^{o})R^{h}; -C₁₋₆ straight- or branched-chain alkyl, unsubstituted or substituted with one to four Rⁱ groups; -C₃₋₇ cycloalkyl, unsubstituted or substituted with one to four Rⁱ groups; and -(CH₂)ₙQ where n and Q are as defined above;
Rⁿ and R^{o} represent hydrogen, phenyl; -C₁₋₆ straight- or branched-chain alkyl unsubstituted or substituted with one to four Rⁱ groups;
each R^{s} independently represents hydrogen; phenyl or -C₁₋₆ straight- or branched-chain alkyl, unsubstituted or substituted with one to four Rⁱ groups;
each R^{t} independently represents hydrogen; halo; phenyl; - CN; -NO₂; -NR^{u}R^{v}; -OR^{u}; -SR^{u}; -CONR^{u}R^{v}; -COOR^{h}; -SOR^{u}; -SO₂R^{u}; - SO₂NR^{u}R^{v}; -NR^{u}SO₂R^{v}; -COR^{u}; -NR^{u}COR^{v}; -OCOR^{u}; -OCONR^{u}R^{v}; - NR^{u}CO₂R^{v}; -NR^{u}CONR^{v}R^{w}; -OCO₂R^{v}; -C₁₋₆ straight- or branched-chain alkyl, unsubstituted or substituted with one to four Rⁱ groups;
R^{u} and R^{v} represent hydrogen or -C₁₋₆ straight- or branched-chain alkyl, unsubstituted or substituted with one to four Rⁱ groups;
   or R^{u} and R^{v} together with any intervening atoms represent a 4-6 membered saturated ring optionally interrupted by one or more of O, S, NR^{w} or -C(O)-, said ring being unsubstituted or substituted with one to four Rⁱ groups;
each R^{w} independently represents hydrogen; -C₁₋₆ straight- or branched-chain alkyl, unsubstituted or substituted with one to four Rⁱ groups; C₃₋₆ cycloalkyl optionally substituted with one to four Rⁱ groups; phenyl optionally substituted with one to four Rⁱ groups, or heteroaryl optionally substituted with 1-4 Rⁱ groups;
   or R^{h} and R^{w} taken together with any intervening atoms represent a 5-6 membered saturated ring, optionally interrupted by one or two of O, S, SO₂, NH or NCH₃;
R^{X} represents hydrogen or a C₁₋₈ straight- or branched-chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched-chain alkyl groups, said alkyl groups being unsubstituted or substituted with one to four Rⁱ groups;
R^{y} and R^{z} represent hydrogen; phenyl; -C₁₋₆ straight or branched chain alkyl, unsubstituted or substituted with one to four Rⁱ groups, and optionally interrupted by O, S, NR^{w}, N⁺R^{h}R^{w} or -C(O)-;
or R^{x} and R^{y} together with any intervening atoms represent a 4-6 membered saturated ring optionally interrupted by O, S, SO₂, NR^{w} , N⁺R^{h}R^{w} or -C(O)-, unsubstituted or substituted with 1 - 4 Rⁱ groups,
   and when R^{x} and R^{y} together represent a 4-6 membered ring as defined above, R^{z} is as defined above or R^{z} represents an additional saturated 4-6 membered ring fused to the ring represented by R^{x} and R^{y} taken together, optionally interrupted by O, S, NR^{w} or -C(O)-, said rings being unsubstituted or substituted with one to four Rⁱ groups.

Pharmaceutical compositions and methods of treatment are also included herein.

### DETAILED DESCRIPTION OF THE INVENTION

The invention is described herein in detail using the terms defined below unless otherwise specified.

Carboxylate anion refers to a negatively charged group -COO⁻.

The term "alkyl" refers to a monovalent alkane (hydrocarbon) derived radical containing from 1 to 10 carbon atoms unless otherwise defined. It may be straight, branched or cyclic. Preferred alkyl groups include methyl, ethyl, propyl, isopropyl, butyl, t-butyl, cyclopentyl and cyclohexyl. When substituted, alkyl groups may be substituted with up to four substituent groups, selected from R^{d} and Rⁱ, as defined, at any available point of attachment. When the alkyl group is said to be substituted with an alkyl group, this is used interchangeably with "branched alkyl group".

Cycloalkyl is a specie of alkyl containing from 3 to 15 carbon atoms, without alternating or resonating double bonds between carbon atoms. It may contain from 1 to 4 rings which are fused.

The term "alkenyl" refers to a hydrocarbon radical straight, branched or cyclic containing from 2 to 10 carbon atoms and at least one carbon to carbon double bond. Preferred alkenyl groups include ethenyl, propenyl, butenyl and cyclohexenyl.

The term "alkynyl" refers to a hydrocarbon radical straight or branched, containing from 2 to 10 carbon atoms and at least one carbon to carbon triple bond. Preferred alkynyl groups include ethynyl, propynyl and butynyl.

Aryl refers to aromatic rings e.g., phenyl, substituted phenyl and the like, as well as rings which are fused, e.g., naphthyl, phenanthrenyl and the like. An aryl group thus contains at least one ring having at least 6 atoms, with up to five such rings being present, containing up to 22 atoms therein, with alternating (resonating) double bonds between adjacent carbon atoms or suitable heteroatoms. The preferred aryl groups are phenyl, naphthyl and phenanthrenyl. Aryl groups may likewise be substituted as defined. Preferred substituted aryls include phenyl and naphthyl.

The term "heteroaryl" refers to a monocyclic aromatic hydrocarbon group having 5 or 6 ring atoms, or a bicyclic aromatic group having 8 to 10 atoms, containing at least one heteroatom, O, S or N, in which a carbon or nitrogen atom is the point of attachment, and in which one or two additional carbon atoms is optionally replaced by a heteroatom selected from O or S, and in which from 1 to 3 additional carbon atoms are optionally replaced by nitrogen heteroatoms, said heteroaryl group being optionally substituted as described herein. Examples of this type are pyrrole, pyridine, oxazole, thiazole and oxazine. Additional nitrogen atoms may be present together with the first nitrogen and oxygen or sulfur, giving, e.g., thiadiazole. Examples include the following:

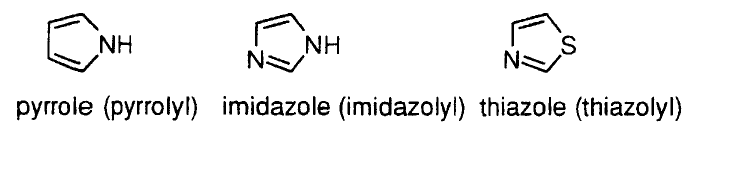

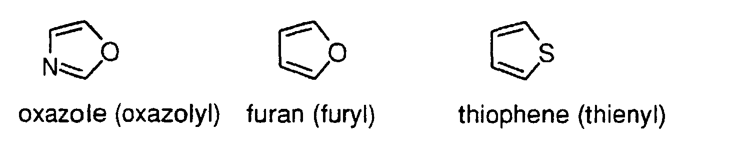

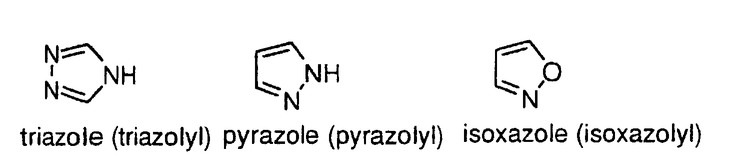

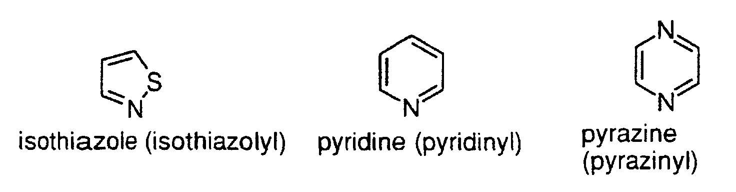

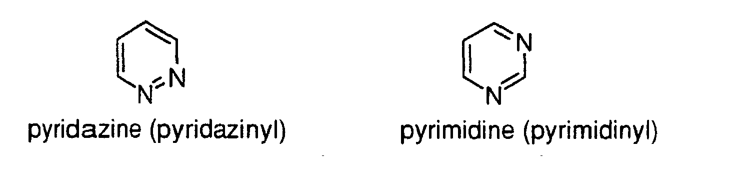

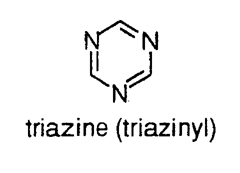

Heteroarylium refers to heteroaryl groups bearing a quaternary nitrogen atom and thus a positive charge. Examples include the following:

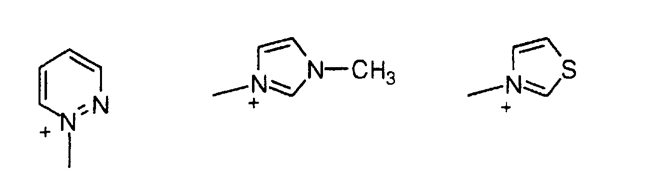

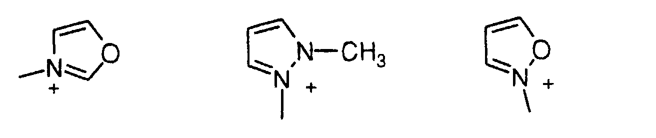

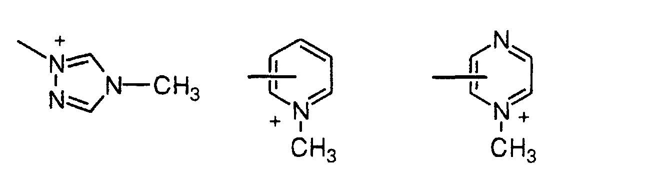

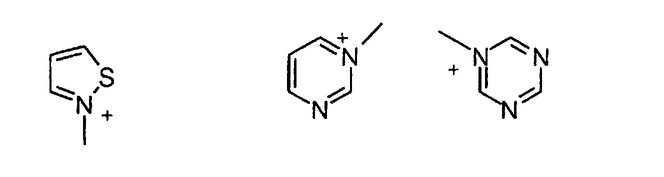

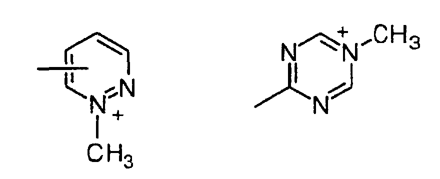

When a charge is shown on a particular nitrogen atom in a ring which contains one or more additional nitrogen atoms, it is understood that the charge may reside on a different nitrogen atom in the ring by virtue of charge resonance that occurs. 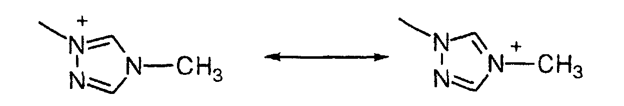 and

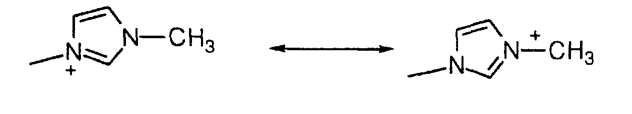

The term "heterocycloalkyl" refers to a cycloalkyl group (nonaromatic) in which one of the carbon atoms in the ring is replaced by a heteroatom selected from O, S or N, and in which up to three additional carbon atoms may be replaced by hetero atoms.

The terms "quaternary nitrogen" and "positive charge" refer to tetravalent, positively charged nitrogen atoms including, e.g., the positively charged nitrogen in a tetraalkylammonium group (e. g. tetramethylammonium), heteroarylium, (e.g., N-methylpyridinium), basic nitrogens which are protonated at physiological pH, and the like. Cationic groups thus encompass positively charged nitrogen-containing groups, as well as basic nitrogens which are protonated at physiologic pH.

The term "heteroatom" means O, S or N, selected on an independent basis.

Halogen and "halo" refer to bromine, chlorine, fluorine and iodine.

Alkoxy refers to C₁-C₄ alkyl-O-, with the alkyl group optionally substituted as described herein.

When a group is termed "substituted", unless otherwise indicated, this means that the group contains from 1 to 4 substituents thereon. With respect to R, R^{a}, R^{b} and R^{c}, the substituents available on alkyl groups are selected from the values of R^{d}. Many of the variable groups are optionally substituted with up to four Rⁱ groups. With respect to R^{e}, R^{f} and R^{g}, when these variables represent substituted alkyl, the substituents available thereon are selected from the values of Rⁱ.

When a functional group is termed "protected", this means that the group is in modified form to preclude undesired side reactions at the protected site. Suitable protecting groups for the compounds of the present invention will be recognized from the present application taking into account the level of skill in the art, and with reference to standard textbooks, such as Greene, T. W. et al. Protective Groups in Organic Synthesis Wiley, New York (1991). Examples of suitable protecting groups are contained throughout the specification.

In some of the carbapenem compounds of the present invention, M is a readily removable carboxyl protecting group, and/or P represents a hydroxyl which is protected by a hydroxyl-protecting group. Such conventional protecting groups consist of known groups which are used to protectively block the hydroxyl or carboxyl group during the synthesis procedures described herein. These conventional blocking groups are readily removable, i.e., they can be removed, if desired, by procedures which will not cause cleavage or other disruption of the remaining portions of the molecule. Such procedures include chemical and enzymatic hydrolysis, treatment with chemical reducing or oxidizing agents under mild conditions, treatment with a transition metal catalyst and a nucleophile and catalytic hydrogenation.

Examples of carboxyl protecting groups include allyl, benzhydryl, 2-naphthylmethyl, benzyl, silyl such as t-butyldimethylsilyl (TBDMS), phenacyl, p-methoxybenzyl, o-nitrobenzyl; p-methoxyphenyl, p-nitrobenzyl, 4-pyridylmethyl and t-butyl.

Examples of suitable C-6 hydroxyethyl protecting groups include triethylsilyl, t-butyldimethylsilyl, o-nitrobenzyloxycarbonyl, p-nitrobenzyloxycarbonyl, benzyloxycarbonyl, allyloxycarbonyl, t-butyloxycarbonyl, 2,2,2-trichloroethyloxycarbonyl and the like.

The carbapenem compounds of the present invention are useful per se and in their pharmaceutically acceptable salt and ester forms for the treatment of bacterial infections in animal and human subjects. The term "pharmaceutically acceptable ester, salt or hydrate," refers to those salts, esters and hydrated forms of the compounds of the present invention which would be apparent to the pharmaceutical chemist. i.e., those which are substantially non-toxic and which may favorably affect the pharmacokinetic properties of said compounds, such as palatability, absorption, distribution, metabolism and excretion. Other factors, more practical in nature, which are also important in the selection, are cost of the raw materials, ease of crystallization, yield, stability, solubility, hygroscopicity and flowability of the resulting bulk drug. Conveniently, pharmaceutical compositions may be prepared from the active ingredients in combination with pharmaceutically acceptable carriers. Thus, the present invention is also concerned with pharmaceutical compositions and methods of treating bacterial infections utilizing as an active ingredient the novel carbapenem compounds.

With respect to -CO₂M, which is attached to the carbapenem nucleus at position 3, this represents a carboxylic acid group (M represents H), a carboxylate anion (M represents a negative charge), a pharmaceutically acceptable ester (M represents an ester forming group) or a carboxylic acid protected by a protecting group (M represents a carboxyl protecting group). The pharmaceutically acceptable salts referred to above may take the form -COOM, where M is a negative charge, which is balanced by a counterion, e.g., an alkali metal cation such as sodium or potassium. Other pharmaceutically acceptable counterions may be calcium, magnesium, zinc, ammonium, or alkylammonium cations such as tetramethylammonium, tetrabutylammonium, choline, triethylhydroammonium, meglumine, triethanolhydroammonium, etc.

The pharmaceutically acceptable salts referred to above also include acid addition salts. Thus, the Formula I compounds can be used in the form of salts derived from inorganic or organic acids. Included among such salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate and undecanoate.

The pharmaceutically acceptable esters are such as would be readily apparent to a medicinal chemist, and include, for example, those described in detail in U.S. Pat. No. 4,309,438. Included within such pharmaceutically acceptable esters are those which are hydrolyzed under physiological conditions, such as pivaloyloxymethyl, acetoxymethyl, phthalidyl, indanyl and methoxymethyl, and others described in detail in U.S. Pat. No. 4,479,947. These are also referred to as "biolabile esters".

Biolabile esters are biologically hydrolizable, and may be suitable for oral administration, due to good absorption through the stomach or intenstinal mucosa, resistance to gastric acid degradation and other factors. Examples of biolabile esters include compounds in which M represents an alkoxyalkyl, alkylcarbonyloxyalkyl, alkoxycarbonyloxyalkyl, cycloalkoxyalkyl, alkenyloxyalkyl, aryloxyalkyl, alkoxyaryl, alkylthioalkyl, cycloalkylthioalkyl, alkenylthioalkyl, arylthioalkyl or alkylthioaryl group. These groups can be substituted in the alkyl or aryl portions thereof with acyl or halo groups. The following M species are examples of biolabile ester forming moieties.: acetoxymethyl, 1-acetoxyethyl, 1-acetoxypropyl, pivaloyloxymethyl, 1-isopropyloxycarbonyloxyethyl, 1-cyclohexyloxycarbonyloxyethyl, phthalidyl and (2-oxo-5-methyl-1,3-dioxolen-4-yl)methyl.

L⁻ can be present or absent as necessary to maintain the appropriate charge balance. When present, L⁻ represents a pharmaceutically acceptable counterion. Most anions derived from inorganic or organic acids are suitable. Representative examples of such counterions are the following: acetate, adipate, aminosalicylate, anhydromethylenecitrate, ascorbate, aspartate, benzoate, benzenesulfonate, bromide, citrate, camphorate, camphorsulfonate, chloride, estolate, ethanesulfonate, fumarate, glucoheptanoate, gluconate, glutamate, lactobionate, malate, maleate, mandelate, methanesulfonate, pantothenate, pectinate, phosphate/diphosphate, polygalacturonate, propionate, salicylate, stearate, succinate, sulfate, tartrate and tosylate. Other suitable anionic species will be apparent to the ordinarily skilled chemist.

Likewise, when L- represents a specie with more than one negative charge, such as malonate, tartrate or ethylenediaminetetraacetate (EDTA), an appropriate number of carbapenem molecules can be found in association therewith to maintain the overall charge balance and neutrality.

Numbering of the naphthosultam platform is as follows:

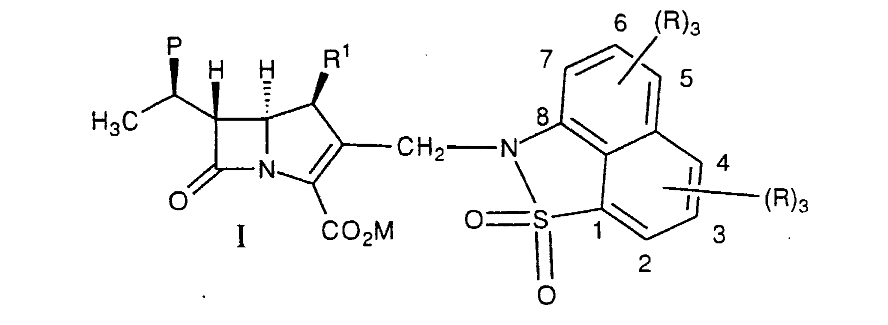

At least one of the R groups attached to the naphthosultam platform contains a positively charged moiety. Thus, it can include -R* or Q, or a moiety which in turn contains a positively charged group.

A subset of compounds of formula I which is of interest relates to those compounds where CO₂M represents a carboxylate anion. Hence, M in this instance represents a negative charge which will be balanced by a positively charged group, such as in the positively charged R group. Likewise, if the positively charged R group contains more than one positive charge, a negatively charged counterion may be present which in combination with the carboxylate anion, provides overall charge neutrality.

Another subset of compounds of formula I which is of interest relates to compounds of formula I wherein one R represents a group which contains a positively charged moiety, and the remaining R groups are selected from hydrogen and C₁₋₆ straight or branched chain alkyl, unsubstituted or substituted with one to four R^{d} groups. More particularly, this subset of interest includes compounds of formula I wherein one R represents a group containing a positively charged moiety and the remaining R groups are hydrogen.

With respect to the positively charged moiety or moieties that are contained in one or more R groups, it is preferred that from 1-3 positive charges be present, and most preferably two positive charges be present, balanced by the carboxylate anion and a negatively charged counterion.

Another subset of compounds which is of interest is represented by formula I wherein one R group represents a -C₁₋₆ straight or branched chain alkyl group, substituted with one to four R^{d} groups, wherein one R^{d} group represents -R* or Q. Hence, a positively charged moiety -R* or Q is attached to an alkyl group.

Another group of compounds of interest is represented by formula I wherein Q is selected from the group consisting of:

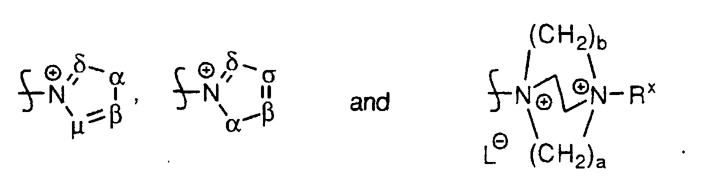

More particularly, the group of compounds which is of interest is represented by formula I wherein Q represents:

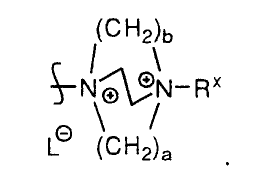

Within this subset of compounds, L⁻, a and b are as originally defined, and R^{x} is as originally defined, and represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups.

Another group of compounds of interest is represented by formula I wherein Q represents -N⁺R^{x}R^{y}R^{z}, wherein R^{x}, RY and R^{z} are as originally defined.

Another group of compounds of interest is represented by formula I wherein one R* group is present and is selected from: 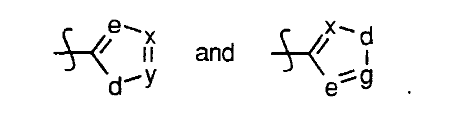 Within this subset, d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

A preferred subset of compounds of formula I which is of particular interest relates to compounds represented by formula I wherein:
CO₂M represents a carboxylate anion;
one R group which is attached to the naphthosultam platform contains at least one positively charged moiety, and the remaining R groups are selected from hydrogen and C₁₋₆ straight or branched chain alkyl, unsubstituted or substituted with one to four R^{d} groups;
Rd is as originally defined;
R^{h} represents hydrogen or a C₁₋₆ straight or branched chain alkyl group;
Q is selected from the group consisting of: 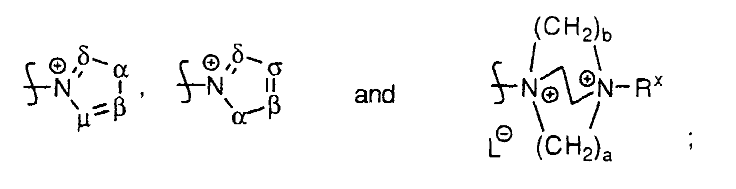 wherein L⁻ , a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R* is selected from: 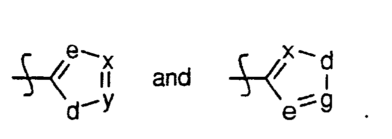 wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

Within this subset, all other variables are as originally defined with respect to formula I.

A more preferred subset of compounds of the invention is represented by formula Ia: 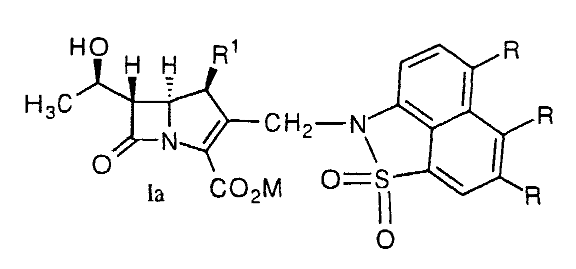 or a pharmaceutically acceptable salt thereof, wherein:
CO₂M represents a carboxylate anion;
one R group is attached to the naphthosultam platform which contains a positively charged moiety, and the other R groups are selected from hydrogen and C₁₋₆ straight or branched chain alkyl, unsubstituted or substituted with one to four R^{d} groups;
R^{d} is as originally defined;
Q is selected from the group consisting of: 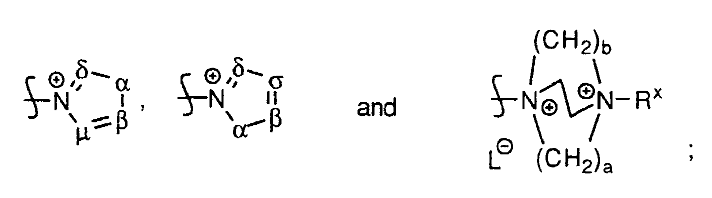 wherein L⁻ , a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R^{h} represents hydrogen or a C₁₋₆ straight or branched chain alkyl group;
R^{w} is as originally defined;
R* is selected from: 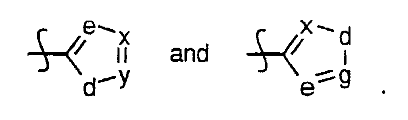
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

Within this subset, all other variables are as originally defined with respect to formula I.

Another more preferred subset of compounds is represented by formula Ib: 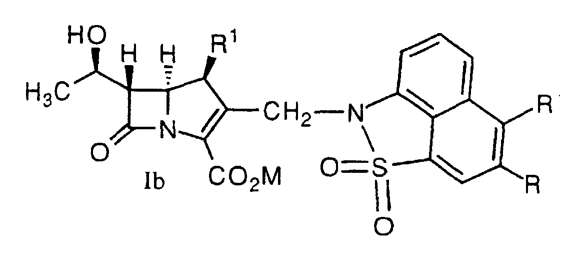 or a pharmaceutically acceptable salt thereof, wherein:
CO₂M represents a carboxylate anion;
one R group is attached to the naphthosultam platform which contains a positively charged moiety, and the other R group is selected from hydrogen and C₁₋₆ straight or branched chain alkyl, unsubstituted or substituted with one to four R^{d} groups;
R^{d} is as originally defined;
Q is selected from the group consisting of: 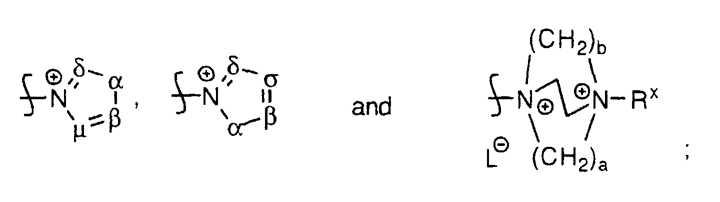 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R^{h} represents hydrogen or a C₁₋₆ straight or branched chain alkyl group;
R^{w} is as originally defined;
R* is selected from: 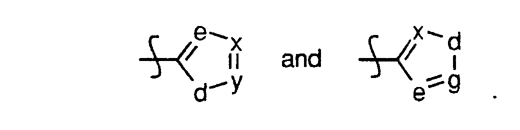
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen. Within this subset, all other variables are as originally defined with respect to formula I.

Another more preferred subset of compounds is represented by formula Ic: 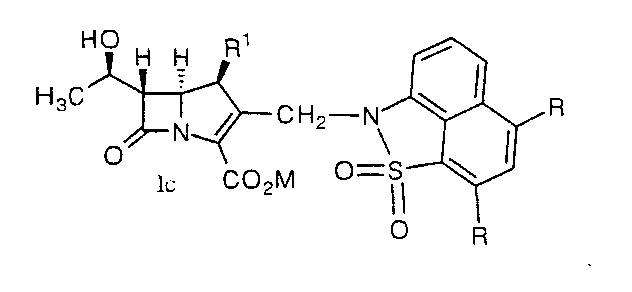 or a pharmaceutically acceptable salt thereof, wherein:
CO₂M represents a carboxylate anion;
one R group is attached to the naphthosultam platform which contains a positively charged moiety, and the other R group is selected from hydrogen, halo and C₁₋₆ straight or branched chain alkyl, unsubstituted or substituted with one to four R^{d} groups;
R^{d} is as originally defined;
Q is selected from the group consisting of: 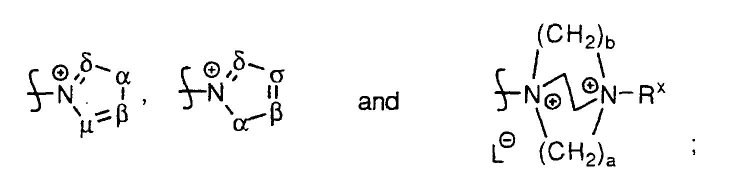 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R^{h} represents hydrogen or a C₁₋₆ straight or branched chain alkyl group;
R^{w} is as originally defined;
R* is selected from: 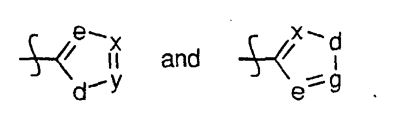
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen. Within this subset, all other variables are as originally defined with respect to formula I.

Another more preferred subset of compounds is represented by formula Id: 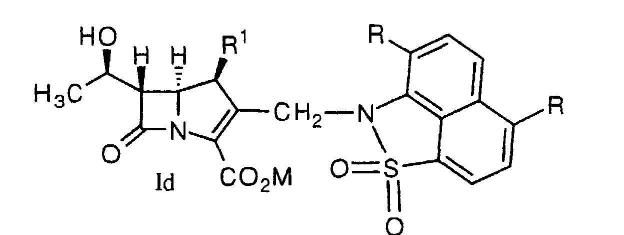 or a pharmaceutically acceptable salt thereof, wherein:
CO₂M represents a carboxylate anion;
one R group is attached to the naphthosultam platform which contains a positively charged moiety, and the other R group is selected from hydrogen, halo and C₁₋₆ straight or branched chain alkyl, unsubstituted or substituted with one to four R^{d} groups;
R^{d} is as originally defined;
Q is selected from the group consisting of: 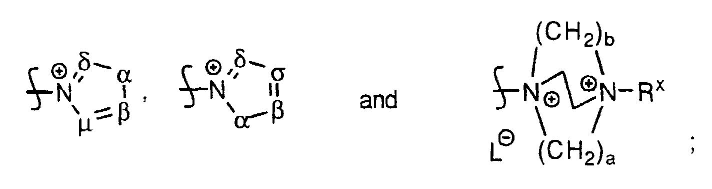 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R^{h} represents hydrogen or a C₁₋₆ straight or branched chain alkyl group;
R^{w} is as originally defined;
R* is selected from: 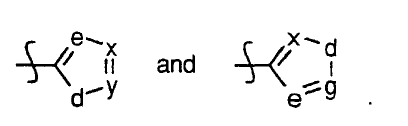
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen. Within this subset, all other variables are as originally defined with respect to formula I.

Still more preferably, the present invention relates to a compound represented by formula Ia wherein the R group at position 4 represents a positively charged moiety, and the R groups at position 3 and 5 represent hydrogen.

In particular, such compounds can be represented by formula Ie: 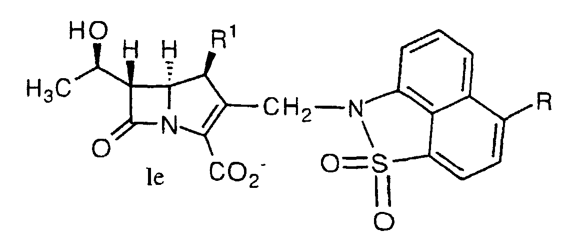 or a pharmaceutically acceptable salt thereof, wherein:
R contains a positively charged moiety selected from the group consisting of: -R*, Q, and a C₁₋₆ straight or branched alkyl chain substituted with one R^{d} group;
R^{d} is independently selected -R* or Q;
Q is selected from the group consisting of: 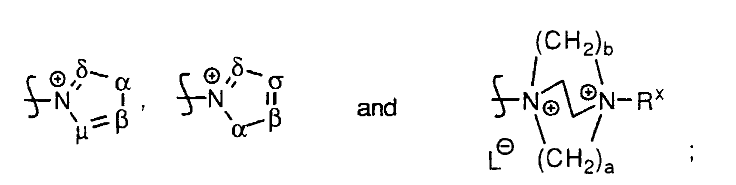 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R* is selected from: 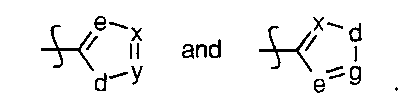
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

Likewise, such compounds can be represented by formula If: 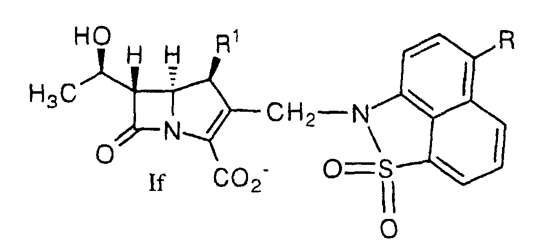 or a pharmaceutically acceptable salt thereof, wherein:
R contains a positively charged moiety selected from the group consisting of: -R*, Q, and a C₁₋₆ straight or branched alkyl chain substituted with one R^{d} group;
R^{d} is independently selected -R* or Q;
Q is selected from the group consisting of: 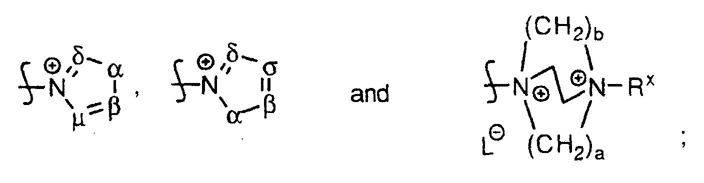 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R* is selected from: 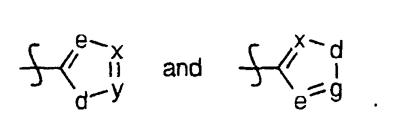
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

A still more preferred subset of compounds of the invention is represented by formula Ie wherein:
R represents 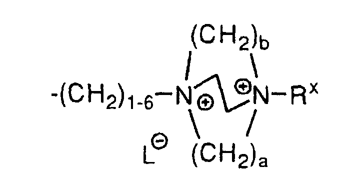 and R^{x}, a, b and L⁻ are as originally defined.

Another more preferred subset of compounds of the invention is represented by formula Ig: 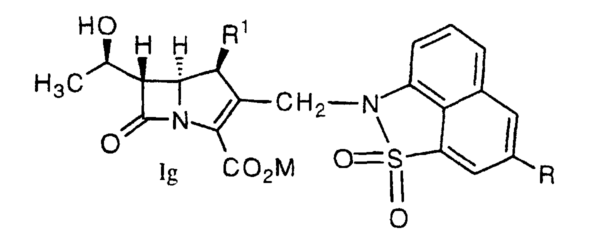 wherein:
R represents 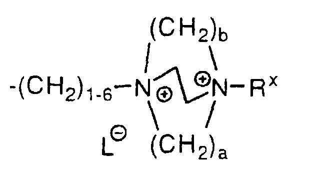 and R^{x}, a, b and L⁻ are as originally defined.

Representative examples of compounds of the invention are as follows:

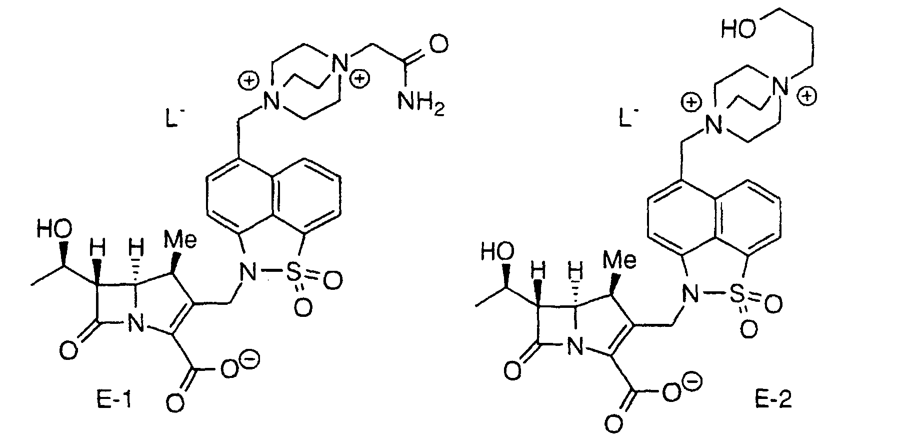

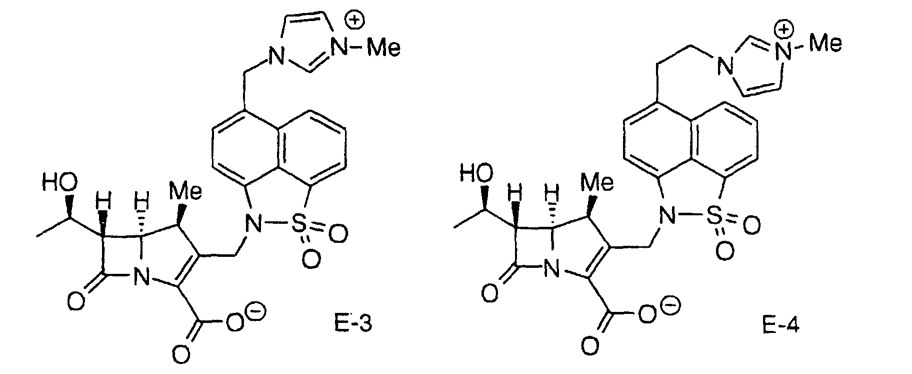

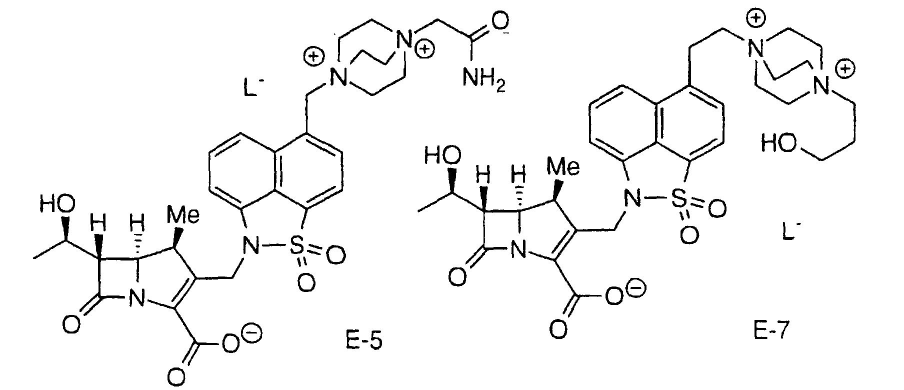

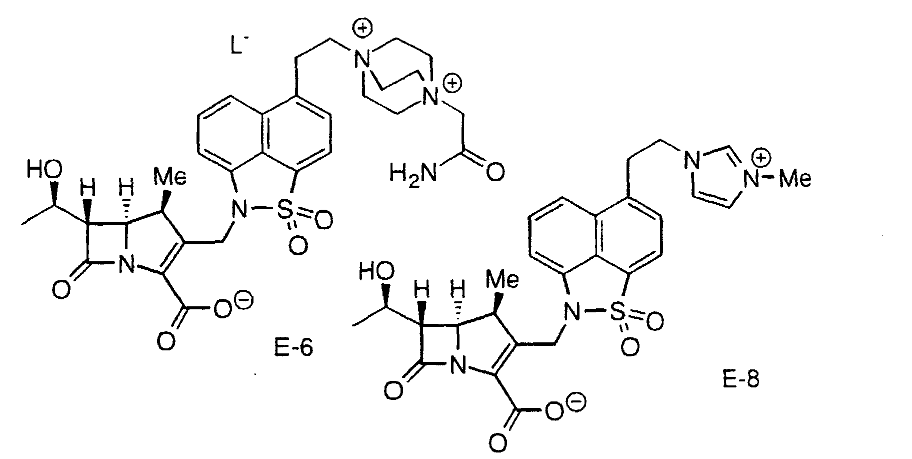

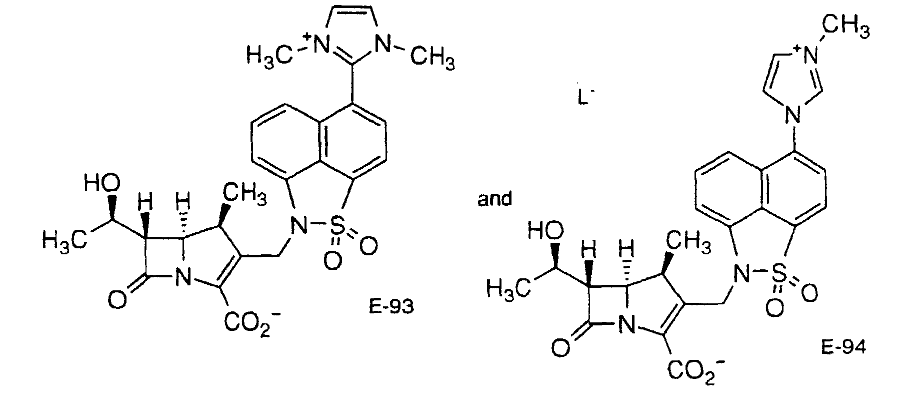

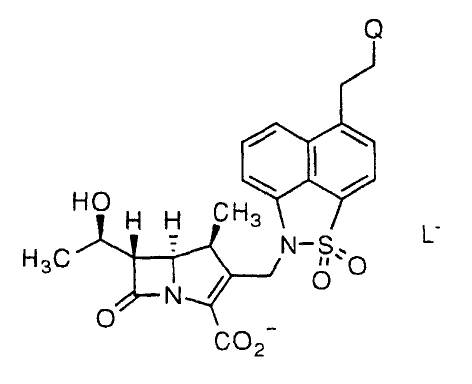

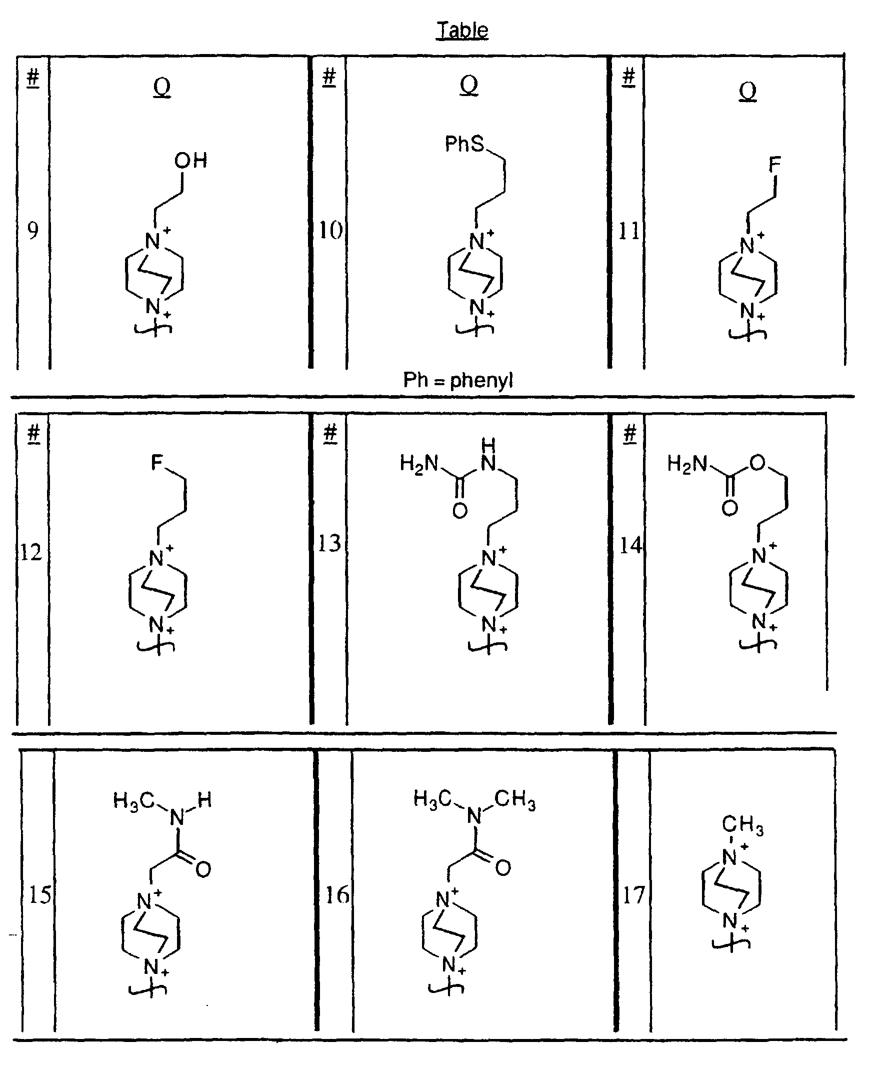

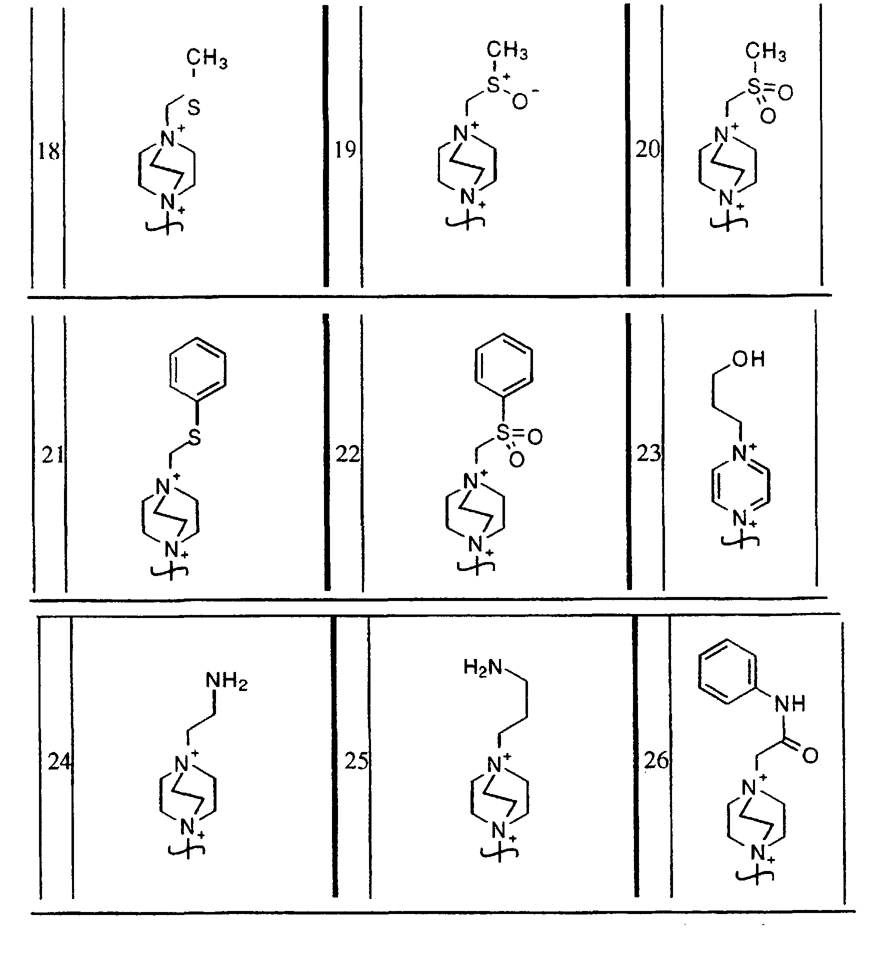

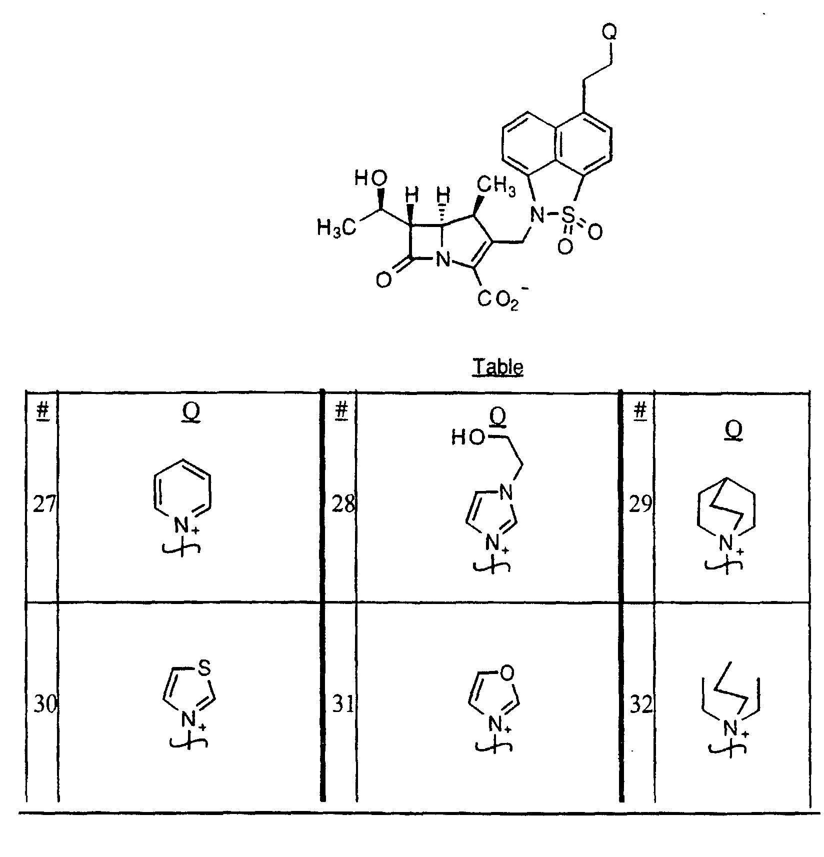

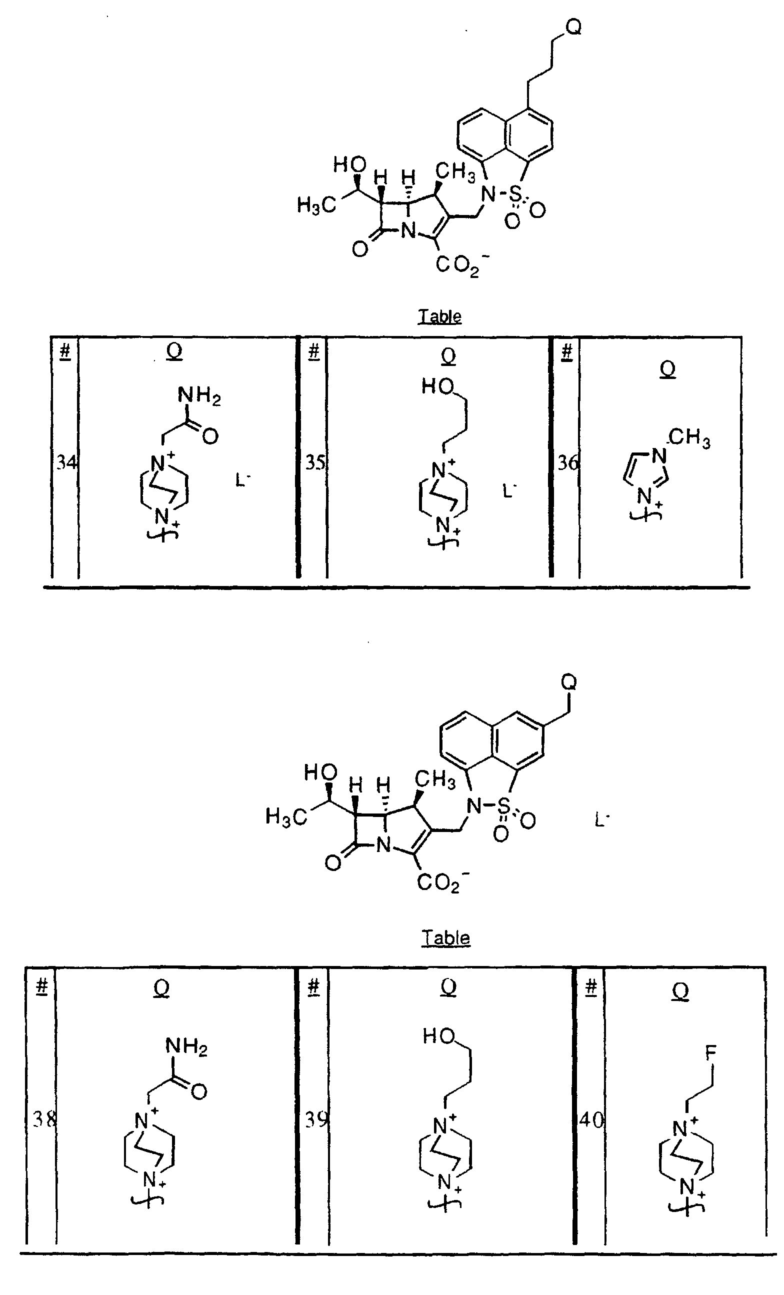

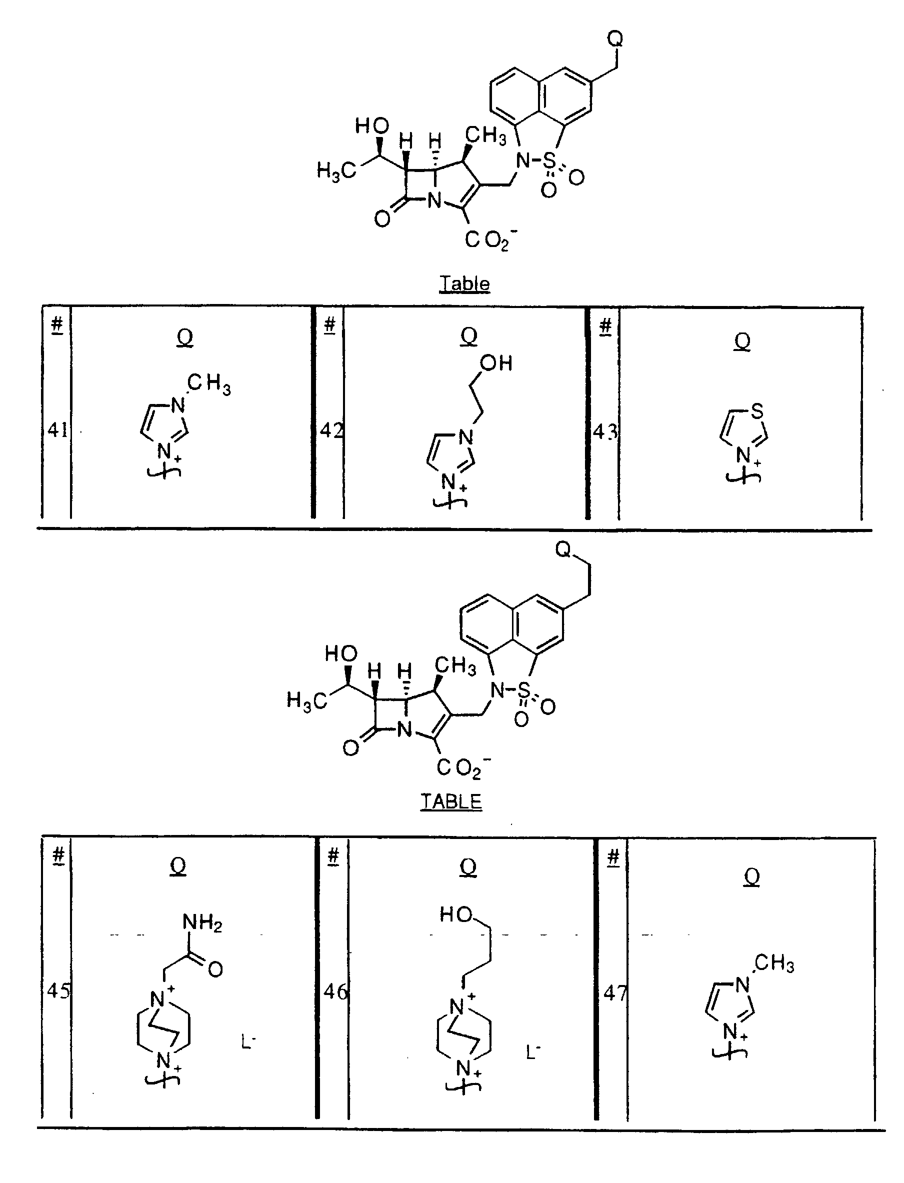

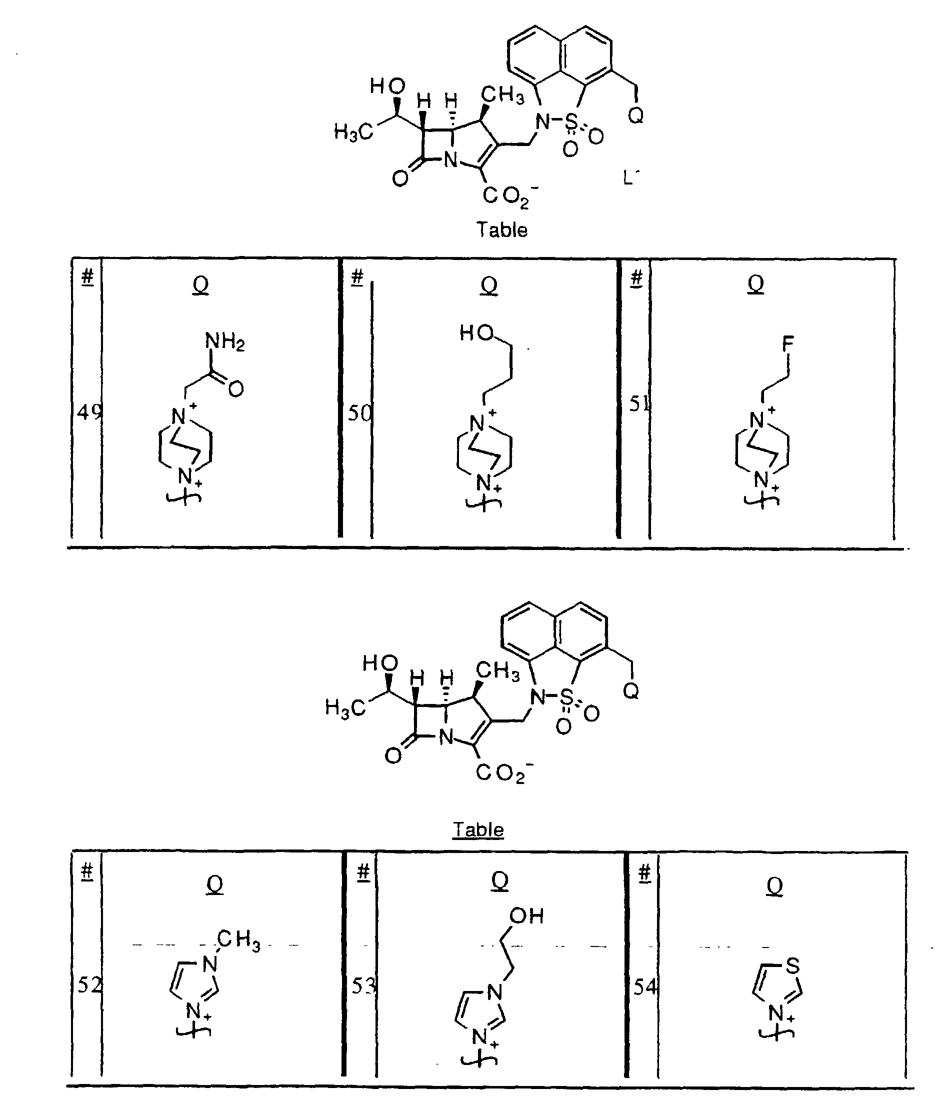

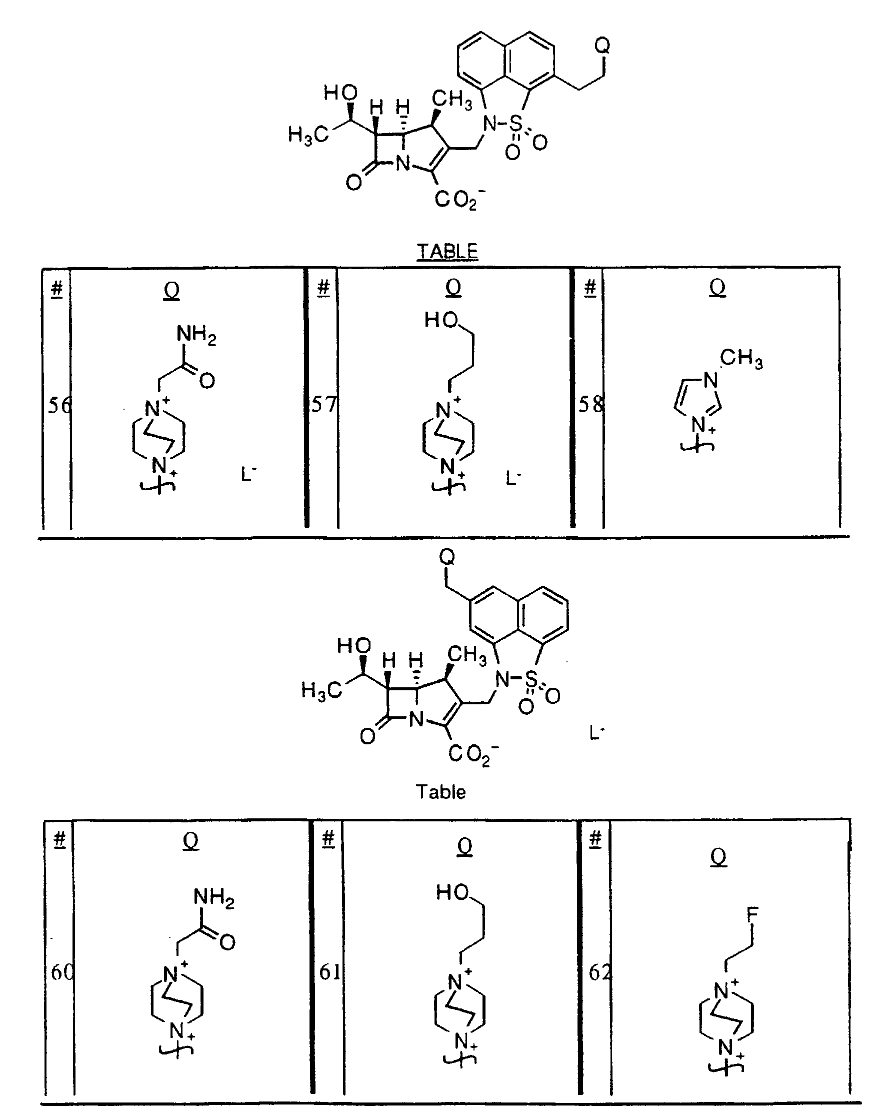

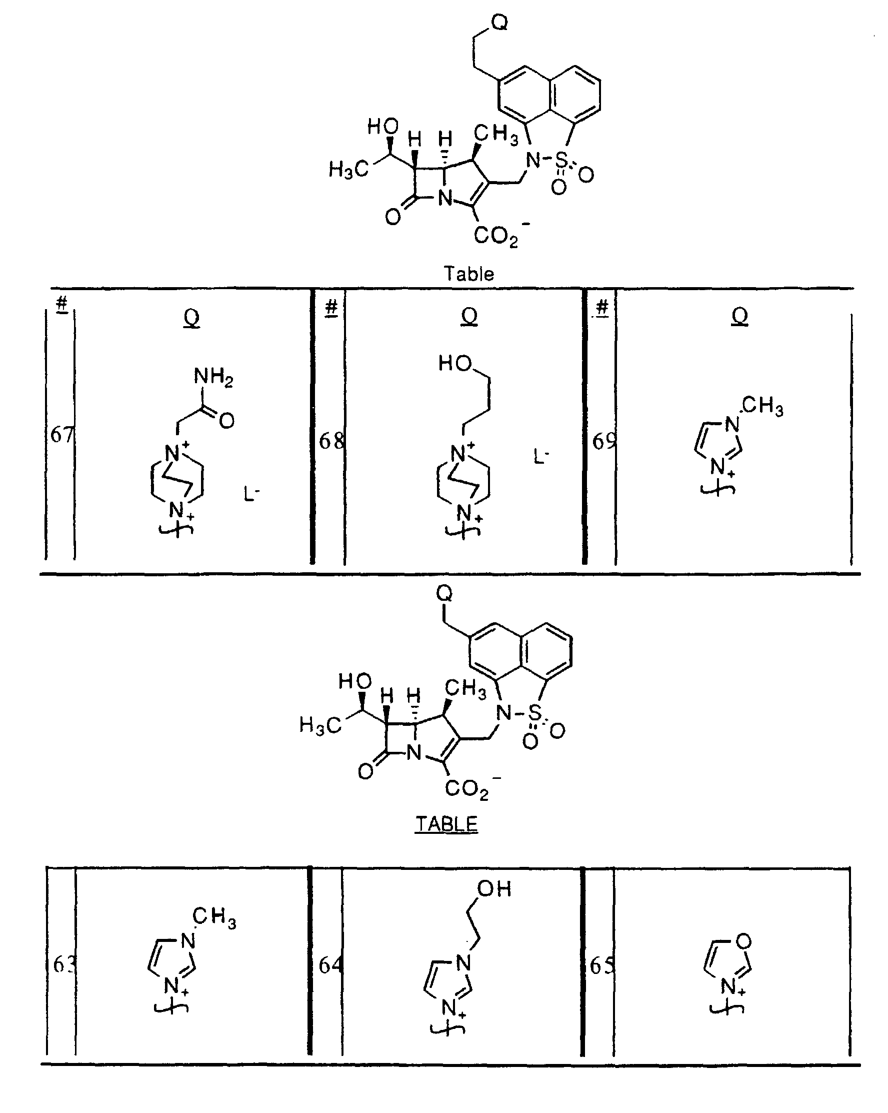

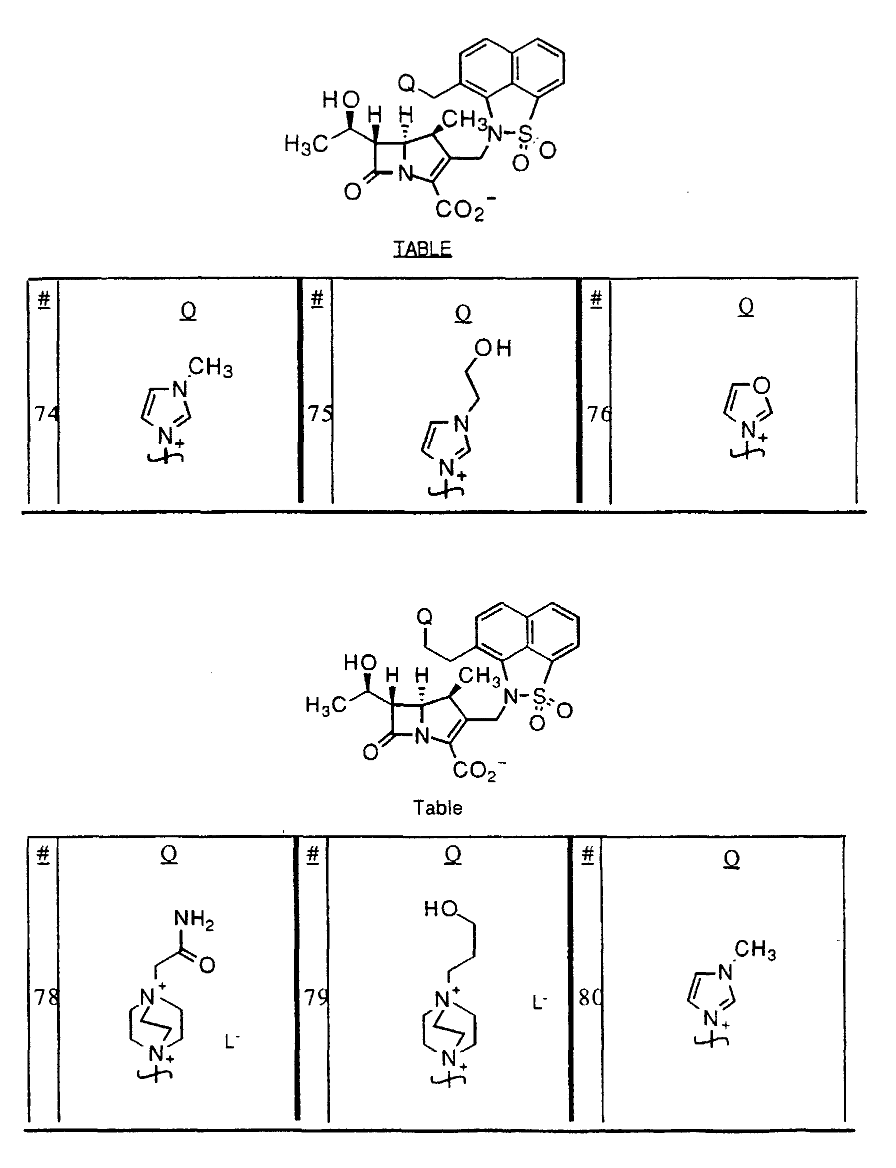

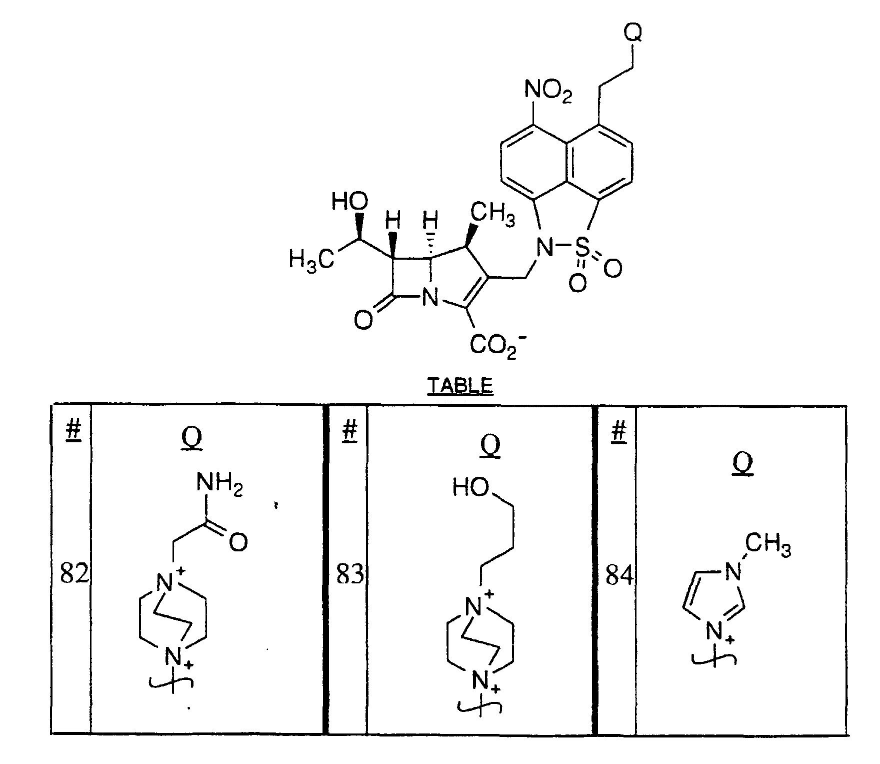

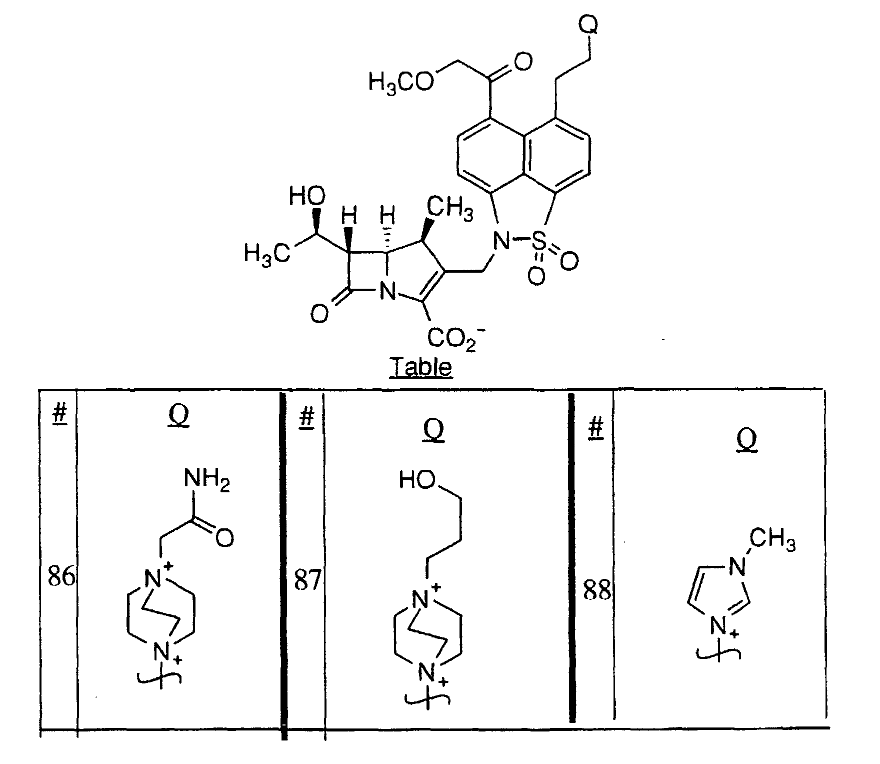

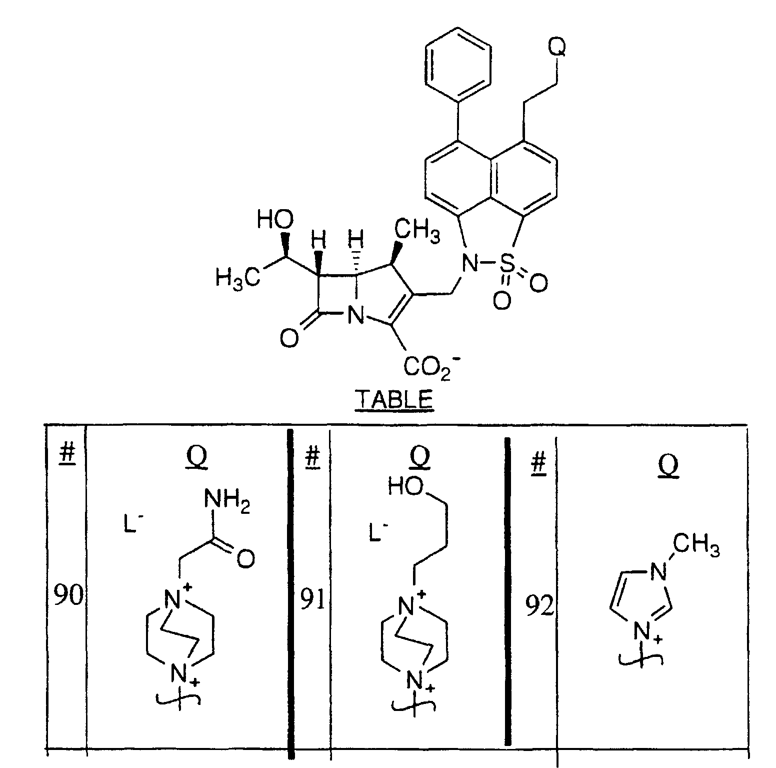

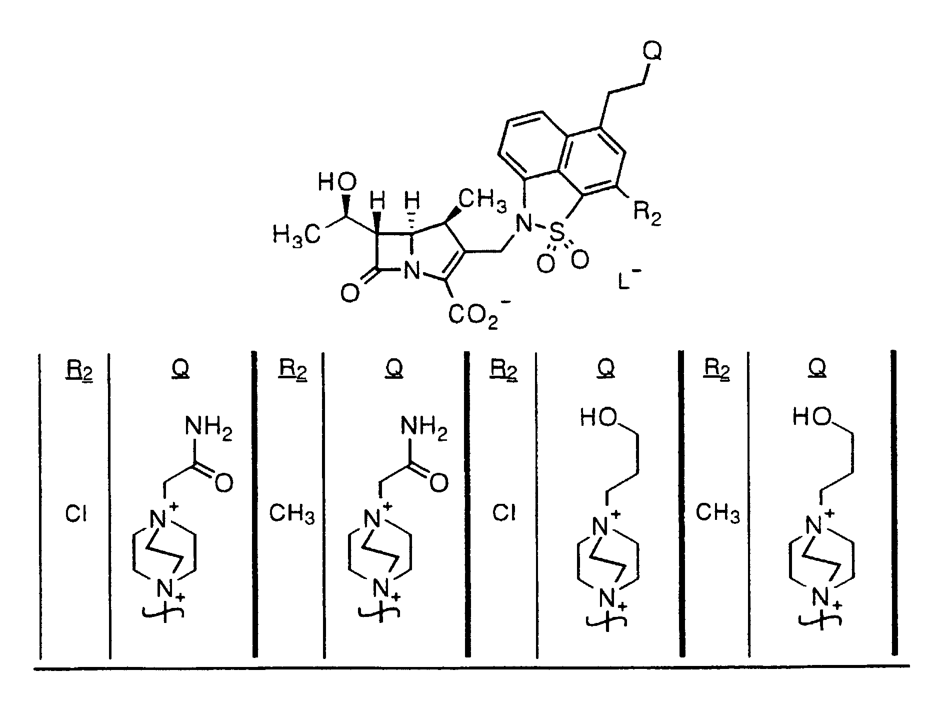

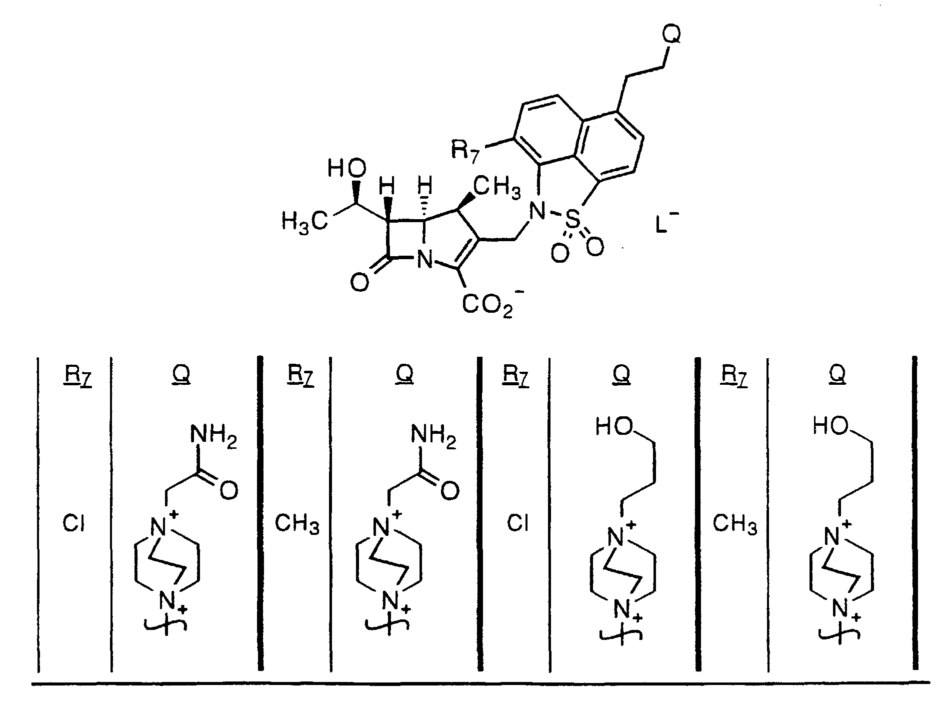

wherein Q is as defined in the tables and L⁻ represents a pharmaceutically acceptable counterion.

The compounds of the present invention are prepared by reacting a suitably protected, activated 2-hydroxymethyl-carbapen-2-em-3-carboxylate with a naphthosultam, modifying the thus-introduced side chain as desired, and then removing any protecting groups which are present to afford the desired final product. The process is illustrated by the following generic scheme:

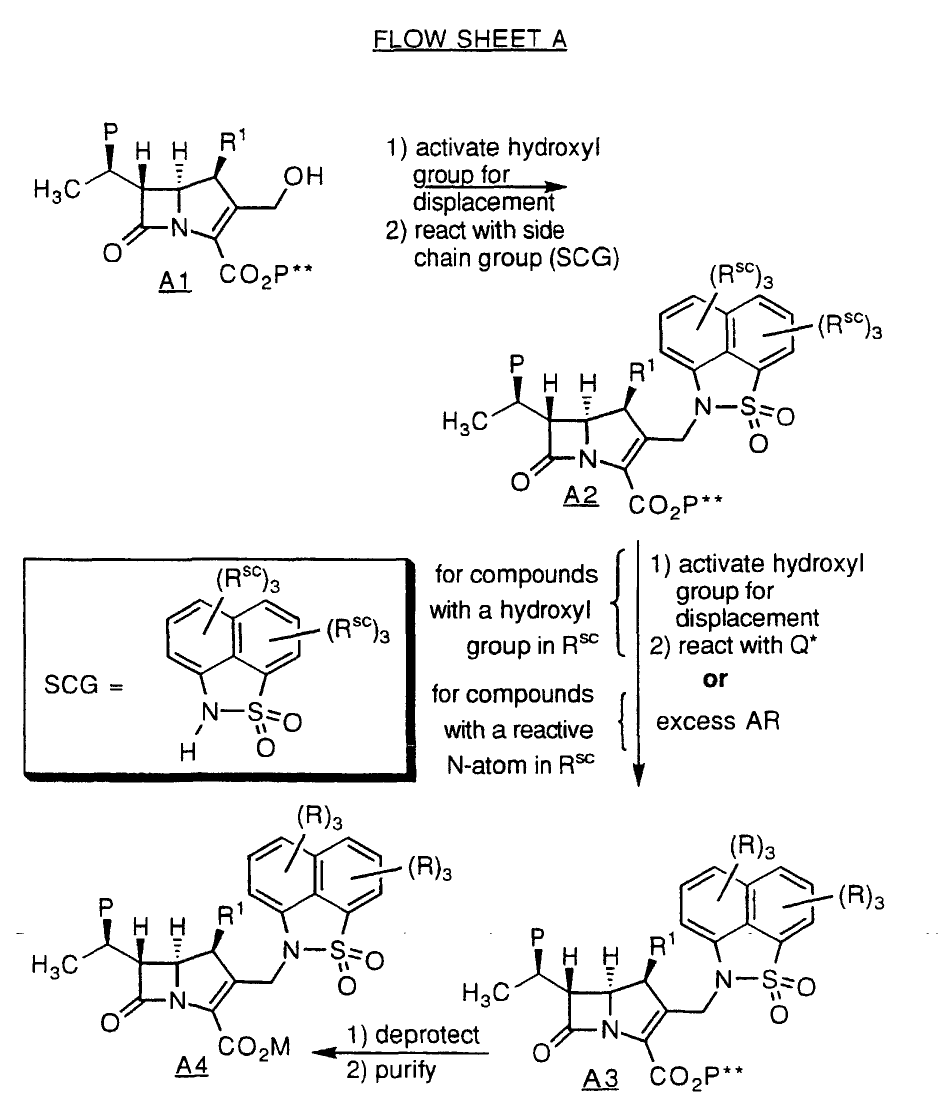

With reference to Flow Sheet A above, P, R¹, R, and M, are as defined with respect to the compounds of formula I.

P** represents a carboxyl protecting group.

R^{sc} represents a group which may or may not be selected from the group comprising R as defined above and is modified as necessary in the course of the synthesis of a compound of formula I to afford a member of that group, thus R^{SC} may be viewed as a precursor for R.

Q* represents a group which reacts with intermediate A2 (upon activation of A2) in a manner which results in the incorporation in the final product of a member of the group defined as Q above, thus Q* may be viewed as a precursor for Q.

AR represents a suitable alkylating reagent, such as methyl iodide, methyl bromide, benzyl trichloroacetimidate, methyl trifluoromethanesulfonate, triethyloxonium tetrafluoroborate and the like.

The naphthosultam side chain group (SCG) used in the synthesis of the compounds of the present invention have, in some cases, been described in the chemical literature. In other cases, precursor compounds which may be readily converted to the requisite naphthosultam have been described in the literature. In cases where the requisite naphthosultam is not known in the literature it is neceessary to synthesize the naphthosultam by a newly developed synthesis. One skilled in the art can adapt a previously published synthesis of an analogous naphthosultam to prepare the requisite compound in a straightforward manner without undue experimentation. Numerous examples of naphthosultam synthesis are described herein (see below).

The naphthosultam side chain group (SCG) is initially reacted with a suitably protected carbapen-2-em-3-carboxylate having an activated hydroxymethyl group at the 2-position.

The carbapenem nucleus having a -CH₂OH substituent at position 2 can be obtained in accordance with Schmitt, S. M. et al., J. Antibiotics 41 (6): 780-787 (1988), the teachings of which are incorporated herein by reference. The carboxylic acid group at C-3 of the carbapenem is generally protected as a carboxyl protecting group such as p-nitrobenzyl (PNB), allyl, p-methoxybenzyl, trichloroethyl, 2-trimethylsilylethyl, and the like. Furthermore, the hydroxyl group of the 6-(hydroxyethyl) side-chain is optionally protected with a hydroxyl protecting group such as trimethylsilyl (TMS), triethylsilyl (TES), tertbutyldimethylsilyl (TBDMS), tert-butyldiphenylsilyl (TBDPS), acetyl, allyloxycarbonyl, 2-trimethylsilylethoxy carbonyl, 2-trichloroethoxycarbonyl and the like.

The addition of the naphthosultam side chain group (SCG) to the carbapenem is accomplished by treating a solution of the hydroxymethyl-carbapenem and the naphthosultam side chain group in a suitable solvent such as tetrahydrofuran (THF), ether, acetonitrile, dimethylformamide (DMF), benzene, dimethylsulfoxide (DMSO), and the like with a (premixed) suitable activating reagent such as diethyl azodicarboxylate (DEAD) / triphenylphosphine, diisopropyl azodicarboxylate (DIAD) / tributylphosphine, and the like, at a temperature between about -20 °C and 35 °C for about 5 to 90 minutes.

Alternatively, the naphthosultam and carbapenem can be mixed together with either the azodicarboxylate or the phosphine reagent in a suitable and the other component of the activating reagent (the phosphine or the azodicarboxylate, respectively) can be added to that mixture. Once the naphthosultam, carbapenem, and activating reagent(s) have been mixed, the reaction is allowed to proceed at a temperature between about -20 °C and 35 °C for about 5 to 90 minutes.

The resulting mixture is then subjected to a standard work-up procedure familiar to those skilled in the art to afford a crude 2-naphthosultam-methyl substituted carbapenem which is purified, if necessary, by recrystallization or by chromatography on silica gel, eluting with a suitable solvent or mixture of two or more solvents, such as hexane, ethyl acetate, ether, benzene, dichloromethane, chloroform, acetone, methanol and the like.

Modification of the naphthosultam side chain of compounds A2, which is generally necessary to introduce the charged substituent of A4, is best accomplished before removal of the protecting groups. For compounds which contain a hydroxyl group in the side chain, i.e. in R^{sc}, a positively charged substituent may be introduced into the side chain by first activating the hydroxyl group by converting it to a suitable leaving group such as a triflate, mesylate, tosylate, iodide, chloride, bromide, and the like, and then displacing the resulting leaving group with a compound Q*, such as N-methyl-imidazole, N-(2-hydroxyethyl)-imidazole, N-methyl-diazabicyclooctane, 1-(carbamoylmethyl)-4-aza-1-azoniabicyclo-[2.2.2.]-octane, 1-(3-hydroxyprop-1-yl)-4-aza-1-azoniabicyclo-[2.2.2.]-octane, pyridine, morpholine and the like which contains a nitrogen atom that can act as a nucleophile. Althematively, in some cases, the charged substituent may be incorporated in the naphthosultam side chain before addition of the naphthosultam to the carbapenem or may be introduced after deprotection of A2. However, introduction of the charged substituent by modification of A2 before deprotection is greatly preferred.

In some cases, activation of the hydroxyl group and displacement by Q* to produce A3 may be accomplished in a single step by taking advantage of the basic character of compound Q* and using it as a base in the activation reaction.

The conversion of the hydroxyl group to a suitable leaving group is accomplished by treating the hydroxyl substituted compound in a suitable solvent such as dichloromethane, tetrahydrofuran, ether, benzene, and the like with an activating reagent, such as trifluoromethanesulfonic anhydride, methanesulfonic anhydride, toluenesulfonic anhydride, methanesulfonyl chloride, benzenesulfonyl chloride, toluenesulfonyl chloride, and the like in the presence of a suitable base such as triethylamine, tributylamine, diisopropylethylamine, and the like at a temperature between about -100°C and 0°C for about 5 to 120 minutes. The intermediate thus obtained contains a leaving group, which may be converted to an alternative leaving group, iodide, by treating a solution of the intermediate in a suitable solvent such as acetone, methyl ethyl ketone, and the like at about -10°C to 50°C with an excess of sodium iodide or potassium iodide for about 0.25 to 24 hours.

In many cases, the iodide is obtained in sufficiently pure form that it may be used without further purification. For ease of handling, the iodide, if not crystalline, may be lyophilized from benzene to afford an amorphous, easily handled, solid.

The activated hydroxyl group or iodide is displaced by reacting the activated intermediate with reagent Q*. In some cases, activation and displacement of the hydroxyl group may be accomplished in a single step. The activating reagent is added to a solution of the hydroxyl substituted compound in the presence of a suitable base in a suitable solvent such as dichloromethane, tetrahydrofuran, ether, DMF, benzene, acetonitrile, DMSO, and the like as described in the preceding paragraphs. The resulting activated intermediate is treated with 1-3 molar equivalents of compound Q* at a temperature between about -78°C and 50°C for about 15 to 120 minutes. In some cases, it is desirable to form the activated intermediate in one solvent, isolate the activated intermediate, and conduct the displacement reaction in a different solvent. In other cases, the displacement may be conducted without isolation of the intermediate and, in cases where Q* is also used as a base, may even be concurrent with the formation of the activated intermediate.

In cases where the displacement reaction is best accomplished by using the iodide, a solution of the iodide is combined with an approximately equivalent amount (0.9 - 1.05 molar equivalents) of compound Q*. A silver salt of a non-nucleophilic acid, such as silver trifluoromethanesulfonate, silver tetrafluoroborate and the like is then added. Although the reaction will proceed in the absence of the silver salt, the reaction proceeds more rapidly in the presence of the silver salt. In addition, the silver salt assists in the removal of the displaced iodide from the reaction mixture which can improve the efficiency of subsequent steps. The resulting mixture is then subjected to a standard work-up procedure familiar to those skilled in the art to afford a crude product which is purified, if necessary, by recrystallization or chromatography.

An alternative method for introducing a positive charge into the side chain may be applied to side chains (i.e. R^{SC} groups) that contain a nitrogen atom which may be quatemized by reaction with a suitable alkylating reagent AR, such as methyl iodide, methyl bromide, benzyl trichloroacetimidate, methyl trifluoromethanesulfonate, triethyloxonium tetrafluoroborate, and the like. Quaternization of the nitrogen atom in the side chain is effected by treating a solution of the compound with a slight excess (1.05 to 1.2 molar equivalents) of the alkylating reagent.

The synthesis of the target compound is completed by removing any protecting groups which are present in the penultimate intermediate using standard techniques which are well known to those skilled in the art. The deprotected final product is then purified, as necessary, using standard techniques such as ion exchange chromatography, HPLC on reverse phase silica gel, MPLC on reverse phase polystyrene gel, and the like or by recrystallization.

The final product may be characterized structurally by standard techniques such as NMR, IR, MS, and UV. For ease of handling, the final product, if not crystalline, may be lyophilized from water to afford an amorphous, easily handled solid.

The compounds of the present invention are valuable antibacterial agents active against various Gram-positive and to a lesser extent Gram-negative bacteria, and accordingly find utility in human and veterinary medicine.

Many of compounds of the present invention are biologically active against MRSA/MRCNS. In vitro antibacterial activity is predictive of in vivo activity when the compounds are administered to a mammal infected with a susceptible bacterial organism.

Using standard susceptibility tests, the compounds of the invention are determined to be active against MRSA.

The compounds of the invention can be formulated in pharmaceutical compositions by combining the compound with a pharmaceutically acceptable carrier. Examples of such carriers are set forth below.

The compounds may be employed in powder or crystalline form, in liquid solution, or in suspension. They may be administered by a variety of means; those of principal interest include: topically, orally and parenterally by injection (intravenously or intramuscularly).

Compositions for injection, a preferred route of delivery, may be prepared in unit dosage form in ampules, or in multidose containers. The injectable compositions may take such forms as suspensions, solutions, or emulsions in oily or aqueous vehicles, and may contain various formulating agents. Alternatively, the active ingredient may be in powder (lyophillized or non-lyophillized) form for reconstitution at the time of delivery with a suitable vehicle, such as sterile water. In injectable compositions, the carrier is typically comprised of sterile water, saline or another injectable liquid, e.g., peanut oil for intramuscular injections. Also, various buffering agents, preservatives and the like can be included.

Topical applications may be formulated in carriers such as hydrophobic or hydrophilic bases to form ointments, creams, lotions, in aqueous, oleaginous or alcoholic liquids to form paints or in dry diluents to form powders.

Oral compositions may take such forms as tablets, capsules, oral suspensions and oral solutions. The oral composions may utilize carriers such as conventional formulating agents, and may include sustained release properties as well as rapid delivery forms.

The dosage to be administered depends to a large extent upon the condition and size of the subject being treated, the route and frequency of administration, the sensitivity of the pathogen to the particular compound selected, the virulence of the infection and other factors. Such matters, however, are left to the routine discretion of the physician according to principles of treatment well known in the antibacterial arts. Another factor influencing the precise dosage regimen, apart from the nature of the infection and peculiar identity of the individual being treated, is the molecular weight of the compound.

The compositions for human delivery per unit dosage, whether liquid or solid, may contain from about 0.01% to as high as about 99% of active material, the preferred range being from about 10-60%. The composition will generally contain from about 15 mg to about 2.5 g of the active ingredient; however, in general, it is preferable to employ dosage amounts in the range of from about 250 mg to 1000 mg. In parenteral administration, the unit dosage will typically include the pure compound in sterile water solution or in the form of a soluble powder intended for solution, which can be adjusted to neutral pH and isotonic.

The invention described herein also includes a method of treating a bacterial infection in a mammal in need of such treatment comprising administering to said mammal a compound of formula I in an amount effective to treat said infection.

The preferred methods of administration of the Formula I antibacterial compounds include oral and parenteral, e.g., i.v. infusion, i.v. bolus and i.m. injection.

For adults, about 5-50 mg of Formula I antibacterial compound per kg of body weight given one to four times daily is preferred. The preferred dosage is 250 mg to 1000 mg of the antibacterial given one to four times per day. More specifically, for mild infections a dose of about 250 mg two or three times daily is recommended. For moderate infections against highly susceptible gram positive organisms a dose of about 500 mg three or four times daily is recommended. For severe, life-threatening infections against organisms at the upper limits of sensitivity to the antibiotic, a dose of about 1000-2000 mg three to four times daily may be recommended.

For children, a dose of about 5-25 mg/kg of body weight given 2, 3, or 4 times per day is preferred; a dose of 10 mg/kg is typically recommended.

The compounds of Formula I are of the broad class known as carbapenems. Many carbapenems are susceptible to attack by a renal enzyme known as dehydropeptidase (DHP). This attack or degradation may reduce the efficacy of the carbapenem antibacterial agent. Many of the compounds of the present invention, on the other hand, are less subject to such attack, and therefore may not require the use of a DHP inhibitor. However, such use is optional and contemplated to be part of the present invention. Inhibitors of DHP and their use with carbapenems are disclosed in, e.g.,[European Patent Application Nos. 79102616.4, filed July 24, 1979 (Patent No. 0 007 614); and 82107174.3, filed August 9, 1982 (Publication No. 0 072 014)].

The compounds of the present invention may, where DHP inhibition is desired or necessary, be combined or used with the appropriate DHP inhibitor as described in the aforesaid patents and published application. The cited European Patent Applications define the procedure for determining DHP susceptibility of the present carbapenems and disclose suitable inhibitors, combination compositions and methods of treatment. A preferred weight ratio of Formula I compound: DHP inhibitor in the combination compositions is about 1:1.

A preferred DHP inhibitor is 7-(L-2-amino-2-carboxyethylthio)-2-(2,2-dimethylcyclopropanecarboxamide)-2-heptenoic acid or a useful salt thereof.

The invention is further described in connection with the following non-limiting examples.

### PREPARATIVE EXAMPLE 1 SYNTHESIS OF 5-(TRIMETHYLSILYLOXYMETHYL)-1,8-NAPHTHOSULTAM

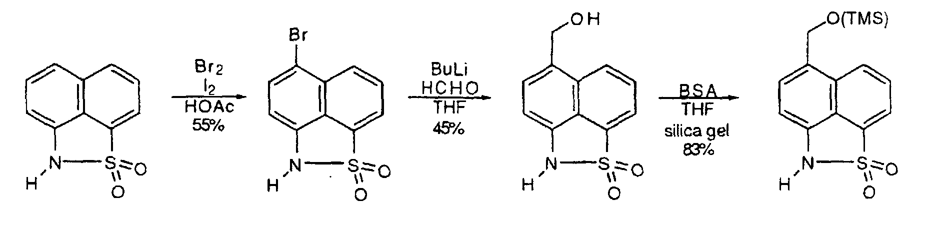

### Step 1: 5-Bromo-1,8-naphthosultam

A suspension of 1,8-naphthosultam (5 g, 24.4 mmol) in acetic acid (20 mL) was treated with a dark solution of iodine (6.5 g, 25.6 mmol) and bromine (1.3 mL, 25.2 mmol) in acetic acid (20 mL) over 10 minutes. The suspension was stirred an additional 95 minutes then placed in a 60°C oil bath for 30 minutes. After cooling to room temperature, the mixture was added to a 1% aqueous NaHSO₃ solution (300 mL). The dark precipitate was filtered and dried overnight under a stream of nitrogen. The resulting solid (6 g) was dissolved in ethyl acetate then silica gel (ca. 6 g) was added and the mixture was evaporated under vacuum. The silica-adsorbed mixture was loaded onto a 4.5 x 30 cm silica column (silica gel 60) and was eluted with 5% ethyl acetate/ methylene chloride, collecting 25 mL fractions. Fractions 24-60 were combined and evaporated to give a green solid which was recrystallized from toluene to give the title compound as a light green solid (3.8 g).
¹H NMR (CDCl₃, 500 MHz) δ 6.82 (d, ArH), 6.83 (br.s, NH), 7.80 (d, ArH), 7.93 (t, ArH), 8.05 (d, ArH) and 8.38 (d, ArH).

### Step 2: 5-(hydroxymethyl)-1,8-naphthosultam

A solution of 5-bromo-1,8-naphthosultam (0.5 g, 1.76 mmol) in anhydrous tetrahydrofuran (10 mL) under nitrogen was cooled in a dry ice / acetone bath in a three neck flask. N-butyllithium (2.75 mL of a 1.6 M solution in hexanes, 4.4 mmol) was added over 2 minutes and the suspension was stirred an additional 7 minutes. Paraformaldehyde (0.317 g, 10.6 mmol), placed in the bulb of a drying tube which was attached to the flask, was heated with a heat gun while a slow stream of nitrogen was blown over the solid. The generated formaldehyde was carried into the flask and the carrier gas vented through a line connected to a Firestone valve over a period of 13 minutes. After an additional 5 minutes, the mixture was removed from the bath and stirred for 10 minutes. Aqueous hydrochloric acid (3 mL of a 2 N solution) was added and the clear suspension was stirred an additional 10 minutes. The mixture was partitioned between ethyl acetate (50 mL) and water (50 mL). The ethyl acetate layer was washed with saturated aqueous sodium chloride (20 mL), dried over magnesium sulfate, filtered, and evaporated. The solid residue (0.5 g) was dissolved in 5% methanol/ methylene chloride and was loaded onto a 24 x 4.5 cm silica gel column (silica gel 60, packed/ loaded/ eluted with 5% methanol/ methylene chloride), collecting 8 mL fractions. Fractions 12-42 were combined and evaporated to give the title compound as a white solid (0.185 g).
¹H NMR (DMSO-d₆, 500 MHz) δ 4.85 (d, CH₂OH), 5.22 (t, CH₂OH), 6.82 (d, ArH), 7.52 (d, ArH), 7.83 (t, ArH), 8.13 (d, ArH) and 8.38 (d, ArH).

### Step 3: 5-(trimethylsilyloxymethyl)-1,8-naphthosultam

A solution of 5-(hydroxymethyl)-1,8-naphthosultam (0.185 g, 0.79 mmol) in tetrahydrofuran (1 mL) was treated with N,O-Bis(trimethylsilyl)acetamide ((BSA), 0.49 mL, 1.98 mmol). The mixture was stirred at room temperature for 1 hour then evaporated. The residual oil was dissolved in methylene chloride (1 mL) and was filtered through silica gel 60 (2.5 g), eluting the silica with additional methylene chloride (50 mL). The solvent was evaporated under vacuum and the residue was lyophilized from benzene (3 mL) to give the title compound as a white solid (0.20 g).
¹H NMR (CDCl₃, 500 MHz) δ 0.19 (s, SiMe₃), 5.07 (s, CH₂), 6.83 (d, ArH), 6.87 (br.s, NH), 7.50 (d, ArH), 7.78 (t, ArH), 7.95 (d, ArH) and 8.26 (d, ArH).

### PREPARATIVE EXAMPLE 2 SYNTHESIS OF 5-(2-(TRIMETHYLSILYLOXY)-ETHYL)-1,8-NAPHTHOSULTAM

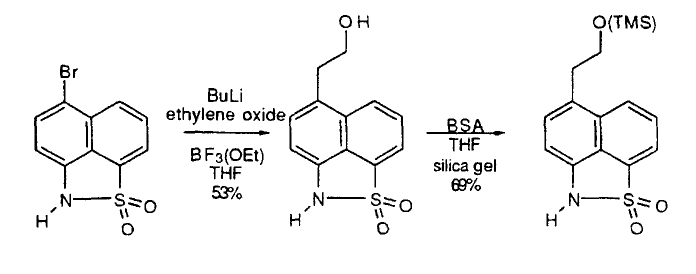

### Step 1: 5-(2-(hydroxy)-ethyl)-1,8-naphthosultam

A solution of 5-bromo-1,8-naphthosultam (0.6 g, 2.11 mmol) in anhydrous tetrahydrofuran (10mL) under nitrogen was cooled in a dry ice/ acetone bath. N-butyllithium (3.3 mL of a 1.6 M solution in hexanes, 5.28 mmol) was added over 7 minutes and the suspension was stirred an additional 8 minutes. An excess of ethylene oxide was slowly bubbled into the mixture over 5 minutes. Boron trifluoride etherate (0.26 mL, 2.11 mmol) was then added over 5 minutes. After an additional 20 minutes, the reaction was quenched with the addition of acetic acid (0.35 mL, 6 mmol). The mixture was partitioned between ethyl acetate (100 mL) and water (100 mL). The ethyl acetate layer was washed with saturated aqueous sodium chloride (50 mL), dried over magnesium sulfate, filtered, and evaporated. The residual oil (0.7 g) was dissolved in 5% methanol/ methylene chloride and was loaded onto a 24 x 2.75cm silica gel column (silica gel 60, packed/ loaded/ eluted with 5% methanol/ methylene chloride), collecting 8 mL fractions. Fractions 26-39 were combined and evaporated to give the title compound as an oil (0.28 g).
¹H NMR (DMSO-d₆, 500 MHz) δ 3.22 (t, CH₂Ar), 3.87 (t, CH₂OH), 6.79 (d, ArH), 7.35 (d, ArH), 7.74 (t, ArH), 7.91 (d, ArH) and 8.21 (d, ArH).

### Step 2: 5-(2-(trimethylsilyloxy)-ethyl)-1,8-naphthosultam

A solution of 5-(2-(hydroxy)-ethyl)-1,8-naphthosultam (0.09 g, 0.36 mmol) in tetrahydrofuran (1 mL) was treated with N,O-Bis(trimethylsilyl)acetamide (0.223 mL, 0.90 mmol). The mixture was stirred at room temperature for 20 minutes and was evaporated. The residual oil was dissolved in methylene chloride (3 mL) and was filtered through silica gel 60 (2.7 g), eluting the silica with additional methylene chloride (50 mL). The solvent was evaporated under vacuum and the residue was lyophilized from benzene (3 mL) to give the title compound as a white solid (0.08 g).

### PREPARATIVE EXAMPLE 3 SYNTHESIS OF 4-(2-(TRIMETHYLSILYLOXY)-ETHYL)-1,8-NAPHTHOSULTAM

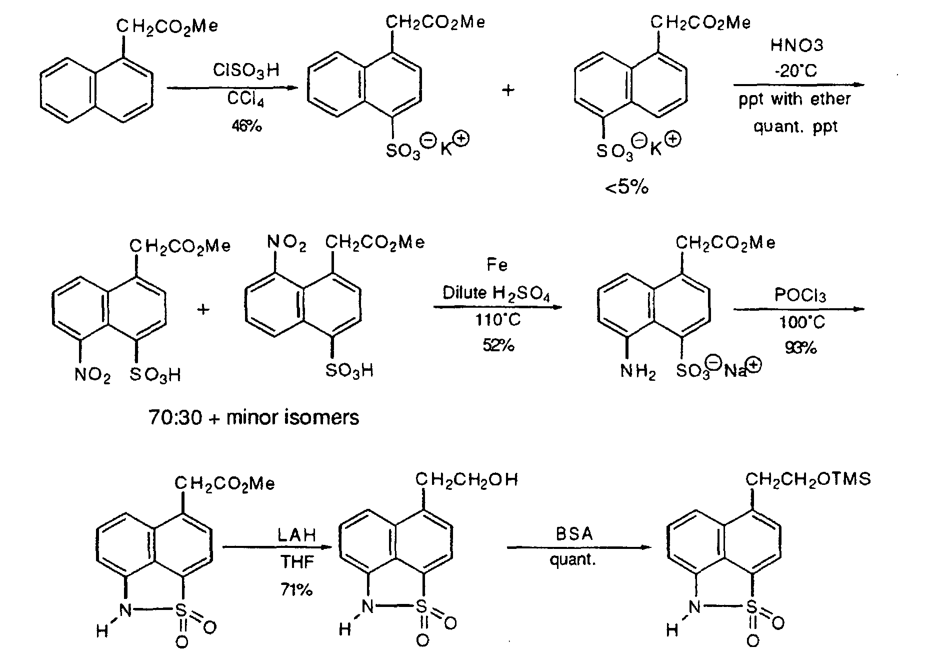

### Step 1: potassium 1-(methoxycarbonylmethyl)-4-naphthalene sulfonate

A solution of methyl 1-naphthaleneacetate (1 mL, 5.77 mmol) in carbon tetrachloride (1 mL) was cooled under nitrogen in an ice bath. Chlorosulfonic acid (0.38 mL, 5.7 mmol) was added dropwise over 8 minutes. After an additional 30 minutes, the viscous mixture was removed from the bath and was stirred at room temperature for 17 hours to give a white solid. The solid was partitioned between methylene chloride (5 mL) and water (5 mL). After filtering through solka-floc, the methylene chloride layer was extracted with more water (2 x 5 mL), and the combined aqueous extracts were basified with potassium carbonate to give a precipitate. The suspension was concentrated to approximately 5 mL and was cooled in an ice bath. The suspension was then filtered and the collected solid was washed with cold water (2 mL). The solid was dried under a stream of nitrogen to give the title compound as a white solid (0.84 g).
¹H NMR (DMSO-d₆, 500 MHz) δ 3.73 (s, OMe), 4.27 (s, CH₂Ar), 7.53 (d, ArH), 7.71 (t, ArH), 7.76 (t, ArH), 8.06 (d, ArH), 8.10 (d, ArH) and 8.73 (d, ArH).

### Step 2: 1-(methoxycarbonylmethyl)-5-nitro-4-naphthalene sulfonic acid

Potassium 1-(methoxycarbonylmethyl)-4-naphthalene sulfonate (10 g, 31.4 mmol) was added portionwise over 30 minutes to 90% nitric acid, which was cooled in a methanol / ice bath to approximately -15°C. After 2 hours, the bath temperature had reached -10°C and diethyl ether (200 mL) was added to the mixture. The precipitated solid was filtered, washed with ether (100 mL) and isopropanol (20 mL), and dried under a stream of nitrogen to give the title compound as an approximately 70:30 mixture of the 5- and 8-nitro isomers (approximately 12 g).
¹H NMR (D₂O, 500 MHz) δ 3.69 (s, OMe), 4.30 (s, CH₂Ar), 7.67 (t, ArH), 7.71 (d, ArH), 8.18 (d, ArH), 8.29 (d, ArH) and 8.33 (d, ArH).

### Step 3: sodium 1-(methoxycarbonylmethyl)-5-amino-4-naphthalene sulfonate

1-(methoxycarbonylmethyl)-5-nitro-4-naphthalene sulfonic acid (2 g, 6.15 mmol) was dissolved in water (20 mL), containing 0.5 mL concentrated sulfuric acid, and was added dropwise over 5 minutes to a refluxing suspension of iron (4 g, 71.6 mmol) in water (100 mL). After refluxing for one hour, the dark mixture was cooled to room temperature, made basic with sodium carbonate, and concentrated to approximately 30mL. The residual mixture was placed on a CG-161 amberchrom resin column (2.5 x 30 cm). The column was washed with water (200 mL), 10% MeCN/ H₂O(200 mL), and 25% MeCN/ H₂O (400 mL), collecting 25 mL fractions. Fractions 21-28 were combined and evaporated to give the title compound as a dark solid (0.675 g).
¹H NMR (D₂O, 500 MHz) δ 3.64 (s, OMe), 4.18 (s, CH₂Ar), 7.04 (d, ArH), 7.38 (d, ArH), 7.41 (d, ArH), 7.45 (t, ArH) and 8.22 (d, ArH).

### Step 4: 4-(methoxycarbonylmethyl)-1,8-naphthosultam

Sodium 1-(methoxycarbonylmethyl)-5-amino-4-naphthalene sulfonate (0.675 g, 2.13 mmol) was suspended in phosphorous oxychloride (10 g, 65.2 mmol) and was refluxed for 1 hour to give a thin suspension. The mixture was cooled to room temperature and was partitioned between ethyl acetate (100 mL) and water (100 mL). The water layer was extracted with ethyl acetate (50 mL) and the combined ethyl acetate layers were washed with saturated aqueous sodium chloride (100 mL), dried over magnesium sulfate, filtered and evaporated to give the title compound as a solid (0.55 g).
¹H NMR (CDCl₃, 500 MHz) δ 3.72 (s, OMe), 4.15 (s, CH₂Ar), 6.86 (br s, NH), 6.97 (d, ArH), 7.60 (t, ArH), 7.67 (d, ArH), 7.71 (d, ArH) and 7.95 (d, ArH).

### Step 5: 4-(2-(hydroxy)-ethyl)-1,8-naphthosultam

A solution of 4-(methoxycarbonylmethyl)-1,8-naphthosultam (0.2 g, 0.72 mmol) in tetrahydrofuran (2 mL) was cooled under nitrogen in an ice bath. Lithium aluminum hydride (1.44 mL of a 1.0 M solution in THF, 1.44 mmol) was added over 1 minute to give a light yellow suspension. After 30 minutes, water was carefully added and the mixture was partitioned between ethyl acetate (30 mL) and 1N hydrochloric acid (10 mL). The aqueous layer was extracted with ethyl acetate (50 mL) and the combined ethyl acetate layers were washed with saturated aqueous sodium chloride (10 mL), dried over magnesium sulfate, filtered and evaporated. The residual solid (0.16g) was purified by preparative thin layer chromatography (2 x 1000 micron silica gel plates, developed/ eluted with 5% MeOH/CH₂Cl₂) to give the title compound as a solid (0.127 g).
¹H NMR (0.14 mL CDCl₃ and 0.01 mL CD₃OD, 500 MHz) δ 3.33 (t, CH₂Ar), 3.91 (t, CH₂OH), 6.84 (d, ArH), 7.49 (dd, ArH), 7.59 (d, ArH), 7.59 (d, ArH) and 7.83 (d, ArH).

### Step 6: 4-(2-(trimethylsilyloxy)-ethyl)-1,8-naphthosultam

N,O-Bistrimethylsilylacetamide (0.31 mL, 1.25 mmol) was added to a solution of 4-(2-(hydroxy)-ethyl)-1,8-naphthosultam (0.125 g, 0.50 mmol) in tetrahydrofuran (1 mL). After one hour the mixture was evaporated and the residue was dissolved in methylene chloride (2 mL) and filtered through silica gel (2.5 g). The silica gel was eluted with methylene chloride (50 mL), the solvent was evaporated and the residue was lyophilized from benzene (3 mL) to give the title compound as an oil (0.16 g, quant.).
¹H NMR (CDCl₃, 500 MHz) δ 0.035 (s, TMS), 3.37 (t, CH₂Ar), 3.94 (t, CH₂O(TMS)), 6.95 (d, ArH), 7.56 (dd, ArH), 7.64 (d, ArH), 7.71 (d, ArH) and 7.92 (d, ArH).

### PREPARATIVE EXAMPLE 4 SYNTHESIS OF 4-(TRIMETHYLSILYLOXYMETHYL)-1,8-NAPHTHOSULTAM

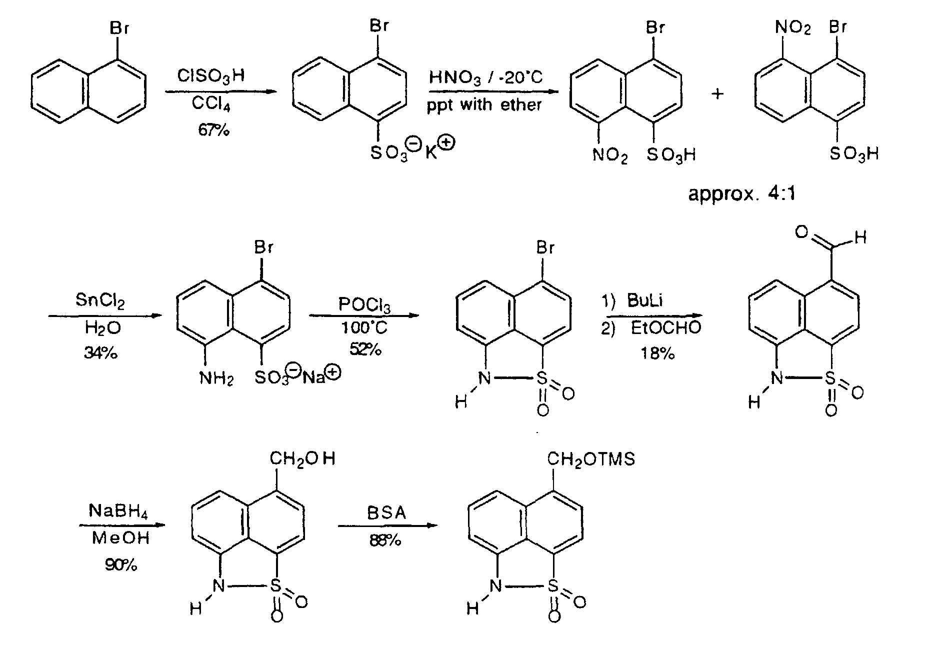

### Step 1: potassium 1-bromo-4-naphthalenesulfonate

A solution of 1-bromonaphthalene (19 mL, 137 mmol) in carbon tetrachloride (24 mL) was cooled in an ice bath under nitrogen. Chlorosulfonic acid (9.1 mL, 137 mmol) was added dropwise over 20 minutes. After an additional 5 minutes, the heavy grey suspension was removed from the ice bath and was stirred at room temperature for 16 hours to give a grey paste. The mixture was partitioned between methylene chloride (100 mL) and water (300 mL). The aqueous layer was made basic with potassium carbonate and the resulting suspension was filtered. The collected solid was washed with methylene chloride (50 mL) and water (50 mL), and dried under vacuum to give the title compound as a white solid (30 g, 67%).
¹H NMR (DMSO-d₆, 500 MHz) δ 7.61 (m, ArH), 7.65 (m, ArH), 7.82 (m, 2ArH), 8.14 (dd, ArH), and 8.90 (dd, ArH).

### Step 2: 1-bromo-5-nitro-4-naphthalene sulfonic acid

Potassium 1-bromo-4-naphthalenesulfonate (1.38 g, 4.24 mmol) was added portionwise over 20 minutes to 90% nitric acid (2 mL), which was cooled in a methanol/ ice bath to approximately -15°C. After 1.5 hours, the mixture was placed in a refrigerator for 20 hours. Diethyl ether (20 mL) was added and the precipitated solid was filtered, washed with ether (100 mL) and isopropanol (20 mL), and dried under a stream of nitrogen to give the title compound as an approximately 4:1 mixture of the 5- and 8-nitro isomers (1.25 g).
¹H NMR (D₂O, 500 MHz) δ 7.70 (dd, ArH), 8.09 (d, ArH), 8.20 (d, ArH), 8.21 (dd, ArH), and 8.63 (d, ArH).

### Step 3: sodium 1-bromo-5-amino-4-naphthalenesulfonate

1-Bromo-5-nitro-4-naphthalenesulfonate (1 g, 3.01 mmol) and tin chloride dihydrate (1.83 g, 8.1 mmol) were suspended in a mixture of water (10 mL) and ethanol (10 mL). The resulting mixture was heated for 3 hours in a 100 °C oil bath. The mixture was cooled to room temperature and filtered. The collected solid was suspended in water (20 mL) and the mixture was made basic with sodium carbonate then placed on a CG-161 amberchrom resin column (3 x 9 cm). The column was washed with water (300 mL) and was eluted with 25% MeCN/ H₂O, collecting 12 mL fractions. Fractions 17-19 were combined and evaporated to give the title compound as a solid (0.33 g).
¹H NMR (D₂O, 500 MHz) δ 7.07 (dd, ArH), 7.49 (t, ArH), 7.83 (d, ArH), 7.85 (dd ArH) and 8.08 (d, ArH).

### Step 4: 4-bromo-1,8-naphthosultam

Sodium 1-bromo-5-amino-4-naphthalenesulfonate (1.2 g, 3.70 mmol) was suspended in phosphorous oxychloride (10 mL, 107 mmol) and the mixture was refluxed for 1 hour to give a thin suspension. The mixture was cooled to room temperature and was added to ice (100 mL). The precipitate was collected and washed with water (20 mL) then dried under vacuum (0.675 g). A second crop was obtained from the filtrate (0.186 g). The combined solids were dissolved in 5% methanol in methylene chloride and were placed on a silica gel column (29 x 3.5cm, packed and eluted with 5% methanol in methylene chloride), collecting 8 mL fractions. Fractions 27-39 were combined and evaporated to give the title compound as a solid (0.55 g).
¹H NMR (0.14mLCDCl₃and 0.01mL CD₃OD, 500 MHz) δ 6.89 (d, ArH), 7.58(dd ArH), 7.68 (d, ArH), 7.73 (d, ArH) and 7.95 (d, ArH).

### Step 5: 4-formyl-1,8-naphthosultam

A solution of 4-bromo-1,8-naphthosultam (0.24 g, 0.845 mmol) in anhydrous tetrahydrofuran (5 mL) was cooled in a dry ice/acetone bath under nitrogen. n-Butyllithium (1.32 mL of a 1.6 M solution in hexanes, 2.11 mmol) was added and the mixture was stirred for 5 minutes. Ethyl formate (1 mL, 12.4 mmol) was then added, and after an additional 5 minutes, 2N aqueous hydrochloric acid (3 mL) was added. The flask was removed from the bath and the yellow solution was partitioned between ethyl acetate (30 mL) and water (30 mL). The ethyl acetate layer was washed with saturated aqueous sodium chloride (20 mL), dried over magnesium sulfate, filtered, and evaporated. The residual oil was purified on preparative silica gel plates (3 x 1000 micron/ developed and eluted with 5% methanol/methylene chloride) to give the title compound as a red solid (0.035 g).
¹H NMR (CDCl₃, 500 MHz) δ 7.09 (d, ArH), 7.78 (dd, ArH), 8.12 (d, ArH), 8.30(d, ArH), 8.70 (d, ArH) and 10.5 (s, CHO).

### Step 6: 4-(hydroxymethyl)-1,8-naphthosultam

A solution of 4-formyl-1,8-naphthosultam (0.035 g, 0.15 mmol) in anhydrous methanol (1 mL) was cooled in an ice bath under nitrogen. Sodium borohydride (0.011 g, 0.3 mmol) was added and the solution was stirred for 30 minutes. The mixture was partitioned between methylene chloride (10 mL) and 0.2N aqueous hydrochloric acid (10 mL). The aqueous layer was extracted with 5% methanol in methylene chloride (2 x 10 mL), and the combined organic layers were evaporated to give the title compound as a yellow solid (0.032 g).
¹H NMR (0.14mLCDCl₃and 0.01mL CD₃OD, 500 MHz) δ 5.13 (s, CH₂OH), 6.85 (d, ArH), 7.50 (dd, ArH), 7.57 (d, ArH), 7.82 (d, ArH) and 7.88 (d, ArH).

### Step 7: 4-(trimethylsilyloxymethyl)-1,8-naphthosultam

A solution of 4-(hydroxymethyl)-1,8-naphthosultam (0.032 g, 0.136 mmol) in tetrahydrofuran (0.5 mL) was treated with N,O-Bis(trimethylsilyl)acetamide (0.084 mL, 0.34 mmol). The mixture was stirred at room temperature for 45 minutes and was evaporated. The residual oil was dissolved in methylene chloride (1 mL) and was filtered through silica gel 60 (1 g), eluting the silica with additional methylene chloride (50 mL). The solvent was evaporated under vacuum and the residue was lyophilized from benzene (3 mL) to give the title compound as a white solid (0.037 g).
¹H NMR (CDCl₃, 500 MHz) δ 0.23 (s, SiMe₃), 6.78 (brs, NH), 5.23 (s, CH₂), 6.97 (d, ArH), 7.58 (dd, ArH), 7.64 (d, ArH), 7.90 (d, ArH) and 7.97 (d, ArH).

### PREPARATIVE EXAMPLE 5 SYNTHESIS OF 4-(3-TRIMETHYLSILYLOXYPROP-1-YL)-1,8-NAPHTHOSULTAM

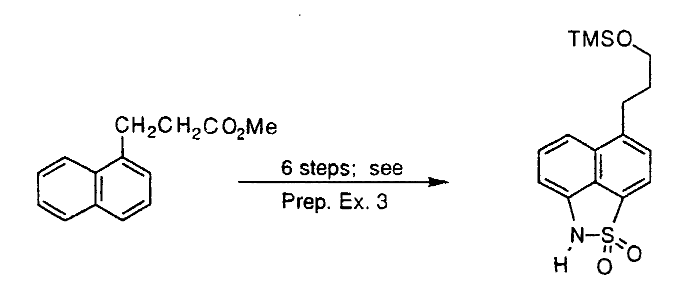

### Steps 1-6 Synthesis of 4-(3-trimethylsilyloxyprop-1-yl)-1,8-naphthosultam

By substitution of methyl 1-naphthalenepropionate for methyl 1-naphthaleneacetate in the procedure of Preparative Example 3, 4-(3-trimethylsilyloxyprop-1-yl)-1,8-naphthosultam is prepared.

### EXAMPLE 1

### SYNTHESIS OF (1S,5R,6S)-2-(5-(((CARBAMOYLMETHYL)-1,4-DIAZONIABICYCLO[2.2.2]OCT-1-YL)METHYL)(1,8-NAPHTHOSULTAM)METHYL)-6-[1(R)-HYDROXYETHYL]-1-METHYLCARBAPEN-2-EM-3-CARBOXYLATE CHLORIDE

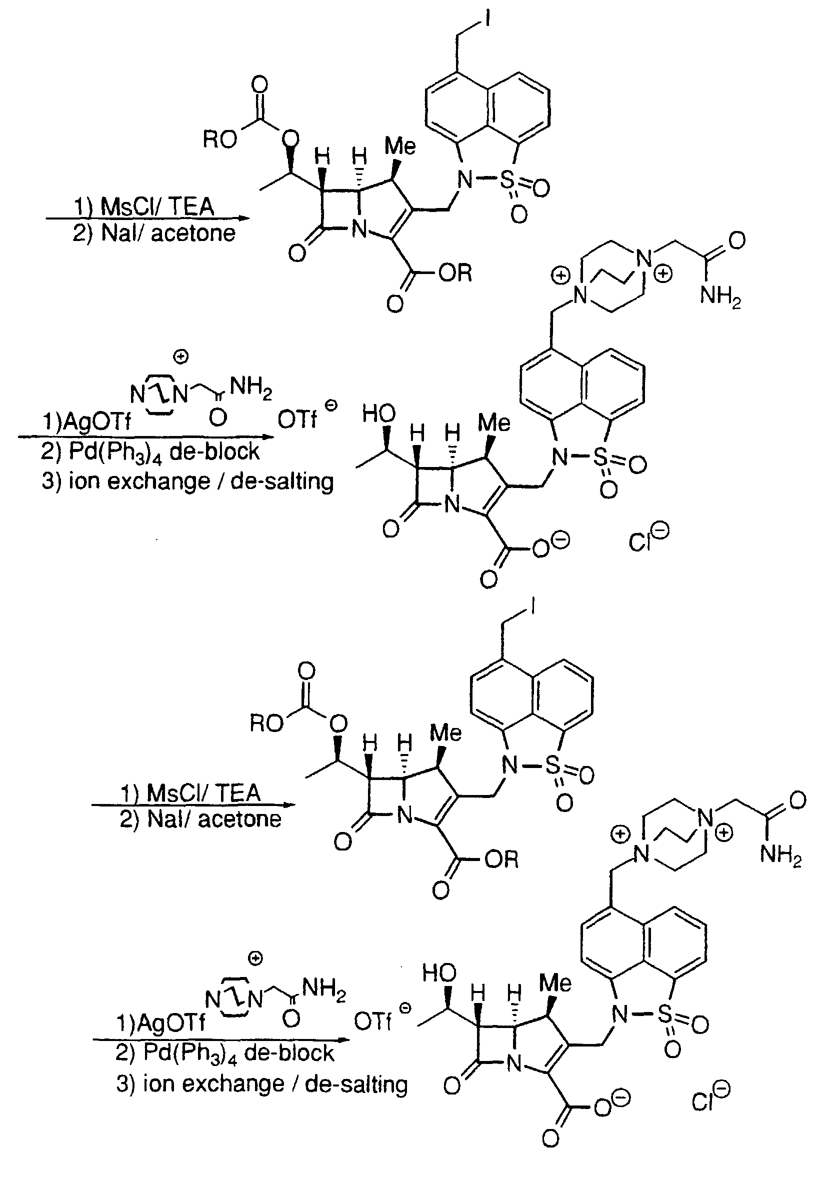

### Step 1: allyl (1S,5R,6S)-2-(5-(trimethylsilyloxymethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(hydroxymethyl)-6-[1(*R*)-(allyloxycarbonyl)oxyethyl]-1-methylcarbapen-2-em-3-carboxylate (0.09 g, 0.246 mmol), 5-(trimethylsilyloxymethyl)-1,8-naphthosultam (0.076 g, 0.246 mmol) and triphenylphosphine (0.097 g, 0.369 mmol) in tetrahydrofuran (1.5 mL), cooled in an ice bath, was added diethylazodicarboxylate (DEAD) (0.058 mL, 0.369 mmol). After 25 minutes the mixture was partitioned between ethyl acetate (20 mL) and water (20 mL). The water layer was extracted with ethyl acetate (10 mL) and the combined ethyl acetate layers were washed with saturated aqueous sodium chloride (20 mL), dried with magnesium sulfate, filtered, and evaporated. The residual oil was purified by preparative thin layer chromatography (3 x 1000 micron silica gel plates, developed/ eluted with 5% EtOAc/CH₂Cl₂) to give the title compound as an oil (0.134 g).
¹H NMR (CDCl₃, 500 MHz) δ 0.18 (s, TMS), 1.31 (d, 1-CH₃), 1.46 (d, CH₃CHO(ALLOC)), 3.38 (dq, H-1), 3.45 (dd, H-6), 4.17 (dd, H-5), 4.60 and 4.89 ( 2m's, 2CH₂vinyl), 5.07 (s, CH₂O), 5.13 (dq, H-8), 5.26, 5.34, 5.36 and 5.53 (4m's, 4 vinyl H's), 4.68 and 5.39 (2d's, CH₂N), 5.91 and 6.05 (2m's, 2CH₂vinyl), 6.67 (d, ArH), 7.49 (d, ArH), 7.83 (dd, ArH), 8.02 (d, ArH) and 8.33 (d, ArH).

### Step 2: allyl (1S,5R,6S)-2-(5-(hydroxymethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(5-(trimethylsilyloxymethyl-1,8-naphthosultam)methyl)-6-[1(*R*)-(allyloxycarbonyl)oxyethyl]-1-methylcarbapen-2-em-3-carboxylate (0.068 g, 0.104 mmol) in a mixture of tetrahydrofuran (1 mL) and water (0.5 mL), was added 1N aqueous trifluoromethane sulfonic acid (0.02 mL, 0.02 mmol). After 5 minutes the mixture was partitioned between methylene chloride (5 mL) and 5% aqueous bicarbonate (5 mL). The water layer was extracted with methylene chloride (5 mL) and the combined methylene chloride layers were dried with magnesium sulfate, filtered and evaporated to give the title compound as an oil (0.06 g, quant).
¹H NMR (CDCl₃, 500 MHz) δ 1.31 (d, 1-CH₃), 1.45 (d, CH₃CHO(ALLOC)), 3.38 (dq, H-1), 3.45 (dd, H-6), 4.16 (dd, H-5), 4.59 and 4.89 ( 2m's, 2CH₂vinyl), 5.08 (m, CH₂O), 5.13 (dq, H-8), 5.26, 5.34, 5.36 and 5.53 (4m's, 4 vinyl H's), 4.68 and 5.41 (2d's, CH₂N), 5.91 and 6.05 (2m's, 2CH₂vinyl), 6.67 (d, ArH), 7.50 (d, ArH), 7.85 (dd, ArH), 8.03 (d, ArH) and 8.41 (d, ArH).

### Step 3: allyl (1S,5R,6S)-2-(5-(iodomethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(5-(hydroxymethyl-1,8-naphthosultam)methyl)-6-[1(*R*)-(allyloxycarbonyl)oxyethyl]-1-methylcarbapen-2-em-3-carboxylate (0.12 g, 0.206 mmol) in dichloromethane (4 mL), cooled in an ice bath under nitrogen, was added triethylamine (0.05 mL, 0.361 mmol) followed by methane sulfonyl chloride (0.024 mL, 0.31 mmol). After 20 minutes, additonal triethylamine (0.025 mL, 0.18 mmol) and methane sulfonyl chloride (0.012 mL, 0.16 mmol) were added. After an additional 50 minutes, still more triethylamine (0.05mL, 0.36mmol) and methane sulfonyl chloride (0.024mL, 0.31 mmol) were added. After a total of 90 minutes the mixture was partitioned between methylene chloride (30 mL) and 0.1N aqueous hydrochloric acid (20 mL). The methylene chloride layer was washed with water (80 mL), dried over magnesium sulfate and evaporated. Examination of the residual oil by ¹H NMR showed mostly the chloromethyl derivative, with a trace of the expected mesylate. (The tlc, 5% ethyl acetate/ methylene chloride, of the mesylate and the starting alcohol are almost identical, apprx. R_{f} of 0.2, with the chloromethyl derivative at approximately 0.5. The addition of the excess reagents was therefore unnecessary). The oil was dissolved in acetone (3 mL) then sodium iodide (0.093 g, 0.618 mmol) was added and the mixture was stirred at room temperature for 3 hours. The reaction mixture was partitioned between methylene chloride (20 mL) and water (20 mL), the methylene chloride layer was washed with 5% aqueous NaHSO₃, dried over magnesium sulfate, filtered and evaporated. The residual oil was lyophilized from benzene (3 mL) to give the title compound as a foam (0.132 g)
¹H NMR (CDCl₃, 500 MHz) δ 1.30 (d, 1-CH₃), 1.42 (d, CH₃CHO(ALLOC)), 3.40 (dq, H-1), 3.43 (dd, H-6), 4.18 (dd, H-5), 4.59 and 4.89 ( 2m's, 2CH₂vinyl), 4.88 (m, CH₂O), 5.15 (dq, H-8), 5.26, 5.34, 5.36 and 5.53 (4m's, 4 vinyl H's), 4.65 and 5.55 (2d's, CH₂N), 5.91 and 6.05 (2m's, 2CH₂vinyl), 6.60 (d, ArH), 7.60 (d, ArH), 7.95 (dd, ArH), 8.03 (d, ArH) and 8.37 (d, ArH).

### Step 4: (1S,5R,6S)-2-(5-(((carbamoylmethyl)-1,4-diazoniabicyclo[2.2.2]oct-1-yl)methyl)(1,8-naphthosultam)methyl)-6-[1(R)-hydroxyethyl]-1-methylcarbapen-2-em-3-carboxylate chloride

To a solution of allyl (1*S*,5*R*,6*S*)-2-(5-(iodomethyl-1,8-naphthosultam)methyl)-6-[1(*R*)-(allyloxycarbonyl)oxyethyl]-1-methylcarbapen-2-em-3-carboxylate (0.025g, 0.036mmol) in acetonitrile (0.3mL) was added 1-carbamoylmethyl-4-aza-1-azoniabicyclo[2.2.2]-octane trifluoromethane sulfonate (0.012 g, 0.036 mmol) and silver trifluoromethane sulfonate (0.036 mL of a 1.0M solution in acetonitrile, 0.036 mmol). The suspension was stirred for 40 minutes at room temperature, filtered and evaporated. The residual oil was dissolved in dimethylformamide (0.5 mL). The solution was cooled in an ice bath and 0.5M sodium ethyl hexanoate in ethyl acetate (0.08 mL, 0.04 mmol) and ethyl hexanoic acid (0.006 mL, 0.04 mmol) were added. The solution was blanketed with nitrogen, using a Firestone valve, and triphenylphosphine (0.003 g, 0.012 mmol) and tetrakis(triphenylphosphine)palladium (0.014 g, 0.012 mmol) were added. After one hour, diethyl ether (5 mL) was added and the supernatant was decanted from the precipitate. The solid was washed with additional diethyl ether (5 mL) and was dried under vacuum. The solid was dissolved in 1:1 acetonitrile/ water (3 mL) and was loaded onto a Bio-Rad weak cation exchange resin (3 mL, macroprep cm ion exchange resin, sodium cycle). The column was washed with 1:1 acetonitrile/ water (2 mL) and water (12 mL). The column was then eluted with 5% aqueous sodium chloride, collecting 3 mL fractions. Fractions 1-5 were cooled in an ice bath and were then loaded onto an amberchrom CG-161 resin (3mL). The column was washed with cold de-ionized water (20mL) and was eluted with 20% isopropanol in water, collecting 4 mL fractions. Fractions 1-4 were combined and concentrated to approximately 1mL which was lyophilized to give the title compound (0.005 g).
¹H NMR (D₂O, 500 MHz) δ 1.16 (d, 1-CH₃), 1.21 (d, CH₃CHOH), 3.10 (dq, H-1), 3.42 (dd, H-6), 4.03 (dd, H-5), 4.18 (dq, H-8), 4.05 and 4.20 (2m, NCH₂CH₂N), 4.36 (s, CH₂CONH₂), 4.63 and 5.25 (2d's, CH₂N), 5.20 (s, ArCH₂), 6.89(d, ArH), 7.78 (d ArH), 7.99 (dd, ArH), 8.13 (d, ArH) and 8.40 (d, ArH).

### EXAMPLE 2

### SYNTHESIS OF (1S,5R,6S)-2-(5-(((3-HYDROXYPROP-1-YL)-1,4-DIAZONIABICYCLO[2.2.2]OCT-1-YL)METHYL)(1,8-NAPHTHOSULTAM)METHYL)-6-[1(R)-HYDROXYETHYL]-1-METHYLCARBAPEN-2-EM-3-CARBOXYLATE CHLORIDE

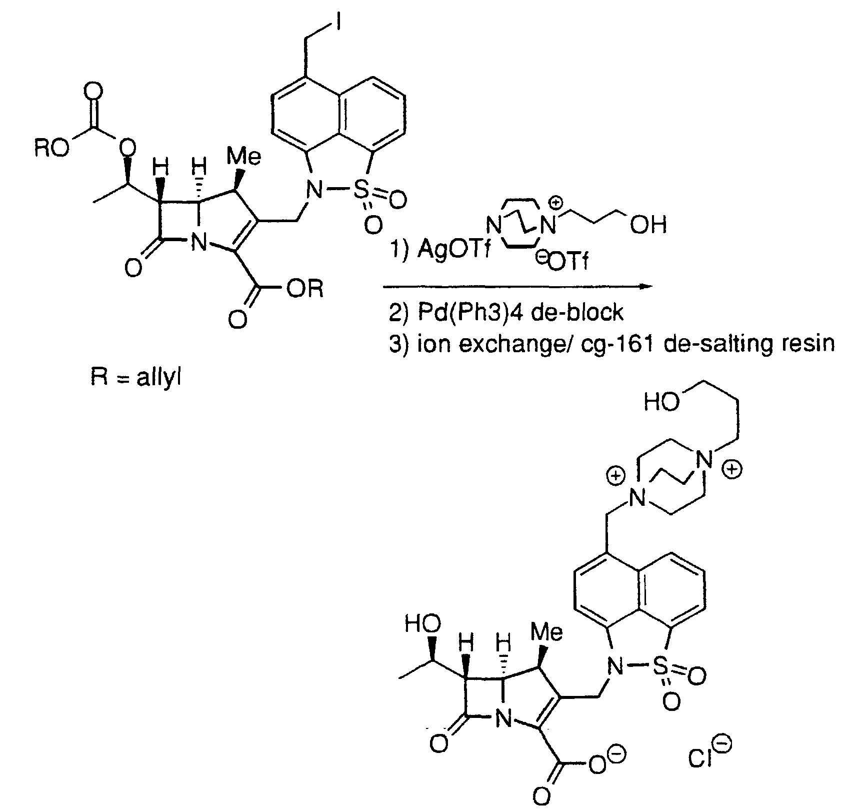

To a solution of allyl (1*S*,5*R*,6*S*)-2-(5-(iodomethyl-1,8-naphthosultam)methyl)-6-[1(*R*)-(allyloxycarbonyl)oxyethyl]-1-methylcarbapen-2-em-3-carboxylate (0.035 g, 0.051 mmol) in acetonitrile (0.3 mL) was added 1-(3-hydroxypropyl)-4-aza-1-azoniabicyclo[2.2.2]octane trifluoromethane sulfonate (0.018 g, 0.056 mmol) and silver trifluoromethane sulfonate (0.05 mL of a 1.0M solution in acetonitrile, 0.05 mmol). The suspension was stirred for 45 minutes at room temperature, filtered and evaporated. The residual oil was dissolved in dimethylformamide (0.5mL). The resulting solution was cooled in an ice bath and 0.5M sodium ethyl hexanoate in ethyl acetate (0.08 mL, 0.04 mmol) and ethyl hexanoic acid (0.006 mL, 0.04 mmol) were added. The solution was blanketed with nitrogen, using a Firestone valve, and triphenylphosphine (0.003 g, 0.012 mmol) and tetrakis(triphenylphosphine)palladium (0.014 g, 0.012 mmol) were added. After one hour, diethyl ether (4 mL) was added and the supernatant was decanted from the precipitate. The solid was washed with additional diethyl ether (4 mL) and was dried under vacuum. The solid was dissolved in 1:1 acetonitrile/ water (3 mL) and was loaded onto a Bio-Rad weak cation exchange resin (3 mL, macroprep cm ion exchange resin, sodium cycle). The column was washed with 1:1 acetonitrile/ water (2 mL) and water (18 mL). The column was then eluted with 5% aqueous sodium chloride, collecting 3 mL fractions. Fractions 1-4 were cooled in an ice bath and were then loaded onto an amberchrom CG-161 resin (3 mL). The column was washed with cold de-ionized water (20 mL) and was eluted with 20% isopropanol in water, collecting 5 mL fractions. Fractions 1-4 were combined and concentrated to approximately 1 mL,which was lyophilized to give the title compound (0.006 g).
¹H NMR (D₂O, 500 MHz) δ 1.16 (d, 1-CH₃), 1.21 (d, CH₃CHOH), 2.00 (m, ArCH₂CH₂CH₂OH), 3.11 (dq, H-1), 3.42 (dd, H-6), 3.62 and 3.65 (m, ArCH₂CH₂CH₂OH), 3.93 and 4.05 (2m, NCH₂CH₂N), 4.05(dd, H-5), 4.17 (dq, H-8), 4.36 (s, CH₂CONH₂), 4.63 and 5.26 (2d's, CH₂N), 5.20 (s, ArCH₂), 6.89(d, ArH), 7.78 (d ArH), 7.99 (dd, ArH), 8.15 (d, ArH) and 8.40 (d, ArH).

### EXAMPLE 3

### SYNTHESIS OF (1S,5R,6S)-2-(5-((1-METHYLIMIDAZOL-3-IUM)METHYL)(1,8-NAPHTHOSULTAM)METHYL)-6-[1(R)-HYDROXYETHYL]-1-METHYLCARBAPEN-2-EM-3-CARBOXYLATE

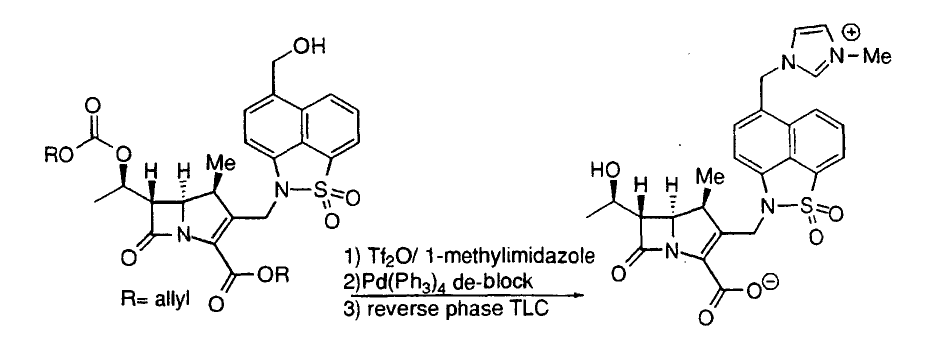

To a solution of allyl (1*S*,5*R*,6*S*)-2-(5-(hydroxymethyl-1,8-naphthosultam)methyl)-6-[1(*R*)-(allyloxycarbonyl)oxyethyl]-1-methylcarbapen-2-em-3-carboxylate (0.065 g, 0.11 mmol) in dichloromethane (2 mL), cooled in a dry ice/ acetone bath under nitrogen, was added 1-methylimidazole (0.035 mL, 0.44 mmol) followed by trifluoromethanesulfonic anhydride (0.038 mL, 0.22 mmol). After 5 minutes the flask was removed from the bath and the mixture was stirred at room temperature for 45 minutes. The mixture was then partitioned between methylene chloride (10 mL) and water (10 mL). The methylene chloride layer was washed with water (10 mL), dried over magnesium sulfate, filtered, and evaporated. The residual oil (0.08 g) was dissolved in methylene chloride (2 mL) then 0.5M sodium ethyl hexanoate in ethyl acetate (0.22 mL, 0.11 mmol) and ethyl hexanoic acid (0.017mL, 0.11mmol) were added. The solution was blanketed with nitrogen, using a Firestone valve, and triphenylphosphine (0.008 g, 0.03 mmol) and tetrakis(triphenylphosphine)palladium (0.035 g, 0.03 mmol) were added. After 40 minutes the methylene chloride was evaporated under a stream of nitrogen and dimethylformamide (1.5 mL) was added. After an additional 15 minutes, diethyl ether (6 mL) was added and the supernatant was decanted from the precipitate. The solid was washed with additional diethyl ether (6 mL) and dried under vacuum. The solid was purified on a 1000 micron reverse phase TLC plate developed with 30% acetonitrile/ water and eluted with 80% acetonitrile/ water (25 mL). The eluent was diluted with water (15 mL), washed with hexanes (25 mL), concentrated to approximately 1 mL and lyophilized to give the title compound (0.024 g).
¹H NMR (D₂O, 500 MHz) δ 1.13 (d, 1-CH₃), 1.21 (d, CH₃CHOH), 3.06 (dq, H-1), 3.40 (dd, H-6), 3.78 (s, ImMe), 4.02 (dd, H-5), 4.17 (dq, H-8), 4.57 and 5.22 (2d's, CH₂N), 5.70 (s, ArCH₂), 6.78 (d, ArH), 7.37 and 7.41 (2m, ImH), 7.59 (d, ArH), 7.83 (t, ArH), 8.05 (d, ArH), 8.14 (d, ArH) and 8.63 (s, ImH).

### EXAMPLE 4

### SYNTHESIS OF (1S,5R,6S)-2-(5-(((2-(1-METHYLIMIDAZOL-3-IUM)-ETHYL)(1,8-NAPHTHOSULTAM)METHYL)-6-[1(R)-HYDROXYETHYL]-1-METHYLCARBAPEN-2-EM-3-CARBOXYLATE

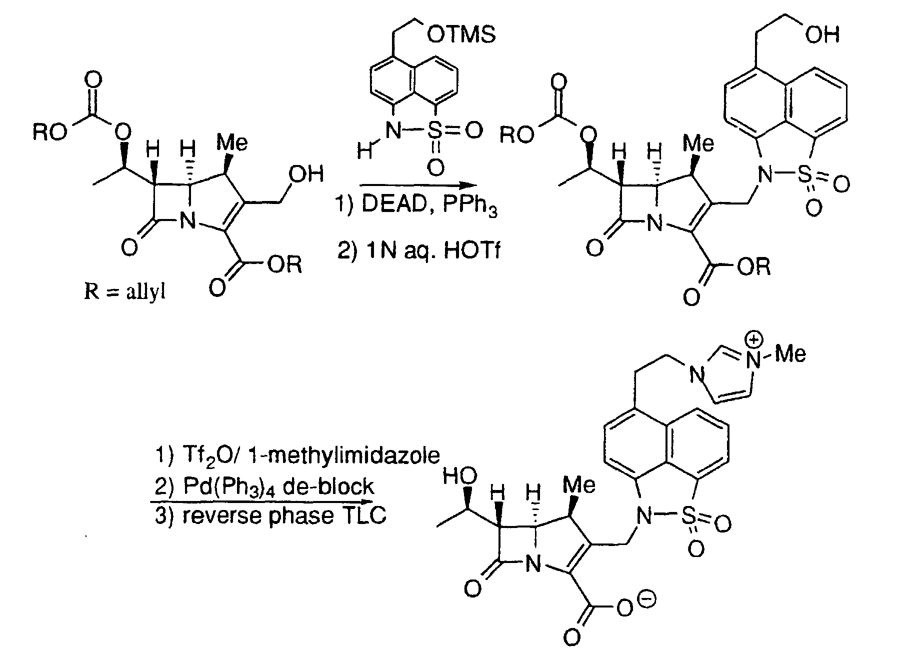

### Step 1: Synthesis of allyl (1S,5R,6S)-2-(5-((2-hydroxy)-ethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(hydroxymethyl)-6-[1(*R*)-(allyloxycarbonyl)oxyethyl]-1-methylcarbapen-2-em-3-carboxylate (0.086 g, 0.235 mmol), 4-((2-trimethylsilyloxy)-ethyl)-1,8-naphthosultam (0.08 g, 0.247 mmol) and triphenylphosphine (0.093 g, 0.353 mmol) in tetrahydrofuran (1 mL), cooled in an ice bath, was added diethylazodicarboxylate (0.056 mL, 0.353 mmol). After 30 minutes the mixture was partitioned between ethyl acetate (20 mL) and water (20 mL). The water layer was extracted with ethyl acetate (10 mL) and the combined ethyl acetate layers were washed with saturated aqueous sodium chloride (20 mL), dried over magnesium sulfate, filtered, and evaporated. The residual oil was purified by preparative thin layer chromatography (2 x 1000 micron silica gel plates, developed/ eluted with 5% EtOAc/CH₂Cl₂) to give an oil (0.074 g, 47%). This oil was dissolved in a mixture of tetrahydrofuran (2 mL) and water (0.5 mL) then 1N aqueous trifluoromethane sulfonic acid (0.02 mL, 0.02 mmol) was added. After 5 minutes the mixture was partitioned between methylene chloride (20 mL) and 1% aqueous bicarbonate (20 mL). The water layer was extracted with methylene chloride (5 mL) and the combined methylene chloride layers were dried over magnesium sulfate, filtered, and evaporated. The residue was lyophilized from benzene (3 mL) to give the title compound as a solid (0.062 g).
¹H NMR (CDCl₃, 500 MHz) δ 1.32 (d, 1-CH₃), 1.46 (d, CH₃CHO(ALLOC)), 3.29 (m, ArCH₂CH₂OH), 3.40 (dq, H-1), 3.45 (dd, H-6), 3.97 (dt, ArCH₂CH₂OH), 4.17 (dd, H-5), 4.59 and 4.88 ( 2m's, 2CH₂vinyl), 5.14 (dq, H-8), 5.26, 5.33, 5.35 and 5.53 (4m's, 4 vinyl H's), 4.67 and 5.40 (2d's, CH₂N), 5.91 and 6.05 (2m's, 2CH₂vinyl), 6.69 (d, ArH), 7.40 (d, ArH), 7.83 (dd, ArH), 8.02 (d, ArH) and 8.28 (d, ArH).

### Step 2: (1S,5R,6S)-2-(5-((2-(1-methylimidazol-3-ium)-ethyl)(1,8-naphthosultam)methyl)-6-[1(R)-hydroxyethyl]-1-methylcarbapen-2-em-3-carboxylate

To a solution of allyl (1*S*,5*R*,6*S*)-2-(5-(2-hydroxy)ethyl-1,8-naphthosultam)methyl)-6-[1(*R*)-(allyloxycarbonyl)oxyethyl]-1-methylcarbapen-2-em-3-carboxylate (0.03 g, 0.05 mmol) in dichloromethane (0.5 mL), cooled in a dry ice/ acetone bath under nitrogen, was added 1-methylimidazole (0.032 mL, 0.40 mmol) followed by trifluoromethane-sulfonic anhydride (0.017 mL, 0.10 mmol). After 5 minutes the flask was removed from the bath and stirred at room temperature for 20 minutes. The mixture was partitioned between methylene chloride (20 mL) and water (20 mL). The methylene chloride layer was washed with water (100 mL), dried over magnesium sulfate, filtered and evaporated. The residual oil (0.053g) was dissolved in dimethylformamide (0.7 mL) and was cooled in an ice bath. A solution of 0.5M sodium ethyl hexanoate in ethyl acetate (0.11 mL, 0.055 mmol) and ethyl hexanoic acid (0.018 mL, 0.11 mmol) were added. The solution was blanketed with nitrogen, using a Firestone valve, and triphenylphosphine (0.004 g, 0.0165 mmol) and tetrakis(triphenylphosphine)palladium (0.019 g, 0.0165 mmol) were added. After 70 minutes, diethyl ether (5 mL) was added and the supernatant was decanted from the precipitate. The solid was washed with additional diethyl ether (5 mL) and was dried under vacuum. The solid was purified on a 1000 micron reverse phase plate developed, in an ice bath, with 30% acetonitrile/ water and eluted with 80% acetonitrile/ water (15 mL). The eluent was diluted with de-ionized water (10 mL), washed with hexanes (40 mL), evaporated to approximately 2 mL and lyophilized to give the title compound (0.020 g).
¹H NMR (D₂O, 500 MHz) δ 1.11 (d, 1-CH₃), 1.20 (d, CH₃CHOH), 3.06 (dq, H-1), 3.29 (m, ArCH₂CH₂Q), 3.38 (dd, H-6), 3.60 (s, ImMe), 4.04 (dd, H-5), 4.17 (dq, H-8), 4.38 (m, ArCH₂CH₂Q), 4.50 and 5.14 (2d's, CH₂N), 6.56 (d, ArH), 6.96 (d, ArH), 7.15 and 7.20 (2m, ImH), 7.69 (dd, ArH), 7.91 (d, ArH), 7.95 (d, ArH) and 8.15 (s, ImH).

### EXAMPLE 5

### SYNTHESIS OF (1S,5R,6S)-2-(4-(((CARBAMOYLMETHYL)-1,4-DIAZONIABICYCLO[2.2.2]OCT-1-YL)METHYL)(1,8-NAPHTHOSULTAM)METHYL)-6-[1(R)-HYDROXYETHYL]-1-METHYLCARBAPEN-2-EM-3-CARBOXYLATE CHLORIDE

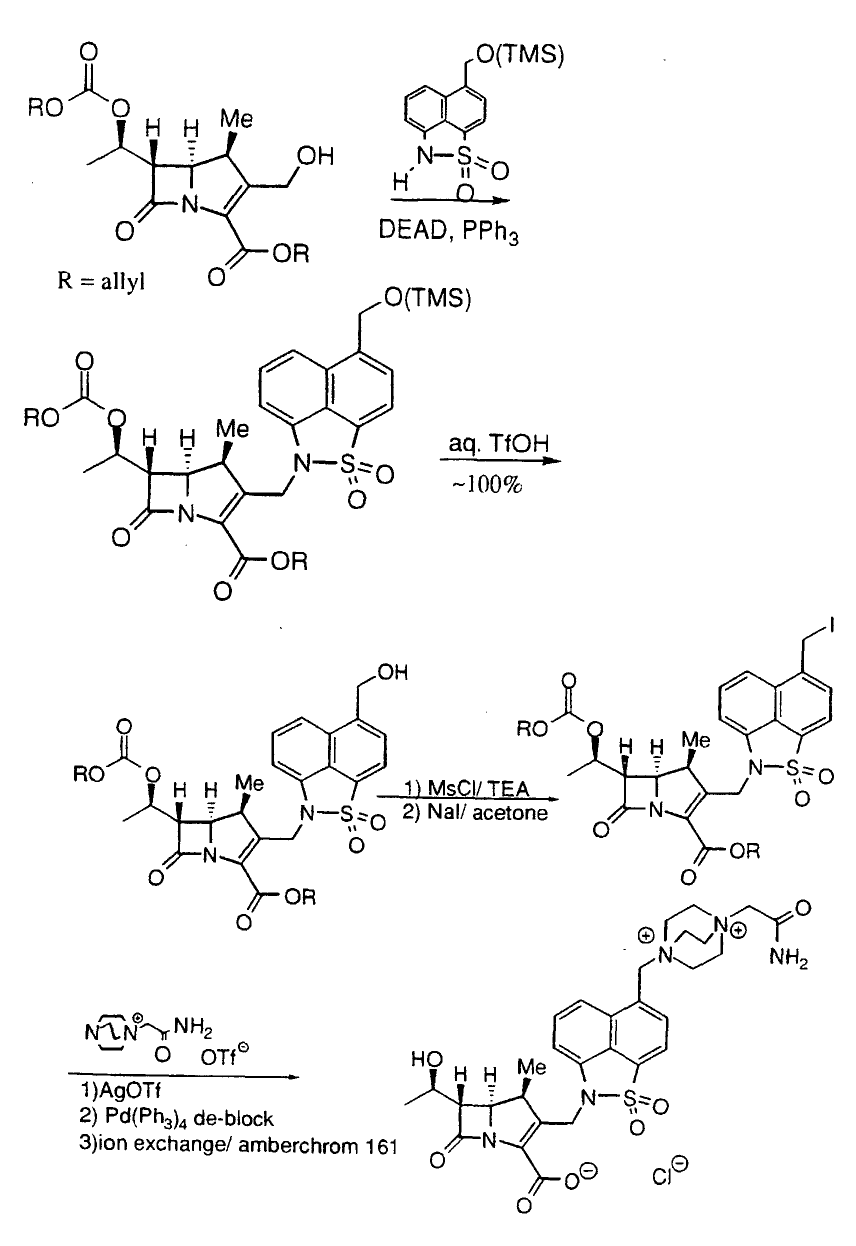

### Step 1: allyl (1S,5R,6S)-2-(4-(trimethylsilyloxymethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(hydroxymethyl)-6-[1(*R*)-(allyloxycarbonyl)oxyethyl)-1-methylcarbapen-2-em-3-carboxylate (0.037 g, 0.12 mmol), 4-(trimethylsilyloxymethyl)-1,8-naphthosultam (0.049 g, 0.12 mmol) and triphenylphosphine (0.047 g, 0.18 mmol) in tetrahydrofuran (0.7 mL), cooled in an ice bath, was added diethylazodicarboxylate (0.028 mL, 0.18 mmol). After 30 minutes, the mixture was partitioned between ethyl acetate (20 mL) and water (20 mL). The water layer was extracted with ethyl acetate (10 mL) and the combined ethyl acetate layers were washed with saturated aqueous sodium chloride (20 mL), dried over magnesium sulfate, filtered and evaporated. The residual oil was purified by preparative thin layer chromatography on a 1000 micron silica gel plate, developed/eluted with 5% EtOAc/CH₂Cl₂) to give the title compound as an oil (0.045 g).
¹H NMR (CDCl₃, 500 MHz) δ 0.23 (s, TMS), 1.31 (d, 1-CH₃), 1.45 (d, CH₃CHO(ALLOC)), 3.40 (dq, H-1), 3.45 (dd, H-6), 4.16 (dd, H-5), 4.59 and 4.88 ( 2m's, 2CH₂vinyl), 5.13 (dq, H-8), 5.22 (s, CH₂O), 5.26, 5.34, 5.35 and 5.53 (4m's, 4 vinyl H's), 4.68 and 5.40 (2d's, CH₂N), 5.91 and 6.05 (2m's, 2CH₂vinyl), 6.72 (m, ArH), 7.53 (m, 2ArH), 7.90 (m, ArH), and 7.98 (d, ArH).

### Step 2: allyl (1S,5R,6S)-2-(4-hydroxymethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(4-(trimethylsilyloxymethyl-1,8-naphthosultam)methyl)-6-[1(*R*)-(allyloxycarbonyl)oxyethyl]-1-methylcarbapen-2-em-3-carboxylate (0.045 g, 0.069 mmol) in a mixture of tetrahydrofuran (1 mL) and water (0.5 mL), was added 1N aqueous trifluoromethane sulfonic acid (0.01 mL, 0.01 mmol). After 5 minutes, the mixture was partitioned between methylene chloride (20 mL) and 5% aqueous bicarbonate (5 mL). The water layer was extracted with methylene chloride (5 mL) and the combined methylene chloride layers were dried over magnesium sulfate, filtered and evaporated to give the title compound as an oil (0.04 g, quant).
¹H NMR (CDCl₃, 500 MHz) δ 1.30 (d, 1-CH₃), 1.44 (d, CH₃CHO(ALLOC)), 3.39 (dq, H-1), 3.44 (dd, H-6), 4.15 (dd, H-5), 4.58 and 4.88 ( 2m's, 2CH₂vinyl), 5.13 (dq, H-8), 5.22 (d, CH₂O), 5.25, 5.33, 5.34 and 5.52 (4m's, 4 vinyl H's), 4.67 and 5.40 (2d's, CH₂N), 5.90 and 6.04 (2m's, 2CH₂vinyl), 6.73 (d, ArH), 7.53 (dd, ArH), 7.59 (dd, ArH), 7.88 (d, ArH) and 7.97 (d, ArH).

### Step 3: allyl (1S,5R,6S)-2-(4-(iodomethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(4-(hydroxymethyl-1,8-naphthosultam)methyl)-6-[1(*R*)-(allyloxycarbonyl)oxyethyl]-1-methylcarbapen-2-em-3-carboxylate (0.043 g, 0.074 mmol) in dichloromethane (1.5 mL), cooled in an ice bath under nitrogen, was added triethylamine (0.018 mL, 0.13 mmol) followed by methane sulfonyl chloride (0.009 mL, 0.111 mmol). After a total of 30 minutes the mixture was partitioned between methylene chloride (20 mL) and 0.1N aqueous hydrochloric acid (20 mL). The methylene chloride layer was dried over magnesium sulfate then filtered and evaporated. Examination of the residual oil by ¹H NMR showed a complete conversion to the mesylate. The oil was dissolved in acetone (2 mL) then sodium iodide (0.067 g, 0.444 mmol) was added and the mixture was stirred at room temperature for 75 minutes. The reaction was partitioned between methylene chloride (20 mL) and water (20 mL). The methylene chloride layer was washed with 5% aqueous NaHSO₃ (20 mL), dried over magnesium sulfate, filtered, and evaporated. The residual oil was lyophilized from benzene (3 mL) to give the title compound as a foam (0.039 g)
¹H NMR (CDCl₃, 500 MHz) δ 1.31 (d, 1-CH₃), 1.46 (d, CH₃CHO(ALLOC)), 3.40 (dq, H-1), 3.46 (dd, H-6), 4.17 (dd, H-5), 4.59 and 4.89 ( 2m's, 2CH₂vinyl), 4.90 (m, CH₂I), 5.14 (dq, H-8), 5.26, 5.34, 5.36 and 5.53 (4m's, 4 vinyl H's), 4.67 and 5.42 (2d's, CH₂N), 5.91 and 6.05 (2m's, 2CH₂vinyl), 6.74 (m, ArH), 7.65 (m, 2ArH), 7.81 (d, ArH), and 7.87 (d, ArH).

### Step 4: (1S,5R,6S)-2-(4-(((carbamoylmethyl)-1,4-diazoniabicyclo[2.2.2]oct-1-yl)methyl)(1,8-naphthosultam)methyl)-6-[1(R)-hydroxyethyl]-1-methylcarbapen-2-em-3-carboxylate chloride

To a solution of allyl (1*S*,5*R*,6*S*)-2-(4-(iodomethyl-1,8-naphthosultam)methyl)-6-[1(*R*)-(allyloxycarbonyl)oxyethyl]-1-methylcarbapen-2-em-3-carboxylate (0.039 g, 0.056 mmol) in acetonitrile (0.5mL) was added 1-carbamoylmethyl-4-aza-1-azoniabicyclo-[2.2.2]octane trifluoromethane sulfonate (0.022 g, 0.0676 mmol) and silver trifluoromethane sulfonate (0.056 mL of a 1.0M solution in acetonitrile, 0.056 mmol). The suspension was stirred for 1 hour at room temperature, then filtered and the solvent was evaporated under vacuum. The residual oil was dissolved in dimethylformamide (0.9 mL) and the solution was cooled in an ice bath as 0.5M sodium ethyl hexanoate in ethyl acetate (0.12 mL, 0.062 mmol) and ethyl hexanoic acid (0.01 mL, 0.062 mmol) were added. The solution was blanketed with nitrogen, using a Firestone valve, and triphenylphosphine (0.0044 g, 0.017 mmol) and tetrakis(triphenylphosphine)palladium (0.02 g, 0.017 mmol) were added. After one hour, diethyl ether (25 mL) was added and the supernatant was decanted from the precipitate. The solid was washed with additional diethyl ether (25 mL) and dried under vacuum. The combined ether layers were extracted with water (10 mL) and the aqueous layer was loaded onto a Bio-Rad weak cation exchange resin (2.5 mL, macroprep CM weak cation exchange resin, sodium cycle). The solid was dissolved in 1:1 acetonitrile/ water (2 mL) and was also loaded onto the ion-exchange resin. The column was washed with 1:1 acetonitrile/ water (10 mL) and water (50 mL). The column was then eluted with 5% aqueous sodium chloride, collecting 8 mL fractions. Fractions 1-6 were cooled in an ice bath and were then loaded onto an amberchrom CG-161 resin (3 mL). The column was washed with cold de-ionized water (50 mL) and was eluted with 20% isopropanol in water, collecting 3 mL fractions. Fractions 1+ 2 were combined and concentrated to approximately 1 mL which was lyophilized to give the title compound (0.01 g).
¹H NMR (D₂O, 500 MHz) δ 1.07 (d, 1-CH₃), 1.22 (d, CH₃CHOH), 2.98 (dq, H-1), 3.37(dd, H-6), 3.95 (dd, H-5), 4.15 (dq, H-8), 4.15 and 4.22 (2m, NCH₂CH₂N), 4.36 (s, CH₂CONH₂), 4.57 and 5.17 (2d's, CH₂N), 5.34 (s, ArCH₂), 6.89 (d, ArH), 7.72(m, 2ArH), 8.10 (dd, ArH) and 8.16 (d, ArH).

### EXAMPLE 6

### SYNTHESIS OF (1S,5R,6S)-2-(4-(2-(((CARBAMOYLMETHYL)-1,4-DIAZONIABICYCLO[2.2.2]OCT-1-YL))-ETHYL)(1,8-NAPHTHOSULTAM)METHYL)-6-[1(R)-HYDROXYETHYL]-1-METHYLCARBAPEN-2-EM-3-CARBOXYLATE CHLORIDE

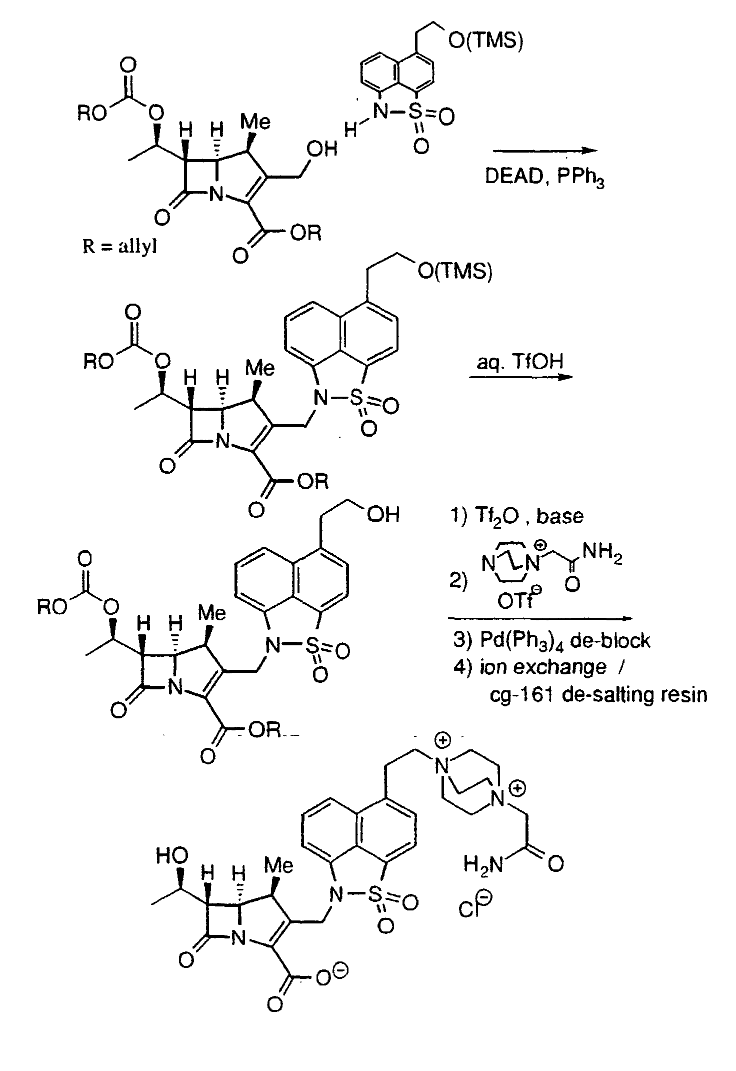

### Step 1: Synthesis of allyl (1S,5R,6S)-2-(4-(trimethylsilyloxyethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(hydroxymethyl)-6-[1(*R*)-(allyloxycarbonyl)oxyethyl]-1-methylcarbapen-2-em-3-carboxylate (0.086 g, 0.235 mmol), 4-(2(-trimethylsilyloxy)-ethyl)-1,8-naphthosultam (0.08 g, 0.247 mmol) and triphenylphosphine (0.093 g, 0.353 mmol) in tetrahydrofuran (1 mL), cooled in an ice bath, was added diethylazodicarboxylate (0.056 mL, 0.353 mmol). After 30 minutes the mixture was partitioned between ethyl acetate (20 mL) and water (20 mL). The aqueous layer was extracted with ethyl acetate (10 mL), and the combined organic layers were washed with saturated aqueous sodium chloride (30 mL), dried over magnesium sulfate, filtered and evaporated. The residual oil was purified by preparative thin layer chromatography (2 x 1000 micron silica gel plates, developed/ eluted with 5% EtOAc/CH₂Cl₂) to give the title compound as an oil (0.105 g).
¹H NMR (CDCl₃, 500 MHz) δ 0.04 (s, TMS), 1.31 (d, 1-CH₃), 1.45 (d, CH₃CHO(ALLOC)), 3.36 (t, ArCH₂CH₂O(TMS)), 3.40 (dq, H-1), 3.45 (dd, H-6), 3.93 (t, ArCH₂CH₂O(TMS), 4.16 (dd, H-5), 4.60 and 4.89 ( 2m's, 2CH₂vinyl), 5.14 (dq, H-8), 5.26, 5.34, 5.36 and 5.53 (4m's, 4 vinyl H's), 4.69 and 5.40 (2d's, CH₂N), 5.91 and 6.05 (2m's, 2CH₂vinyl), 6.72 (d, ArH), 7.52 (t, ArH), 7.63 (d, ArH), 7.64 (d, ArH) and 7.93 (d, ArH).

### Step 2: allyl (1S,5R,6S)-2-(4-(2-(hydroxy)-ethyl-1,8-naphthosultam)methyl)-6-[1(R)-(allyloxycarbonyl)oxyethyl]-1-methylcarbapen-2-em-3-carboxylate

To a solution of allyl (1*S*,5*R*,6*S*)-2-(4-(2-(trimethylsilyloxy)-ethyl-1,8-naphthosultam)methyl)-6-[1(*R*)-(allyloxycarbonyl)oxyethyl]-1-methylcarbapen-2-em-3-carboxylate (0.105 g, 0.157 mmol) in a mixture of tetrahydrofuran (2 mL) and water (0.5 mL), was added I N aqueous trifluoromethanesulfonic acid (0.02 mL, 0.02 mmol). After 5 minutes, the mixture was partitioned between methylene chloride (20 mL) and 5% aqueous bicarbonate (20 mL). The water layer was extracted with methylene chloride (5 mL), and the combined methylene chloride layers were dried over magnesium sulfate, filtered and evaporated to give the title compound as an oil (0.096 g, quant).
¹H NMR (CDCl₃, 500 MHz) δ 1.31 (d, 1-CH₃), 1.45 (d, CH₃CHO(ALLOC)), 3.41 (t, ArCH₂CH₂OH), 3.41 (dq, H-1), 3.45 (dd, H-6), 4.04 (dt, ArCH₂CH₂O(TMS), 4.16 (dd, H-5), 4.59 and 4.89 ( 2m's, 2CH₂vinyl), 5.14 (dq, H-8), 5.26, 5.33, 5.35 and 5.53 (4m's, 4 vinyl H's), 4.69 and 5.41 (2d's, CH₂N), 5.91 and 6.05 (2m's, 2CH₂vinyl), 6.73 (d, ArH), 7.54 (dd, ArH), 7.63 (d, ArH), 7.68 (d, ArH) and 7.95 (d, ArH).

### Step 3: (1S,5R,6S)-2-(4-(2-(((carbamoylmethyl)-1,4-diazoniabicyclo[2.2.2]oct-1-yl))-ethyl)(1,8-naphthosultam)methyl)-6-[1(R)-hydroxyethyl]-1-methylcarbapen-2-em-3-carboxylate chloride

To a solution of allyl (1*S*,5*R*,6*S*)-2-(4-((2-hydroxy)-ethyl-1,8-naphthosultam)methyl)-6-[1(*R*)-(allyloxycarbonyl)oxyethyl]-1-methylcarbapen-2-em-3-carboxylate (0.25 g, 0.419 mmol) in dichloromethane (8 mL), cooled in a methanol/ ice bath (-15°C) under nitrogen, was added 2,6-lutidine (0.147 mL, 1.26 mmol) followed by trifluoromethanesulfonic anhydride (Tf₂O) (0.106 mL, 0.629 mmol). After 30 minutes the mixture was partitioned between methylene chloride (80 mL) and 0.05N aqueous hydrochloric acid (80 mL). The methylene chloride layer was washed with water (80 mL), dried over magnesium sulfate and filtered into a flask containing a solution of 1-carbamoylmethyl-4-aza-1-azoniabicyclo[2.2.2]octane trifluoromethanesulfonate (0.147 g, 0.461 mmol) in acetonitrile (4 mL). The mixture was concentrated under vacuum to give a viscous oil (ca. 1 mL). After 30 minutes, additional 1-carbamoylmethyl-4-aza-1-azoniabicyclo [2.2.2]octane trifluoromethanesulfonate (0.015 g, 0.047 mmol) was added and the mixture was diluted with dimethylformamide (5 mL). The solution was cooled in an ice bath and 0.5M sodium ethyl hexanoate in ethyl acetate (0.84 mL, 0.419 mmol) and ethyl hexanoic acid (0.067 mL, 0.419 mmol) were added. The solution was blanketed with nitrogen, using a Firestone valve, and triphenylphosphine (0.033 g, 0.126 mmol) and tetrakis(triphenylphosphine)palladium (0.146 g, 0.126 mmol) were added. After one hour, diethyl ether (50 mL) was added and the supernatant was decanted from the precipitate. The solid was washed with additional diethyl ether (50 mL) and was dried under vacuum. The solid was dissolved in 1:1 acetonitrile/ water (5 mL) and was loaded onto a Bio-Rad weak cation exchange resin (21 mL, 2.75 x 4 cm, macroprep cm ion exchange resin, sodium cycle). The column was washed with 1:1 acetonitrile/ water (20 mL) and water (100 mL). The column was then eluted with 5% aqueous sodium chloride, collecting 8 mL fractions. Fractions 4-36 were cooled in an ice bath and were then loaded onto an amberchrom CG-161 resin (30 mL, 8 x 2.5 cm). The column was washed with cold de-ionized water (200 mL) and was eluted with 20% isopropanol in water, collecting 8 mL fractions. Fractions 5-10 were combined and concentrated to approximately 15 mL, which was lyophilized to give the title compound (0.157 g).
¹H NMR (D₂O, 500 MHz) δ 1.08 (d, 1-CH₃), 1.18 (d, CH₃CHOH), 3.01 (dq, H-1), 3.36 (dd, H-6), 3.67 (m, ArCH₂CH₂Q), 3.93 (m, ArCH₂CH₂Q), 3.93 (dd, H-5), 4.10 (dq, H-8), 4.25 and 4.37 (2m, NCH₂CH₂N), 4.46 (s, CH₂CONH₂), 4.51 and 5.12 (2d's, CH₂N), 6.63 (d, ArH), 7.37 (t, ArH), 7.42 (d, ArH), 7.73 (d, ArH) and 8.00 (d, ArH).

### EXAMPLE 7

### SYNTHESIS OF (1S,5R,6S)-2-(4-(2-(((3-HYDROXYPROP-1-YL)-1,4-DIAZONIABICYCLO[2.2.2]OCT-1-YL))-ETHYL)(1,8-NAPHTHOSULTAM)METHYL)-6-[1(R)-HYDROXYETHYL]-1-METHYLCARBAPEN-2-EM-3-CARBOXYLATE CHLORIDE

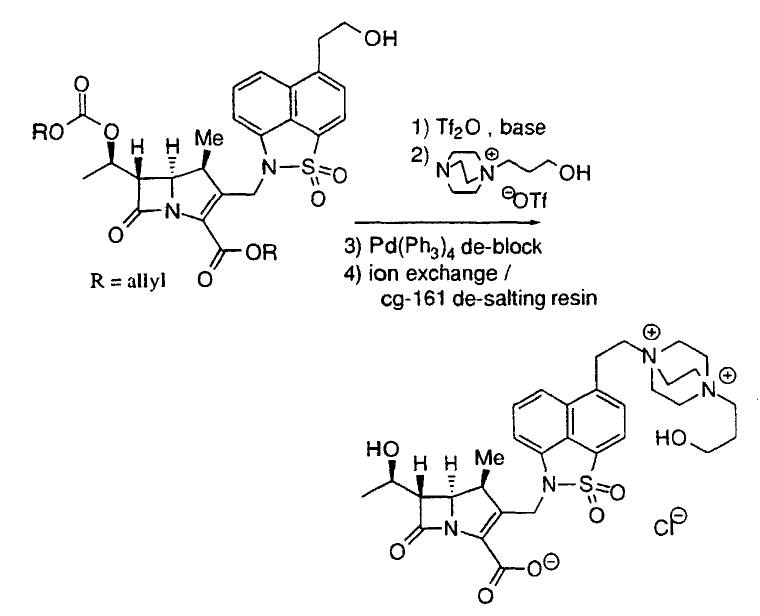

To a solution of allyl (1*S*,5*R*,6*S*)-2-(4-((2-hydroxy)-ethyl-1,8-naphthosultam)methyl)-6-[1 (*R*)-(allyloxycarbonyl)oxyethyl]-1-methylcarbapen-2-em-3-carboxylate (0.29 g, 0.486 mmol) in dichloromethane (9 mL), cooled in a methanol/ ice bath (-15°C) under nitrogen, was added 2,6-lutidine (0.17 mL, 1.46 mmol) followed by trifluoromethane sulfonic anhydride (0.123 mL, 0.729 mmol). After 30 minutes the mixture was partitioned between methylene chloride (100 mL) and 0.05N aqueous hydrochloric acid (100 mL). The methylene chloride layer was washed with water (100 mL), dried over magnesium sulfate and filtered into a flask containing a solution of 1-carbamoylmethyl-4-aza-1-azoniabicyclo[2.2.2]octane trifluoromethanesulfonate (0.187 g, 0.461 mmol) in acetonitrile (4 mL). The solvents were evaporated under vacuum to give a viscous oil (ca. 1 mL). After 30 minutes the mixture was diluted with dimethylformamide (5 mL) and was cooled in an ice bath. A solution of 0.5M Sodium ethyl hexanoate in ethyl acetate (0.84 mL, 0.419 mmol) and ethyl hexanoic acid (0.067 mL, 0.419 mmol) were added, the solution was blanketed with nitrogen, using a Firestone valve, and triphenylphosphine (0.033 g, 0.126 mmol) and tetrakis(triphenylphosphine)palladium (0.146 g, 0.126 mmol) were added. After one hour, diethyl ether (50 mL) was added and the supernatant was decanted from the precipitate. The solid was washed with additional diethyl ether (50 mL) and was dried under vacuum. The solid was dissolved in 1:1 acetonitrile/ water (5 mL) and was loaded onto a Bio-Rad weak cation exchange resin (21 mL, 2.75 x 4 cm, macroprep cm ion exchange resin, sodium cycle). The column was washed with 1:1 acetonitrile/ water (22 mL) and then water (100 mL). The column was then eluted with 5% aqueous sodium chloride, collecting 8 mL fractions. Fractions 5-30 were cooled in an ice bath and were then loaded onto an amberchrom 161 resin (30 mL, 8 x 2.5 cm). The column was washed with cold de-ionized water (200 mL) and was eluted with 20% isopropanol in water, collecting 8 mL fractions. Fractions 5-9 were combined and concentrated to approximately 15 mLwhich was lyophilized to give the title compound (0.18 g).
¹H NMR (D₂O, 500 MHz) δ 0.97 (d, 1-CH₃), 1.14 (d, CH₃CHOH), 2.05 (m, ArCH₂CH₂CH₂OH), 2.89 (dq, H-1), 3.29 (dd, H-6), 3.61 (m, ArCH₂CH₂Q), 3.74 (2m, ArCH₂CH₂CH₂OH), 3.83 (dd, H-5), 3.91 (m, ArCH₂CH₂Q), 4.05 (dq, H-8), 4.13 and 4.24 (2m, NCH₂CH₂N), 4.37 and 5.03 (2d's, CH₂N), 6.47 (d, ArH), 7.19 (t, ArH), 7.29 (d, ArH), 7.67 (d, ArH) and 7.91 (d, ArH).

### EXAMPLE 8

### SYNTHESIS OF (1S,5R,6S)-2-(4-(2-((1-METHYLIMIDAZOL-3-IUM))-ETHYL)(1,8-NAPHTHOSULTAM)METHYL)-6-[1(R)-HYDROXYETHYL]-1-METHYLCARBAPEN-2-EM-3-CARBOXYLATE

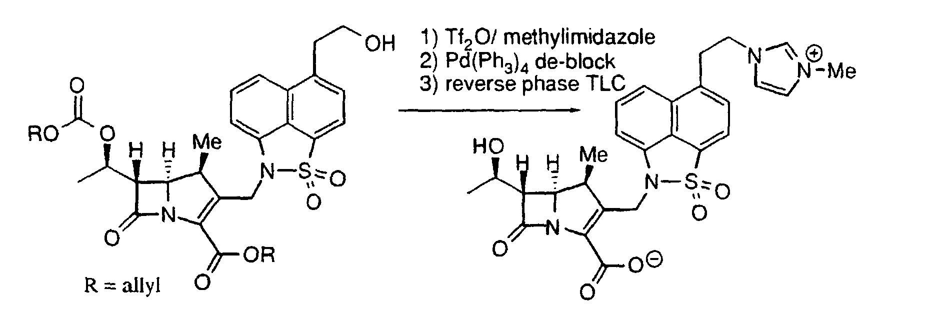

To a solution of allyl (1*S*,5*R*,6*S*)-2-(4-(2-(hydroxy)-ethyl-1,8-naphthosultam)methyl)-6-[1(*R*)-(allyloxycarbonyl)oxyethyl]-1-methylcarbapen-2-em-3-carboxylate (0.03 g, 0.05 mmol) in dichloromethane (0.5 mL), cooled in a dry ice/ acetone bath under nitrogen, was added 1-methylimidazole (0.016 mL, 0.20 mmol) followed by trifluoromethane-sulfonic anhydride (0.017 mL, 0.10 mmol). After 5 minutes the flask was removed from the bath and stirred at room temperature for 50 minutes. Aditional 1-methylimidazole (0.016 mL, 0.20 mmol) was added and after 20 minutes the mixture was partitioned between methylene chloride (20 mL) and water (20 mL). The methylene chloride layer was washed with water (100 mL), dried over magnesium sulfate, filtered and evaporated to an oil (0.0436 g). The oil was dissolved in dimethylformamide (0.7 mL) and cooled in an ice bath. A solution of 0.5M sodium ethyl hexanoate in ethyl acetate (0.11 mL, 0.055 mmol) and ethyl hexanoic acid (0.018 mL, 0.11 mmol) were added, the solution was blanketed with nitrogen, using a Firestone valve, and triphenylphosphine (0.004 g, 0.0165 mmol) and tetrakis(triphenylphosphine)palladium (0.019 g, 0.0165 mmol) were added. After 70 minutes, diethyl ether (6 mL) was added and the supernatant was decanted from the precipitate. The solid was washed with additional diethyl ether (6 mL) and was dried under vacuum. The solid was purified on a 1000 micron reverse phase plate developed with 30% acetonitrile/ water and eluted with 80% acetonitrile/ water (15 mL). The eluent was diluted with water (15 mL), washed with hexanes (40 mL), evaporated to approximately 2 mL and lyophilized to give the title compound (0.017 g).
¹H NMR (D₂O, 500 MHz) δ 1.14 (d, 1-CH₃), 1.22 (d, CH₃CHOH), 3.08 (dq, H-1), 3.40 (dd, H-6), 3.55 (m, ArCH₂CH₂Q), 3.60 (s, ImMe), 4.04 (dd, H-5), 4.18 (dq, H-8), 4.52 (m, ArCH₂CH₂Q), 4.57 and 5.19 (2d's, CH₂N), 6.75 (d, ArH), 7.20 and 7.22 (2m, ImH), 7.24 (d, ArH), 7.39 (t, ArH), 7.47 (d, ArH), 7.93 (d, ArH) and 8.20 (s, ImH).

### EXAMPLES 9-23

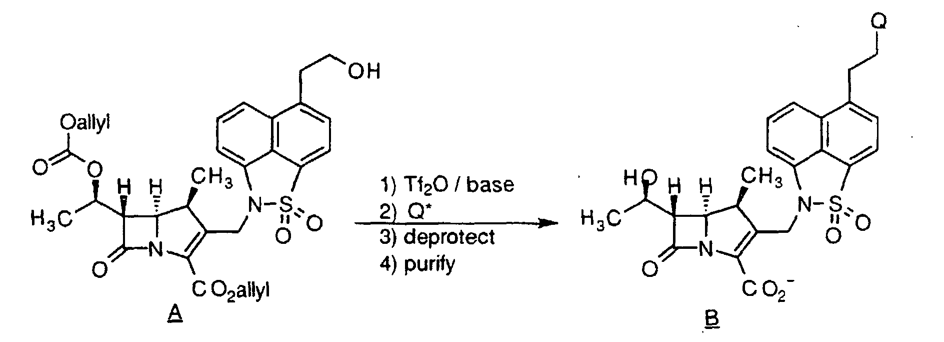

By appropriately modifying the procedure of Example 7, allyl (1*S*,5*R*,6*S*)-2-[N-(4-(2-hydroxy)-ethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

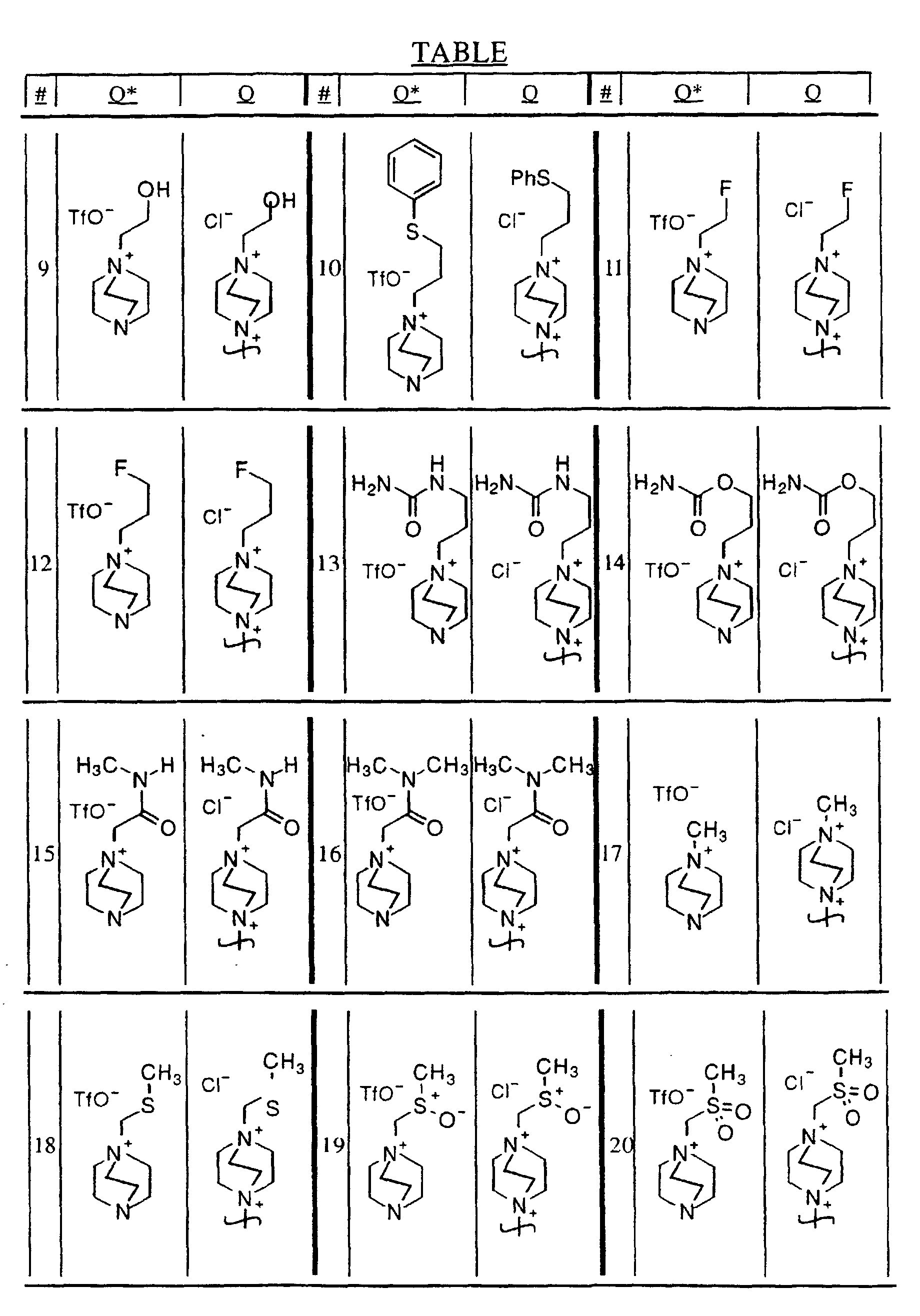

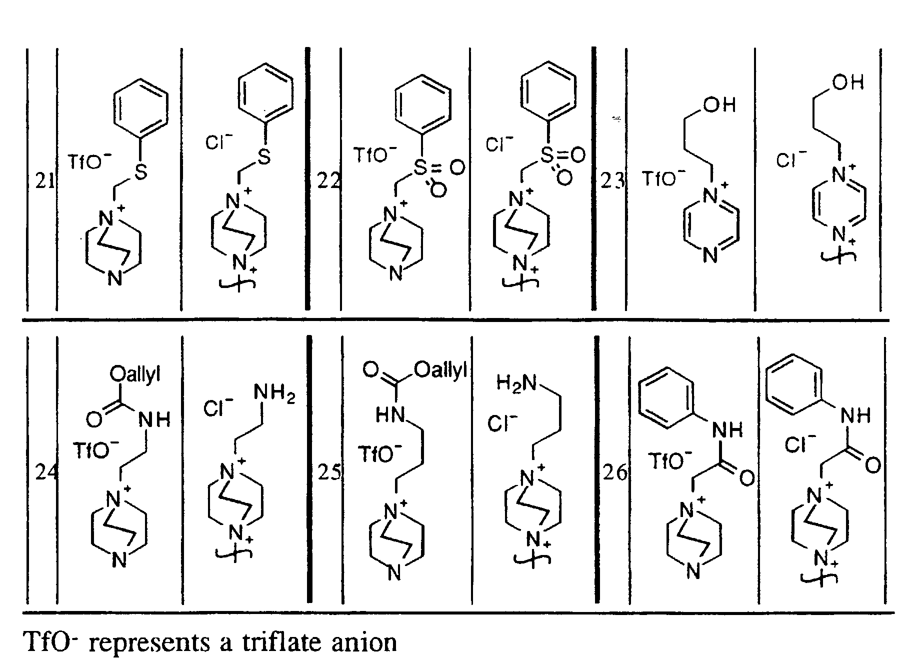

### EXAMPLES 27-32

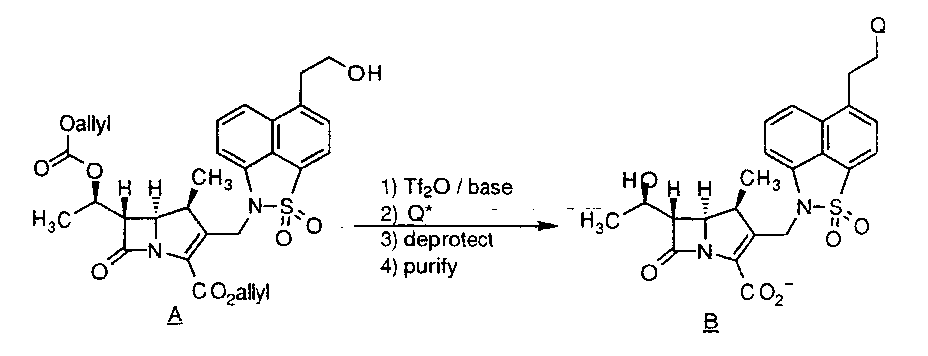

By appropriately modifying the procedure of Example 8, allyl (1*S*,5*R*,6*S*)-2-[N-(4-(2-hydroxy)-ethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

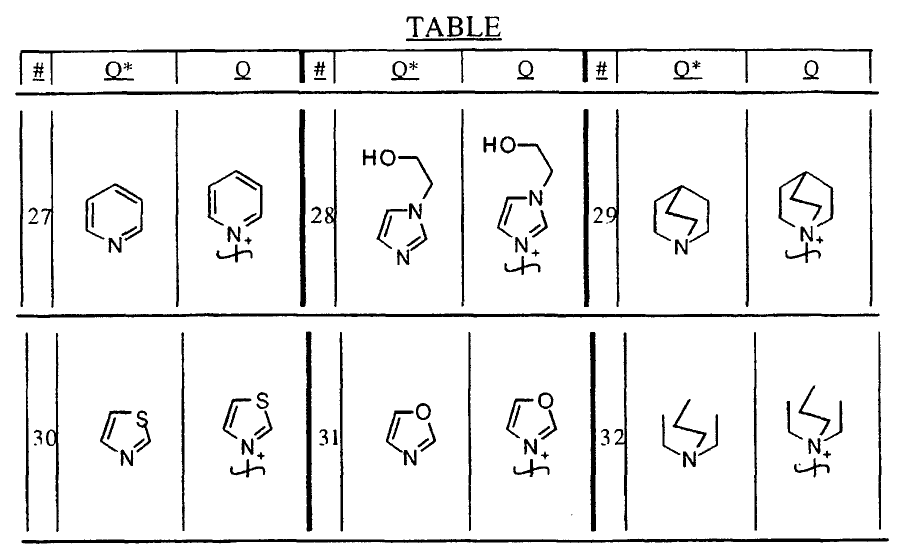

### EXAMPLE 33

### SYNTHESIS OF ALLYL (1S,5R,6S)-2-(4-(3-(HYDROXY)-PROPYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

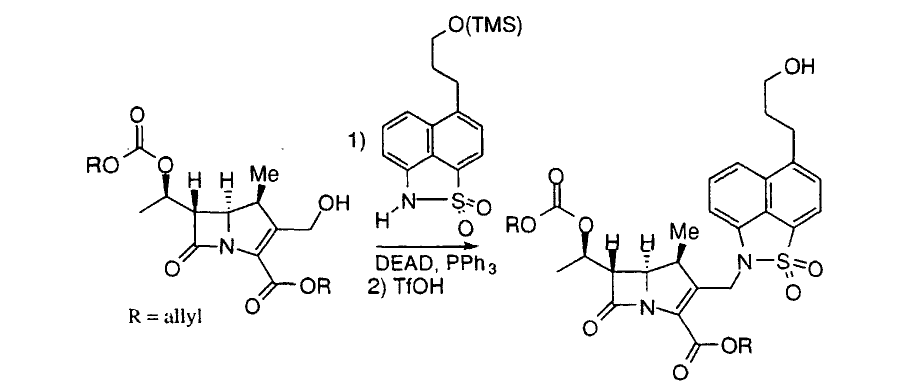

### Steps 1 and 2: allyl (1S,5R,6S)-2-(4-(3-(hydroxy)-propyl-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 6, allyl (1*S*,5*R*,6*S*)-2-(hydroxymethyl)-6-[1(*R*)-(allyloxycarbonyl)oxyethyl)-1-methylcarbapen-2-em-3-carboxylate is reacted with 4-(3-trimethylsilyloxyprop-1-yl)-1,8-naphthosultam to afford allyl (1*S*,5*R*,6*S*)-2-[N-(4-(3-hydroxy)-propyl)-1,8-naphthosultam)-methyl]-6-[(1*R*)-(allyloxycarbonyloxy)ethyl]-1-methylcarbapen-2-em-3-carboxylate.

### EXAMPLES 34-36

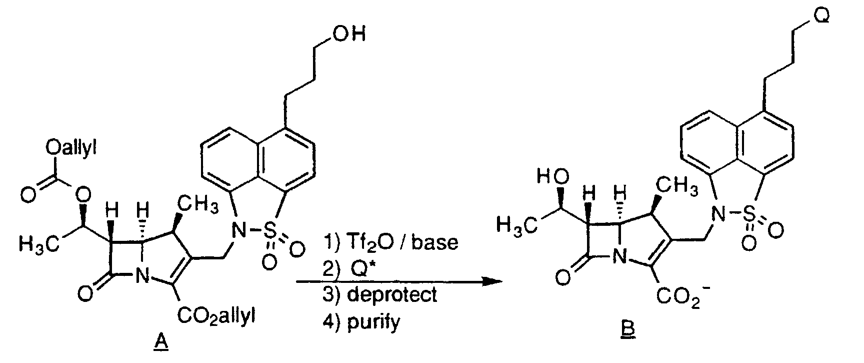

By appropriately modifying the procedures of Example 7 and 8, allyl (1*S*,5*R*,6*S*)-2-[N-(4-(3-hydroxy)-propyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

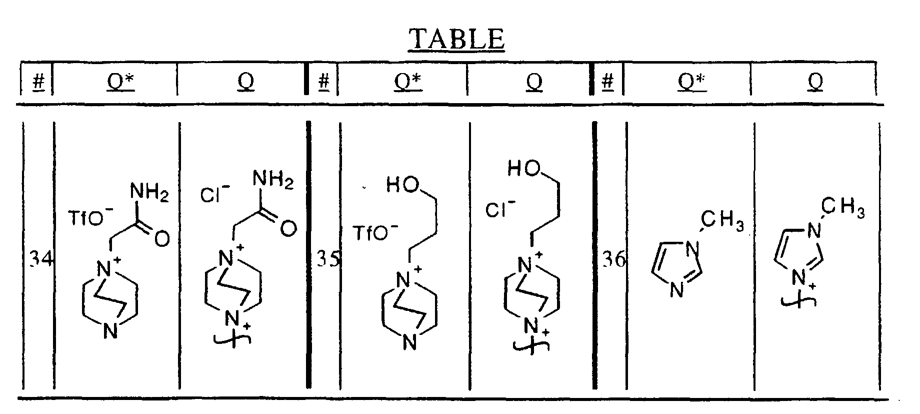

### EXAMPLE 37

### SYNTHESIS OF ALLYL (1S,5R,6S)-2-(3-(HYDROXYMETHYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

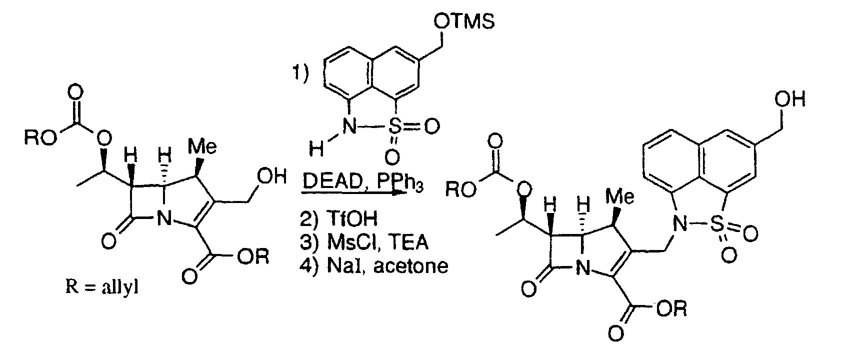

### Steps 1 and 2: allyl (1S,5R,6S)-2-(3-(hydroxymethyl-1,8-naphthosultam)methyl)-6-[1(R)-(allyloxycarbonyl)oxyethyl]-1-methylcarbapen-2-em-3-carboxylate

By appropriately modifying the procedures of Steps I and 2 of Example 1, allyl (1*S*,5*R*,6*S*)-2-(hydroxymethyl)-6-[1(*R*)-(allyloxycarbonyl) oxyethyl]-1-methylcarbapen-2-em-3-carboxylate is reacted with 3-(trimethylsilyloxymethyl)-1,8-naphthosultam to afford allyl (1*S*,5*R*,6*S*)-2-[N-(3-(hydroxymethyl)-1,8-naphthosultam)-methyl]-6-[(1*R*)-(allyloxycarbonyloxy)ethyl]-1-methylcarbapen-2-em-3-carboxylate.

### EXAMPLES 38-40

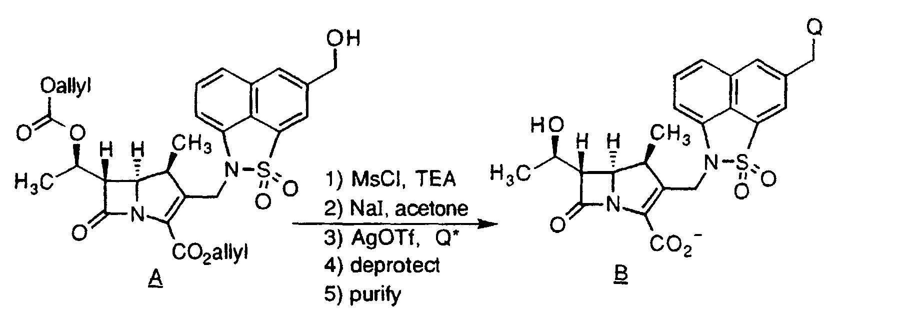

By appropriately modifying the procedures of Steps 3 and 4 of Example 1, allyl (1*S*,5*R*,6*S*)-2-[N-(3-(hydroxymethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

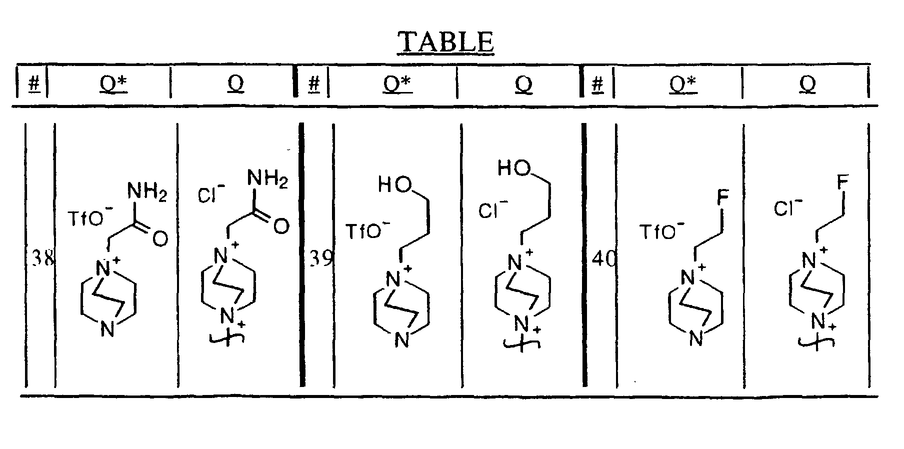

### EXAMPLES 41-43

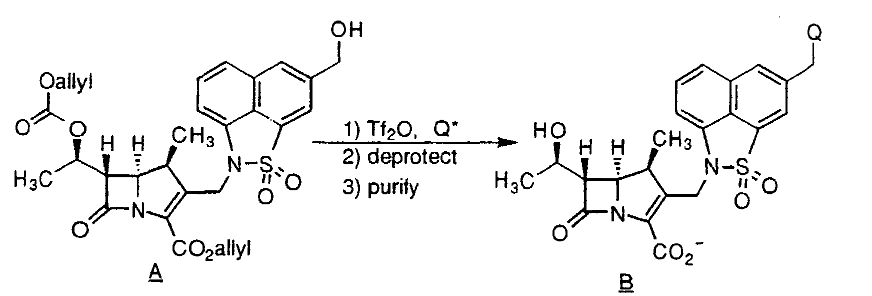

By appropriately modifying the procedure of Example 3, allyl (1*S*,5*R*,6*S*)-2-[N-(3-(hydroxymethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

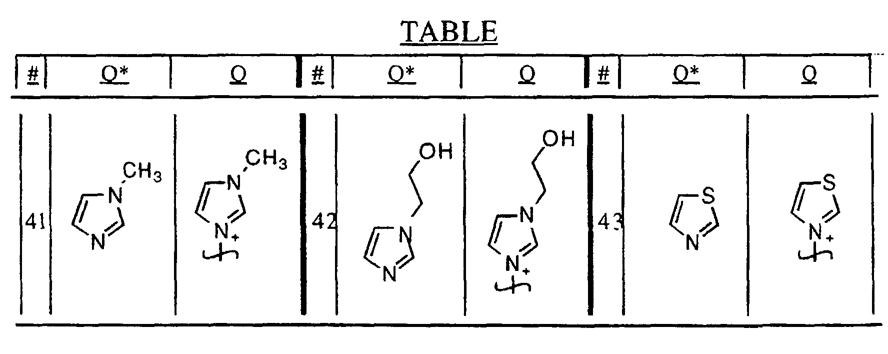

### EXAMPLE 44

### SYNTHESIS OF ALLYL (1S,5R,6S)-2-(3-(2-(HYDROXY)-ETHYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

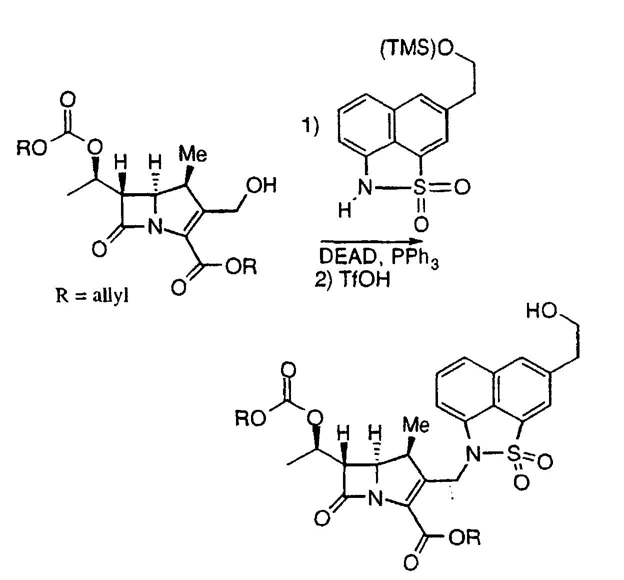

### Steps 1 and 2: allyl (1S,5R,6S)-2-(3-(2-(hydroxy)-ethyl-1,8-naphthosultam)methyl)-6-[1(R)-(allyloxycarbonyl)oxyethyl]-1-methylcarbapen-2-em-3-carboxylate

By appropriately modifying the procedures of Steps I and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 3-(2-trimethylsilyloxyeth-1-yl)-1,8-naphthosultam to afford allyl (1S,5R,6S)-2-[N-(3-(2-hydroxy)-ethyl)-1,8-naphthosultam)-methyl]-6-[(1R)-(allyloxycarbonyloxy)ethyl]-1-methylcarbapen-2-em-3-carboxylate.

### EXAMPLES 45-47

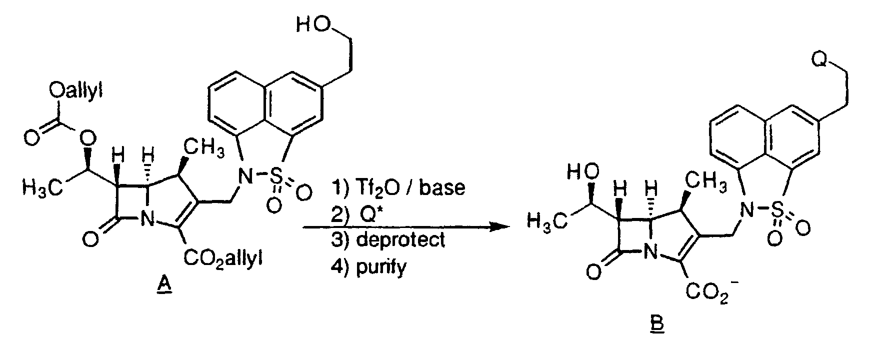

By appropriately modifying the procedures of Example 7 and 8, allyl (1*S*,5*R*,6*S*)-2-[N-(3-(2-hydroxy)-ethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

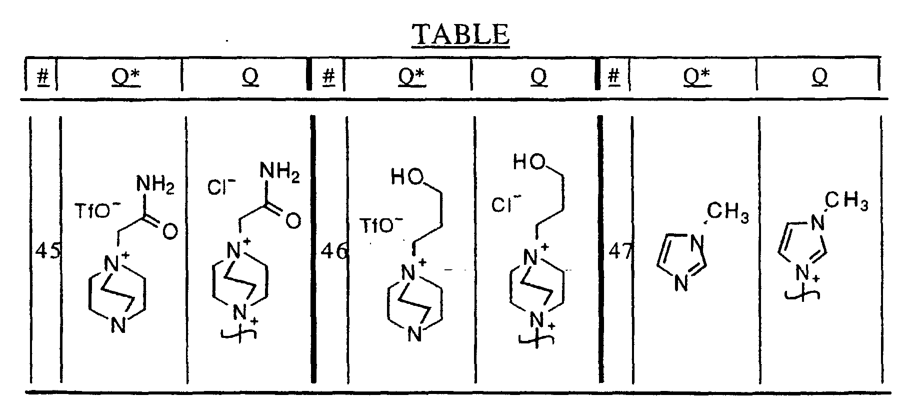

### EXAMPLE 48

### SYNTHESIS OF ALLYL (1S,5R,6S)-2-(2-(HYDROXYMETHYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

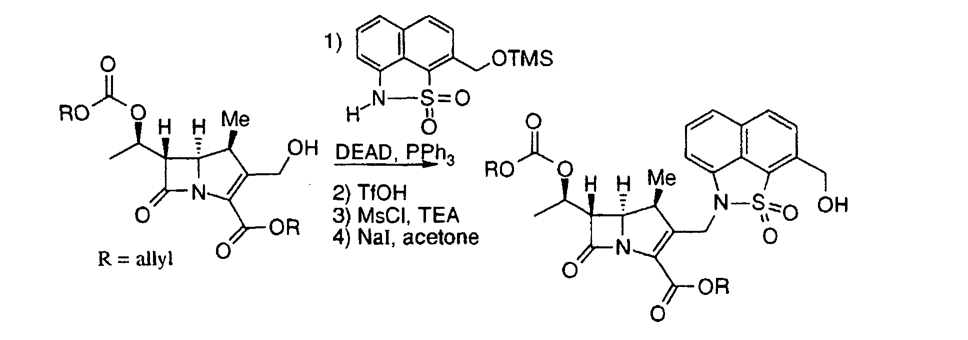

### Steps 1 and 2: allyl (1S,5R,6S)-2-(2-(hydroxymethyl-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 1, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 2-(trimethylsilyloxymethyl)-1,8-naphthosultam to afford allyl (1*S*,5*R*,6*S*)-2-[N-(2-(hydroxymethyl)-1,8-naphthosultam)-methyl]-6-[(1*R*)-(allyloxycarbonyloxy)ethyl]-1-methylcarbapen-2-em-3-carboxylate.

### EXAMPLES 49-51

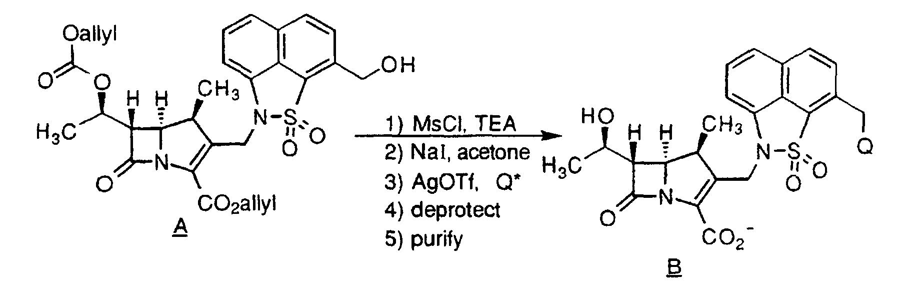

By appropriately modifying the procedures of Steps 3 and 4 of Example 1, allyl (1*S*,5*R*,6*S*)-2-[N-(2-(hydroxymethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

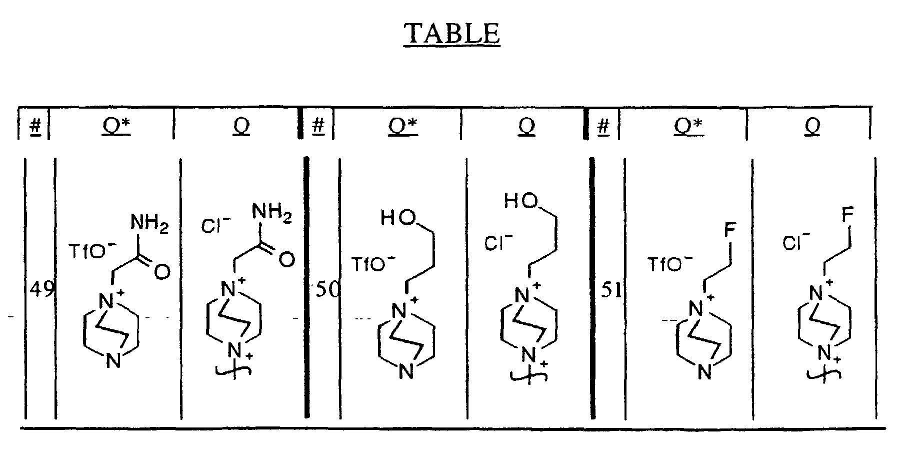

### EXAMPLES 52-54

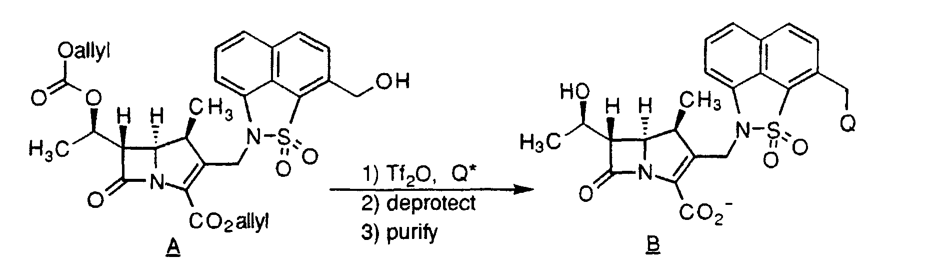

By appropriately modifying the procedure of Example 3, allyl (1*S*,5*R*,6*S*)-2-[N-(2-(hydroxymethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

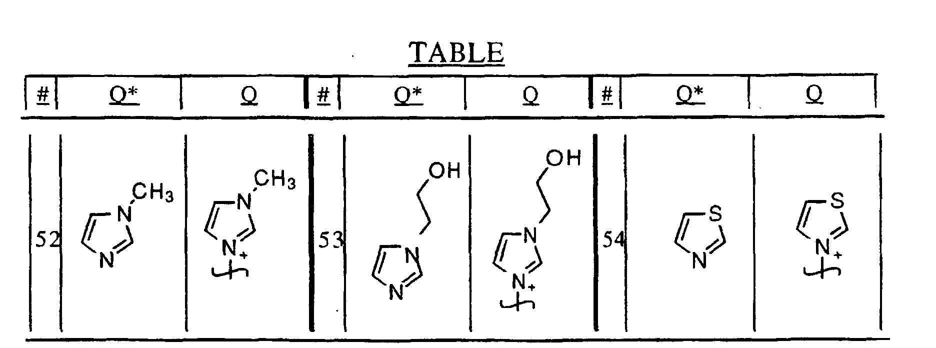

### EXAMPLE 55

### SYNTHESIS OF ALLYL (1S,5R,6S)-2-(2-(2-(HYDROXY)-ETHYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

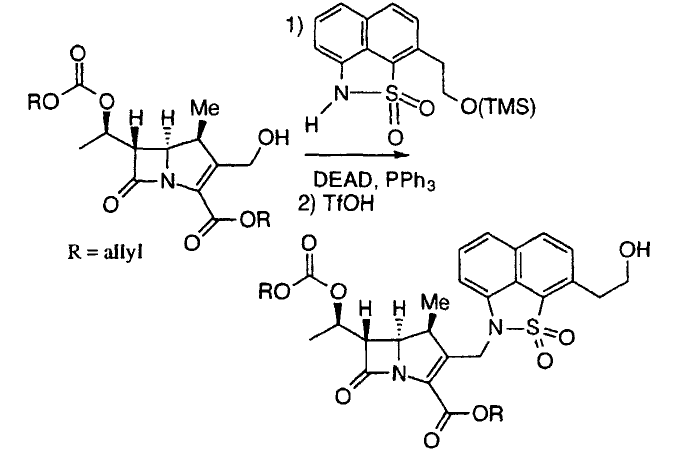

### Steps 1 and 2: allyl (1S,5R,6S)-2-(2-(2-(hydroxy)-ethyl-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 2-(2-trimethylsilyloxyeth-1-yl)-1,8-naphthosultam to afford allyl (1*S*,5*R*,6*S*)-2-[N-(2-(2-hydroxy)-ethyl)-1,8-naphthosultam)-methyl]-6-[(1*R*)-(allyloxycarbonyloxy)ethyl]-1-methylcarbapen-2-em-3-carboxylate.

### EXAMPLES 56-58

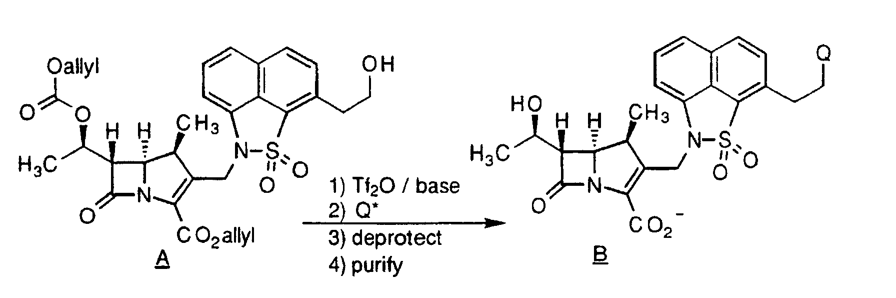

By appropriately modifying the procedures of Example 7 and 8, allyl (1*S*,5*R*,6*S*)-2-[N-(2-(2-hydroxy)-ethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

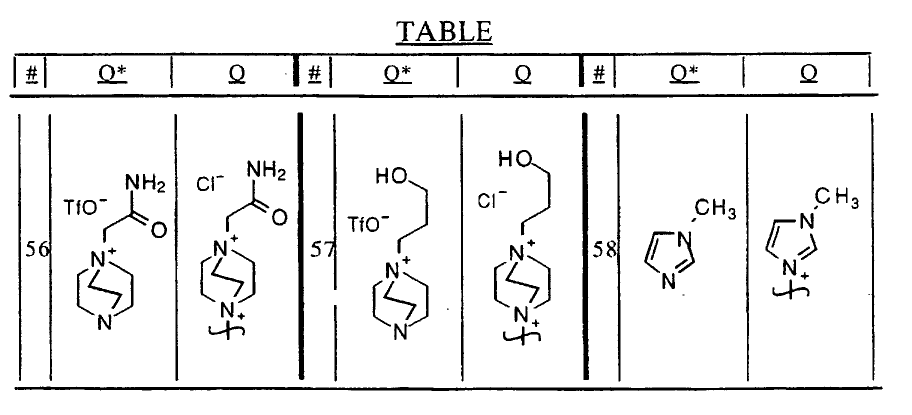

### EXAMPLE 59

### SYNTHESIS OF ALLYL (1S,5R,6S)-2-(6-(HYDROXYMETHYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

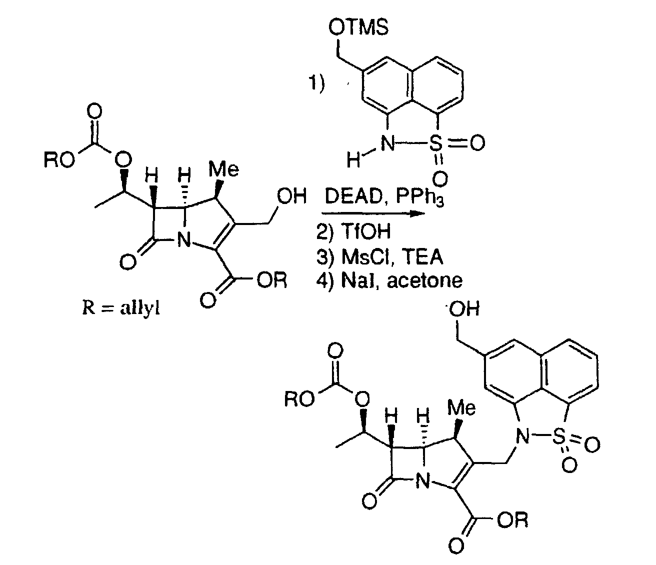

### Steps 1 and 2: allyl (1S,5R,6S)-2-(6-(hydroxymethyl-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 1, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 6-(trimethylsilyloxymethyl)-1,8-naphthosultam to afford allyl (1*S*,5*R*,6*S*)-2-[N-(6-(hydroxymethyl)-1,8-naphthosultam)-methyl]-6-[((1*R*)-(allyloxycarbonyloxy)ethyl]-1-methylcarbapen-2-em-3-carboxylate.

### EXAMPLES 60-62

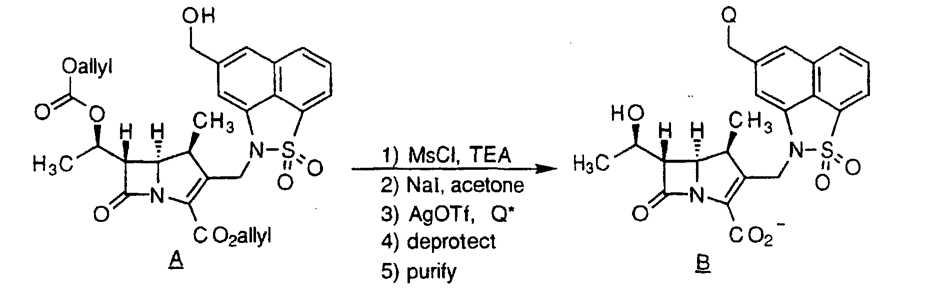

By appropriately modifying the procedures of Steps 3 and 4 of Example 1, allyl (1*S*,5*R*,6*S*)-2-[N-(6-(hydroxymethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

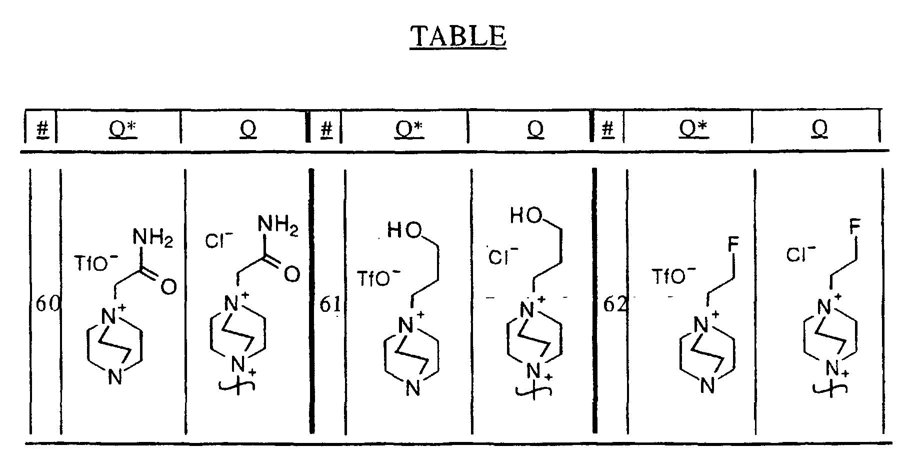

### EXAMPLES 63-65

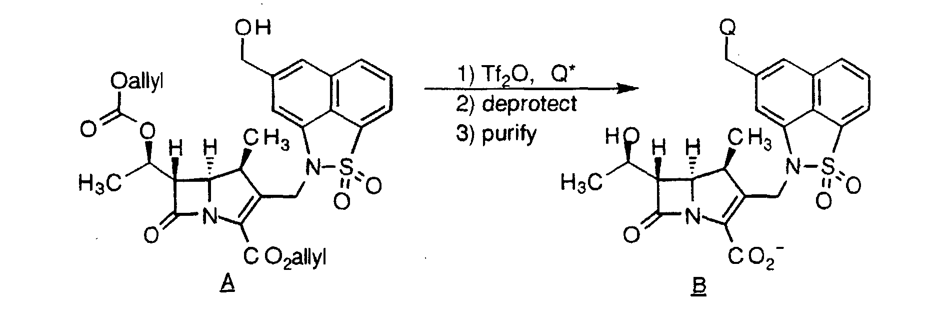

By appropriately modifying the procedure of Example 3, allyl (1*S*,5*R*,6*S*)-2-[N-(6-(hydroxymethyl)-1,8-naphthosultam)-methyl]-6-[( 1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

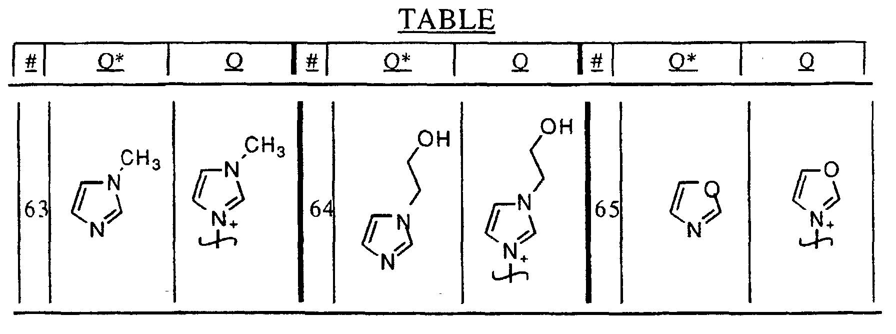

### EXAMPLE 66

### SYNTHESIS OF ALLYL (1S,5R,6S)-2-(6-(2-(HYDROXY)-ETHYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

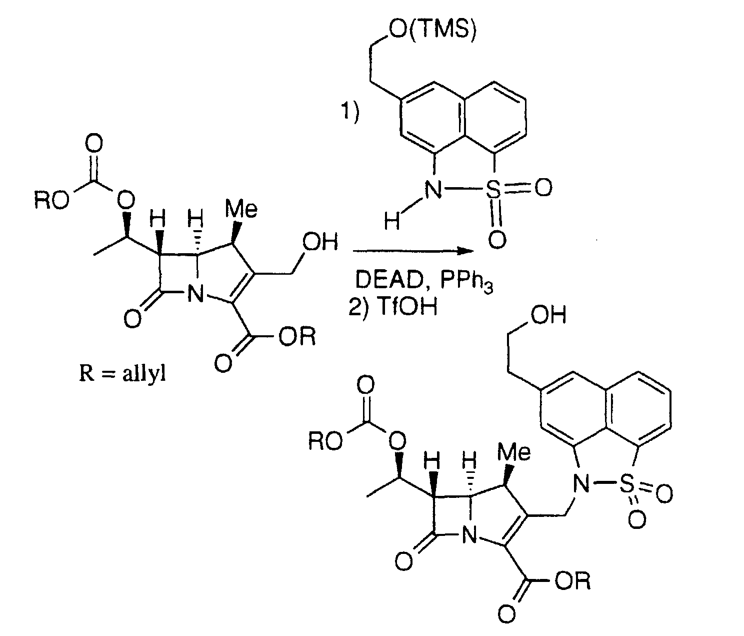

### Steps 1 and 2: allyl (1S,5R,6S)-2-(6-(2-(hydroxy)-ethyl-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 6-(2-trimethylsilyloxyeth-1-yl)-1,8-naphthosultam to afford allyl (1*S*,5*R*,6*S*)-2-[N-(6-(2-hydroxy)-ethyl)-1,8-naphthosultam)-methyl]-6-[(1*R*)-(allyloxycarbonyloxy)ethyl]-1-methylcarbapen-2-em-3-carboxylate.

### EXAMPLES 67-69

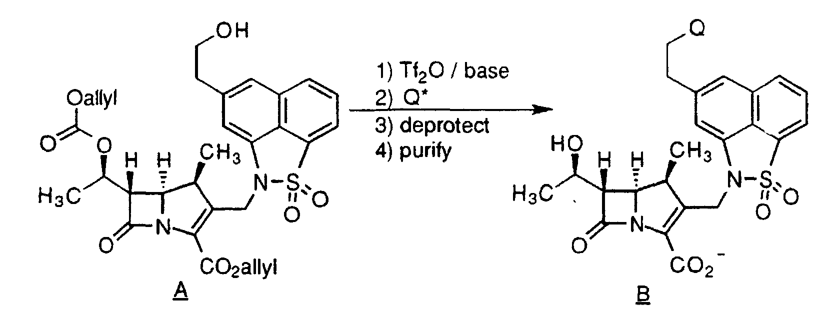

By appropriately modifying the procedures of Example 7 and 8, allyl (1*S*,5*R*,6*S*)-2-[N-(6-(2-hydroxy)-ethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

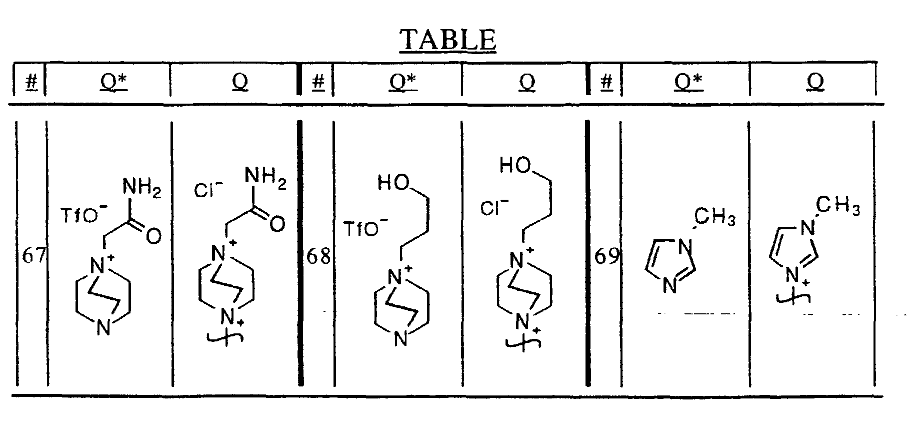

### EXAMPLE 70

### SYNTHESIS OF ALLYL (1S,5R,6S)-2-(7-(HYDROXYMETHYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

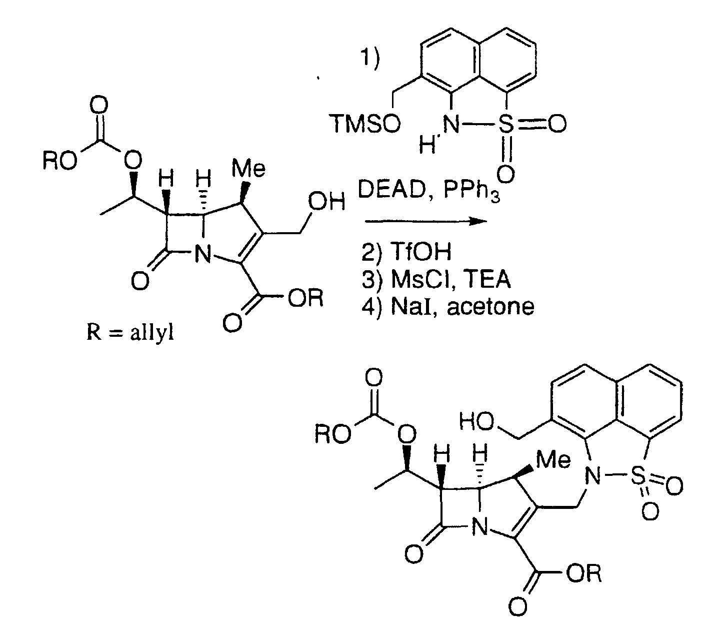

### Steps 1 and 2: allyl (1S,5R,6S)-2-(7-(hydroxymethyl-1,8-naphthosultam)methyl)-6-[1(R)-(allyloxycarbonyl)oxyethyl]-1-methylcarbapen-2-em-3-carboxylate

By appropriately modifying the procedures of Steps I and 2 of Example 1, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 7-(trimethylsilyloxymethyl)-1,8-naphthosultam to afford allyl (1*S*,5*R*,6*S*)-2-[N-(7-(hydroxymethyl)-1,8-naphthosultam)-methyl]-6-[(1*R*)-(allyloxycarbonyloxy)ethyl]-1-methylcarbapen-2-em-3-carboxylate.

### EXAMPLES 71-73

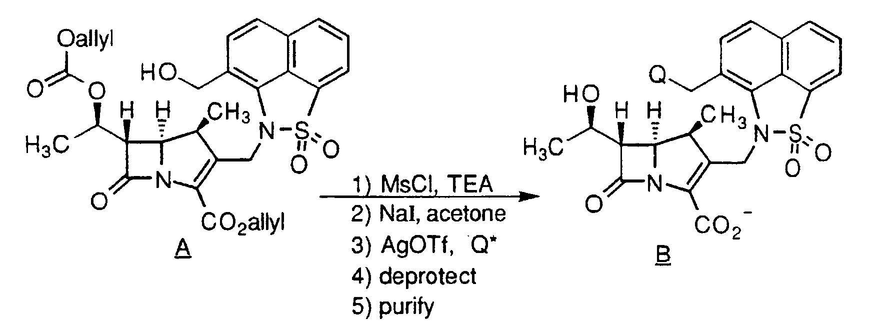

By appropriately modifying the procedures of Steps 3 and 4 of Example 1, allyl (1*S*,5*R*,6*S*)-2-[N-(7-(hydroxymethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

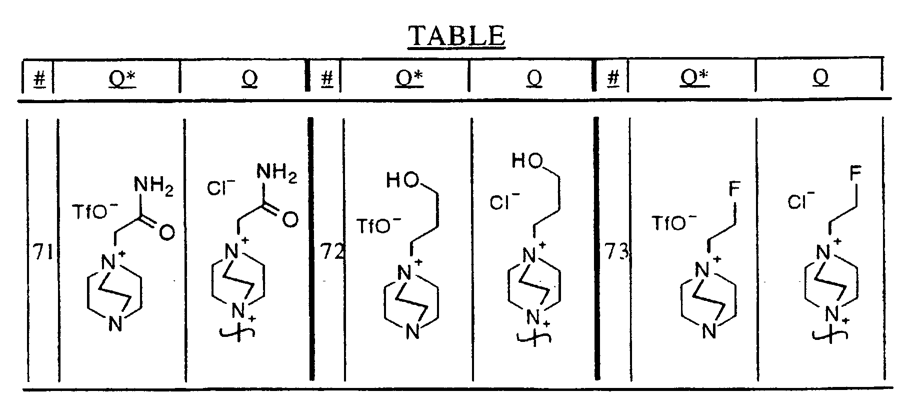

### EXAMPLES 74-76

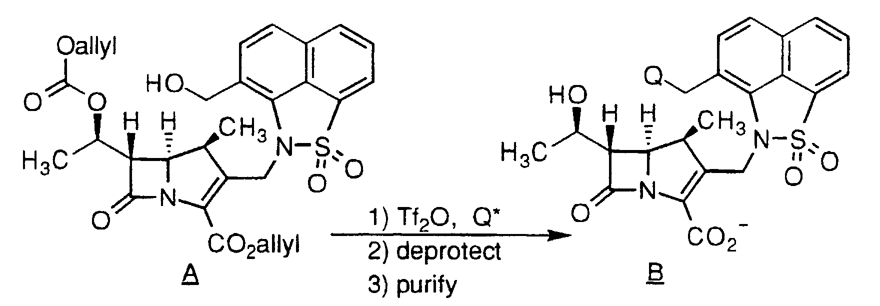

By appropriately modifying the procedure of Example 3, allyl (1*S*,5*R*,6*S*)-2-[N-(7-(hydroxymethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

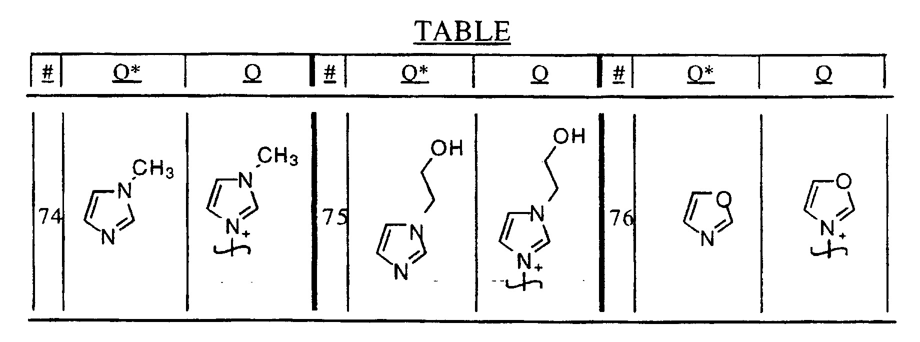

### EXAMPLE 77

### SYNTHESIS OF ALLYL (1S,5R,6S)-2-(7-(2-(HYDROXY)-ETHYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

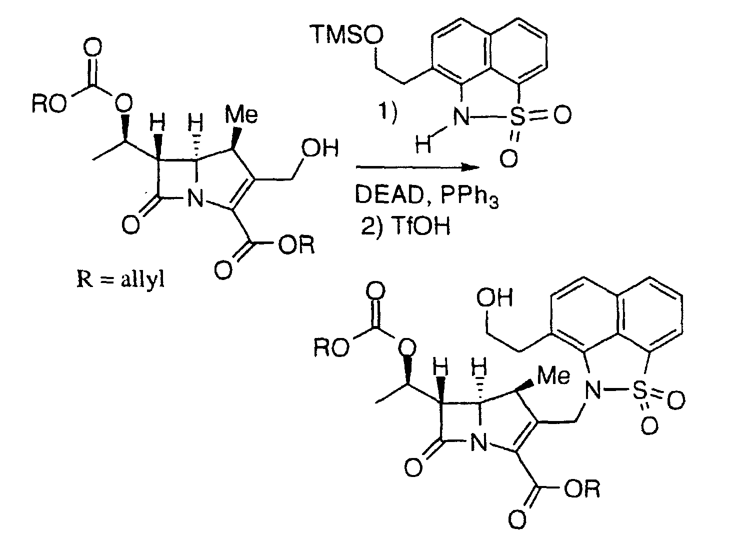

### Steps 1 and 2: allyl (1S,5R,6S)-2-(7-(2-(hydroxy)-ethyl-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 7-(2-trimethylsilyloxyeth-1-yl)-1,8-naphthosultam to afford allyl (1*S*,5*R*,6*S*)-2-[N-(7-(2-hydroxy)-ethyl)-1,8-naphthosultam)-methyl]-6-[(1R)-(allyloxycarbonyloxy)ethyl]-1-methylcarbapen-2-em-3-carboxylate.

### EXAMPLES 78-80

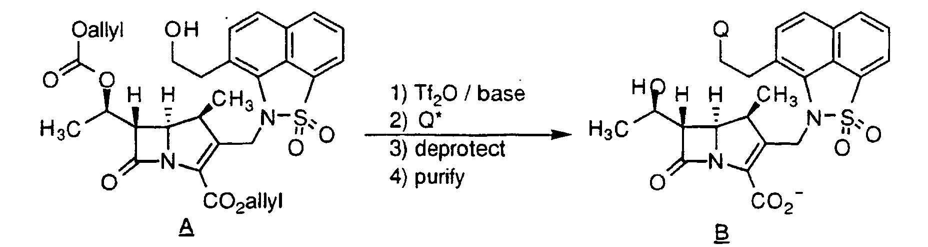

By appropriately modifying the procedures of Example 7 and 8, allyl (1*S*,5*R*,6*S*)-2-[N-(7-(2-hydroxy)-ethyl)-1,8-naphthosultam)-methyl]-6-[(1*R*)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

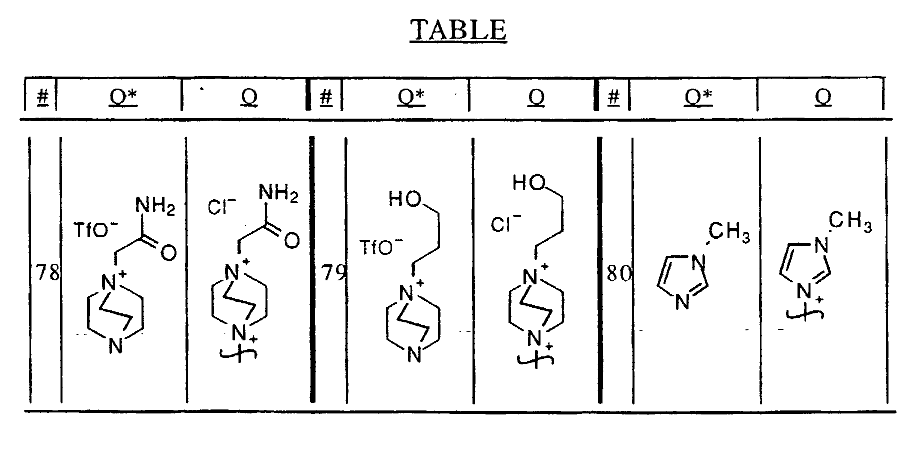

### EXAMPLE 81

### SYNTHESIS OF ALLYL (1S,5R,6S)-2-(4-(2-(HYDROXY)-ETHYL-5-NITRO-1,8-NAPHTHOSULTAM)METHYL)-6-[1(R)-(ALLYLOXYCARBONYL)OXYETHYL]-1-METHYLCARBAPEN-2-EM-3-CARBOXYLATE

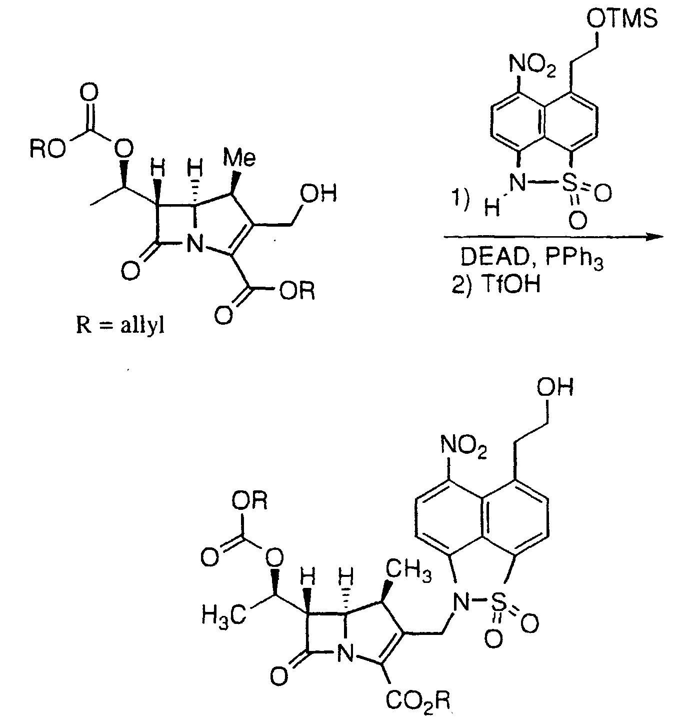

### Steps 1 and 2: allyl (1S,5R,6S)-2-(4-(2-(hydroxy)-ethyl-5-nitro-1,8-naphthosultam)methyl)-6-[1(R)-(allyloxycarbonyl)oxyethyl]-1-methylcarbapen-2-em-3-carboxylate

By appropriately modifying the procedures of Steps I and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 4-(2-trimethylsilyloxyeth-1-yl)-5-nitro-1,8-naphthosultam to afford allyl (1S,5R,6S)-2-[N-(4-(2-hydroxy)-ethyl)-5-nitro-1,8-naphthosultam)-methyl]-6-[(1R)-(allyloxycarbonyloxy)ethyl]-1-methylcarbapen-2-em-3-carboxylate.

### EXAMPLES 82-84

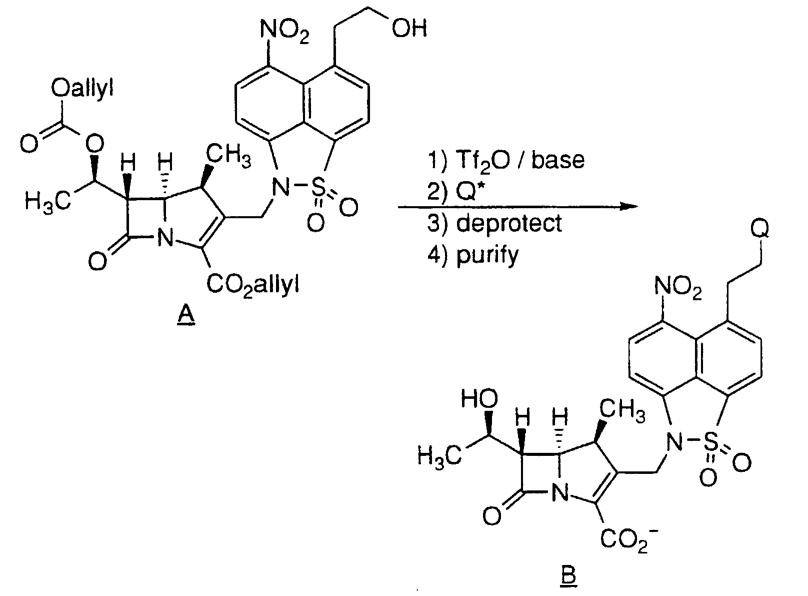

By appropriately modifying the procedures of Example 7 and 8, allyl (1S,5R,6S)-2-[N-(4-(2-hydroxy)-ethyl)-5-nitro-1,8-naphthosultam)-methyl]-6-[(1R)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

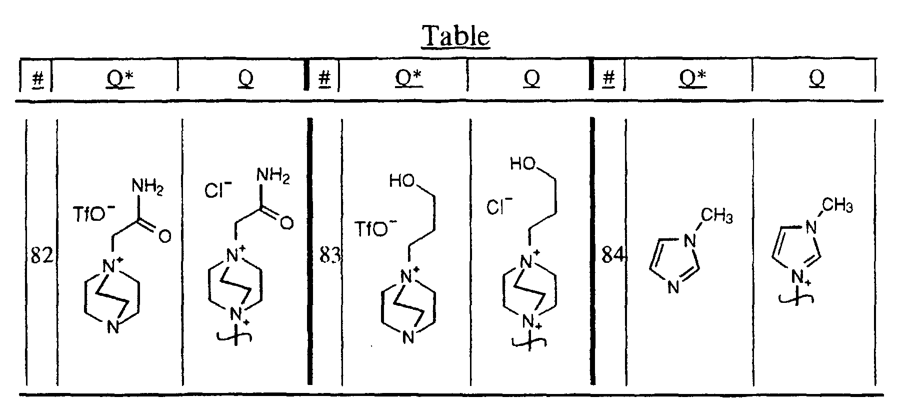

### EXAMPLE 85

### SYNTHESIS OF ALLYL (1S,5R,6S)-2-(4-(2-(HYDROXY)-ETHYL-5-METHOXYACETYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

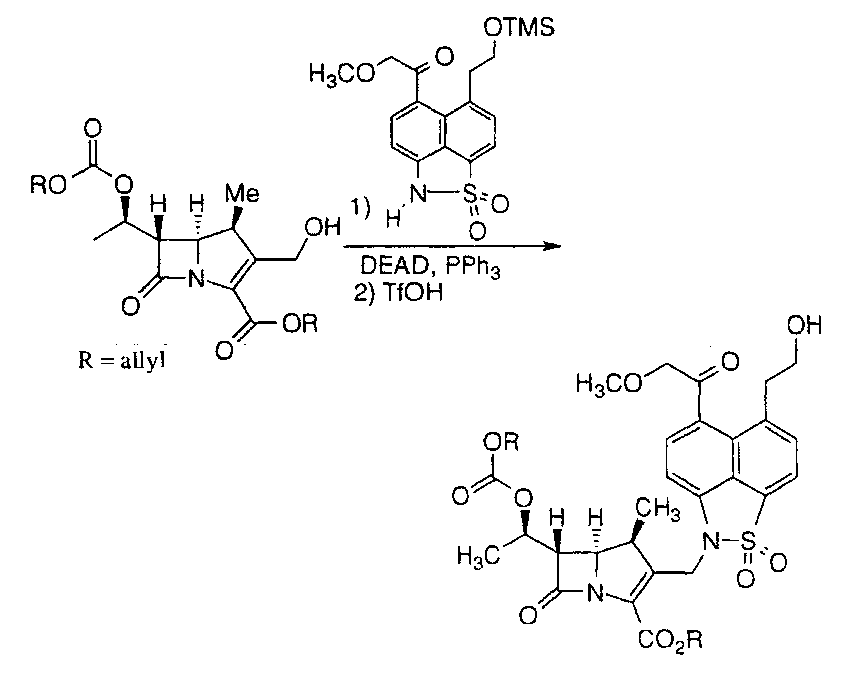

### Steps 1 and 2: allyl (1S,5R,6S)-2-(4-(2-(hydroxy)-ethyl-5-methoxyacetyl-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 4-(2-trimethylsilyloxyeth-1-yl)-5-methoxyacetyl-1,8-naphthosultam to afford allyl (1S,5R,6S)-2-[N-(4-(2-hydroxy)-ethyl)-5-methoxyacetyl-1,8-naphthosultam)-methyl]-6-[(1R)-(allyloxycarbonyloxy)ethyl]-1-methylcarbapen-2-em-3-carboxylate.

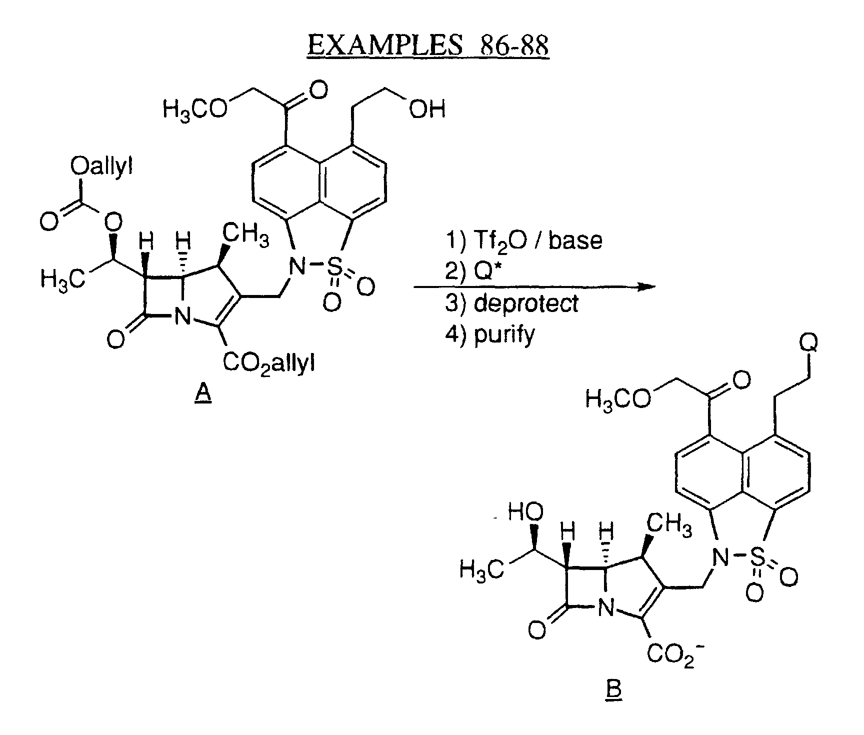

By appropriately modifying the procedures of Example 7 and 8, allyl (1S,5R,6S)-2-[N-(4-(2-hydroxy)-ethyl)-5-methoxyacetyl-1,8-naphthosultam)-methyl]-6-[(1R)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

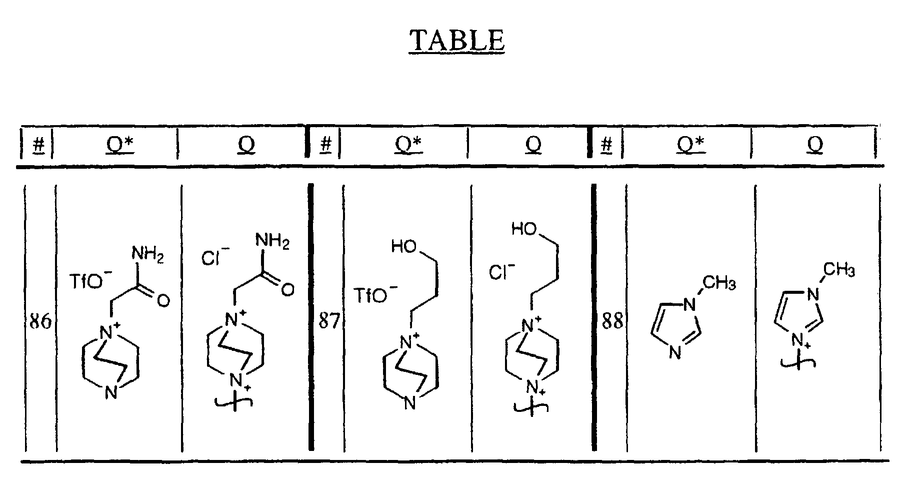

### EXAMPLE 89

### SYNTHESIS OF ALLYL (1S,5R,6S)-2-(4-(2-(HYDROXY)-ETHYL-5-PHENYL-1,8-NAPHTHOSULTAM)METHYL)-6-[1(R)-(ALLYLOXYCARBONYL)OXYETHYL]-1-METHYLCARBAPEN-2-EM-3-CARBOXYLATE

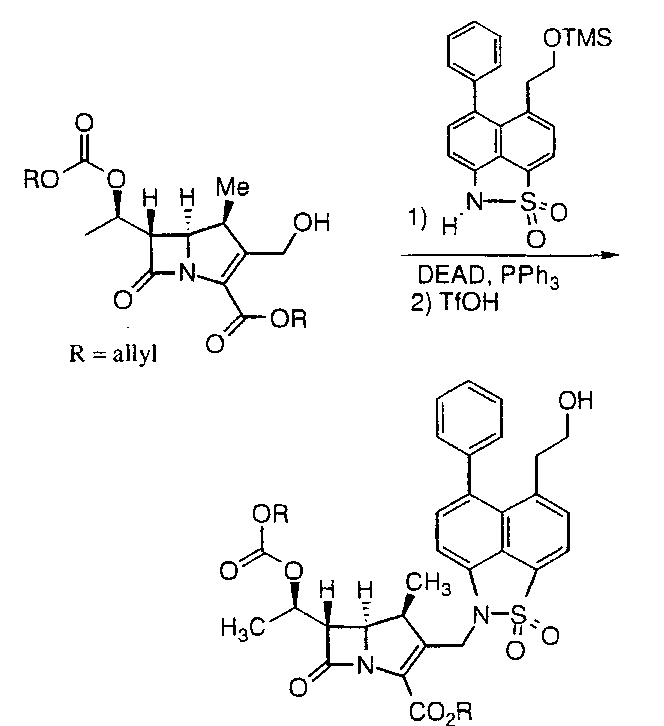

### Steps 1 and 2: allyl (1S,5R,6S)-2-(4-(2-(hydroxy)-ethyl-5-phenyl-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 4-(2-trimethylsilyloxyeth-1-yl)-5-phenyl-1,8-naphthosultam to afford allyl (1S,5R,6S)-2-[N-(4-(2-hydroxy)-ethyl)-5-phenyl-1,8-naphthosultam)-methyl]-6-[(1R)-(allyloxycarbonyloxy)ethyl]-1-methylcarbapen-2-em-3-carboxylate.

### EXAMPLES 90-92

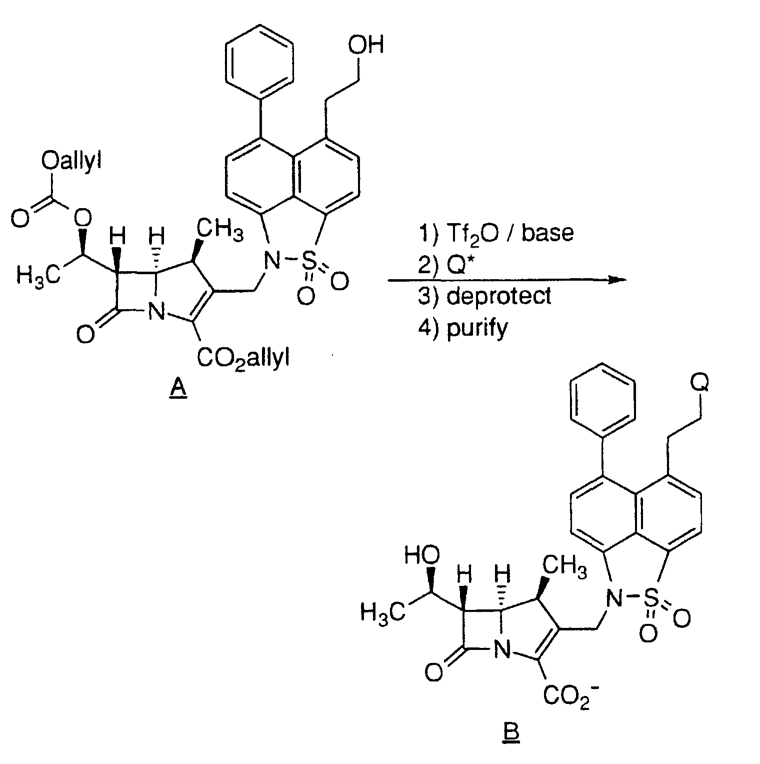

By appropriately modifying the procedures of Example 7 and 8, allyl (1S,5R,6S)-2-[N-(4-(2-hydroxy)-ethyl)-5-methoxyacetyl-1,8-naphthosultam)-methyl]-6-[(1R)-(allyloxycarbonyloxy)ethyl]-1-methylcarbapen-2-em-3-carboxylate (compound A) is reacted with compound Q* as set forth in the following Table to produce compounds of formula B in which Q is as defined in the following Table.

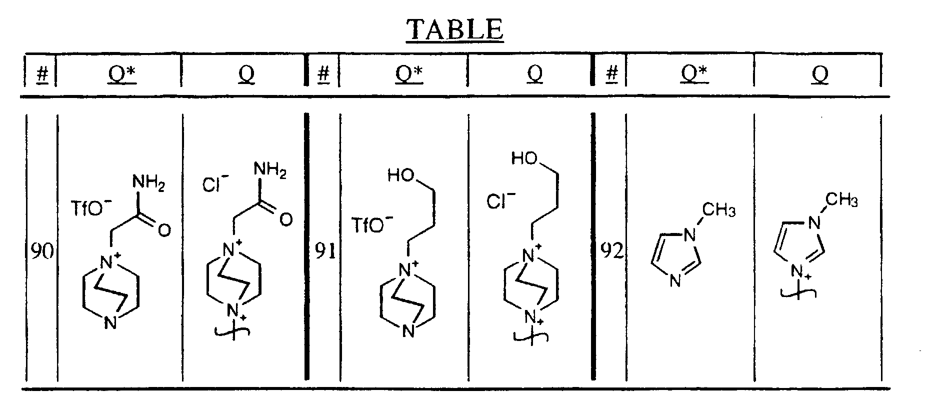

### EXAMPLE 93

### SYNTHESIS OF (1S,5R,6S)-2-(4-((1,3-DIMETHYLIMIDAZOL-2-IUM)-1,8-NAPHTHOSULTAM)METHYL)-6-[1(R)-(ALLYLOXYCARBONYL)OXYETHYL]-1-METHYLCARBAPEN-2-EM-3-CARBOXYLATE

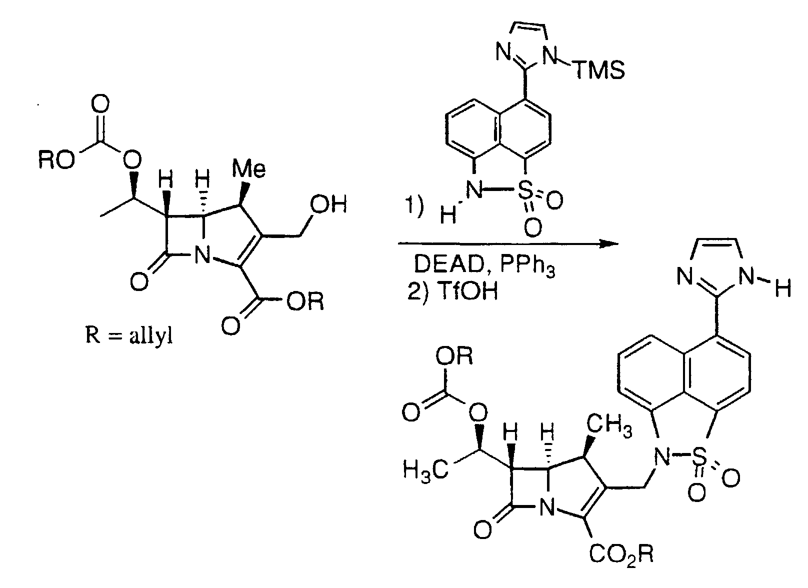

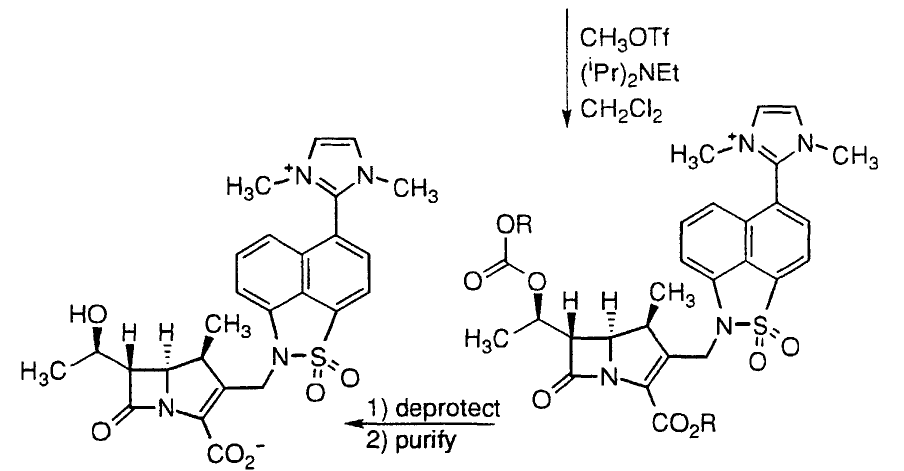

### Steps 1 and 2: allyl (1S,5R,6S)-2-(4-(imidazol-2-yl)-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 4-(imidazol-2-yl)-1,8-naphthosultam to afford allyl (1S,5R,6S)-2-[N-(4-(imidazol-2-yl)-1,8-naphthosultam)-methyl]-6-[(1R)-(allyloxycarbonyloxy)ethyl]-1-methylcarbapen-2-em-3-carboxylate.

### Step 3: allyl (1S,5R,6S)-2-(4-(1,3-dimethyl-imidazol-2-ium)-1,8-naphthosultam)methyl)-6-[1(R)-(allyloxycarbonyl)oxyethyl]-1-methylcarbapen-2-em-3-carboxylate trifluoromethanesulfonate

Allyl (1S,5R,6S)-2-[N-(4-(imidazol-2-yl)-1,8-naphthosultam)-methyl]-6-[(1R)-(allyloxycarbonyloxy)ethyl]-1-methylcarbapen-2-em-3-carboxylate (0.10 mmol) and diisopropylethylamine (0.11 mmol) are dissolved in methylene chloride (5 mL) under a nitrogen atmosphere. The mixture is cooled in an ice bath and treated with methyl trifluoromethanesulfonate (0.21 mmol). After 30 minutes the mixture is removed from the cooling bath and allowed to warm to room temperature. The mixture is partitioned between methylene chloride (10 mL) and 0.1N pH7 potassium phosphate buffer (20 mL). The methylene chloride layer is washed again with 0.1N pH7 potassium phosphate buffer (20 mL), dried over magnesium sulfate, filtered, and evaporated to give the title compound.

### Step 4: (1S,5R,6S)-2-(4-(1,3-dimethyl-imidazol-2-ium)-1,8-naphthosultam)methyl)-6-[1(R)-hydroxyethyl]-1-methylcarbapen-2-em-3-carboxylate

The crude allyl (1*S*,5*R*,6*S*)-2-(4-(1,3-dimethyl-imidazol-2-ium)-1,8-naphthosultam)methyl)-6-[1 (*R*)-(allyloxycarbonyl)oxyethyl]-1-methylcarbapen-2-em-3-carboxylate trifluoromethanesulfonate from the previous step is dissolved in dimethylformamide (1.5 mL) and cooled in an ice bath. A solution of 0.5M sodium ethyl hexanoate in ethyl acetate (0.22 mL, 0.11 mmol) and ethyl hexanoic acid (0.036 mL, 0.22 mmol) are added. The solution is blanketed with nitrogen, using a Firestone valve, and triphenylphosphine (0.033 mmol) and tetrakis(triphenylphosphine)palladium (0.033 mmol) are added. After 70 minutes, diethyl ether (10 mL) is added and the supernatant is decanted from the precipitate. The solid is washed with additional diethyl ether (10 mL) and dried under vacuum. The solid is purified on a 1000 micron reverse phase plate developed, in an ice bath, with 30% acetonitrile/water and eluted with 80% acetonitrile/ water (15 mL). The eluent is diluted with de-ionized water (10 mL), washed with hexanes (40 mL), evaporated to approximately 2 mL and lyophilized to give the title compound.

### EXAMPLE 94

### SYNTHESIS OF (1S,5R,6S)-2-(4-((3-METHYLIMIDAZOL-1-IUM)-1,8-NAPHTHOSULTAM)METHYL)-6-[1(R)-(ALLYLOXYCARBONYL)OXYETHYL]-1-METHYLCARBAPEN-2-EM-3-CARBOXYLATE

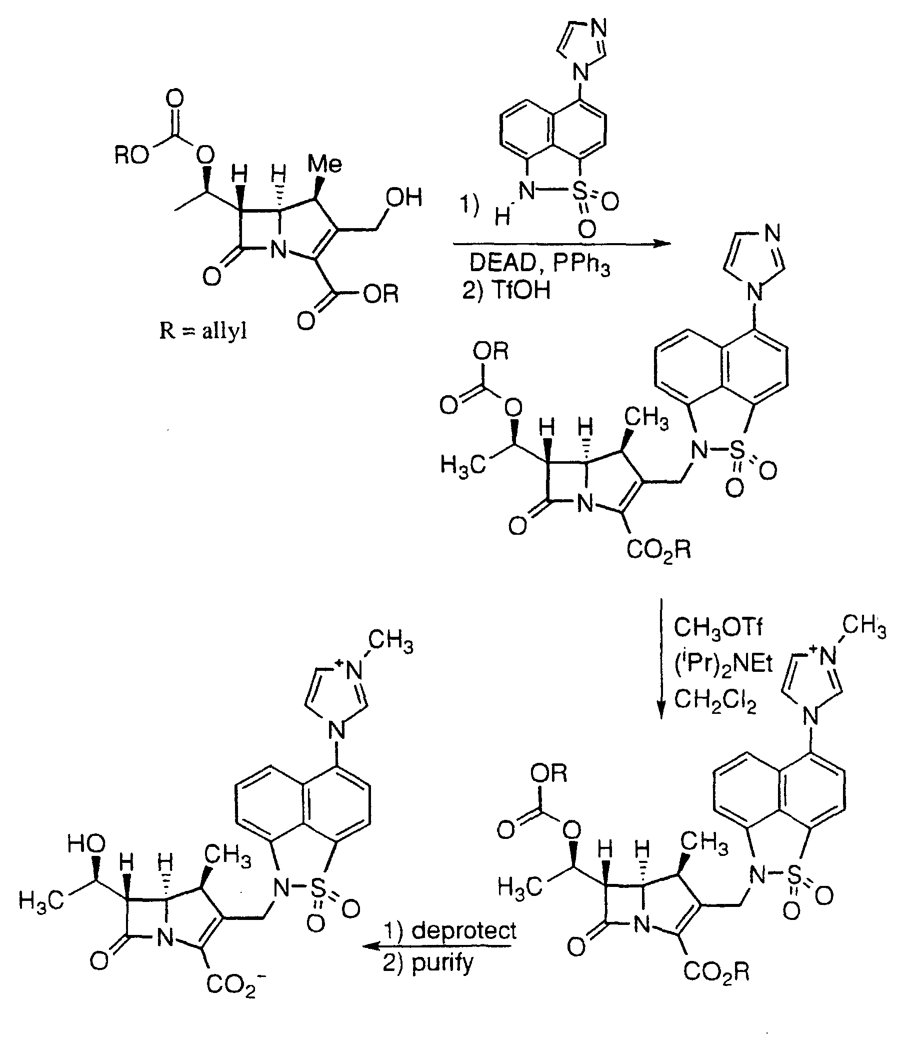

### Steps 1 and 2: allyl (1S,5R,6S)-2-(4-(imidazol-1-yl)-1,8-naphthosultam)methyl)-6-[1(R)-(allyloxycarbonyl)oxyethyl]-1-methylcarbapen-2-em-3-carboxylate

By appropriately modifying the procedures of Steps 1 and 2 of Example 6, allyl (1*S*,5*R*,6*S*)-2-(hydroxymethyl)-6-[1(*R*)-(allyloxycarbonyl)oxyethyl]-1-methylcarbapen-2-em-3-carboxylate is reacted with 4-(imidazol-1-yl)-1,8-naphthosultam to afford allyl (1*S*,5*R*,6*S*)-2-[N-(4-(imidazol-1-yl)-1,8-naphthosultam)-methyl]-6-[(1R)-(allyloxycarbonyloxy)ethyl]-1-methylcarbapen-2-em-3-carboxylate.

### Step 3: allyl(1S,5R,6S)-2-(4-(3-methyl-imidazol-1-ium)-1,8-naphthosultam)methyl)-6-[1(R)-(allyloxycarbonyl)oxyethyl]-1-methylcarbapen-2-em-3-carboxylate trifluoromethanesulfonate

By appropriately modifying the procedure of Step 3 of Example 93, allyl (1S,5R,6S)-2-[N-(4-(imidazol-1-yl)-1,8-naphthosultam)-methyl]-6-[(1R)-(allyloxycarbonyloxy)ethyl]-1-methylcarbapen-2-em-3-carboxylate is reacted with methyl trifluoromethanesulfonate to afford the title compound.

### Step 4: (1S,5R,6S)-2-(4-(3-methyl-imidazol-1-ium)-1,8-naphthosultam)methyl)-6-[1(R)-hydroxyethyl]-1-methylcarbapen-2-em-3-carboxylate

By appropriately modifying the procedure of Step 4 of Example 93, allyl (1S,SR,6S)-2-[N-(4-(3-methyl-imidazol-1-ium)-1,8-naphthosultam)-methyl]-6-[(1R)-(allyloxycarbonyloxy)ethyl]-1-methylcarbapen-2-em-3-carboxylate is deprotected and purified to afford the title compound.

## Claims

1. A compound represented by formula I: 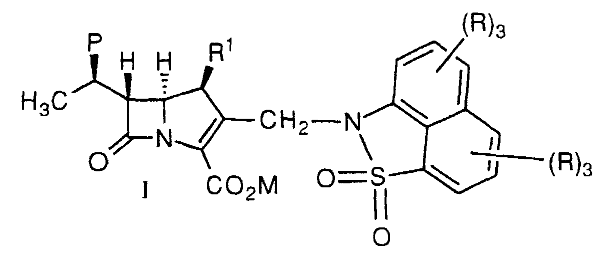 or a pharmaceutically acceptable salt thereof, wherein:
R¹ represents H or methyl;
CO₂M represents a carboxylic acid, a carboxylate anion, a pharmaceutically acceptable ester group or a carboxylic acid protected by a protecting group;
P represents hydrogen, hydroxyl, F or hydroxyl protected by a hydroxyl-protecting group;
each R is independently selected from: -R*; -Q; hydrogen; halo; -CN; -NO₂; -NR^{a}R^{b}; -OR^{c}; -SR^{c}; -C(O)NR^{a}R^{b}; -C(O)OR^{h}; -S(O)R^{c}; -SO₂R^{c}; -SO₂NR^{a}R^{b}; -NR^{a}SO₂R^{b}; -C(O)R^{a}; - OC(O)R^{a}; -OC(O)NR^{a}R^{b}; -NR^{a}C(O)NR^{b}R^{c}; -NR^{a}CO₂R^{h}; -OCO₂R^{h}; - NR^{a}C(O)R^{b}; -C₁₋₆ straight- or branched-chain alkyl, unsubstituted or substituted with one to four R^{d} groups; and -C₃₋₇ cycloalkyl, unsubstituted or substituted with one to four R^{d} groups;
with the proviso that at least one R is present which contains at least one positive charge;
each R^{a}, R^{b} and R^{c} independently represents hydrogen, -R*, -C₁₋₆ straight- or branched-chain alkyl, unsubstituted or substituted with one to four R^{d} groups, or -C₃₋₇ cycloalkyl, unsubstituted or substituted with one to four R^{d} groups;
or R^{a} and R^{b} taken together with any intervening atoms represent a 4-6 membered saturated ring optionally interrupted by one or more of O, S, NR^{c}, with R^{c} as defined above, or -C(O)-, said ring being unsubstituted or substituted with one to four Rⁱ groups;
or R^{b} and R^{c} taken together with any intervening atoms represent a 4-6 membered saturated ring optionally interrupted by one to three of O, S, NR^{a}, with R^{a} as defined above, or -C(O)-, said ring being unsubstituted or substituted with one to four Rⁱ groups;
each R^{d} independently represents halo; -CN; -NO₂; -NR^{e}R^{f}; -OR^{g}; -SR^{g}; -CONR^{e}R^{f}; -COOR^{g}; -SOR^{g}; -SO₂R^{g}; -SO₂NR^{e}R^{f}; -NR^{e}SO₂R^{f}; -COR^{e}; -NR^{e}COR^{f}; -OCOR^{e}; -OCONR^{e}R^{f}; -NR^{e}CONR^{f}R^{g}; -NR^{e}CO₂R^{h}; -OCO₂R^{h}; -C(NR^{e})NR^{f}R^{g}; -NR^{e}C(NH)NR^{f}R^{g}; - NR^{e}C(NR^{f})R^{g}; -R* or -Q;
R^{e}, R^{f} and R^{g} represent hydrogen; -R*; -C₁₋₆ straight- or branched-chain alkyl unsubstituted or substituted with one to four Rⁱ groups;
or R^{e} and R^{f} taken together with any intervening atoms represent a 4-6 membered saturated ring optionally interrupted by one to three of O, S, -C(O)- or NR^{g} with R^{g} as defined above, said ring being unsubstituted or substituted with one to four Rⁱ groups;
each Rⁱ independently represents halo; -CN; -NO₂; phenyl; -NHSO₂R^{h}; -OR^{h}, -SR^{h}; -N(R^{h})₂; -N⁺(R^{h})₃; -C(O)N(R^{h})₂; -SO₂N(R^{h})₂; heteroaryl; heteroarylium; -CO₂R^{h}; -C(O)R^{h}; -OCOR^{h}; -NHCOR^{h}; guanidinyl; carbamimidoyl or ureido;
each R^{h} independently represents hydrogen, a -C₁₋₆ straight or branched-chain alkyl group, a -C₃-C₆ cycloalkyl group or phenyl, or when two R^{h} groups are present, said R^{h} groups may be taken in combination and represent a 4-6 membered saturated ring, optionally interrupted by one or two of O, S, SO₂, -C(O)-, NH and NCH₃;
Q is selected from the group consisting of: 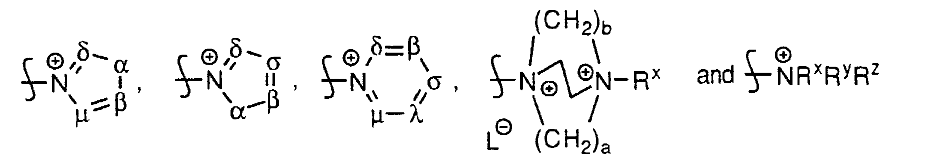 wherein:
a and b are 1, 2 or 3;
L⁻ is a pharmaceutically acceptable counterion;
α represents O, S or NR^{s};
β, δ, λ, µ and σ represent CR^{t}, N or N⁺R^{s}, provided that no more than one of β, δ, λ, µ and σ is N⁺R^{s};
R* is selected from the group consisting of: 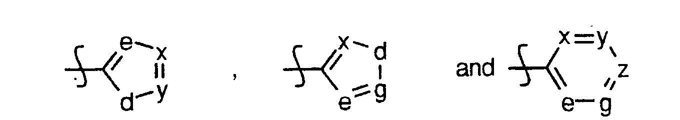 wherein:
d represents 0, S or NR^{k};
e, g, x, y and z represent CR^{m}, N or N⁺R^{k}, provided that no more than one of e, g, x, y and z in any given structure represents N⁺R^{k};
R^{k} represents hydrogen; -C₁₋₆ straight- or branched-chain alkyl, unsubstituted or substituted with one to four Rⁱ groups; or -(CH₂)ₙQ where n = 1, 2 or 3 and Q is as previously defined;
each R^{m} independently represents a member selected from the group consisting of: hydrogen; halo; -CN; -NO₂; -NRⁿR^{o}; -ORⁿ; -SRⁿ; -CONRⁿR^{o}; -COOR^{h}; -SORⁿ; -SO₂Rⁿ; -SO₂NRⁿR^{o}; -NRⁿSO₂R^{o}; -CORⁿ; -NRⁿCOR^{o}; -OCORⁿ; -OCONRⁿR^{o}; -NRⁿCO₂R^{h}; - NRⁿCONR^{o}R^{h}; -OCO₂R^{h}; -CNRⁿNR^{o}R^{h}; -NRⁿCNHNR^{o}R^{h}; - NRⁿC(NR^{o})R^{h}; -C₁₋₆ straight- or branched-chain alkyl, unsubstituted or substituted with one to four Rⁱ groups; -C₃₋₇ cycloalkyl, unsubstituted or substituted with one to four Rⁱ groups; and -(CH₂)ₙQ where n and Q are as defined above;
Rⁿ and R^{o} represent hydrogen, phenyl; -C₁₋₆ straight- or branched-chain alkyl unsubstituted or substituted with one to four Rⁱ groups;
each R^{s} independently represents hydrogen; phenyl or -C₁₋₆ straight- or branched-chain alkyl, unsubstituted or substituted with one to four Rⁱ groups;
each R^{t} independently represents hydrogen; halo; phenyl; - CN; -NO₂; -NR^{u}R^{v}; -OR^{u}; -SR^{u}; -CONR^{u}R^{v}; -COOR^{h}; -SOR^{u}; -SO₂R^{u}; - SO₂NR^{u}R^{v}; -NR^{u}SO₂R^{v}; -COR^{u}; -NR^{u}COR^{v}; -OCOR^{u}; -OCONR^{u}R^{v}; - NR^{u}CO₂R^{v}; -NR^{u}CONR^{v}R^{w}; -OCO₂R^{v}; -C₁₋₆ straight- or branched-chain alkyl, unsubstituted or substituted with one to four Rⁱ groups;
R^{u} and R^{v} represent hydrogen or -C₁₋₆ straight- or branched-chain alkyl, unsubstituted or substituted with one to four Rⁱ groups;
or R^{u} and R^{v} together with any intervening atoms represent a 4-6 membered saturated ring optionally interrupted by one or more of O, S, NR^{w} or -C(O)-, said ring being unsubstituted or substituted with one to four Rⁱ groups;
each R^{w} independently represents hydrogen; -C₁₋₆ straight- or branched-chain alkyl, unsubstituted or substituted with one to four Rⁱ groups; C₃₋₆ cycloalkyl optionally substituted with one to four Rⁱ groups; phenyl optionally substituted with one to four Rⁱ groups, or heteroaryl optionally substituted with 1-4 Rⁱ groups;
or R^{h} and R^{w} taken together with any intervening atoms represent a 5-6 membered saturated ring, optionally interrupted by one or two of O, S, SO₂, NH or NCH₃;
R^{x} represents hydrogen or a C₁₋₈ straight- or branched-chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched-chain alkyl groups, said alkyl groups being unsubstituted or substituted with one to four Rⁱ groups;
R^{y} and R^{z} represent hydrogen; phenyl; -C₁₋₆ straight or branched chain alkyl, unsubstituted or substituted with one to four Rⁱ groups, and optionally interrupted by O, S, NR^{w}, N⁺R^{h}R^{w} or -C(O)-;
or R^{x} and R^{y} together with any intervening atoms represent a 4-6 membered saturated ring optionally interrupted by O, S, SO₂, NR^{w} , N⁺R^{h}R^{w} or -C(O)-, unsubstituted or substituted with 1 - 4 Rⁱ groups,
and when R^{x} and R^{y} together represent a 4-6 membered ring as defined above; R^{z} is as defined above or R^{z} represents an additional saturated 4-6 membered ring fused to the ring represented by R^{x} and R^{y} taken together, optionally interrupted by O, S, NR^{w} or -C(O)-, said rings being unsubstituted or substituted with one to four Rⁱ groups.

2. A compound in accordance with claim I wherein CO₂M represents a carboxylate anion.

3. A compound in accordance with claim 1 wherein wherein one R represents a group which contains a positively charged moiety, and the remaining R groups are selected from hydrogen and C₁₋₆ straight or branched chain alkyl, unsubstituted or substituted with one to four R^{d} groups.

4. A compound in accordance with claim 3 wherein one R represents a group containing a positively charged moiety and the remaining R groups are hydrogen.

5. A compound in accordance with claim 1 wherein the R groups contain from 1-3 positive charges.

6. A compound in accordance with claim 5 wherein the R groups contain two positive charges, balanced by a carboxylate anion and a negatively charged counterion.

7. A compound in accordance with claim 1 wherein one R group represents a -C₁₋₆ straight or branched chain alkyl group, substituted with one to four R^{d} groups, wherein one R^{d} group represents -R* or Q.

8. A compound in accordance with claim 1 wherein Q is selected from the group consisting of:

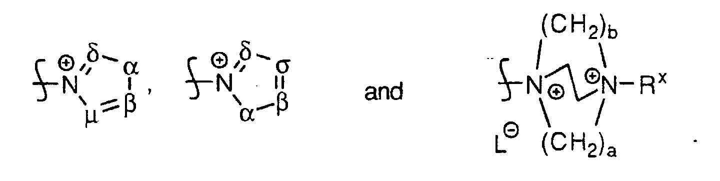

9. A compound in accordance with claim 8 wherein wherein Q represents: 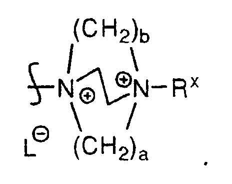
L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups, and
R^{h}, Rⁱ and R^{w} are as originally defined.

10. A compound in accordance with claim 1 wherein Q represents -N⁺R^{x}R^{y}R^{z}, wherein R^{x}, R^{y} and R^{z} are as originally defined.

11. A compound in accordance with claim 1 wherein one R* group is present and is selected from: 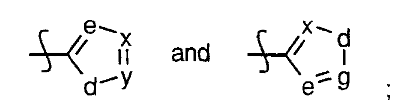 d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

12. A compound in accordance with claim 1 wherein:
CO₂M represents a carboxylate anion;
one R group which is attached to the naphthosultam platform contains at least one positively charged moiety, and the remaining R groups are selected from hydrogen and C₁₋₆ straight or branched chain alkyl, unsubstituted or substituted with one to four R^{d} groups.
Rd is as originally defined;
R^{h} represents hydrogen or a C₁₋₆ straight or branched chain alkyl group;
Q is selected from the group consisting of: 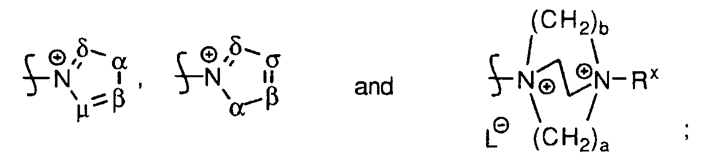 wherein L⁻ is as originally defined; a and b represent 2, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or - C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R* is selected from: 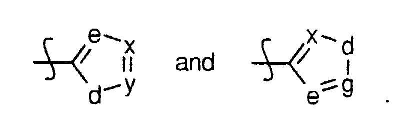
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

13. A compound in accordance with claim 1 represented by formula Ia: 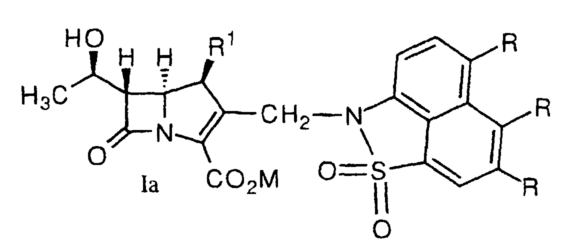 or a pharmaceutically acceptable salt thereof, wherein:
CO₂M represents a carboxylate anion;
one R contains a positively charged moiety, and the other R groups are selected from hydrogen and C₁₋₆ straight or branched chain alkyl, unsubstituted or substituted with one to four R^{d} groups;
R^{d} is as originally defined;
Q is selected from the group consisting of: 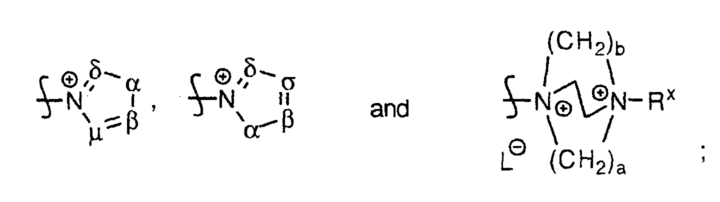 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or - C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R^{h} represents hydrogen or a C₁₋₆ straight or branched chain alkyl group;
R^{w} is as originally defined;
R* is selected from: 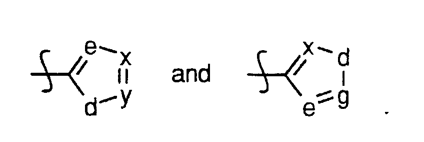
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

14. A compound in accordance with claim 1 represented by formula Ib: 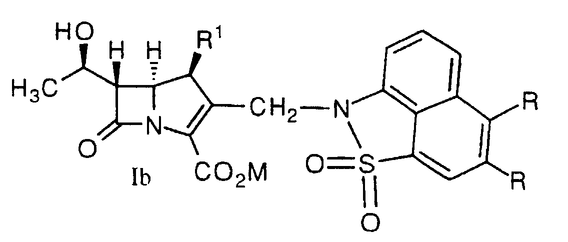 or a pharmaceutically acceptable salt thereof, wherein:
CO₂M represents a carboxylate anion;
one R group is attached to the naphthosultam platform which contains a positively charged moiety, and the other R group is selected from hydrogen and C₁₋₆ straight or branched chain alkyl, unsubstituted or substituted with one to four R^{d} groups;
R^{d} is as originally defined;
Q is selected from the group consisting of: 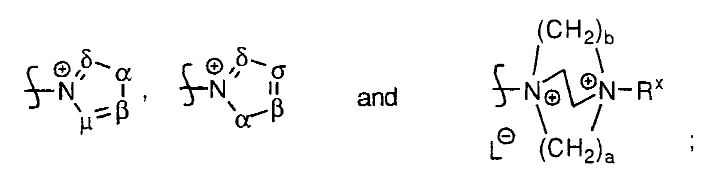 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or - C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R^{h} represents hydrogen or a C₁₋₆ straight or branched chain alkyl group;
R^{w} is as originally defined;
R* is selected from: 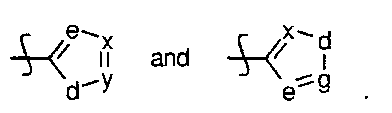
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen. Within this subset, all other variables are as originally defined with respect to formula I.

15. A compound in accordance with claim 1 represented by formula Ic: 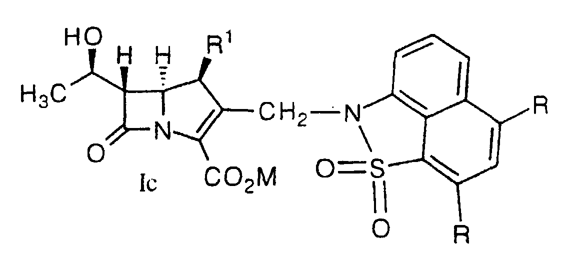 or a pharmaceutically acceptable salt thereof, wherein:
CO₂M represents a carboxylate anion;
one R group is attached to the naphthosultam platform which contains a positively charged moiety, and the other R group is selected from hydrogen, halo and C₁₋₆ straight or branched chain alkyl, unsubstituted or substituted with one to four R^{d} groups;
R^{d} is as originally defined;
Q is selected from the group consisting of: 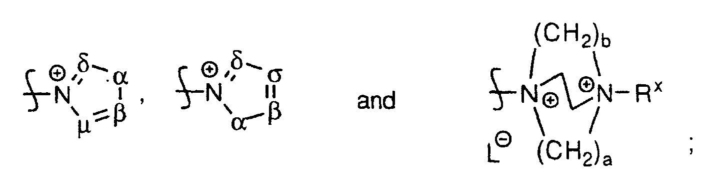 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or -C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R^{h} represents hydrogen or a C₁₋₆ straight or branched chain alkyl group;
R^{w} is as originally defined;
R* is selected from: 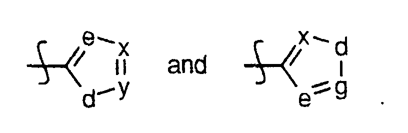
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

16. A compound in accordance with claim 1 represented by formula Id: 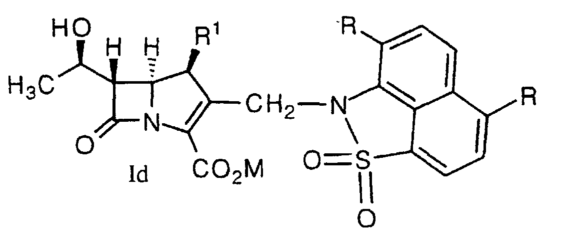 or a pharmaceutically acceptable salt thereof, wherein:
CO₂M represents a carboxylate anion;
one R group is attached to the naphthosultam platform which contains a positively charged moiety, and the other R group is selected from hydrogen, halo and C₁₋₆ straight or branched chain alkyl, unsubstituted or substituted with one to four R^{d} groups;
R^{d} is as originally defined;
Q is selected from the group consisting of: 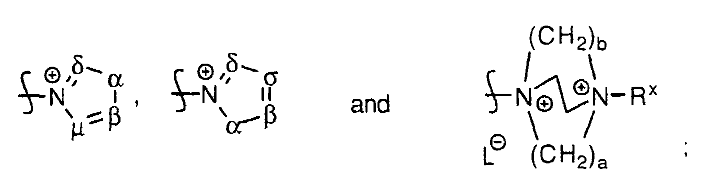 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or - C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R^{h} represents hydrogen or a C₁₋₆ straight or branched chain alkyl group;
R^{w} is as originally defined;
R* is selected from: 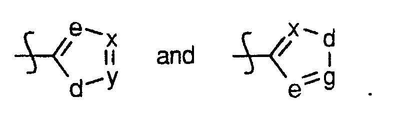
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

17. A compound in accordance with claim 1 represented by formula Ie: 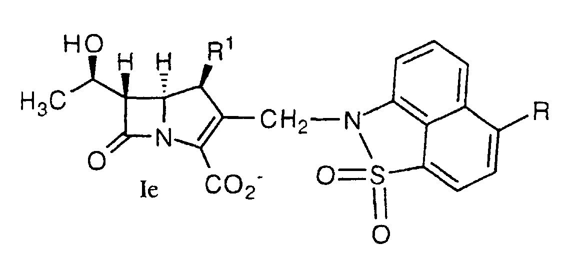 or a pharmaceutically acceptable salt thereof, wherein:
R contains a positively charged moiety selected from the group consisting of: -R*, Q, and a C₁₋₆ straight or branched alkyl chain substituted with one R^{d} group;
R^{d} is independently selected -R* or Q;
Q is selected from the group consisting of: 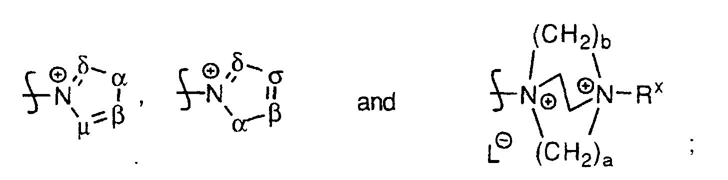 wherein L⁻, a and b are as originally defined, and R^{x} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or - C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R* is selected from: 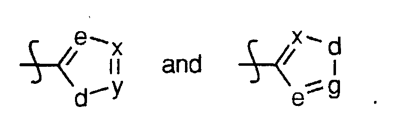
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

18. A compound in accordance with claim 1 represented by formula If: 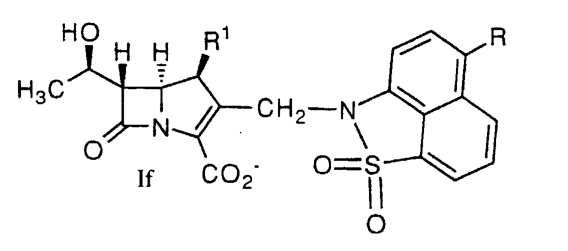 or a pharmaceutically acceptable salt thereof, wherein:
R contains a positively charged moiety selected from the group consisting of: -R*, Q, and a C₁₋₆ straight or branched alkyl chain substituted with one R^{d} group;
R^{d} is independently selected -R* or Q;
Q is selected from the group consisting of: 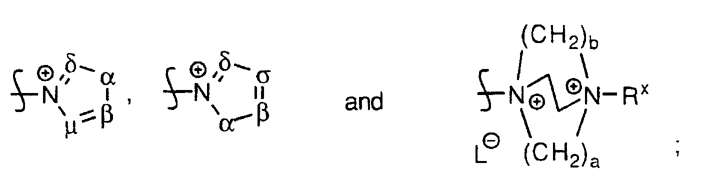 wherein L⁻ , a and b are as originally defined, and R^{X} represents a member selected from the group consisting of: hydrogen or a C₁₋₈ straight- or branched- chain alkyl, optionally interrupted by one or two of O, S, SO, SO₂, NR^{w}, N⁺R^{h}R^{w}, or - C(O)-, said chain being unsubstituted or substituted with one to four of halo, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, or a phenyl or heteroaryl group which is in turn optionally substituted with from one to four Rⁱ groups or with one to two C₁₋₃ straight- or branched- chain alkyl groups, said alkyl groups being unsubstituted or substituted with one to four Rⁱ groups;
R* is selected from: 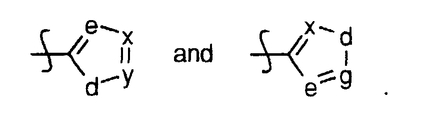
wherein d represents NR^{k}; R^{k} represents -C₁₋₆ straight or branched chain alkyl; and e, g, x and y represent CR^{m} or N⁺R^{k}, with R^{k} as defined above and R^{m} representing hydrogen.

19. A compound in accordance with claim 17 wherein:
R represents 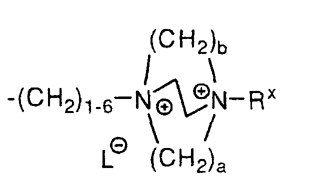 and R^{x}, a, b and L⁻ are as originally defined.

20. A compound in accordance with claim 1 represented by formula Ig: 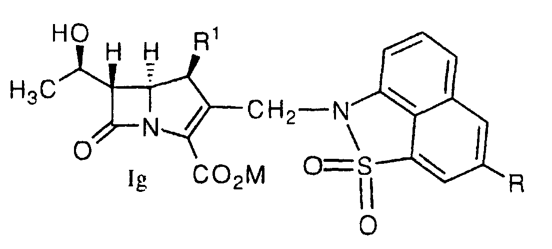 wherein:
R represents 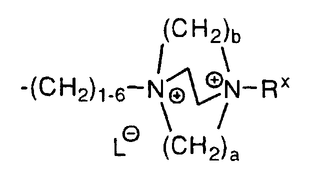 and R^{x}, a, b and L⁻ are as originally defined.

21. A compound in accordance with claim 1 represented by the structural formula: 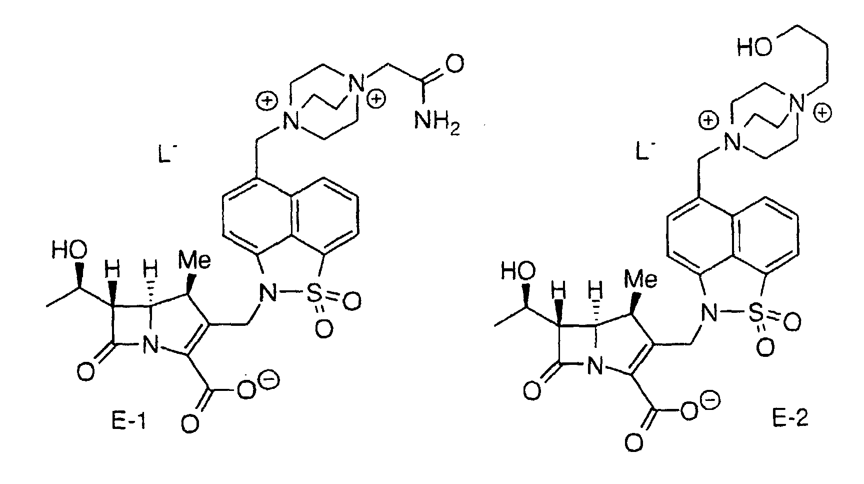 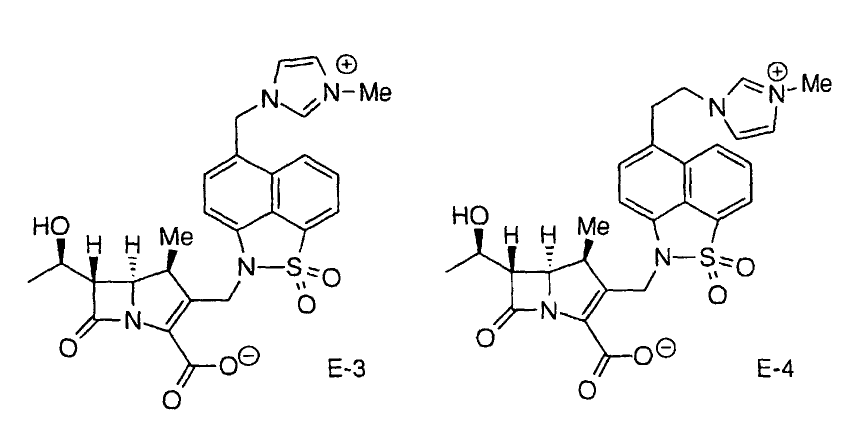 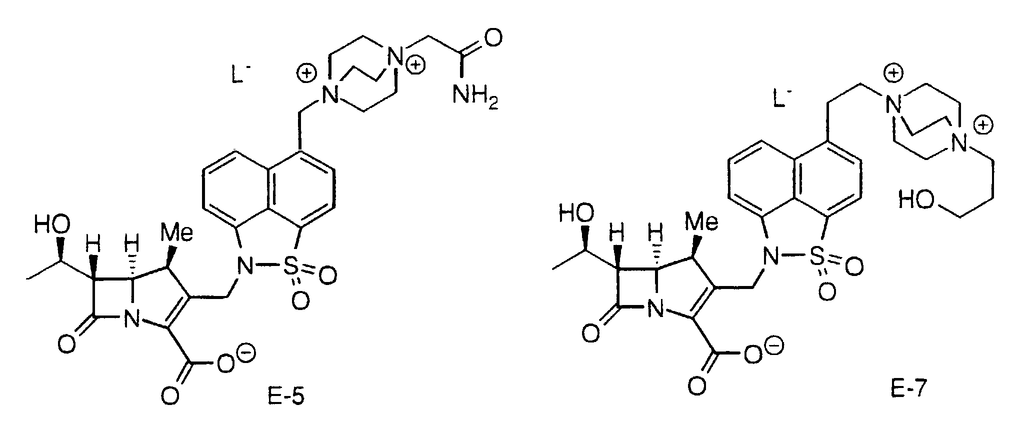 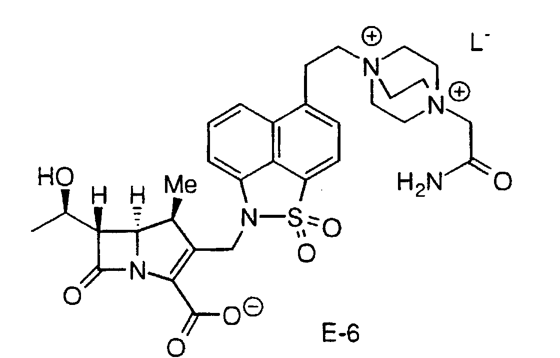 or 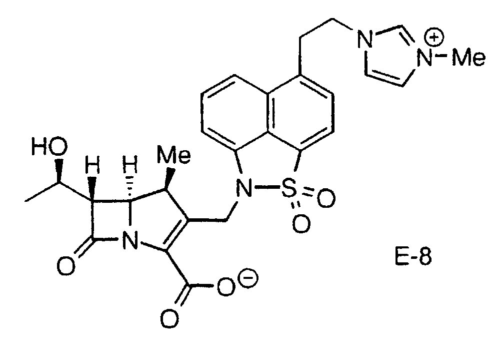 wherein L⁻ is a pharmaceutically acceptable counterion.

22. A compound in accordance with claim 1 represented by the structural formula: 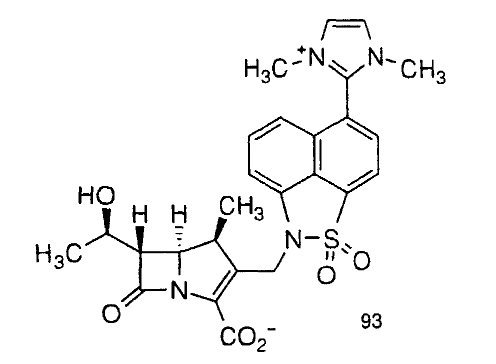 or

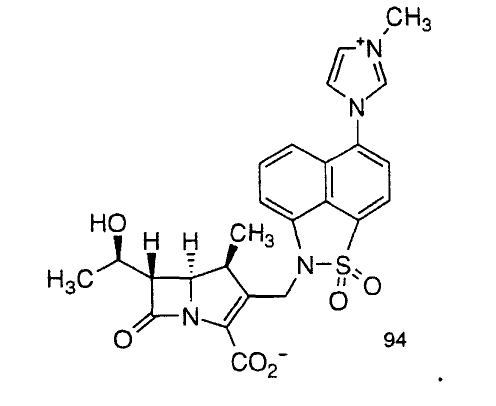

23. A compound represented by the structure: 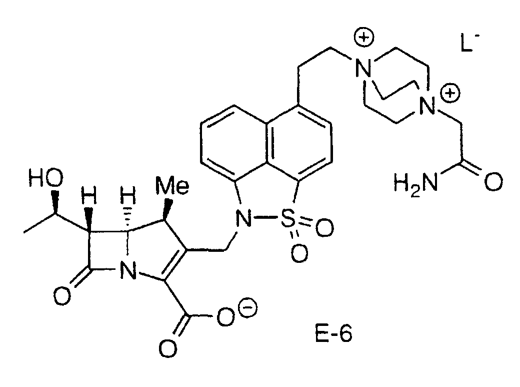 wherein L- represents a pharmaceutically acceptable cation.

24. A compound in accordance with claim 1 falling within one of the following tables: 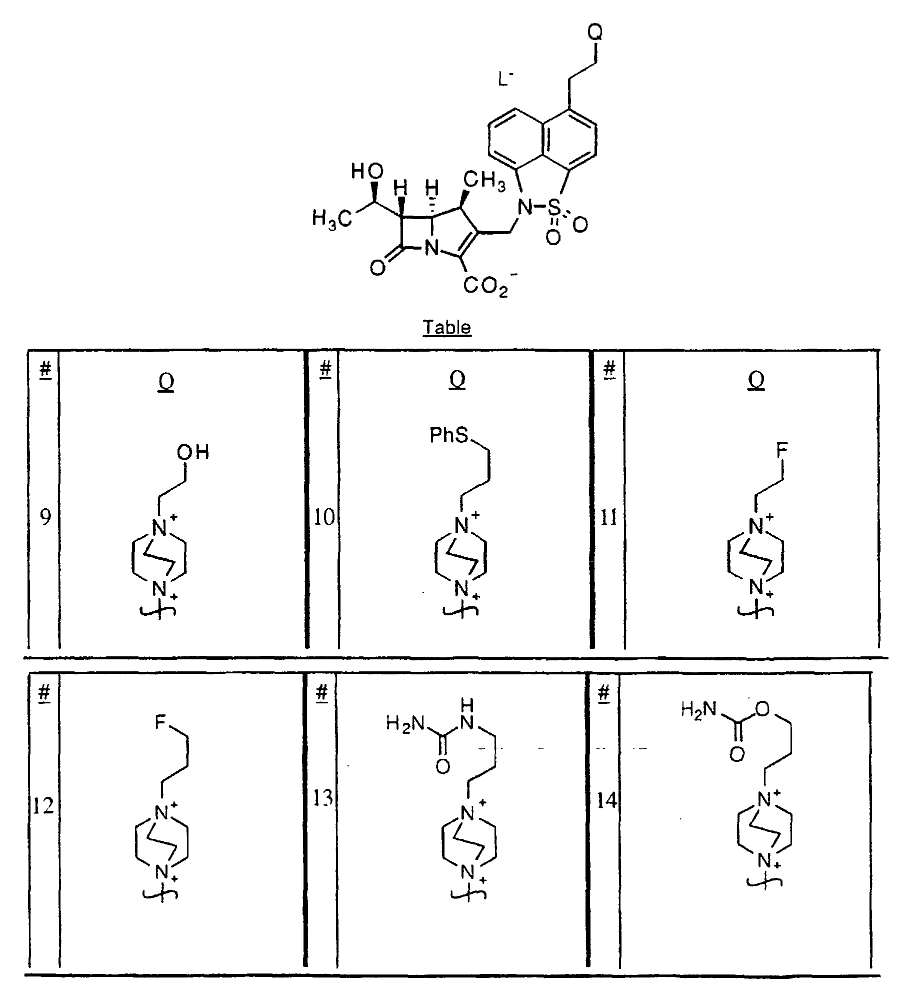 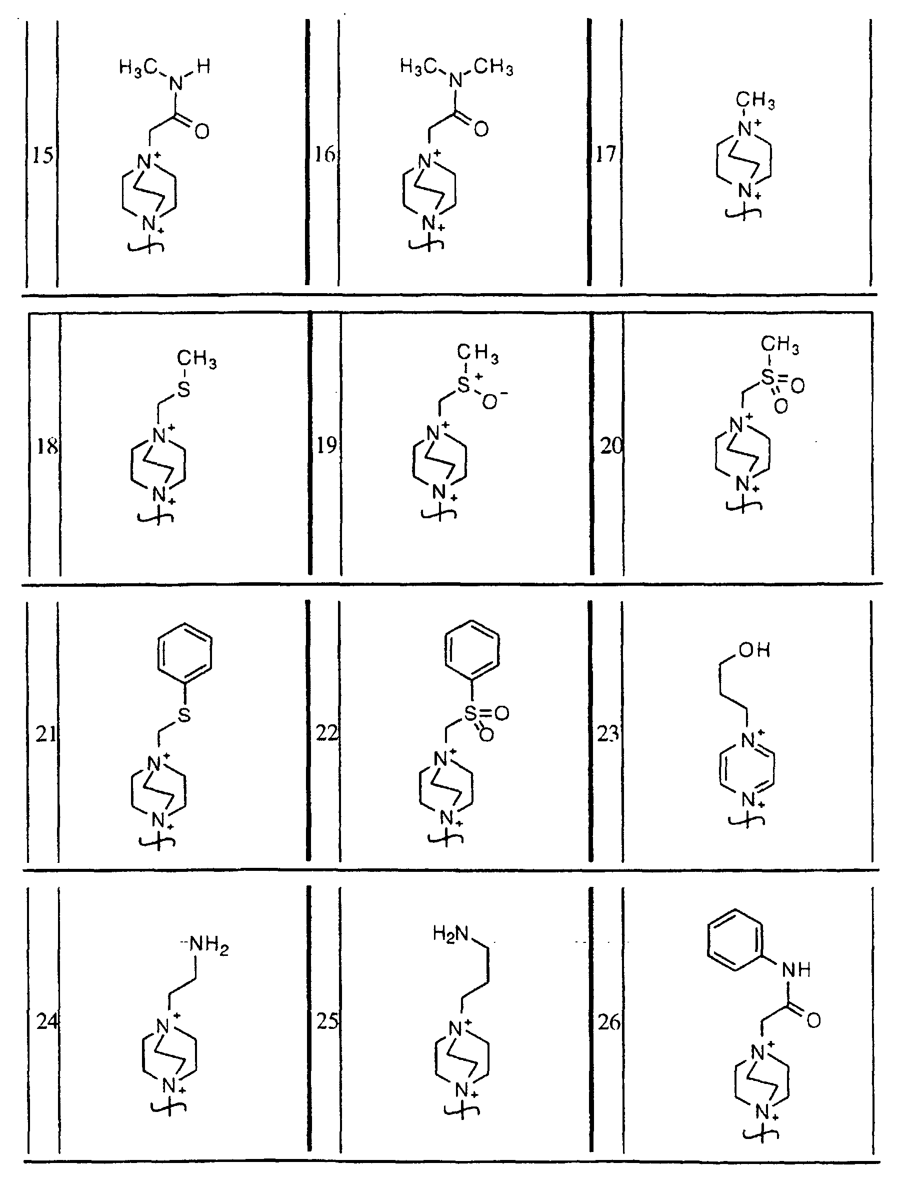 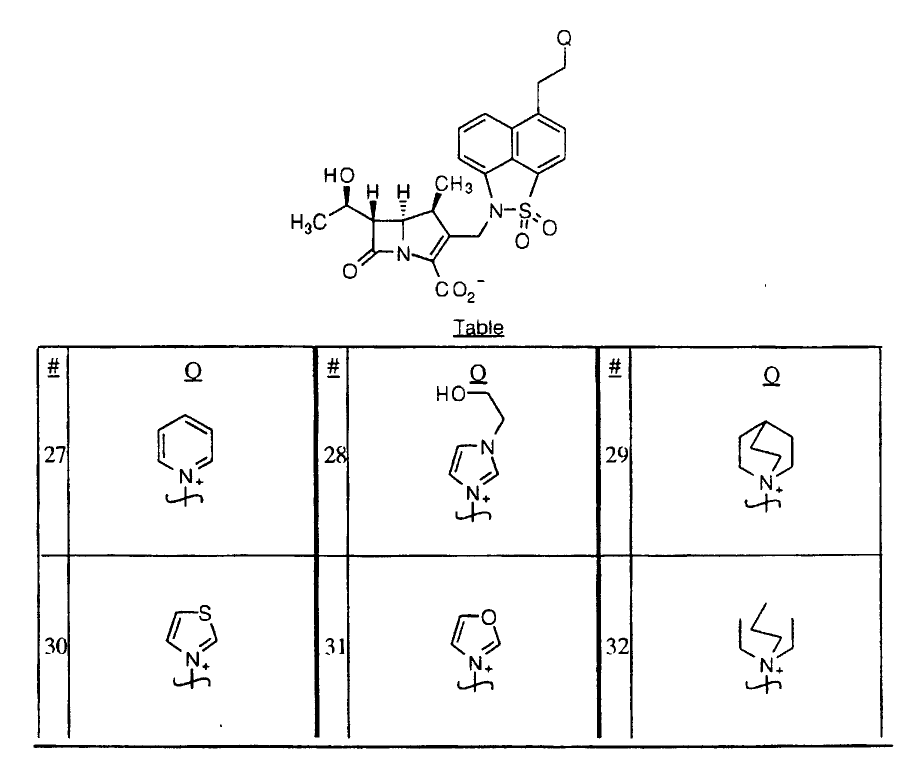 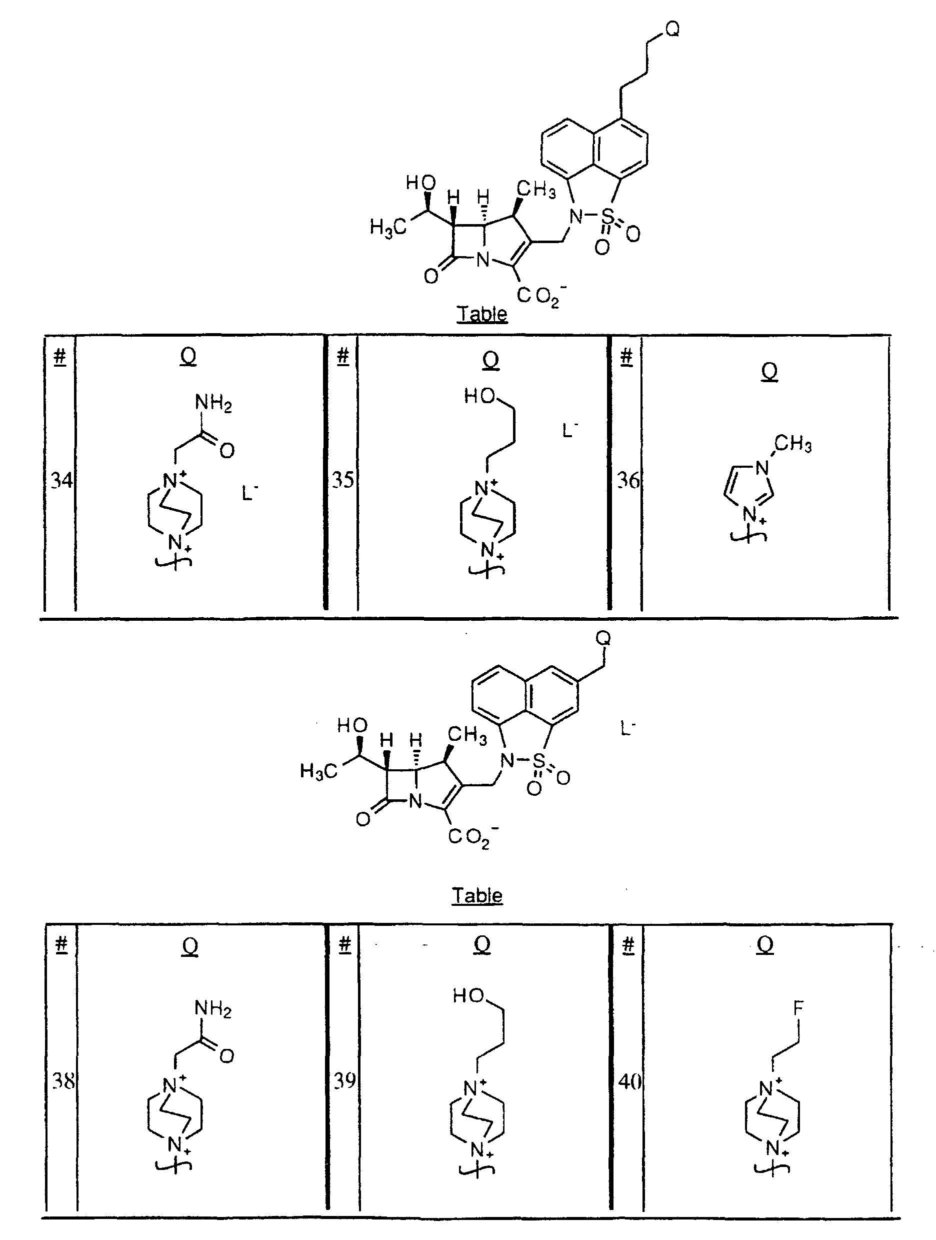 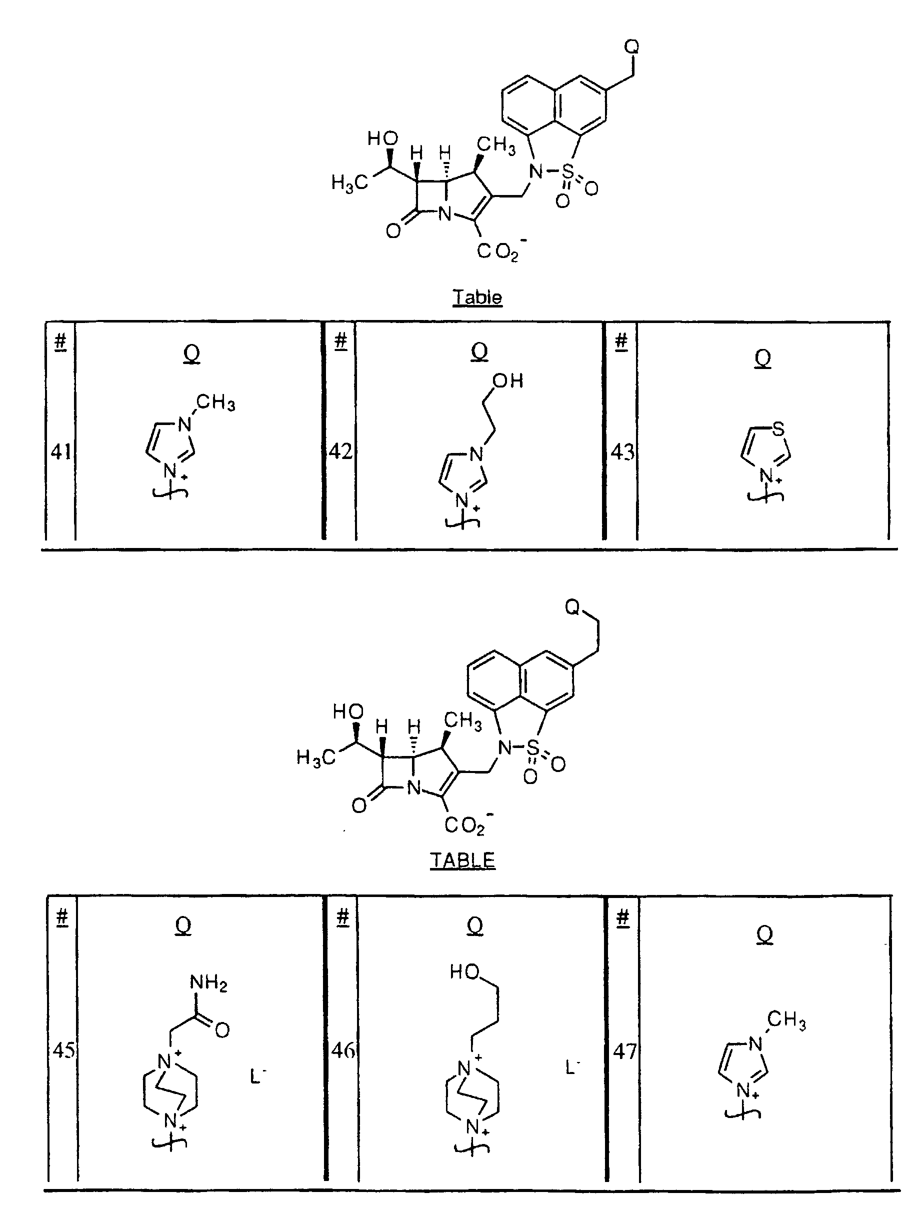 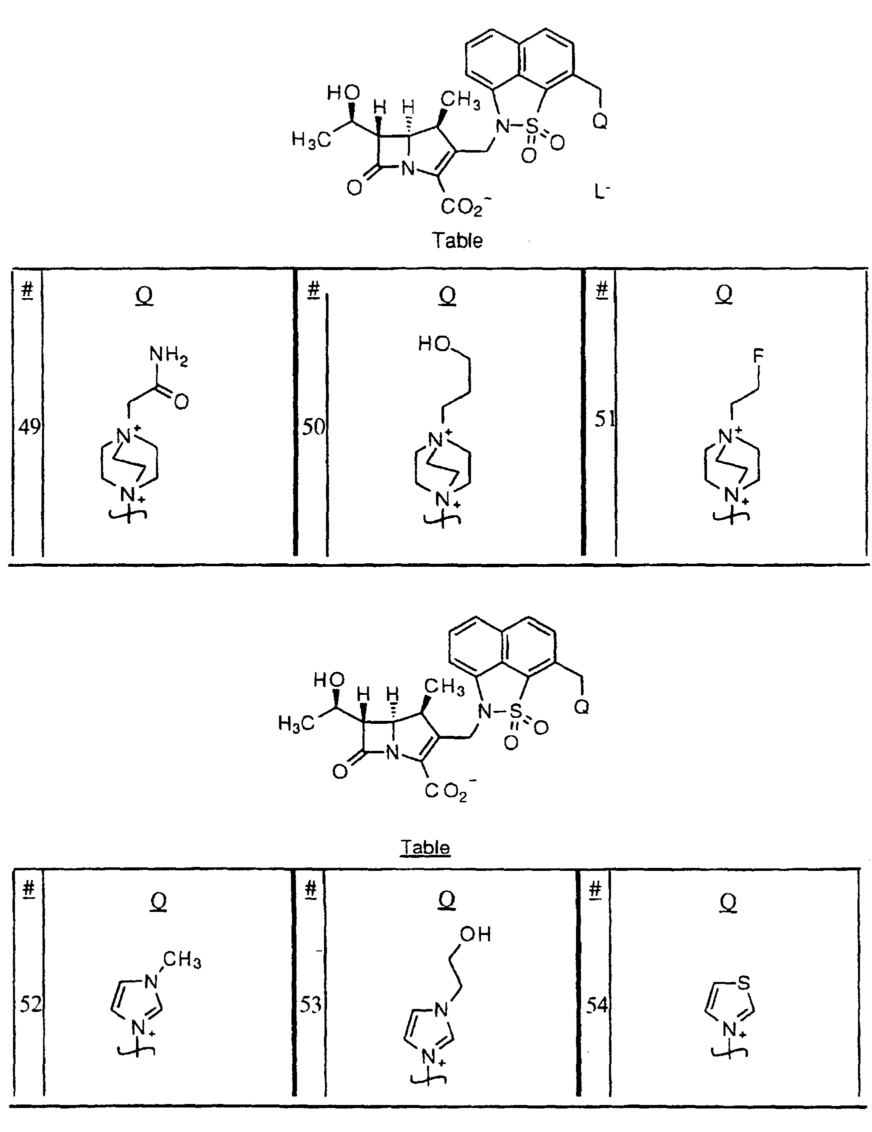 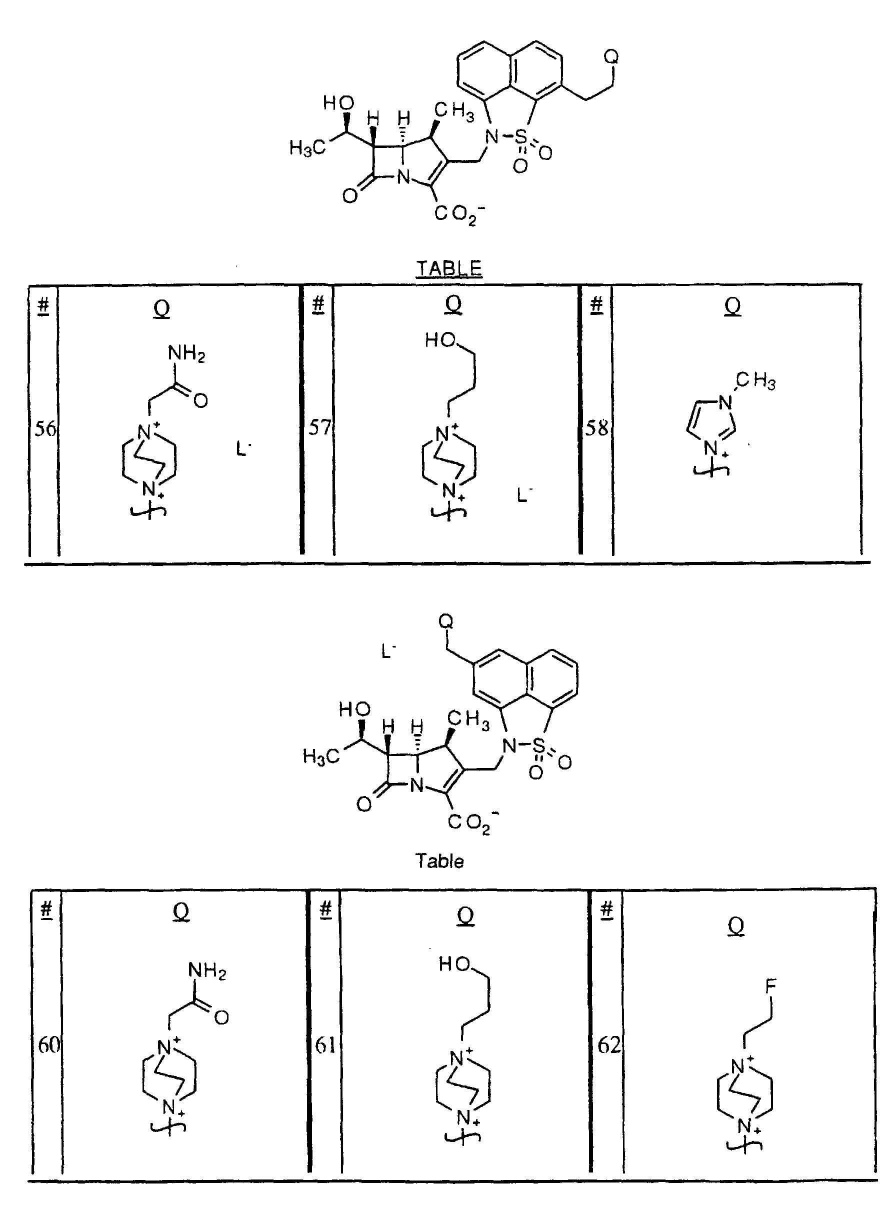 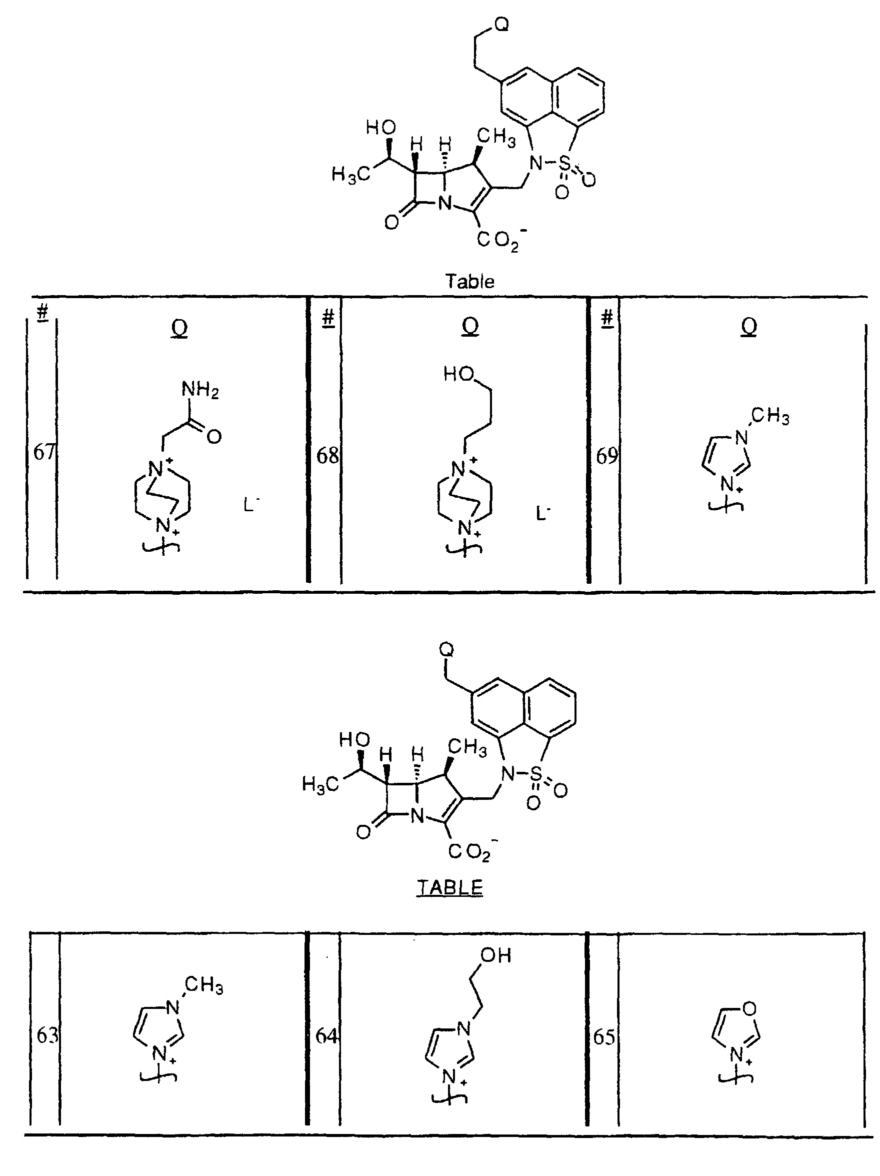 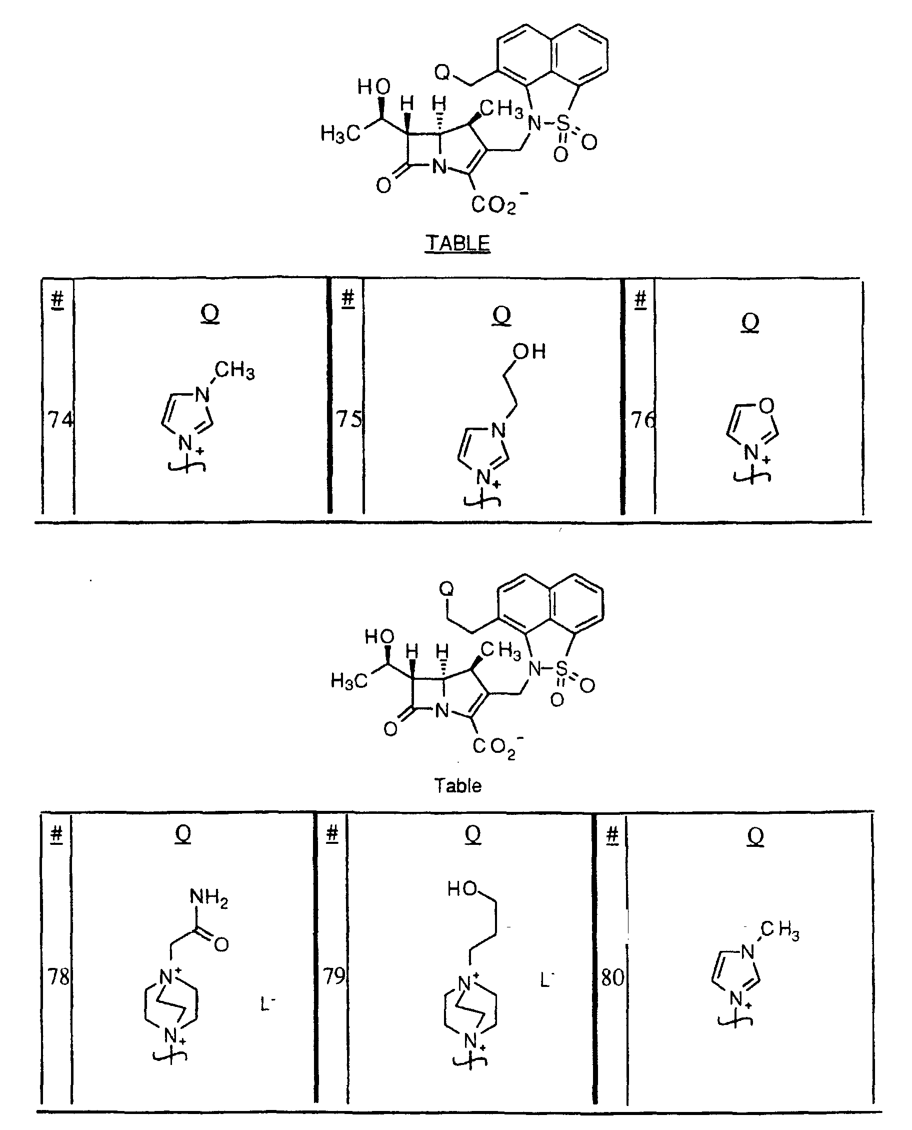 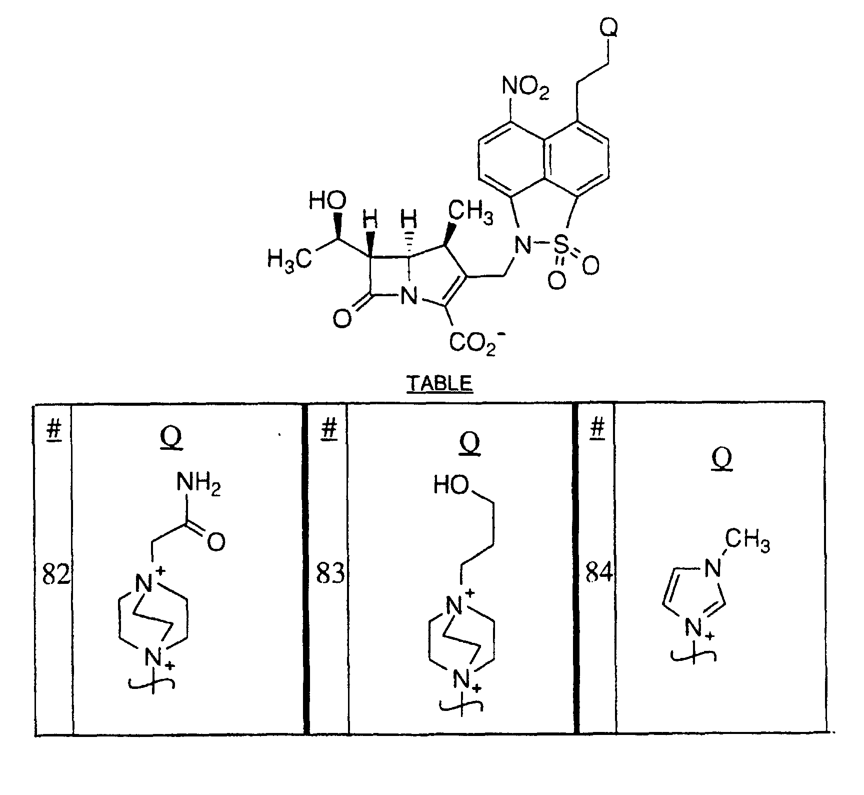 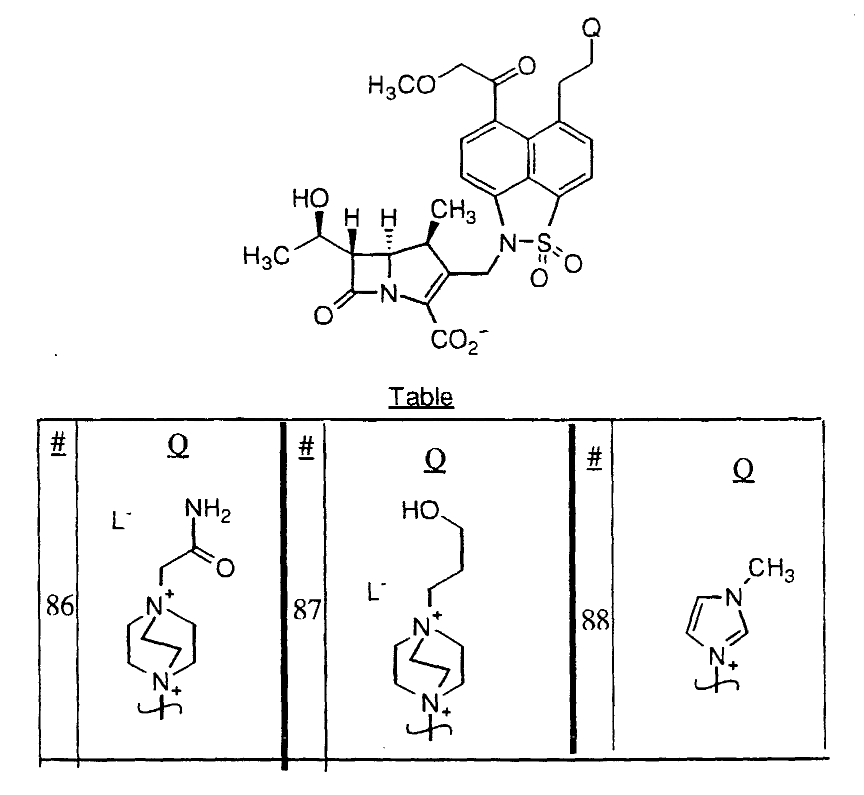 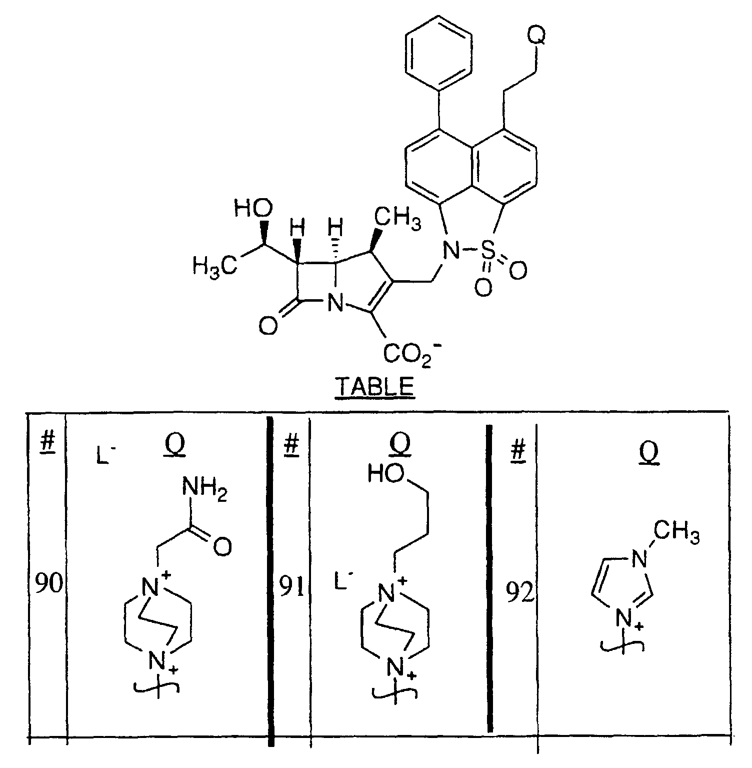 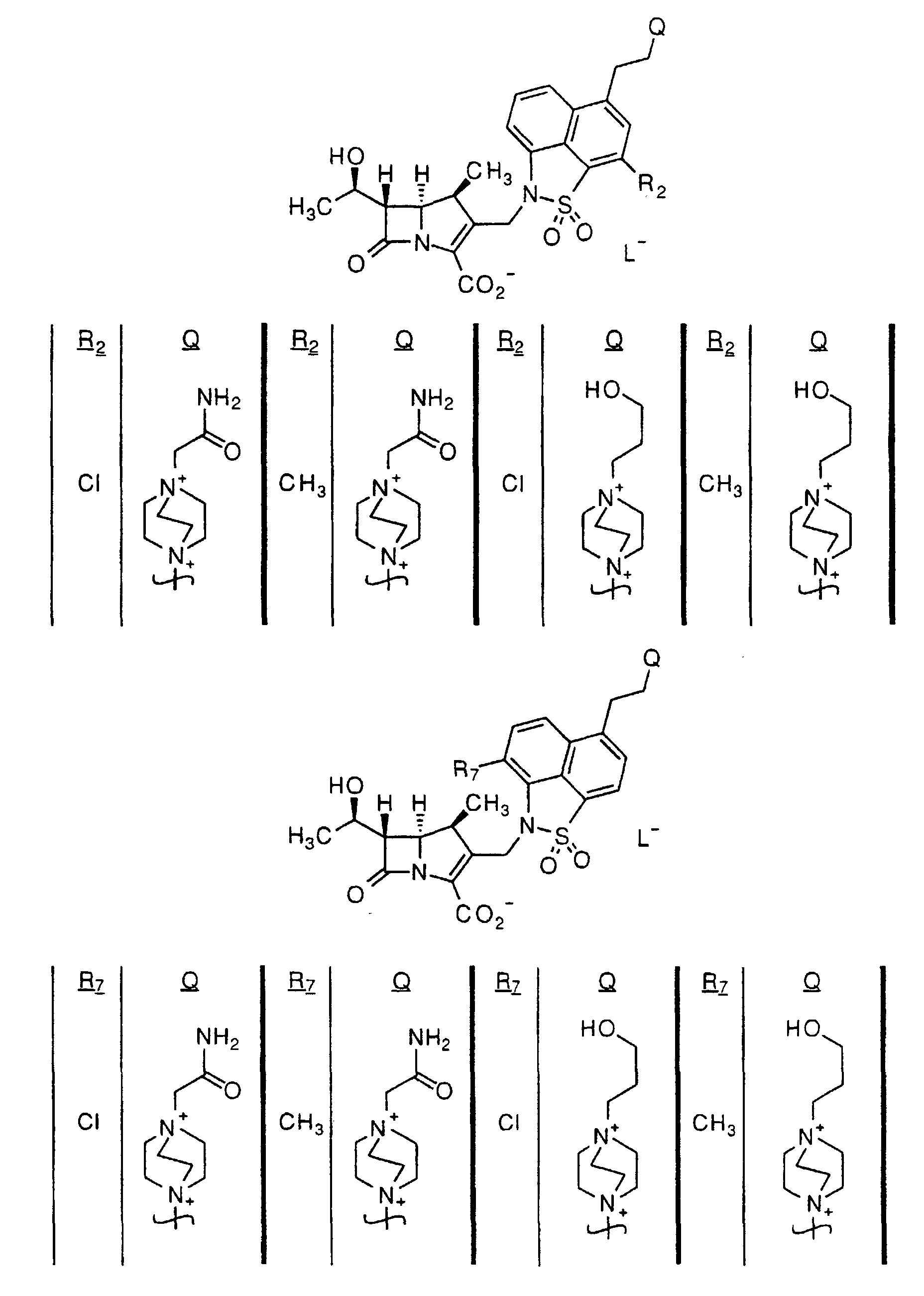 wherein L⁻ represents a pharmaceutically acceptable counterion.

25. A pharmaceutical composition comprised of a compound in accordance with any one of Claims 1 to 24 in combination with a pharmaceutically acceptable carrier.

26. A pharmaceutical composition produced by combining a compound in accordance with any one of Claims 1 to 24 with a pharmaceutically acceptable carrier.

27. Use of a compound in accordance with any one of Claims 1 to 24 for the manufacture of a medicament for the treatment or prevention of a bacterial infection.

## Patentansprüche

1. Eine Verbindung, dargestellt durch Formel I: 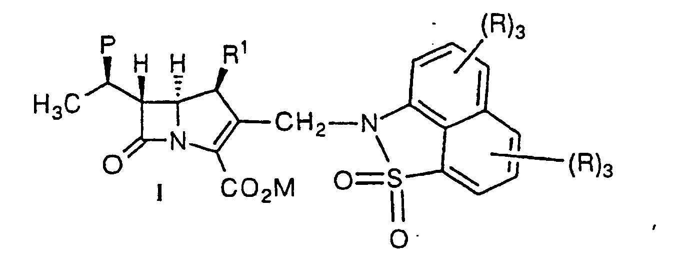 oder ein pharmazeutisch annehmbares Salz davon, wobei:
R¹ H oder Methyl bedeutet,
CO₂M eine Carbonsäure, ein Carboxylatanion, eine pharmazeutisch annehmbare Estergruppe oder eine durch eine Schutzgruppe geschützte Carbonsäure bedeutet,
P Wasserstoff, Hydroxyl, F oder durch eine Hydroxyl-Schutzgruppe geschütztes Hydroxyl bedeutet,
jedes R unabhängig ausgewählt ist aus: -R*, -Q, Wasserstoff, Halogen, -CN, -NO₂, -NR^{a}R^{b}, -OR^{c}, -SR^{c}, -C(O)NR^{a}R^{b}, -C(O)OR^{h}, -S(O)R^{c}, -SO₂R^{c}, -SO₂NR^{a}R^{b}, -NR^{a}SO₂R^{b}, -C(O)R^{a}, -OC(O)R^{a}, -OC(O)NR^{a}R^{b}, -NR^{a}C(O)NR^{b}R^{c}, -NR^{a}CO₂R^{h}, - OCO₂R^{h}, -NR^{a}C(O)R^{b}, gerad- oder verzweigtkettiges -C₁₋₆-Alkyl, unsubstituiert oder substituiert mit ein bis vier R^{d}-Gruppen, und -C₃₋₇-Cycloalkyl, unsubstituiert oder substituiert mit ein bis vier R^{d}-Gruppen,
mit der Maßgabe, daß wenigstens 1 R vorhanden ist, welches wenigstens 1 positive Ladung enthält,
jedes R^{a}, R^{b} und R^{c} unabhängig Wasserstoff, -R*, gerad- oder verzweigtkettiges -C₁₋₆-Alkyl, unsubstituiert oder substituiert mit ein bis vier R^{d}-Gruppen, oder -C₃₋₇-Cycloalkyl, unsubstituiert oder substituiert mit ein bis vier R^{d}-Gruppen, bedeutet
oder R^{a} und R^{b}, zusammengefaßt mit etwaigen dazwischenliegenden Atomen, einen 4-6gliedrigen gesättigten Ring bedeuten, der gegebenenfalls durch ein oder mehrere O, S, NR^{c}, wobei R^{c} wie oben definiert ist, oder - C(O)- unterbrochen ist, wobei der Ring unsubstituiert oder mit ein bis vier Rⁱ-Gruppen substituiert ist,
oder R^{b} und R^{c}, zusammengefaßt mit etwaigen dazwischenliegenden Atomen, einen 4-6gliedrigen gesättigten Ring bedeuten, der gegebenenfalls durch ein bis drei O, S, NR^{a}, wobei R^{a} wie oben definiert ist, oder - C(O)- unterbrochen ist, wobei der Ring unsubstituiert oder mit ein bis vier Rⁱ-Gruppen substituiert ist,
jedes R^{d} unabhängig Halogen, -CN, -NO₂, -NR^{e}R^{f}, -OR^{g}, -SR^{g}, -CONR^{e}R^{f}, - COOR^{g}, -SOR^{g}, SO₂R^{g}, -SO₂NR^{e}R^{f}, -NR^{e}SO₂R^{f}, -COR^{e}, -NR^{e}COR^{f}, -OCOR^{e}, -OCONR^{e}R^{f}, - NR^{e}CONR^{f}R^{g}, -NR^{e}CO₂R^{h}, -OCO₂R^{h}, -C(NR^{e})NR^{f}R^{g}, -NR^{e}C(NH)NR^{f}R^{g}, -NR^{e}C(NR^{f})R^{g}, -R* oder -Q bedeutet,
R^{e}, R^{f} und R^{g} Wasserstoff, -R*, gerad- oder verzweigtkettiges -C₁₋₆-Alkyl, unsubstituiert oder substituiert mit ein bis vier Rⁱ-Gruppen, bedeuten
oder R^{e} und R^{f}, zusammengefaßt mit etwaigen dazwischenliegenden Atomen, einen 4-6gliedrigen gesättigten Ring bedeuten, der gegebenenfalls mit ein bis drei O, S, -C(O)- oder NR^{g}, wobei R^{g} wie oben definiert ist, unterbrochen ist, wobei der Ring unsubstituiert oder mit ein bis vier Rⁱ-Gruppen substituiert ist,
jedes Rⁱ unabhängig Halogen, -CN, -NO₂, Phenyl, -NHSO₂R^{h}, -OR^{h}, -SR^{h}, -N(R^{h})₂, -N⁺(R^{h})₃, -C(O)N(R^{h})₂, -SO₂N(R^{h})₂, Heteroaryl, Heteroarylium, -CO₂R^{h}, -C(O)R^{h}, -OCOR^{h}, -NHCOR^{h}, Guanidinyl, Carbamimidoyl oder Ureido bedeutet,
jedes R^{h} unabhängig Wasserstoff, eine gerad- oder verzweigtkettige - C₁₋₆-Alkylgruppe, eine -C₃-C₆-Cycloalkylgruppe oder Phenyl bedeutet oder, wenn zwei R^{h}-Gruppen vorhanden sind, die R^{h}-Gruppen zusammengefaßt sein können und einen 4-6gliedrigen gesättigten Ring bedeuten, der gegebenenfalls durch ein oder zwei O, S, SO₂, -C(O)-, NH und NCH₃ unterbrochen ist,
Q ausgewählt ist aus der Gruppe, bestehend aus: 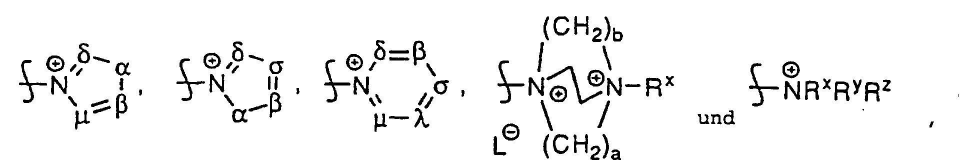 wobei:
a und b 1, 2 oder 3 sind,
L⁻ ein pharmazeutisch annehmbares Gegenion ist,
α O, S oder NR^{s} bedeutet,
β, δ, λ, µ und σ CR^{t}, N oder N⁺R^{s} bedeuten, mit der Maßgabe, daß nicht mehr als ein β, δ, λ, µ und σ N⁺R^{s} ist,
R* ausgewählt ist aus der Gruppe, bestehend aus: 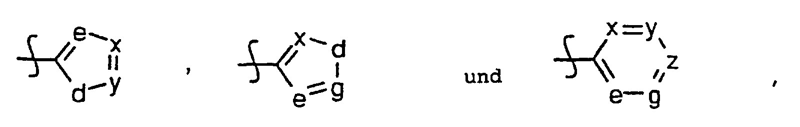 wobei:
d O, S oder NR^{k} bedeutet,
e, g, x, y und z CR^{m}, N oder N⁺R^{k} bedeuten, mit der Maßgabe, daß nicht mehr als eines von e, g, x, y und z in irgendeiner bestimmten Struktur N⁺R^{k} bedeutet,
R^{k} Wasserstoff, gerad- oder verzweigtkettiges -C₁₋₆-Alkyl, unsubstituiert oder substituiert mit ein bis vier Rⁱ-Gruppen, oder -(CH₂)ₙQ, wobei n = 1, 2 oder 3 und Q wie zuvor definiert ist, bedeutet,
jedes R^{m} unabhängig ein Element bedeutet, ausgewählt aus der Gruppe, bestehend aus: Wasserstoff, Halogen, -CN, -NO₂, -NRⁿR^{o}, -ORⁿ, -SRⁿ, - CONRⁿR^{o}, -COOR^{h}, -SORⁿ, -SO₂Rⁿ, -SO₂NRⁿR^{o}, -NRⁿSO₂R^{o}, -CORⁿ, -NRⁿCOR^{o}, -OCORⁿ, - OCONRⁿR^{o}, -NRⁿCO₂R^{h}, -NRⁿCONR^{o}R^{h}, -OCO₂R^{h}, -CNRⁿNR^{o}R^{h}, -NRⁿCNHNR^{o}R^{h}, -NRⁿC(NR^{o})R^{h}, gerad- oder verzweigtkettigem -C₁₋₆-Alkyl, unsubstituiert oder substituiert mit ein bis vier Rⁱ-Gruppen, -C₃₋₇-Cycloalkyl, unsubstituiert oder substituiert mit ein bis vier Rⁱ-Gruppen, und -(CH₂)ₙQ, wobei n und Q wie oben definiert sind,
Rⁿ und R^{o} Wasserstoff, Phenyl, gerad- oder verzweigtkettiges -C₁₋₆-Alkyl, unsubstituiert oder substituiert mit ein bis vier Rⁱ-Gruppen, bedeuten,
jedes R^{s} unabhängig Wasserstoff, Phenyl oder gerad- oder verzweigtkettiges -C₁₋₆-Alkyl, unsubstituiert oder substituiert mit ein bis vier Rⁱ-Gruppen, bedeutet,
jedes R^{t} unabhängig Wasserstoff, Halogen, Phenyl, -CN, -NO₂, -NR^{u}R^{v}, - OR^{u}, -SR^{u}, -CONR^{u}R^{v}, -COOR^{h}, -SOR^{u}, -SO₂R^{u}, -SO₂NR^{u}R^{v}, -NR^{u}SO₂R^{v}, -COR^{u}, - NR^{u}COR^{v}, -OCOR^{u}, -OCONR^{u}R^{v}, -NR^{u}CO₂R^{v}, -NR^{u}CONR^{v}R^{w}, -OCO₂R^{v}, gerad- oder verzweigtkettiges -C₁₋₆-Alkyl, unsubstituiert oder substituiert mit ein bis vier Rᵢ-Gruppen, bedeutet,
R^{u} und R^{v} Wasserstoff oder gerad- oder verzweigtkettiges -C₁₋₆-Alkyl bedeuten, unsubstituiert oder substituiert mit ein bis vier Rⁱ-Gruppen,
oder R^{u} und R^{v}, zusammen mit etwaigen dazwischenliegenden Atomen, einen 4-6gliedrigen gesättigten Ring bedeuten, der gegebenenfalls durch ein oder mehrere O, S, NR^{w} oder -C(O)- unterbrochen ist, wobei der Ring unsubstituiert oder mit ein bis vier Rⁱ-Gruppen substituiert ist,
jedes R^{w} unabhängig Wasserstoff, gerad- oder verzweigtkettiges -C₁₋₆-Alkyl, unsubstituiert oder substituiert mit ein bis vier Rⁱ-Gruppen, C₃₋₆-Cycloalkyl, gegebenenfalls substituiert mit ein bis vier Rⁱ-Gruppen, Phenyl, gegebenenfalls substituiert mit ein bis vier Rⁱ-Gruppen, oder Heteroaryl, gegebenenfalls substituiert mit 1-4 Rⁱ-Gruppen, bedeutet
oder R^{h} und R^{w}, zusammengefaßt mit etwaigen dazwischenliegenden Atomen, einen 5-6gliedrigen gesättigten Ring bedeuten, gegebenenfalls unterbrochen durch ein oder zwei O, S, SO₂, NH oder NCH₃,
R^{x} Wasserstoff oder ein gerad- oder verzweigtkettiges C₁₋₈-Alkyl bedeutet, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei geradoder verzweigtkettigen C₁₋₃-Alkylgruppen substituiert ist, wobei die Alkylgruppen unsubstituiert oder mit ein bis vier Rⁱ-Gruppen substituiert sind,
R^{y} und R^{z} Wasserstoff, Phenyl, gerad- oder verzweigtkettiges -C₁₋₆-Alkyl, unsubstituiert oder substituiert mit ein bis vier Rⁱ-Gruppen und gegebenenfalls unterbrochen durch O, S, NR^{w}, N⁺R^{h}R^{w} oder -C(O)-, bedeuten
oder R^{x} und R^{y}, zusammen mit etwaigen dazwischenliegenden Atomen, einen 4-6gliedrigen gesättigten Ring bedeuten, der gegebenenfalls durch O, S, SO₂, NR^{w}, N⁺R^{h}R^{w} oder -C(O)- unterbrochen ist, unsubstituiert oder substituiert mit 1-4 Rⁱ-Gruppen,
und wenn R^{x} und R^{y} zusammen einen wie oben definierten 4-6gliedrigen Ring bedeuten, R^{z} wie oben definiert ist oder R^{z} einen zusätzlichen gesättigten 4-6gliedrigen Ring bedeutet, der an den Ring kondensiert ist, der durch die zusammengefaßten Reste R^{x} und R^{y} dargestellt wird, gegebenenfalls unterbrochen durch O, S, NR^{w} oder -C(O)-, wobei die Ringe unsubstituiert oder mit ein bis vier Rⁱ-Gruppen substituiert sind.

2. Eine Verbindung gemäß Anspruch 1, wobei CO₂M ein Carboxylatanion bedeutet.

3. Eine Verbindung gemäß Anspruch 1, wobei 1 R eine Gruppe bedeutet, die einen positiv geladenen Rest enthält, und die restlichen R-Gruppen ausgewählt sind aus Wasserstoff und gerad- oder verzweigtkettigem C₁₋₆-Alkyl, unsubstituiert oder substituiert mit ein bis vier R^{d}-Gruppen.

4. Eine Verbindung gemäß Anspruch 3, wobei 1 R eine Gruppe bedeutet, die einen positiv geladenen Rest enthält, und die restlichen R-Gruppen Wasserstoff sind.

5. Eine Verbindung gemäß Anspruch 1, wobei die R-Gruppen 1-3 positive Ladungen enthalten.

6. Eine Verbindung gemäß Anspruch 5, wobei die R-Gruppen zwei positive Ladungen enthalten, ausgeglichen mit einem Carboxylatanion und einem negativ geladenen Gegenion.

7. Eine Verbindung gemäß Anspruch 1, wobei 1 R-Gruppe eine geradoder verzweigtkettige -C₁₋₆-Alkylgruppe bedeutet, substituiert mit ein bis vier R^{d}-Gruppen, wobei 1 R^{d}-Gruppe -R* oder Q bedeutet.

8. Eine Verbindung gemäß Anspruch 1, wobei Q ausgewählt ist aus der Gruppe, bestehend aus:

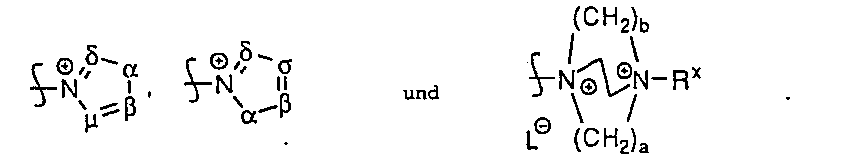

9. Eine Verbindung gemäß Anspruch 8, wobei Q bedeutet: 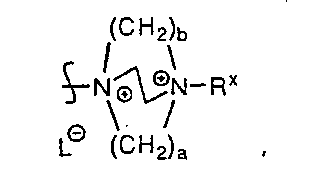
L⁻, a und b wie ursprünglich definiert sind und R^{x} ein Element bedeutet, ausgewählt aus der Gruppe, bestehend aus: Wasserstoff oder einem gerad- oder verzweigtkettigen C₁₋₈-Alkyl, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei gerad- oder verzweigtkettigen C₁₋₃-Alkylgruppen substituiert ist, wobei die Alkylgruppen unsubstituiert oder mit ein bis vier Rⁱ-Gruppen substituiert sind, und
R^{h}, Rⁱ und R^{w} wie ursprünglich definiert sind.

10. Eine Verbindung gemäß Anspruch 1, wobei Q -N⁺R^{x}R^{y}R^{z} bedeutet, wobei R^{x}, R^{y} und R^{z} wie ursprünglich definiert sind.

11. Eine Verbindung gemäß Anspruch 1, wobei 1 R*-Gruppe vorliegt und ausgewählt ist aus: 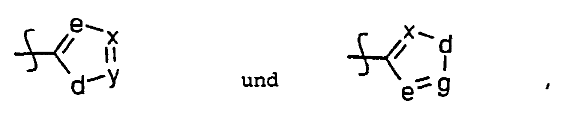 d NR^{k} bedeutet, R^{k} gerad- oder verzweigtkettiges -C₁₋₆-Alkyl bedeutet und e, g, x und y CR^{m} oder N⁺R^{k} bedeuten, wobei R^{k} wie oben definiert ist und R^{m} Wasserstoff bedeutet.

12. Eine Verbindung gemäß Anspruch 1, wobei:
CO₂M ein Carboxylatanion bedeutet,
1 R-Gruppe, die an die Naphthosultam-Plattform gebunden ist, wenigstens 1 positiv geladenen Rest enthält und die restlichen R-Gruppen ausgewählt sind aus Wasserstoff und gerad- oder verzweigtkettigem C₁₋₆-Alkyl, unsubstituiert oder substituiert mit ein bis vier R^{d}-Gruppen,
R^{d} wie ursprünglich definiert ist,
R^{h} Wasserstoff oder eine gerad- oder verzweigtkettige C₁₋₆-Alkylgruppe bedeutet,
Q ausgewählt ist aus der Gruppe, bestehend aus: 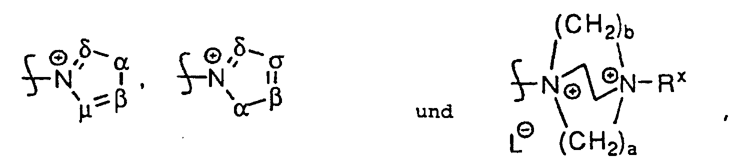 wobei L⁻ wie ursprünglich definiert ist, a und b 2 bedeuten und R^{x} ein Element bedeutet, ausgewählt aus der Gruppe, bestehend aus: Wasserstoff oder einem gerad- oder verzweigtkettigen C₁₋₈-Alkyl, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei gerad- oder verzweigtkettigen C₁₋₃-Alkylgruppen substituiert ist, wobei die Alkylgruppen unsubstituiert oder mit ein bis vier Rⁱ-Gruppen substituiert sind,
R* ausgewählt ist aus: 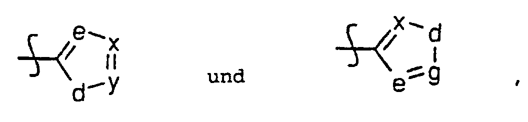
wobei d NR^{k} bedeutet, R^{k} gerad- oder verzweigtkettiges -C₁₋₆-Alkyl bedeutet und e, g, x und y CR^{m} oder N⁺R^{k} bedeuten, wobei R^{k} wie oben definiert ist und R^{m} Wasserstoff bedeutet.

13. Eine Verbindung gemäß Anspruch 1, dargestellt durch Formel Ia: 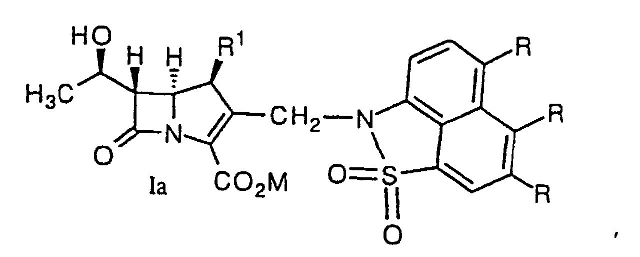 oder ein pharmazeutisch annehmbares Salz davon, wobei:
CO₂M ein Carboxylatanion bedeutet,
1 R einen positiv geladenen Rest enthält und die anderen R-Gruppen ausgewählt sind aus Wasserstoff und gerad- oder verzweigtkettigem C₁₋₆-Alkyl, unsubstituiert oder substituiert mit ein bis vier R^{d}-Gruppen,
R^{d} wie ursprünglich definiert ist,
Q ausgewählt ist aus der Gruppe, bestehend aus: 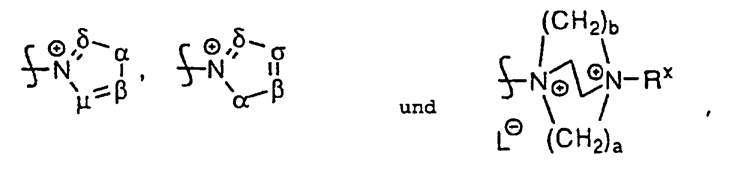 wobei L⁻, a und b wie ursprünglich definiert sind und R^{x} ein Element bedeutet, ausgewählt aus der Gruppe, bestehend aus: Wasserstoff oder einem gerad- oder verzweigtkettigen C₁₋₈-Alkyl, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei gerad- oder verzweigtkettigen C₁₋₃-Alkylgruppen substituiert ist, wobei die Alkylgruppen unsubstituiert oder mit ein bis vier Rⁱ-Gruppen substituiert sind,
R^{h} Wasserstoff oder eine gerad- oder verzweigtkettige C₁₋₆-Alkylgruppe bedeutet,
R^{w} wie ursprünglich definiert ist,
R* ausgewählt ist aus: 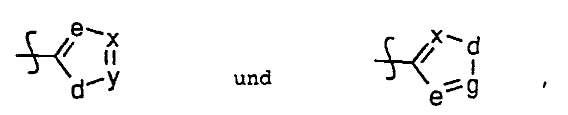
wobei d NR^{k} bedeutet, R^{k} gerad- oder verzweigtkettiges -C₁₋₆-Alkyl bedeutet und e, g, x und y CR^{m} oder N⁺R^{k} bedeuten, wobei R^{k} wie oben definiert ist und R^{m} Wasserstoff bedeutet.

14. Eine Verbindung gemäß Anspruch 1, dargestellt durch Formel Ib: 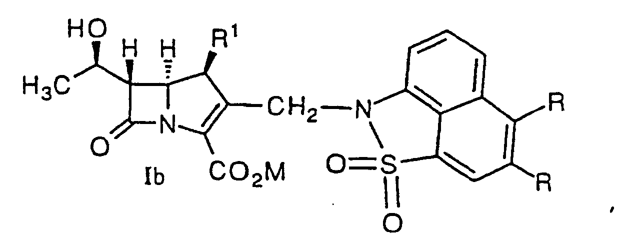 oder ein pharmazeutisch annehmbares Salz davon, wobei:
CO₂M ein Carboxylatanion bedeutet,
1 R-Gruppe an die Naphthosultam-Plattform gebunden ist, die einen positiv geladenen Rest enthält, und die andere R-Gruppe ausgewählt ist aus Wasserstoff und gerad- oder verzweigtkettigem C₁₋₆-Alkyl, unsubstituiert oder substituiert mit ein bis vier R^{d}-Gruppen,
R^{d} wie ursprünglich definiert ist,
Q ausgewählt ist aus der Gruppe, bestehend aus: 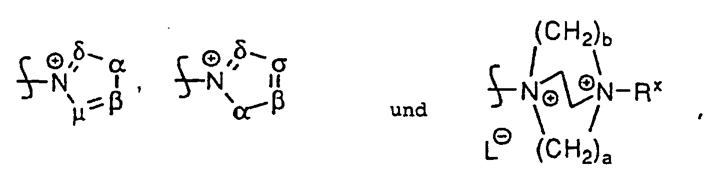 wobei L⁻, a und b wie ursprünglich definiert sind und R^{x} ein Element bedeutet, ausgewählt aus der Gruppe, bestehend aus: Wasserstoff oder einem gerad- oder verzweigtkettigen C₁₋₈-Alkyl, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei gerad- oder verzweigtkettigen C₁₋₃-Alkylgruppen substituiert ist, wobei die Alkylgruppen unsubstituiert oder mit ein bis vier Rⁱ-Gruppen substituiert sind,
R^{h} Wasserstoff oder eine gerad- oder verzweigtkettige C₁₋₆-Alkylgruppe bedeutet,
R^{w} wie ursprünglich definiert ist,
R* ausgewählt ist aus: 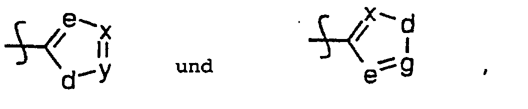
wobei d NR^{k} bedeutet, R^{k} gerad- oder verzweigtkettiges -C₁₋₆-Alkyl bedeutet und e, g, x und y CR^{m} oder N⁺R^{k} bedeuten, wobei R^{k} wie oben definiert ist und R^{m} Wasserstoff bedeutet. Innerhalb dieser Unterklasse sind alle anderen Variablen wie ursprünglich in Bezug auf Formel I definiert.

15. Eine Verbindung gemäß Anspruch 1, dargestellt durch Formel Ic: 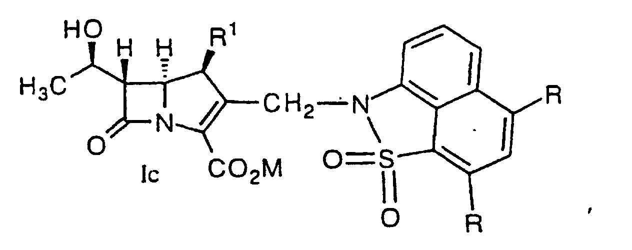 oder ein pharmazeutisch annehmbares Salz davon, wobei:
CO₂M ein Carboxylatanion bedeutet,
1 R-Gruppe an die Naphthosultam-Plattform gebunden ist, die einen positiv geladenen Rest enthält, und die andere R-Gruppe ausgewählt ist aus Wasserstoff, Halogen und gerad- oder verzweigtkettigem C₁₋₆-Alkyl, unsubstituiert oder substituiert mit ein bis vier R^{d}-Gruppen,
R^{d} wie ursprünglich definiert ist,
Q ausgewählt ist aus der Gruppe, bestehend aus: 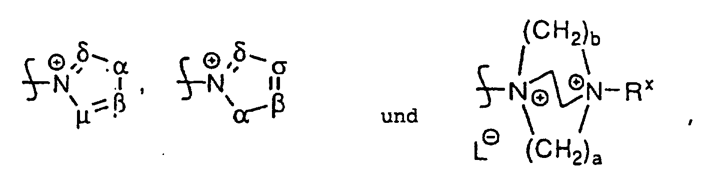 wobei L⁻, a und b wie ursprünglich definiert sind und R^{x} ein Element bedeutet, ausgewählt aus der Gruppe, bestehend aus: Wasserstoff oder einem gerad- oder verzweigtkettigen C₁₋₈-Alkyl, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei gerad- oder verzweigtkettigen C₁₋₃-Alkylgruppen substituiert ist, wobei die Alkylgruppen unsubstituiert oder mit ein bis vier Rⁱ-Gruppen substituiert sind,
R^{h} Wasserstoff oder eine gerad- oder verzweigtkettige C₁₋₆-Alkylgruppe bedeutet,
R^{w} wie ursprünglich definiert ist,
R* ausgewählt ist aus: 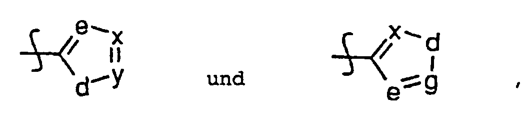
wobei d NR^{k} bedeutet, R^{k} gerad- oder verzweigtkettiges -C₁₋₆-Alkyl bedeutet und e, g, x und y CR^{m} oder N⁺R^{k} bedeuten, wobei R^{k} wie oben definiert ist und R^{m} Wasserstoff bedeutet.

16. Eine Verbindung gemäß Anspruch 1, dargestellt durch Formel Id: 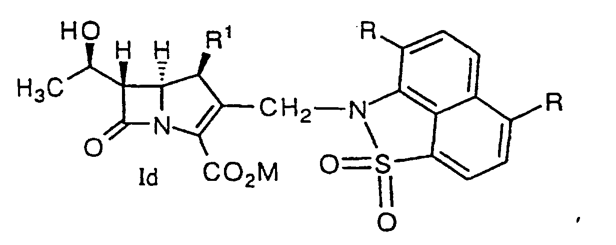 oder ein pharmazeutisch annehmbares Salz davon, wobei:
CO₂M ein Carboxylatanion bedeutet,
1 R-Gruppe an die Naphthosultam-Plattform gebunden ist, die einen positiv geladenen Rest enthält, und die andere R-Gruppe ausgewählt ist aus Wasserstoff, Halogen und gerad- oder verzweigtkettigem C₁₋₆-Alkyl, unsubstituiert oder substituiert mit ein bis vier R^{d}-Gruppen,
R^{d} wie ursprünglich definiert ist,
Q ausgewählt ist aus der Gruppe, bestehend aus: 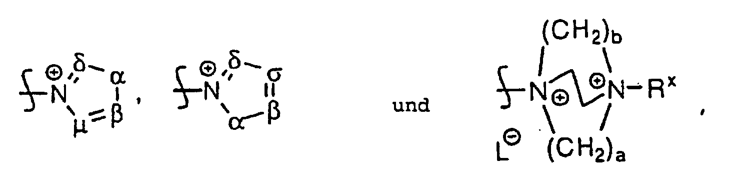 wobei L⁻, a und b wie ursprünglich definiert sind und R^{x} ein Element bedeutet, ausgewählt aus der Gruppe, bestehend aus: Wasserstoff oder einem gerad- oder verzweigtkettigen C₁₋₈-Alkyl, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei gerad- oder verzweigtkettigen C₁₋₃-Alkylgruppen substituiert ist, wobei die Alkylgruppen unsubstituiert oder mit ein bis vier Rⁱ-Gruppen substituiert sind,
R^{h} Wasserstoff oder eine gerad- oder verzweigtkettige C₁₋₆-Alkylgruppe bedeutet,
R^{w} wie ursprünglich definiert ist,
R* ausgewählt ist aus: 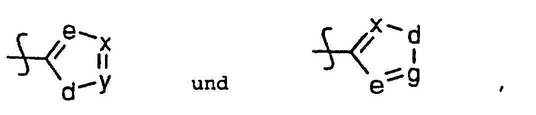
wobei d NR^{k} bedeutet, R^{k} gerad- oder verzweigtkettiges -C₁₋₆-Alkyl bedeutet und e, g, x und y CR^{m} oder N⁺R^{k} bedeuten, wobei R^{k} wie oben definiert ist und R^{m} Wasserstoff bedeutet.

17. Eine Verbindung gemäß Anspruch 1, dargestellt durch Formel Ie: 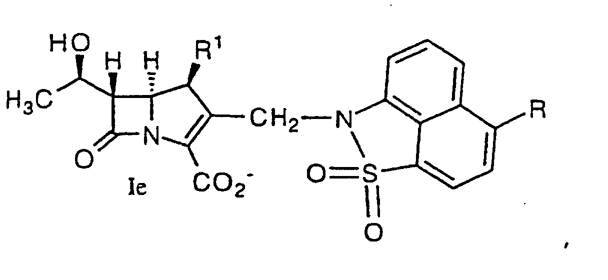 oder ein pharmazeutisch annehmbares Salz davon, wobei:
R einen positiv geladenen Rest enthält, ausgewählt aus der Gruppe, bestehend aus: -R*, Q und einem mit 1 R^{d}-Gruppe substituierten gerad- oder verzweigtkettigen C₁₋₆-Alkyl,
R^{d} unabhängig ausgewählt ist aus -R* oder Q,
Q ausgewählt ist aus der Gruppe, bestehend aus: 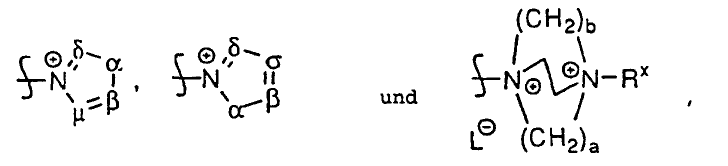 wobei L⁻, a und b wie ursprünglich definiert sind und R^{x} ein Element bedeutet, ausgewählt aus der Gruppe, bestehend aus: Wasserstoff oder einem gerad- oder verzweigtkettigen C₁₋₈-Alkyl, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei gerad- oder verzweigtkettigen C₁₋₃-Alkylgruppen substituiert ist, wobei die Alkylgruppen unsubstituiert oder mit ein bis vier Rⁱ-Gruppen substituiert sind,
R* ausgewählt ist aus: 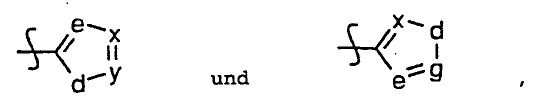
wobei d NR^{k} bedeutet, R^{k} gerad- oder verzweigtkettiges -C₁₋₆-Alkyl bedeutet und e, g, x und y CR^{m} oder N⁺R^{k} bedeuten, wobei R^{k} wie oben definiert ist und R^{m} Wasserstoff bedeutet.

18. Eine Verbindung gemäß Anspruch 1, dargestellt durch Formel If: 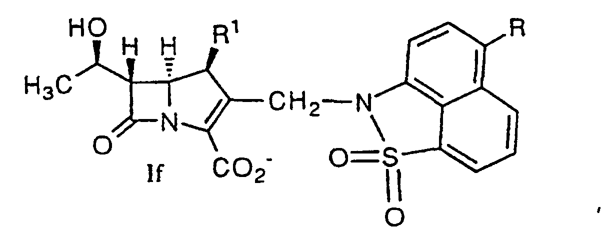 oder ein pharmazeutisch annehmbares Salz davon, wobei:
R einen positiv geladenen Rest enthält, ausgewählt aus der Gruppe, bestehend aus: -R*, Q und einem mit 1 R^{d}-Gruppe substituierten gerad- oder verzweigtkettigen C₁₋₆-Alkyl,
R^{d} unabhängig ausgewählt ist aus -R* oder Q,
Q ausgewählt ist aus der Gruppe, bestehend aus: 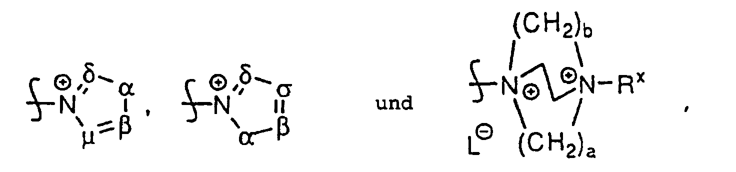 wobei L⁻, a und b wie ursprünglich definiert sind und R^{x} ein Element bedeutet, ausgewählt aus der Gruppe, bestehend aus: Wasserstoff oder einem gerad- oder verzweigtkettigen C₁₋₈-Alkyl, gegebenenfalls unterbrochen durch ein oder zwei O, S, SO, SO₂, NR^{w}, N⁺R^{h}R^{w} oder -C(O)-, wobei die Kette unsubstituiert oder substituiert ist mit ein bis vier Halogen, CN, NO₂, OR^{w}, SR^{w}, SOR^{w}, SO₂R^{w}, NR^{h}R^{w}, N⁺(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w} oder einer Phenyl- oder Heteroarylgruppe, die ihrerseits gegebenenfalls mit ein bis vier Rⁱ-Gruppen oder mit ein bis zwei gerad- oder verzweigtkettigen C₁₋₃-Alkylgruppen substituiert ist, wobei die Alkylgruppen unsubstituiert oder mit ein bis vier Rⁱ-Gruppen substituiert sind,
R* ausgewählt ist aus: 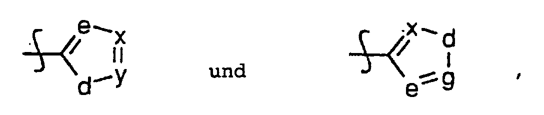
wobei d NR^{k} bedeutet, R^{k} gerad- oder verzweigtkettiges -C₁₋₆-Alkyl bedeutet und e, g, x und y CR^{m} oder N⁺R^{k} bedeuten, wobei R^{k} wie oben definiert ist und R^{m} Wasserstoff bedeutet.

19. Eine Verbindung gemäß Anspruch 17, wobei:
R 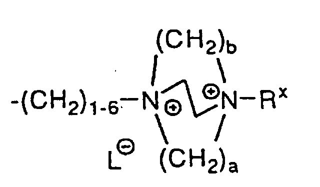 bedeutet
und R^{x}, a, b und L⁻ wie ursprünglich definiert sind.

20. Eine Verbindung gemäß Anspruch 1, dargestellt durch Formel Ig: 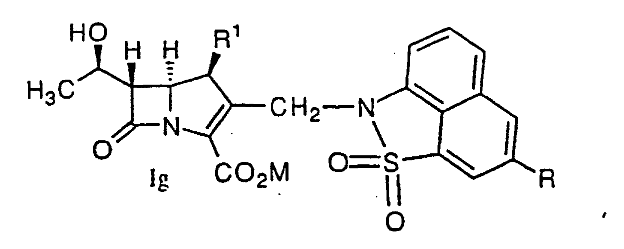 wobei:
R 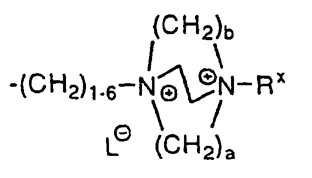 bedeutet
und R^{x}, a, b und L⁻ wie ursprünglich definiert sind.

21. Eine Verbindung gemäß Anspruch 1, dargestellt durch die Strukturformel: 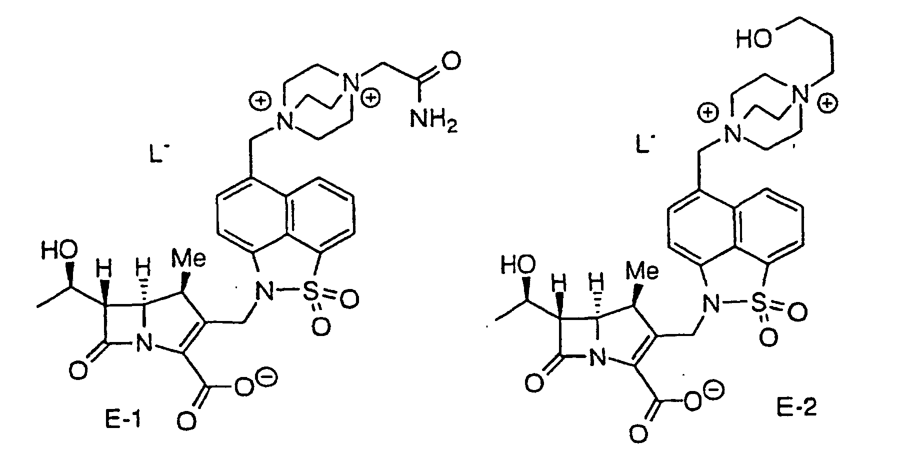 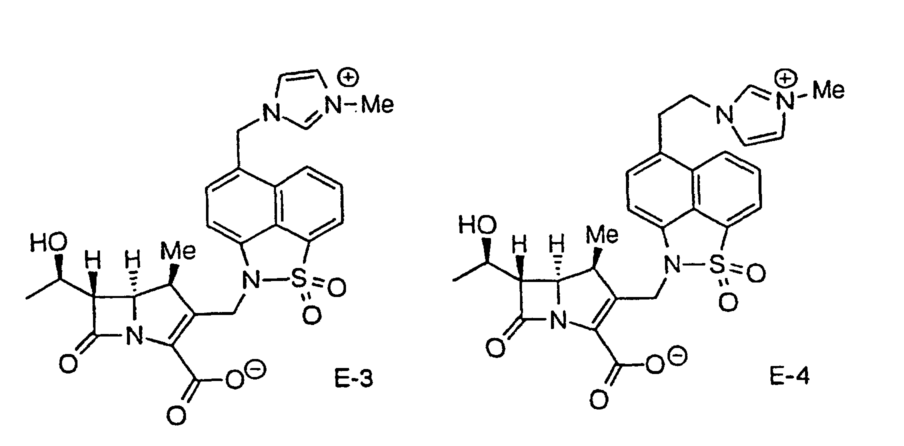 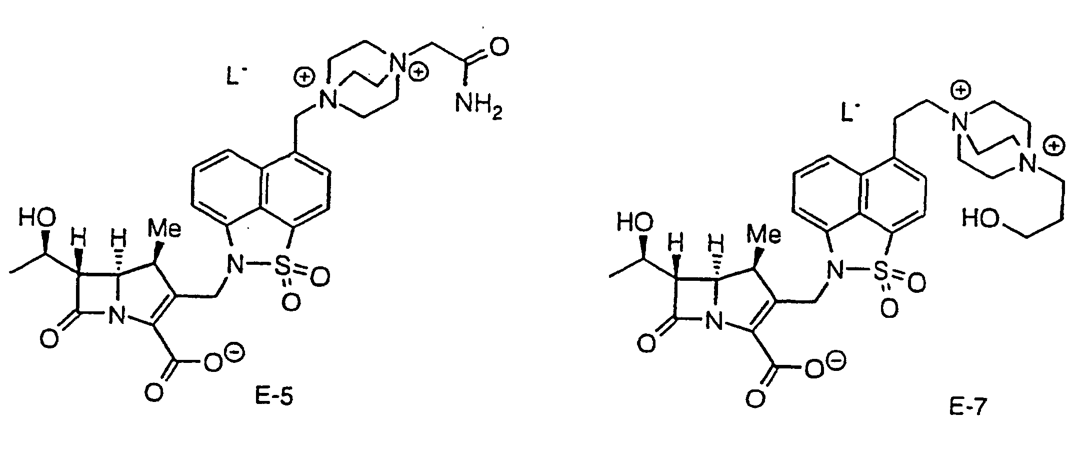 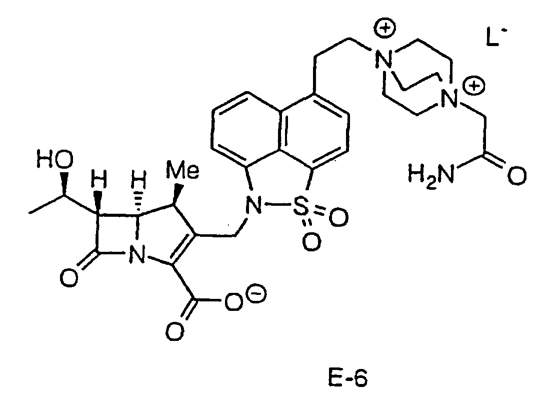 oder 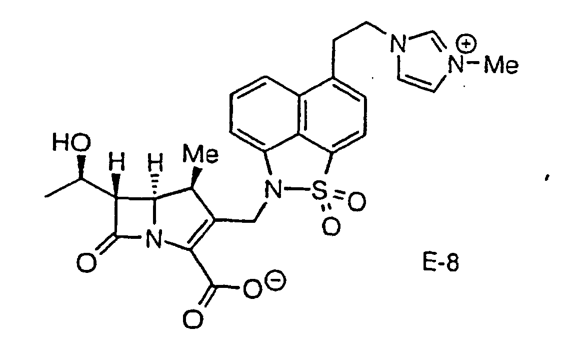 wobei L⁻ ein pharmazeutisch annehmbares Gegenion ist.

22. Eine Verbindung gemäß Anspruch 1, dargestellt durch die Strukturformel: 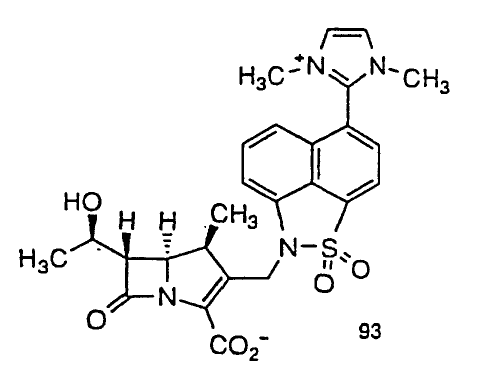 oder

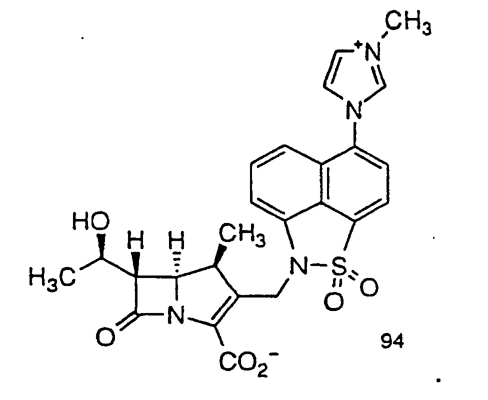

23. Eine durch die Struktur: 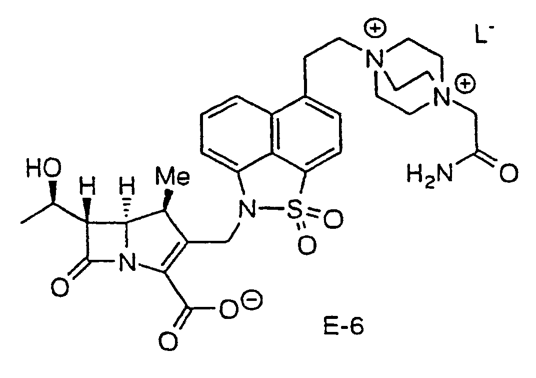 dargestellte Verbindung,
wobei L⁻ ein pharmazeutisch annehmbares Kation bedeutet.

24. Eine Verbindung gemäß Anspruch 1, die in eine der folgenden Tabellen paßt: 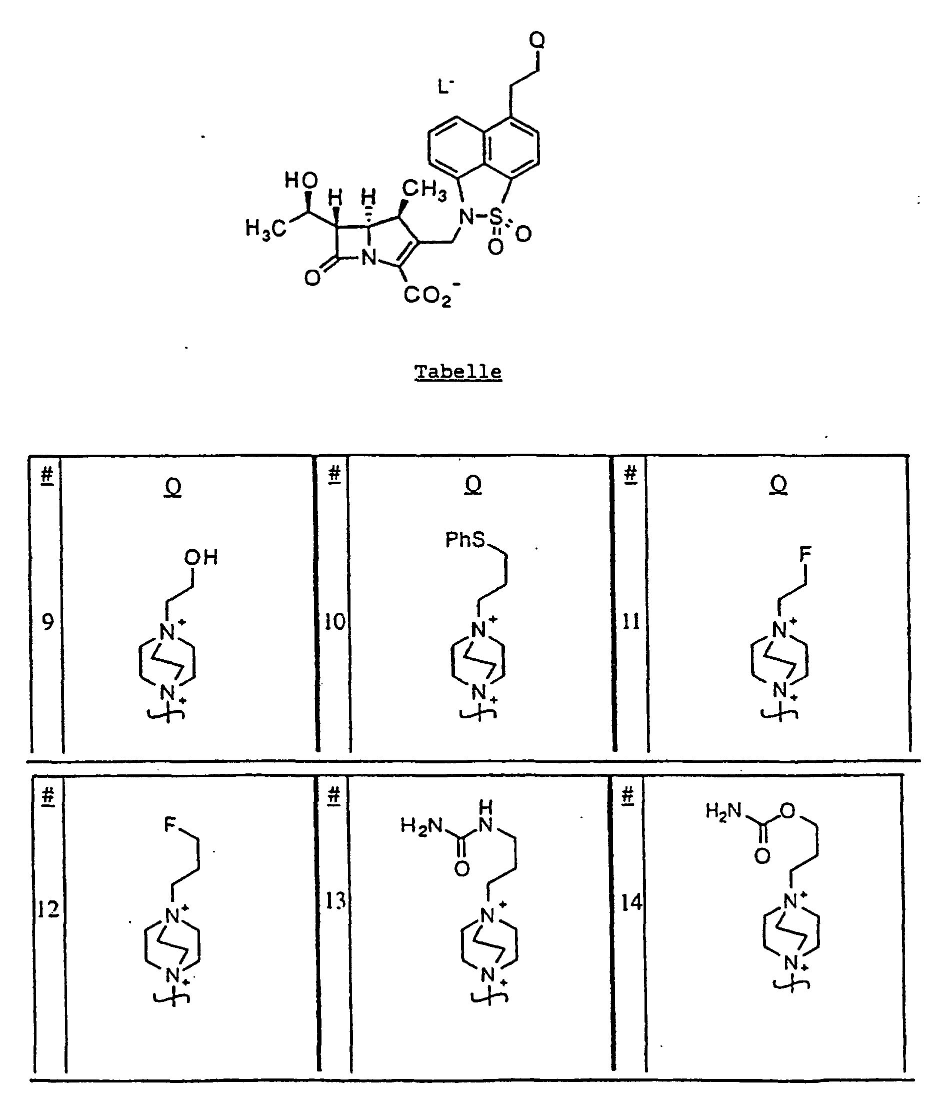 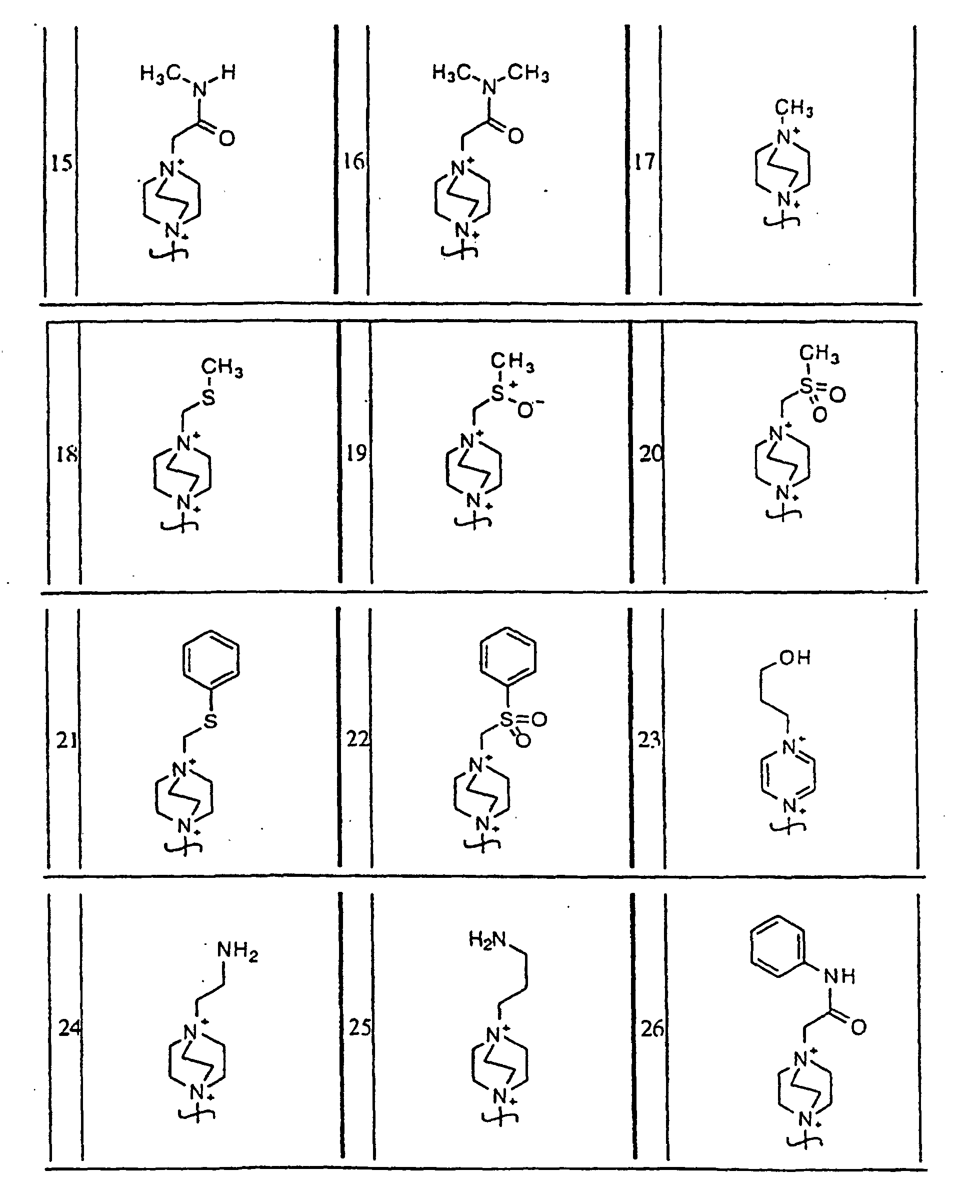 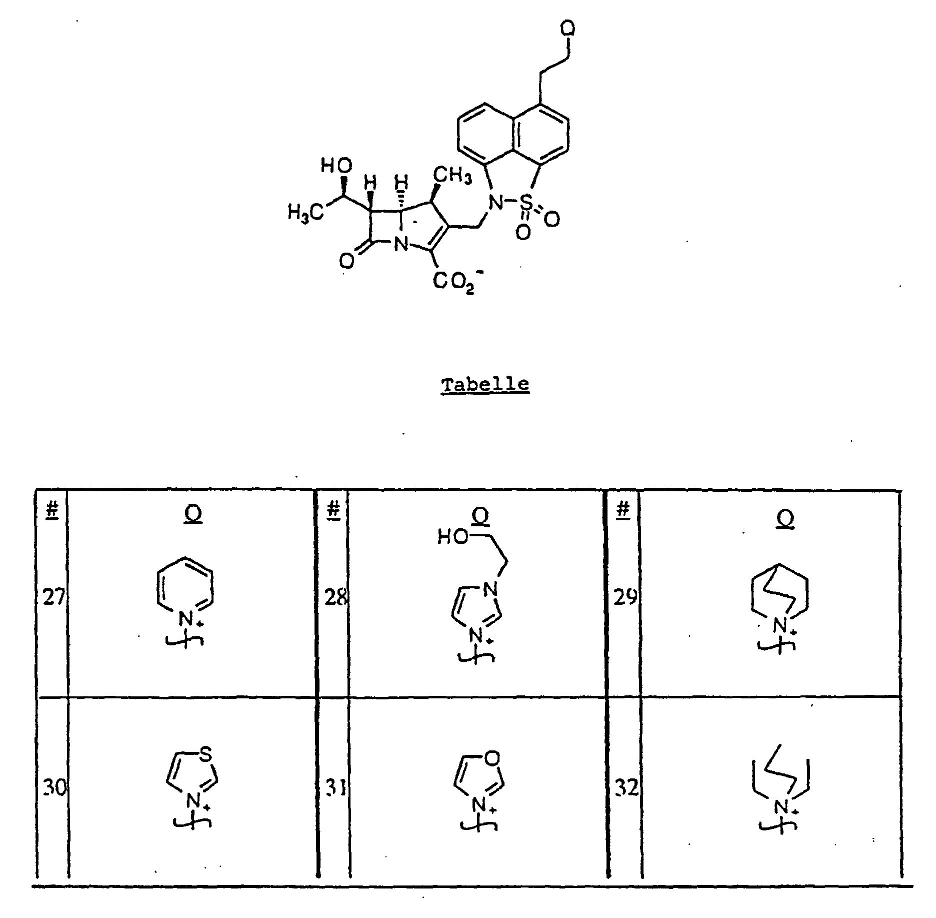 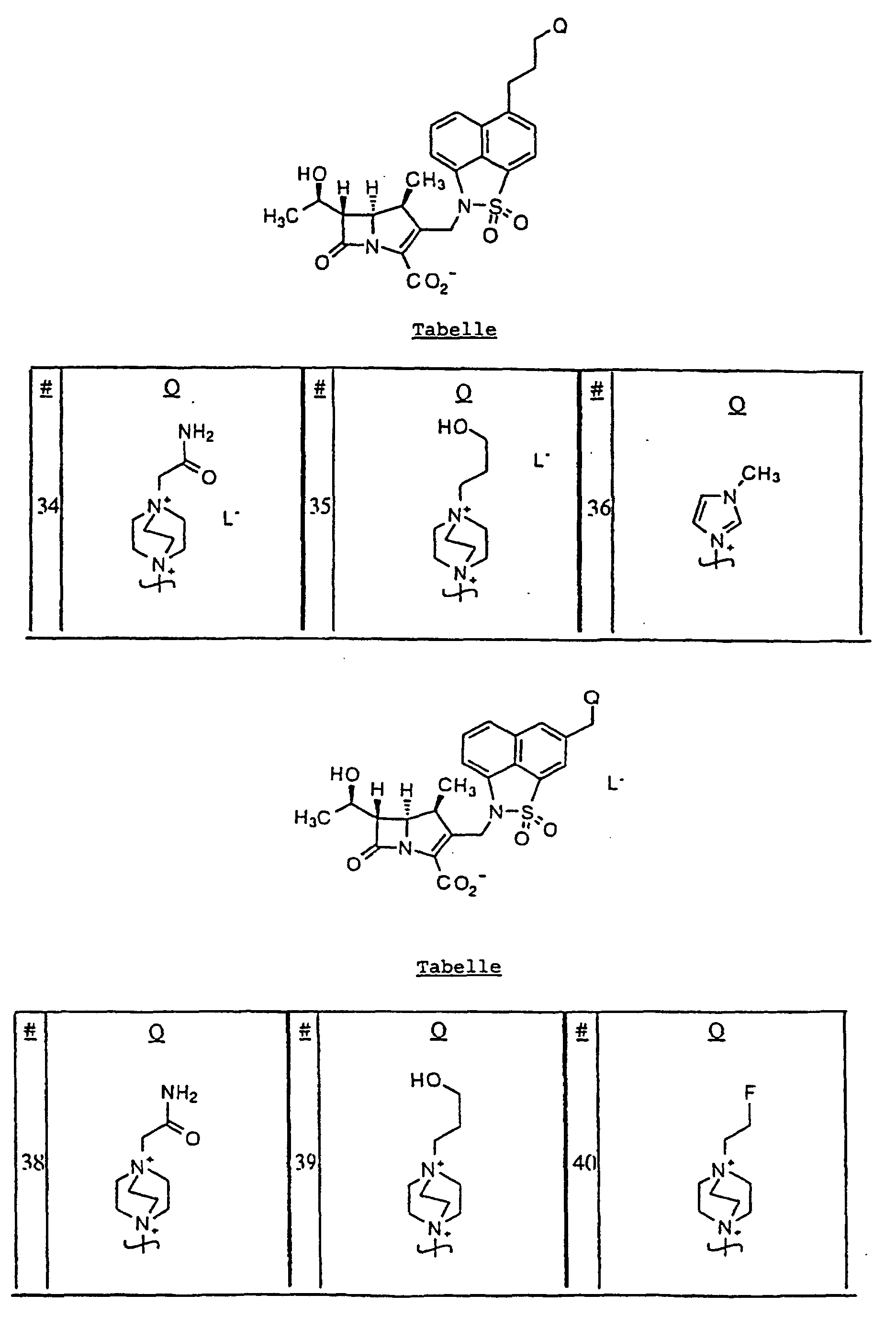 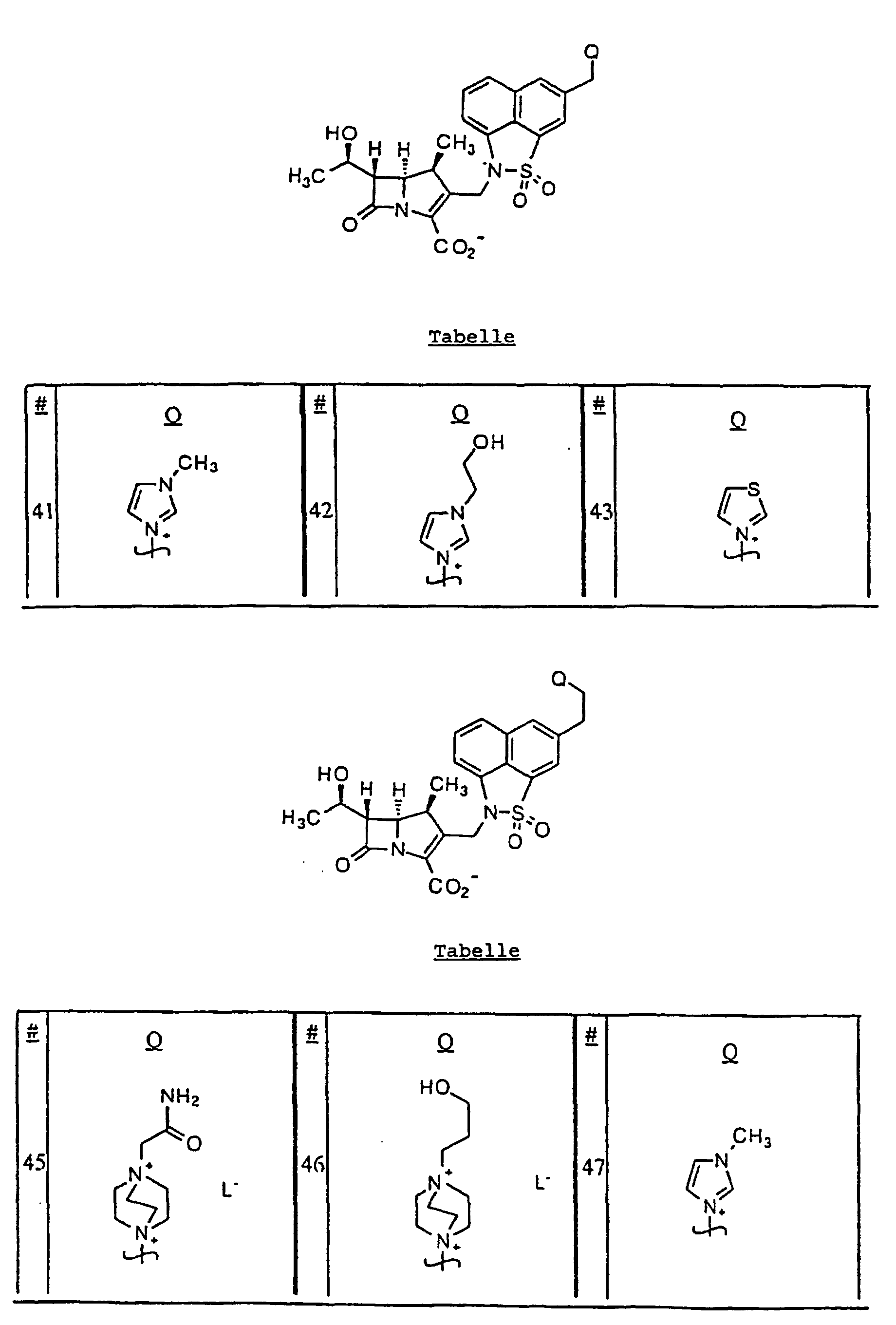 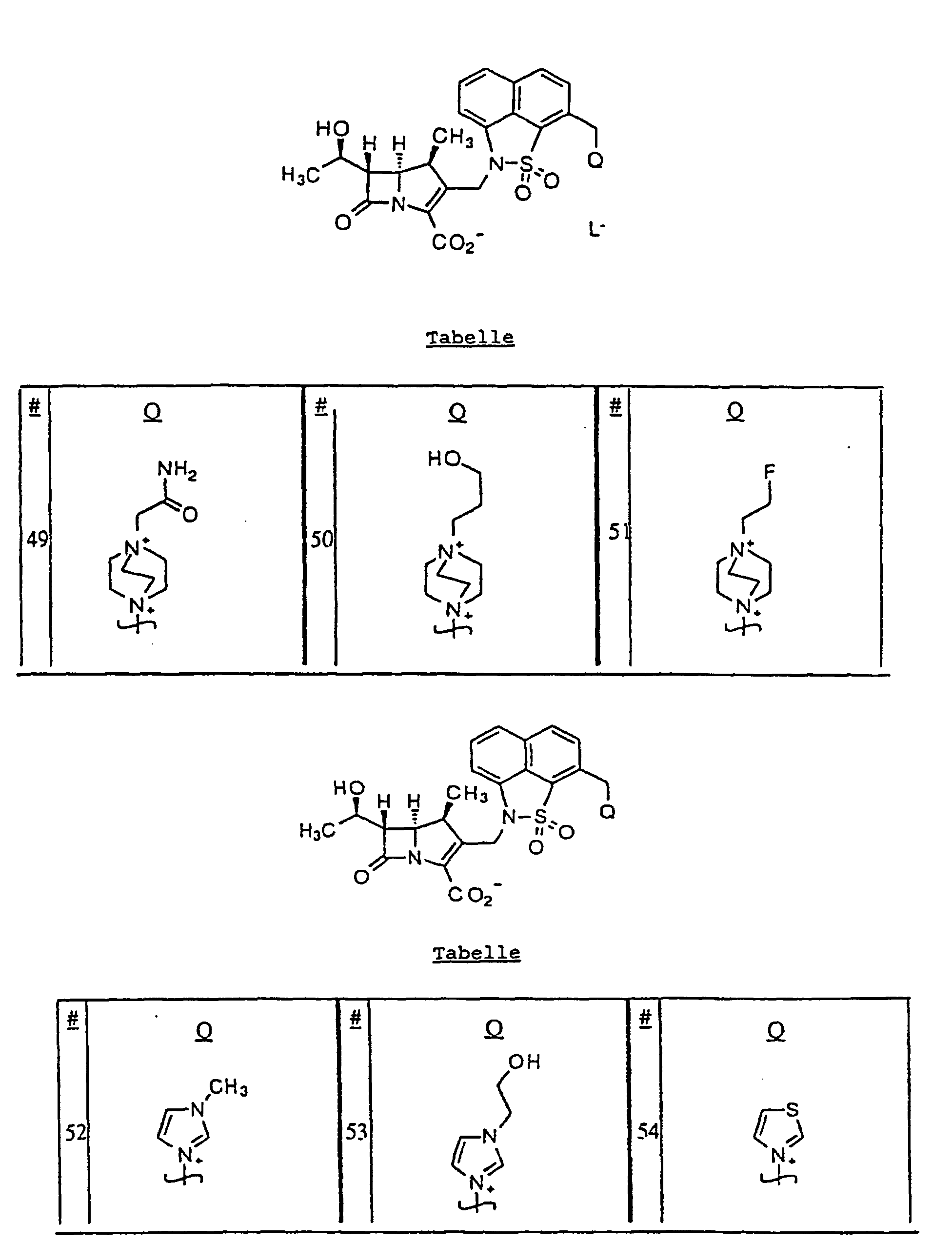 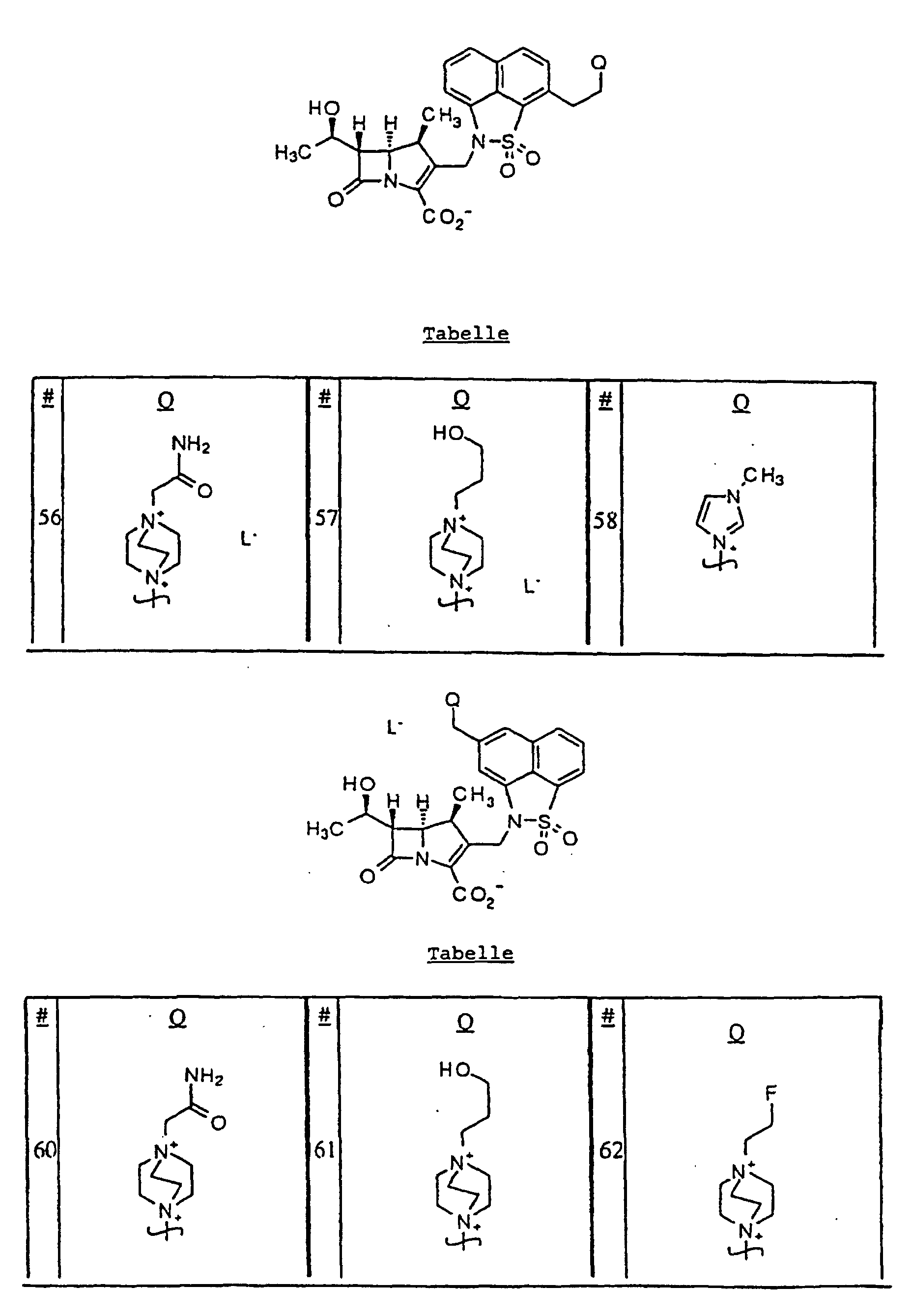 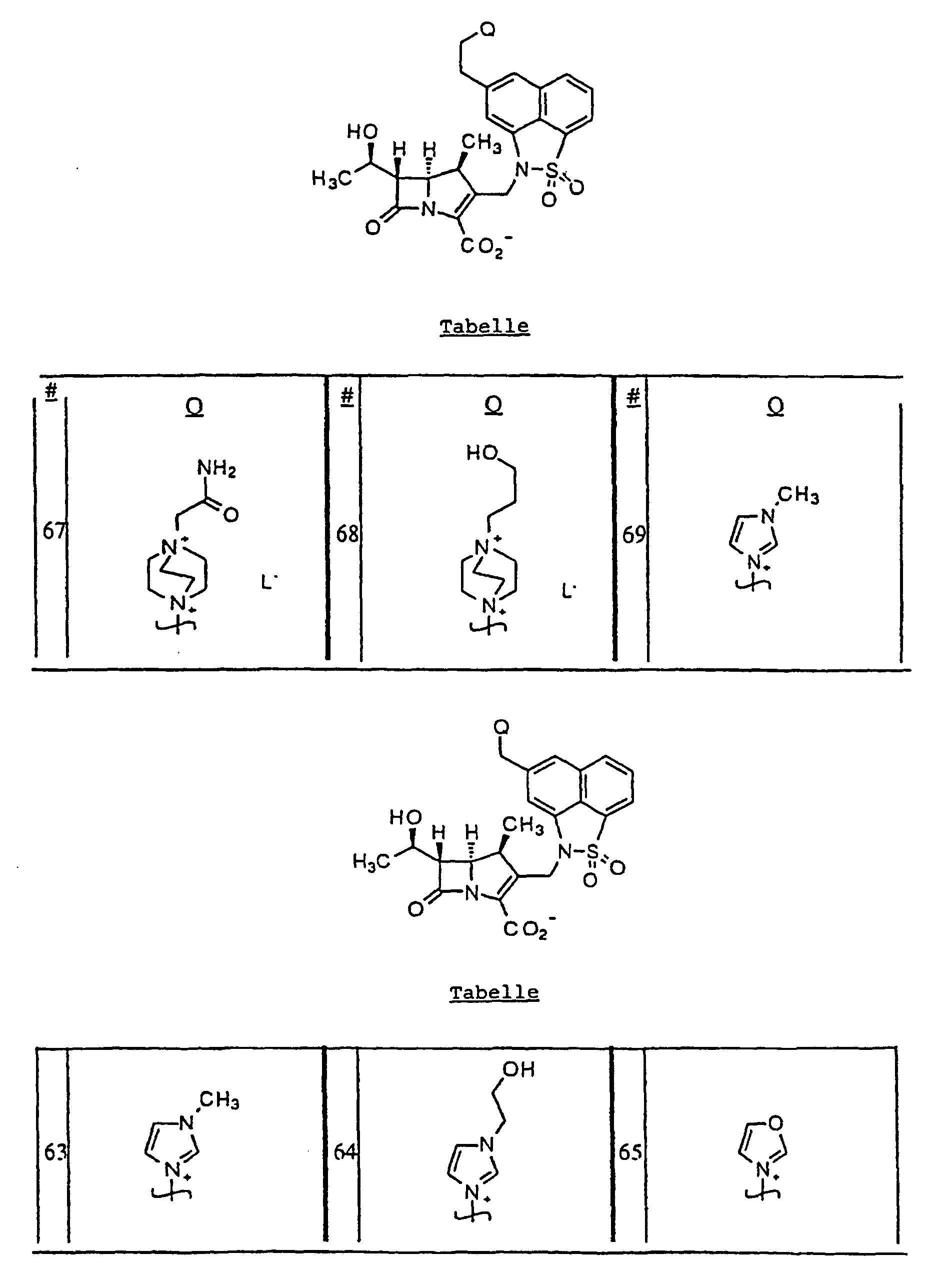 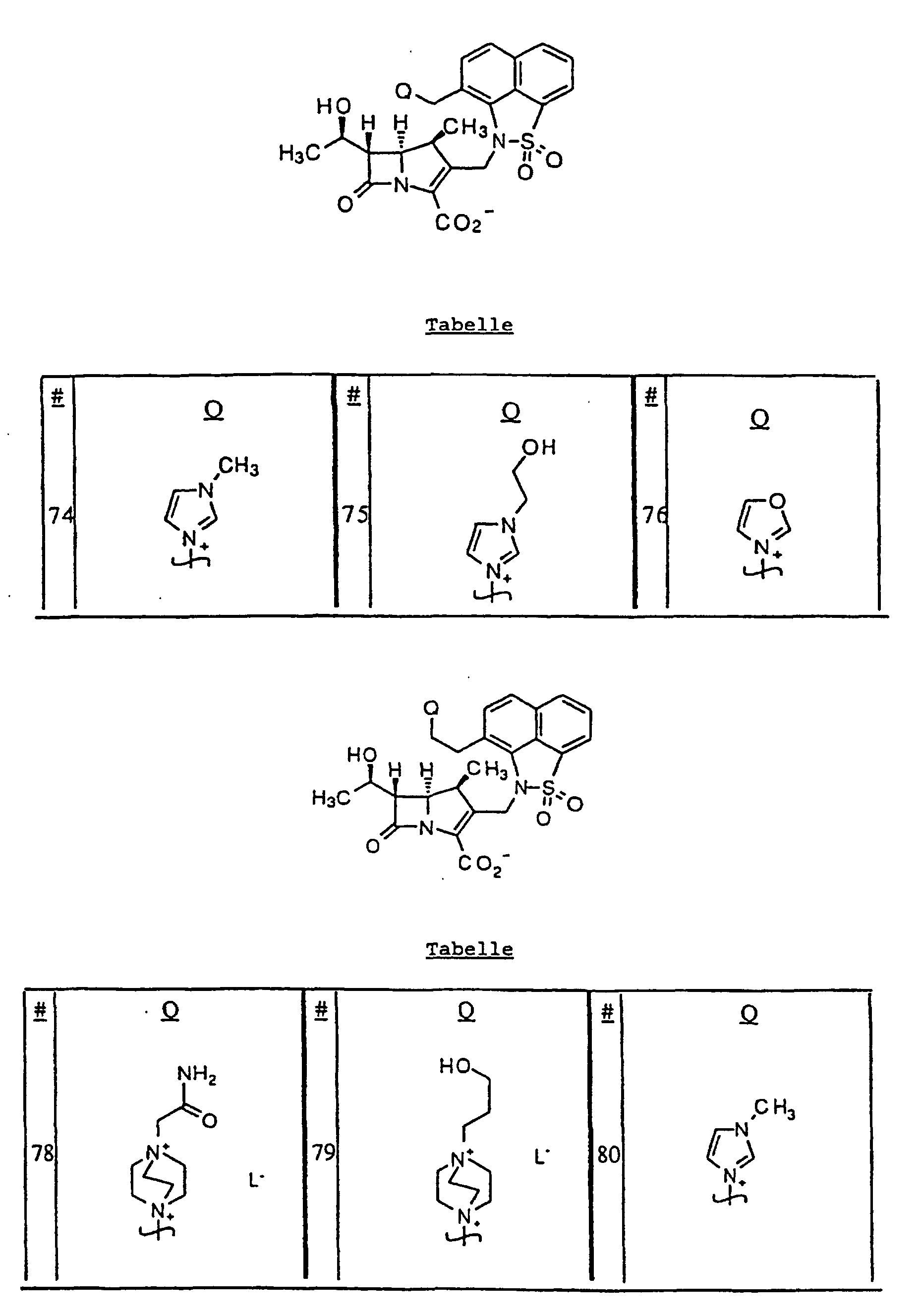 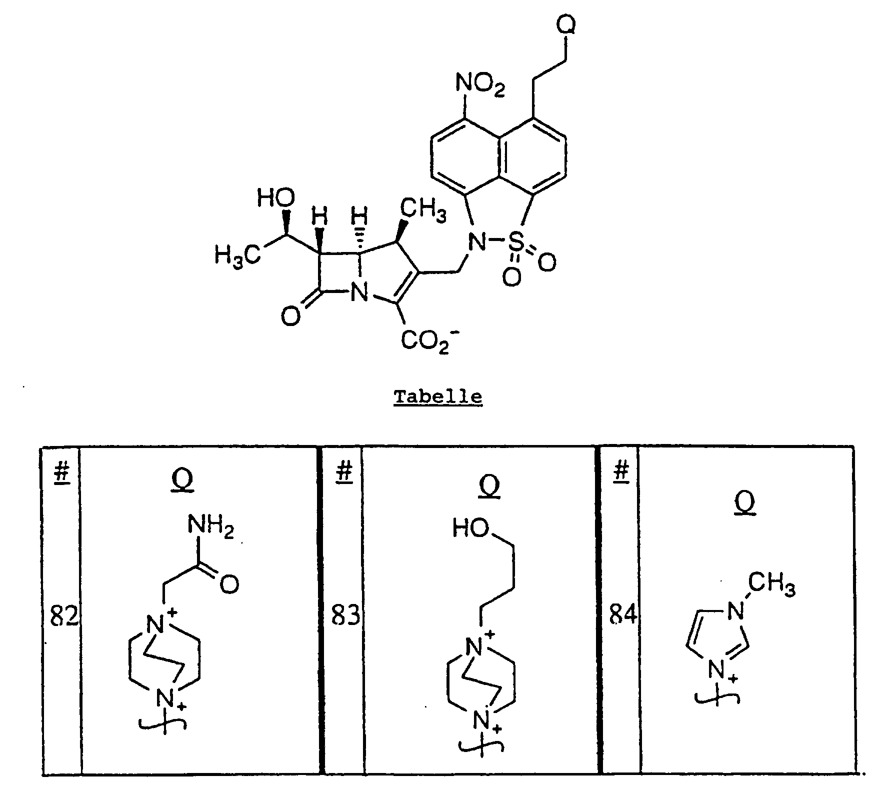 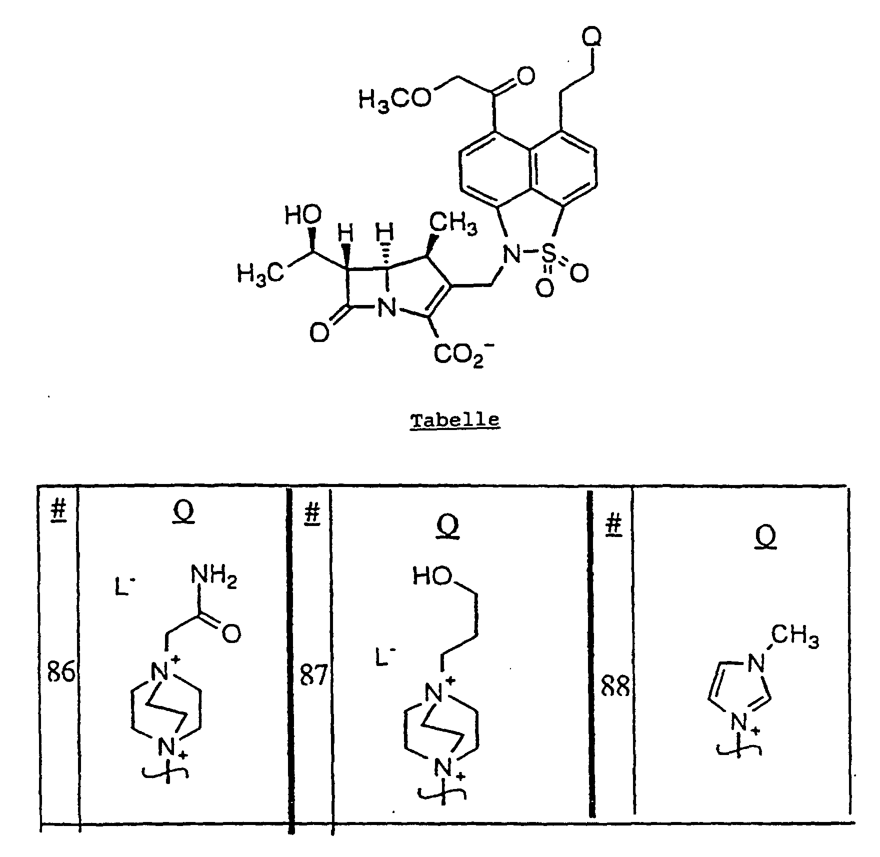 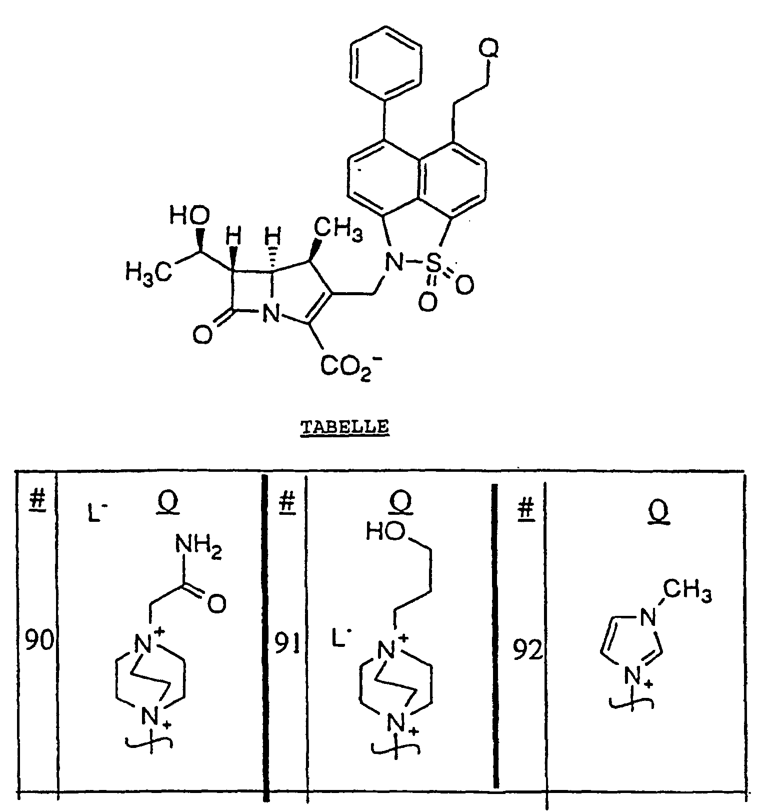 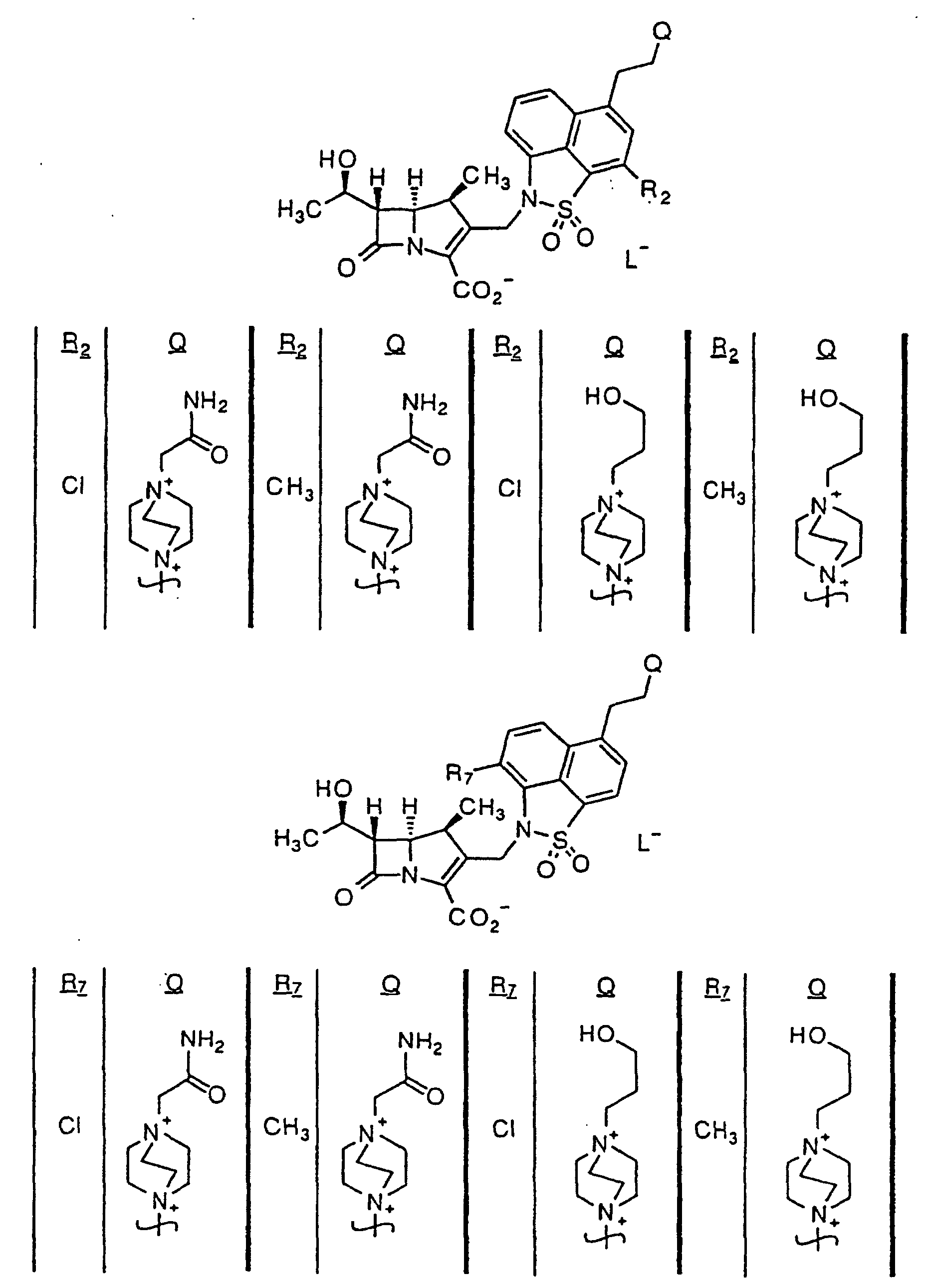 wobei L⁻ ein pharmazeutisch annehmbares Gegenion bedeutet.

25. Eine pharmazeutische Zusammensetzung, die eine Verbindung gemäß irgendeinem der Ansprüche 1 bis 24 in Kombination mit einem pharmazeutisch annehmbaren Träger enthält.

26. Eine pharmazeutische Zusammensetzung, die durch Vereinen einer Verbindung gemäß irgendeinem der Ansprüche 1 bis 24 mit einem pharmazeutisch annehmbaren Träger hergestellt wird.

27. Die Verwendung einer Verbindung gemäß irgendeinem der Ansprüche 1 bis 24 zur Herstellung eines Medikaments zur Behandlung oder Prävention einer Bakterieninfektion.

## Revendications

1. Composé représenté par la formule I : 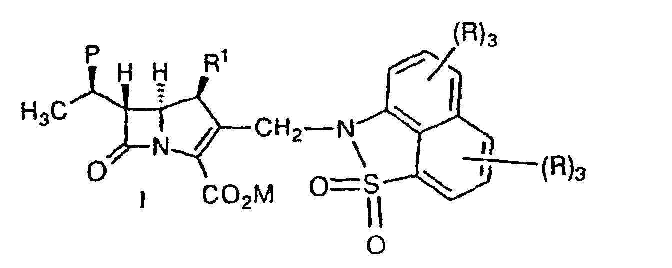 ou l'un de ses sels pharmaceutiquement acceptables, dans lequel :
R¹ représente un H ou un groupe méthyle ;
CO₂M représente un acide carboxylique, un anion carboxylate, un groupe ester pharmaceutiquement acceptable ou un acide carboxylique protégé par un groupe protecteur ; P représente un atome d'hydrogène, un groupe hydroxyle, F ou un groupe hydroxyle protégé par un groupe protecteur de groupe hydroxyle ;
chaque R est indépendamment choisi parmi : -R* ; -Q ; un atome d'hydrogène ; un atome d'halogène ; -CN ; -NO₂ ; -NR^{a}R^{b} ; -OR^{c} ; -SR^{c} ; -C(O)NR^{a}R^{b} ; -C(O)OR^{h} ; -S(O)R^{c} ; -SO₂R^{c} ; -SO₂NR^{a}R^{b} ; -NR^{a}SO₂R^{b} ; -C(O)R^{a} ; -OC(O)R^{a} ; -OC(O)NR^{a}R^{b} ; -NR^{a}C(O)NR^{b}R^{c} ; -NR^{a}CO₂R^{h} ; -OCO₂R^{h} ; -NR^{a}C(O)R^{b} ; un groupe alkyle à chaîne linéaire ou ramifiée en C₁₋₆, non substitué ou substitué par un à quatre groupes R^{d} ; et un groupe cycloalkyle en C₃₋₇ non substitué ou substitué par un à quatre groupes R^{d} ;
à la condition qu'il y ait au moins un R présent qui contienne au moins une charge positive ;
chaque R^{a}, R^{b} et R^{c} représentent indépendamment un atome d'hydrogène, -R*, un groupe alkyle à chaîne linéaire ou ramifiée en C₁₋₆, non substitué ou substitué par un à quatre groupes R^{d} ou un groupe cycloalkyle en C₃₋₇ non substitué ou substitué par un à quatre groupe R^{d} ;
ou bien R^{a} et R^{b}, pris ensemble avec des atomes intermédiaires quelconques représentent un cycle saturé à 4-6 chaînons éventuellement interrompu parmi un à trois parmi O, S, NR^{c}, R^{c} étant tel que défini ci-dessus, ou -C(O)-, ledit cycle étant non substitué ou substitué par un à quatre groupe Rⁱ ;
ou bien R^{b} et R^{c}, pris ensemble avec des atomes intermédiaires quelconques représentent un cycle saturé à 4-6 chaînons éventuellement interrompu parmi un à trois substituants parmi O, S, NR^{a}, R^{a} étant tel que défini ci-dessus, ou -C(O)-, ledit cycle étant non substitué ou substitué par un à quatre groupe Rⁱ ;
chaque R^{d} représente indépendamment un atome d'halogène ; -CN ; -NO₂ ; -NR^{e}R^{f} ; -OR^{g} ; -SR^{g} ; -CONR^{e}R^{f} ; -COOR^{g} ; -SOR^{g} ; -SO₂R^{g} ; SO₂NR^{e}R^{f} ; -NR^{e}SO₂R^{f} ; -COR^{e} ; -NR^{e}COR^{f} ; -OCOR^{e} ; OCONR^{e}R^{f} ; -NR^{e}CONR^{f}R^{g} ; -NR^{e}CO₂R^{h} ; -OCO₂R^{h} ; -C(NR^{e})NR^{f}R^{g} ; -NR^{e}C(N^{h})NR^{f}R^{g} ; -NR^{e}C(NR^{f})R^{g} ; -R* ou -Q ;
R^{e}, R^{f} et R^{g} représentent un atome d'hydrogène ; R* ; un groupe alkyle à chaîne linéaire ou ramifiée en C₁₋₆ non substitué ou substitué par un à quatre groupes Rⁱ ;
ou bien R^{e} et R^{f}, pris ensemble avec des atomes intermédiaires quelconques représentent un cycle saturé à 4-6 chaînons éventuellement interrompu parmi un à trois substituants parmi O, S, -C(O)- ou NR^{g}, R^{g} étant tel que défini ci-dessus, ledit cycle étant non substitué ou substitué par un à quatre groupe Rⁱ ;
chaque Rⁱ représente indépendamment un atome d'halogène ; -CN ; -NO₂ ; phényle ; -NHSO₂R^{h} ; -OR^{h} ; -SR^{h} ; -N(R^{h})₂ ; -N⁺(R^{h})₃ ; -C(O)N(R^{h})₂ ; -SO₂N(R^{h})₂ ; un groupe hétéroaryle ; hétéroarylium ; -CO₂R^{h} ; -C(O)R^{h} ; -OCOR^{h} ; -NHCOR^{h} ; guanidinyle ; carbamimidoyle ou uréido ;
chaque R^{h} représente indépendamment un atome d'hydrogène, un groupe alkyle à chaîne linéaire ou ramifiée en C₁₋₆, un groupe cycloalkyle en C₃₋₆ ou un groupe phényle, ou lorsque deux groupes R^{h} sont présents, lesdits groupes R^{h} peuvent être pris en combinaison et représentent un cycle saturé à 4-6 chaînons, éventuellement interrompus par un ou deux parmi O, S, SO₂, -C(O)-, NH et NCH₃ ;
Q est choisi dans l'ensemble consistant en : 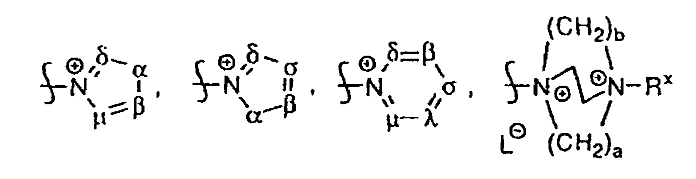 et 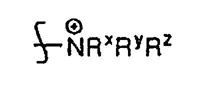 dans lesquels :
a et b valent 1, 2 ou 3 ;
L⁻ est un contre-ion pharmaceutiquement acceptable ;
a représente O, S ou NR^{s} ;
β, δ, λ, µ et σ représentent CR^{t}, N ou N⁺R^{s}, à condition que pas plus d'un parmi β, δ, λ, µ et σ ne représente N⁺R^{s ;}
R* est choisi dans l'ensemble consistant en : 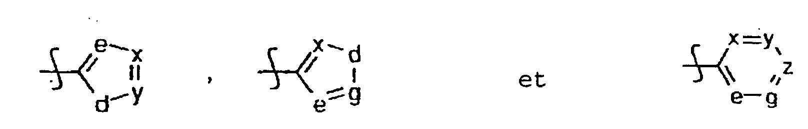 dans lesquels :
d représente O, S ou NR^{k} ;
e, g, x, y et z représentent CR^{m}, N ou N⁺R^{k}, à condition que pas plus d'un parmi e, g, x, y et z dans une structure donnée quelconque représente N⁺R^{k}.
R^{k} représente un atome d'hydrogène ; un groupe alkyle à chaîne linéaire ou ramifiée en C₁₋₆, non substitué ou substitué par un à quatre groupe Rⁱ ; ou -(CH₂)ₙQ, où n = 1, 2 ou 3 et Q est tel que précédemment défini.
chaque R^{m} représente indépendamment un élément choisi dans l'ensemble consistant en un atome d'hydrogène ; un atome d'halogène ; -CN ; -NO₂ ; -NRⁿR^{o} ; -ORⁿ ; -SRⁿ ; -CONRⁿR^{o} ; -COOR^{h} ; -SORⁿ ; -SO₂Rⁿ ; -SO₂NRⁿR^{o} ; -NRⁿSO₂R^{o} ; -CORⁿ ; -NRⁿCOR^{o} ; -OCORⁿ ; -OCONRⁿR^{o} ; -NRⁿCO₂R^{h} ; -NRⁿCONR^{o}R^{h} ; -OCO₂R^{h} ; CNRⁿNR^{o}R^{h} ,; -NRⁿCNHNR^{o}R^{h} ; -NRⁿC(NR^{o})R^{h} ; un groupe alkyle à chaîne linéaire ou ramifiée en C₁₋₆ non substitué ou substitué par un à quatre groupes Rⁱ ; un groupe cycloalkyle en C₃₋₇ non substitué ou substitué par un à quatre groupes Rⁱ ; et -(CH₂)ₙQ où n et Q sont tels que définis ci-dessus ;
Rⁿ et R^{o} représente un atome d'hydrogène, un groupe phényle, un groupe alkyle à chaîne linéaire ou ramifiée en C₁₋₆ non substitué ou substitué par un à quatre groupes Rⁱ ;
chaque R^{s} représente indépendamment un atome d'hydrogène ; un groupe phényle ou un groupe alkyle à chaîne linéaire ou ramifiée en C₁₋₆ non substitué ou substitué par un à quatre groupes Rⁱ ;
chaque R^{t} représente indépendamment un atome d'hydrogène ; un atome d'halogène, un groupe phényle, -CN ; -NO₂ ; -NR^{u}R^{v} ; -OR^{u} ; -SR^{u} ; -CONR^{u}R^{v} ; -COOR^{h} ; -SOR^{u} ; -SO₂R^{u} ; SO₂NR^{u}R^{v} ; -NR^{u}SO₂R^{v} ; -COR^{u} ; -NR^{u}COR^{v} ; -OCOR^{u} ; -OCONR^{u}R^{v} ; - NR^{u}CO₂R^{v} ; -NR^{u}CONR^{v}R^{w} ; -OCO₂R^{v} ; un groupe alkyle à chaîne linéaire ou ramifiée en C₁₋₆ non substitué ou substitué par un à quatre groupes Rⁱ ;
R^{u} et R^{v} représentent un atome d'hydrogène ou un groupe alkyle à chaîne linéaire ou ramifiée en C₁₋₆ non substitué ou substitué par un à quatre groupes Rⁱ ;
ou bien R^{u} et R^{v}, pris ensemble avec des atomes intermédiaires quelconques représentent un cycle saturé à 4-6 chaînons éventuellement interrompu parmi un ou plusieurs parmi O, S, NR^{w}, ou -C(O)-, ledit cycle étant non substitué ou substitué par un à quatre groupe Rⁱ ;
chaque R^{w} représente indépendamment un atome d'hydrogène ; un groupe alkyle à chaîne linéaire ou ramifiée en C₁₋₆ non substitué ou substitué par un à quatre groupes Rⁱ ; un groupe cycloalkyle en C₃₋₆ non substitué ou substitué par un à quatre groupes Rⁱ ; un groupe phényle éventuellement substitué par un à quatre groupes Rⁱ ; ou un groupe hétéroaryle éventuellement substitué par un à quatre groupes Rⁱ ;
ou bien R^{h} et R^{w}, pris ensemble avec des atomes intermédiaires quelconques représentent un cycle saturé à 5-6 chaînons éventuellement interrompu parmi un ou deux parmi O, S, SO₂, NH, ou NCH₃ ;
R^{x} représente un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne pouvant être non substituée ou substituée par un à quatre parmi un atome d'halogène CN, NO₂, OR^{w,} SR^{w}, SOR^{w}, SO₂R^{w}, NR^{h}R^{w}, N+(R^{h})₂R^{w}, -C (O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyles à chaîne linéaire ou ramifiée en C₁₋₃, lesdits groupes alkyles étant non substitués ou substitués par un à quatre groupes Rⁱ ; R^{y} et R^{z} représentent un atome d'hydrogène ; un groupe phényle ; un groupe alkyle à chaîne linéaire ou ramifiée en C₁₋₆ non substitué ou substitué par un à quatre groupes Rⁱ ; et éventuellement interrompu par O, S, NR^{w}, N⁺R^{h}R^{w} ou -C(O)-;
ou bien R^{x} et R^{y} ensemble avec des atomes intermédiaires quelconques représentent un cycle saturé à 4-6 chainons éventuellement interrompus par O, S, SO₂, NR^{w}, N⁺R^{h}R^{w} ou -C(O)- non substitué ou substitué par un à quatre groupes Rⁱ ;
et lorsque R^{x} et R^{y} représentent ensemble un cycle à 4 à 6 chaînons tel que défini ci-dessus, R^{z} est tel que défini ci-dessus ou bien R^{z} représente un autre cycle à 4-6 chaînons saturé condensé au cycle représenté par R^{x} ou R^{y} pris ensemble, éventuellement interrompu par O, S, NR^{w}, ou -C(O)-, lesdits cycles étant non substitués ou substitués par un à quatre groupes Rⁱ .

2. Composé selon la revendication 1, dans lequel CO₂M représente un anion carboxylate.

3. Composé selon la revendication 1, dans lequel un R représente un groupe qui contient une partie positivement chargée et les autres groupes R sont choisis parmi un atome d'hydrogène et un groupe alkyle à chaîne linéaire ou ramifiée en C₁₋₆ non substitué ou substitué par un à quatre groupes R^{d}.

4. Composé selon la revendication 3, dans lequel un R représente un groupe contenant une partie positivement chargée et les autres groupes R représentent un atome d'hydrogène.

5. Composé selon la revendication 1 dans lequel les groupes R contiennent une à trois charges positives.

6. Composé selon la revendication 5, dans lequel les groupes R contiennent deux charges positives, équilibrées par un anion carboxylate et un contre-ion négativement chargé.

7. Composé selon la revendication 1, dans lequel un groupe R représente un groupe alkyle à chaîne linéaire ou ramifiée en C₁₋₆ substitué par un à quatre groupes R^{d}, dans lequel un groupe R^{d} représente -R* ou Q.

8. Composé selon la revendication 1, dans lequel Q est choisi dans l'ensemble consistant en :

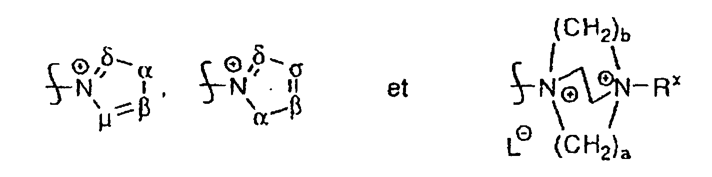

9. Composé selon la revendication 8, dans lequel Q représente : 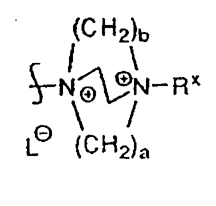
L⁻, a et b sont tels que définis à l'origine et R^{x} représente un élément choisi dans l'ensemble consistant en un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne étant non substituée ou substituée par un à quatre substituants parmi un atome d'halogène CN, NO₂, OR^{w}, SR^{w}, SOR^{w}, SO₂R^{w}, NR^{h}R^{w}, N+(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyles à chaîne linéaire ou ramifiée en C₁₋₃, lesdits groupes alkyles étant non substitués ou substitués par un à quatre groupes Rⁱ ; et
R^{h}, Rⁱ et R^{w} sont tels que définis à l'origine.

10. Composé selon la revendication 1 dans lequel Q représente -N+R^{x}R^{y}R^{z}, où R^{x}, R^{y} et R^{z} sont tels que définis à l'origine.

11. Composé selon la revendication 1, dans lequel un groupe R* est présent et est choisi parmi : 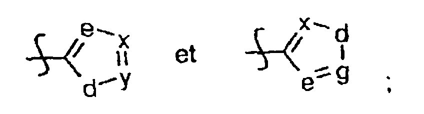 d représente NR^{k} ; R^{k} représente un groupe alkyle à chaîne linéaire ou ramifiée en C₁₋₆ et e, g, x et y représentent CR^{m} ou N+R^{k}, R^{k} étant défini ci-dessus et R^{m} représentant un atome d'hydrogène.

12. Composé selon la revendication 1, dans lequel :
CO₂M représente un anion carboxylate ;
un groupe R qui est attaché à la plate-forme naphtosultame contient au moins une partie positivement chargée et les autres groupes R sont choisis parmi un atome d'hydrogène et un groupe alkyle à chaîne linéaire ou ramifiée en C₁₋₆, non substitué ou substitué par un à quatre groupes R^{d} ;
R^{d} est tel que défini à l'origine ;
R^{h} représente un atome d'hydrogène ou un groupe alkyle à chaîne linéaire ou ramifiée en C₁₋₆ ;
Q est choisi dans l'ensemble consistant en : 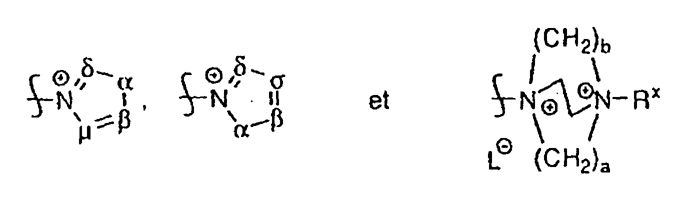 dans lesquels L⁻ est tel que défini à l'origine ; a et b valent 2 et R^{x} représente un élément choisi dans l'ensemble consistant en un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne pouvant être non substituée ou substituée par un à quatre substituants parmi un atome d'halogène CN, NO₂, OR^{w}, SR^{w}, SOR^{w}, SO₂R^{w}, NR^{h}R^{w}, N⁺(R^{h})₂R^{w}, -C(O)-R^{w}, C (O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyles à chaîne linéaire ou ramifiée en C₁₋₃, lesdits groupes alkyles étant non substitués ou substitués par un à quatre groupes Rⁱ ;
R* est choisi parmi : 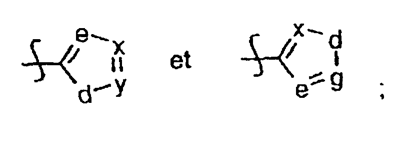
dans lesquels d représente NR^{k} ; R^{k} représente un groupe alkyle à chaîne linéaire ou ramifiée en C₁₋₆ et e, g, x et y représentent CR^{m} ou N+R^{k}, R^{k} étant défini ci-dessus et R^{m} représentant un atome d'hydrogène.

13. Composé selon la revendication 1, représenté par la formule Ia : 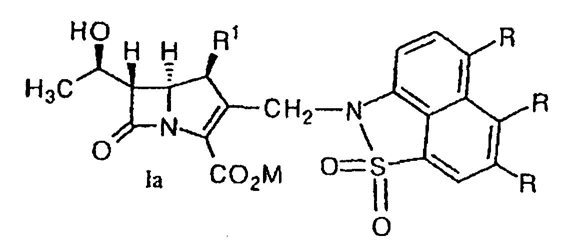 ou l'un de ses sels pharmaceutiquement acceptable, dans lequel :
CO₂M représente un anion carboxylate ;
un R contient une partie positivement chargée et les autres groupes R sont choisis parmi un atome d'hydrogène et un groupe alkyle à chaîne linéaire ou ramifiée en C₁₋₆, non substitué ou substitué par un à quatre groupes R^{d} ;
R^{d} est tel que défini à l'origine ;
Q est choisi dans l'ensemble consistant en : 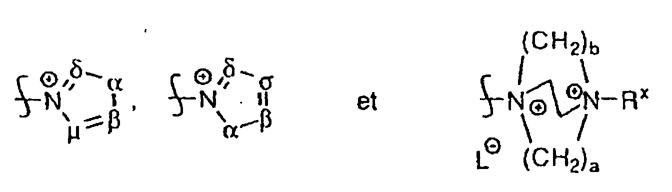 dans lesquels L⁻, a et b sont tels que définis à l'origine, et R^{x} représente un élément choisi dans l'ensemble consistant en un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne pouvant être non substituée ou substituée par un à quatre substituants parmi un atome d'halogène CN, NO₂, OR^{w}, SR^{w}, SOR^{w}, SO₂R^{w}, NR^{h}R^{w}, N+(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyles à chaîne linéaire ou ramifiée en C₁₋₃, lesdits groupes alkyles étant non substitués ou substitués par un à quatre groupes Rⁱ ;
R^{h} représente un atome d'hydrogène ou un groupe alkyle à chaîne linéaire ou ramifiée en C₁₋₆ ;
R^{w} est tel que défini à l'origine ;
R* est choisi parmi : 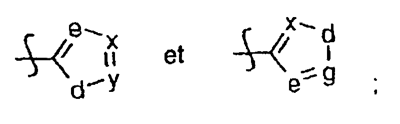
dans lesquels d représente NR^{k} ; R^{k} représente un groupe alkyle à chaîne linéaire ou ramifiée en C₁₋₆ et e, g, x et y représentent CR^{m} ou N+R^{k}, R^{k} étant défini ci-dessus et R^{m} représentant un atome d'hydrogène.

14. Composé selon la revendication 1 représenté par la formule Ib : 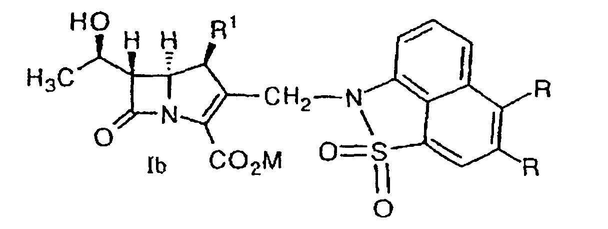 ou l'un de ses sels pharmaceutiquement acceptables, dans lequel
CO₂M représente un anion carboxylate ;
un groupe R qui est attaché à la plate-forme naphtosultame contient au moins une partie positivement chargée et les autres groupes R sont choisis parmi un atome d'hydrogène et un groupe alkyle à chaîne linéaire ou ramifiée en C₁₋₆, non substitué ou substitué par un à quatre groupes R^{d} ;
R^{d} est tel que défini à l'origine ;
Q est choisi dans l'ensemble consistant en : 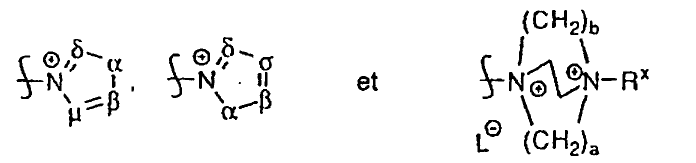 dans lesquels L⁻, a et b sont tels que définis à l'origine ; valent 2 et R^{x} représente un élément choisi dans l'ensemble consistant en un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne pouvant être non substituée ou substituée par un à quatre substituants parmi un atome d'halogène CN, NO₂, OR^{w}, SR^{w}, SOR^{w}, SO₂R^{w}, NR^{h}R^{w}, N+(R^{h})₂R^{w}, -C (O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyles à chaîne linéaire ou ramifiée en C₁₋₃, lesdits groupes alkyles étant non substitués ou substitués par un à quatre groupes Rⁱ ;
R^{h} représente un atome d'hydrogène ou un groupe alkyle à chaîne linéaire ou ramifiée en C_{1-6;}
R^{w} est tel que défini à l'origine
R* est choisi parmi : 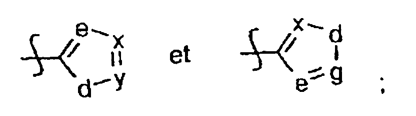
dans lesquels d représente NR^{k} ; R^{k} représente un groupe alkyle à chaîne linéaire ou ramifiée en C₁₋₆ et e, g, x et y représentent CR^{m} ou N+R^{k}, R^{k} étant défini ci-dessus et R^{m} représentant un atome d'hydrogène. Dans ce sous-ensemble, toutes les autres variable sont telles que définies à l'origine relativement à la formule I.

15. Composé selon la revendication 1 représenté par la formule Ic : 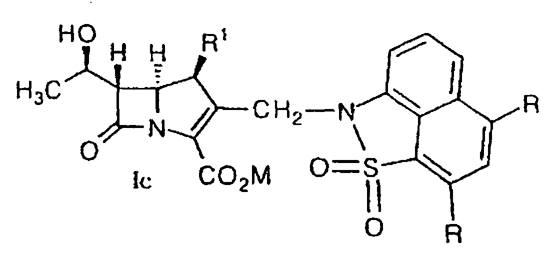 ou l'un de ses sels pharmaceutiquement acceptables, dans lequel
CO₂M représente un anion carboxylate ;
un groupe R qui est attaché à la plate-forme naphtosultame contient au moins une partie positivement chargée et l'autre groupe R est choisi parmi un atome d'hydrogène, un atome d'halogène et un groupe alkyle à chaîne linéaire ou ramifiée, non substitué ou substitué par un à quatre groupes R^{d} ;
R^{d} est tel que défini à l'origine ;
Q est choisi dans l'ensemble consistant en : 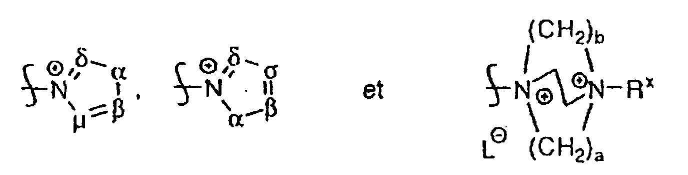 dans lesquels L⁻, a et b sont tels que définis à l'origine et R^{x} représente un élément choisi dans l'ensemble consistant en un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne pouvant être non substituée ou substituée par un à quatre parmi un atome d'halogène CN, NO₂, OR^{w}, SR^{w}, SOR^{w}, SO₂R^{w}, NR^{h}R^{w}, N+ (R^{h})₂R^{w}, -C (O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyles à chaîne linéaire ou ramifiée en C₁₋₃, lesdits groupes alkyles étant non substitués ou substitués par un à quatre groupes Rⁱ ;
R^{h} représente un atome d'hydrogène ou un groupe alkyle à chaîne linéaire ou ramifiée en C₁₋₆ ;
R^{w} est tel que défini à l'origine
R* est choisi parmi : 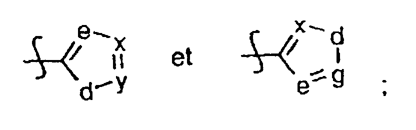
dans lesquels d représente NR^{k} ; R^{k} représente un groupe alkyle à chaîne linéaire ou ramifiée en C₁₋₆ et e, g, x et y représentent CR^{m} ou N+R^{k}, R^{k} étant défini ci-dessus et R^{m} représentant un atome d'hydrogène.

16. Composé selon la revendication 1 représenté par la formule Id : 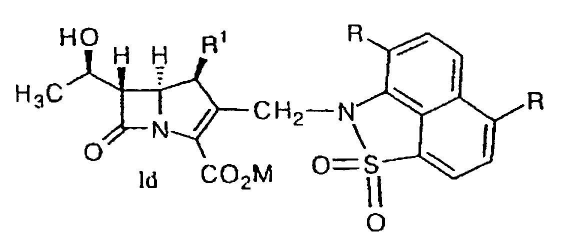 ou l'un de ses sels pharmaceutiquement acceptables, dans lequel
CO₂M représente un anion carboxylate ;
un groupe R qui est attaché à la plate-forme naphtosultame contient au moins une partie positivement chargée et les autres groupes R sont choisis parmi un atome d'hydrogène, un atome d'halogène et un groupe alkyle à chaîne linéaire ou ramifiée en C₁₋₆, non substitué ou substitué par un à quatre groupes R^{d} ;
R^{d} est tel que défini à l'origine ;
Q est choisi dans l'ensemble consistant en : 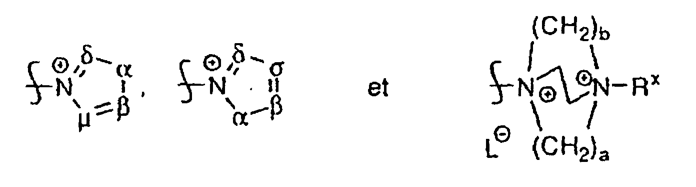 dans lesquels L⁻, a et b sont tels que définis à l'origine et R^{x} représente un élément choisi dans l'ensemble consistant en un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne pouvant être non substituée ou substituée par un à quatre parmi un atome d'halogène CN, NO₂, OR^{w}, SR^{w}, SOR^{w}, SO₂R^{w}, NR^{h}R^{w}, N+(R^{h})₂R^{w}, -C(O)-R^{w}, C(O)NR^{h}R^{w}, SO₂NR^{h}R^{w}, CO₂R^{w}, OC(O) R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyles à chaîne linéaire ou ramifiée en C₁₋₃, lesdits groupes alkyles étant non substitués ou substitués par un à quatre groupes Rⁱ ;
R^{h} représente un atome d'hydrogène ou un groupe alkyle à chaîne linéaire ou ramifiée en C₁₋₆ ;
R^{w} est tel que défini à l'origine
R* est choisi parmi : 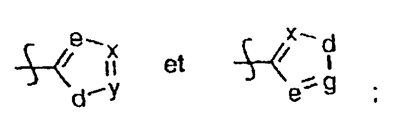
dans lesquels d représente NR^{k} ; R^{k} représente un groupe alkyle à chaîne linéaire ou ramifiée en C₁₋₆ et e, g, x et y représentent CR^{m} ou N+R^{k}, R^{k} étant défini ci-dessus et R^{m} représentant un atome d'hydrogène.

17. Composé selon la revendication 1 représenté par la formule le : 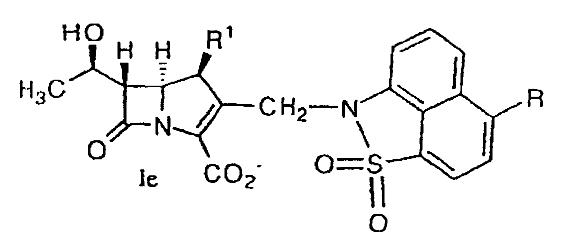 ou l'un de ses sels pharmaceutiquement acceptables, dans lequel
R contient une partie positivement chargée choisie dans l'ensemble consistant en -R*, Q et un groupe alkyle à chaîne linéaire ou ramifiée en C₁₋₆ substitué par un groupe R^{d} ;
R^{d} est indépendamment choisi parmi -R* ou Q ;
Q est choisi dans l'ensemble consistant en : 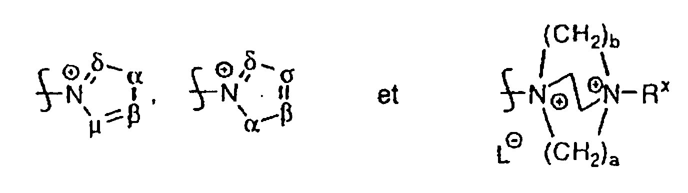 dans lesquels L⁻, a et sont tels que définis à l'origine et R^{x} représente un élément choisi dans l'ensemble consistant en un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne pouvant être non substituée ou substituée par un à quatre substituants parmi un atome d'halogène CN, NO₂, OR^{w}, SR^{w}, SOR^{w}, SO₂R^{w}, NR^{h}R^{w}, N+(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyle à chaîne linéaire ou ramifiée en C₁₋₃, lesdits groupes alkyles étant non substitués ou substitués par un à quatre groupes Rⁱ ;
R* est choisi parmi : 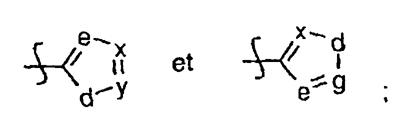
dans lesquels d représente NR^{k} ; R^{k} représente un groupe alkyle à chaîne linéaire ou ramifiée en C₁₋₆ et e, g, x et y représentent CR^{m} ou N+R^{k}, R^{k} étant défini ci-dessus et R^{m} représentant un atome d'hydrogène.

18. Composé selon la revendication 1 représenté par la formule If : 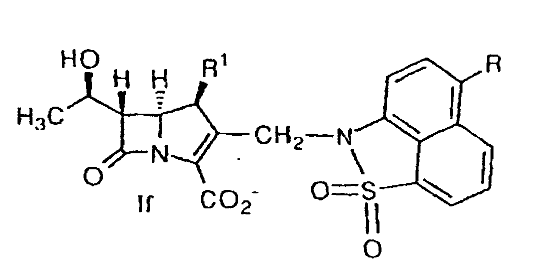 ou l'un de ses sels pharmaceutiquement acceptables, dans lequel
R contient une partie positivement chargée choisie dans l'ensemble consistant en : -R*, Q, et un groupe alkyle à chaîne linéaire ou ramifiée en C₁₋₆ substitué par un groupe R^{d} ;
R^{d} est indépendamment choisi parmi -R* ou Q ;
Q est choisi dans l'ensemble consistant en : 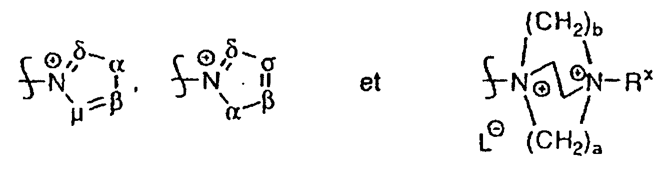 dans lesquels L⁻, a et b sont tels que définis à l'origine ; et R^{x} représente un élément choisi dans l'ensemble consistant en un atome d'hydrogène ou un groupe alkyle à chaîne linéaire ou ramifiée en C₁₋₈ éventuellement interrompu par un ou deux parmi O, S, SO, SO₂, NR^{w}, N⁺R^{h}R^{w}, ou -C(O)-, ladite chaîne pouvant être non substituée ou substituée par un à quatre substituants parmi un atome d'halogène CN, NO₂, OR^{w}, SR^{w}, SOR^{w}, SO₂R^{w}, NR^{h}R^{w}, N+(R^{h})₂R^{w}, -C(O)-R^{w}, C(O)NR^{h}R^{w}, SO₂NR^{h}R^{w}, CO₂R^{w}, OC(O)R^{w}, OC(O)NR^{h}R^{w}, NR^{h}C(O)R^{w}, NR^{h}C(O)NR^{h}R^{w}, ou un groupe phényle ou hétéroaryle qui est quant à lui éventuellement substitué par un à quatre groupes Rⁱ ou par un à deux groupes alkyles à chaîne linéaire ou ramifiée en C₁₋₃, lesdits groupes alkyles étant non substitués ou substitués par un à quatre groupes Rⁱ ;
R* est choisi parmi : 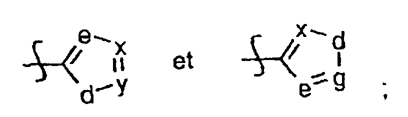
dans lesquels d représente NR^{k} ; R^{k} représente un groupe alkyle à chaîne linéaire ou ramifiée en C₁₋₆ et e, g, x et y représentent CR^{m} ou N+R^{k}, R^{k} étant défini ci-dessus et R^{m} représentant un atome d'hydrogène.

19. Composé selon la revendication 17, dans lequel :
R représente : 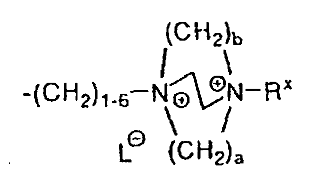
et Rₓ, a, b et L⁻ sont tels que définis à l'origine.

20. Composé selon la revendication 1 représenté par la formule Ig : 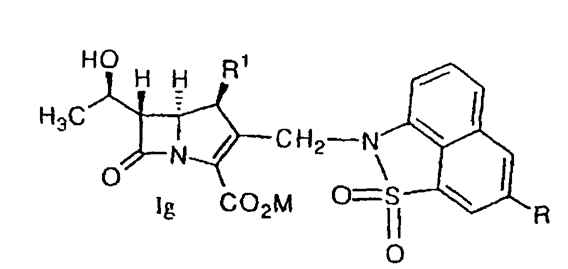 dans laquelle :
R représente 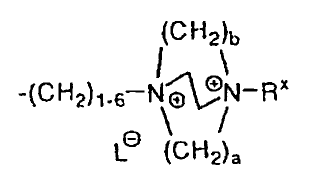
et Rₓ, a, b et L⁻ sont tels que définis à l'origine.

21. Composé selon la revendication 1, représenté par la formule structurale 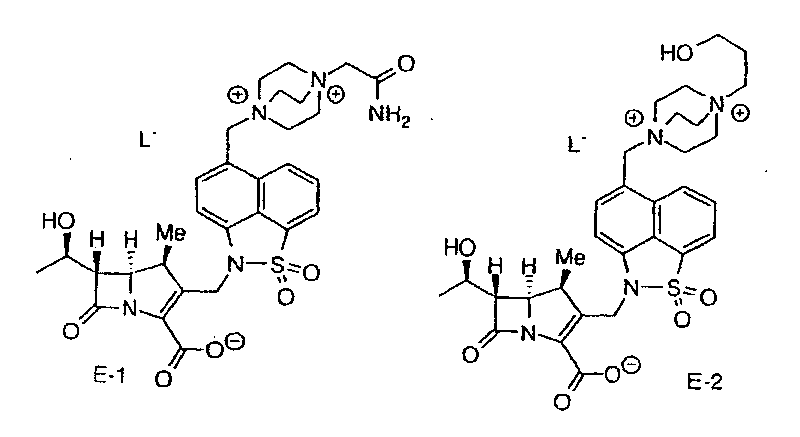 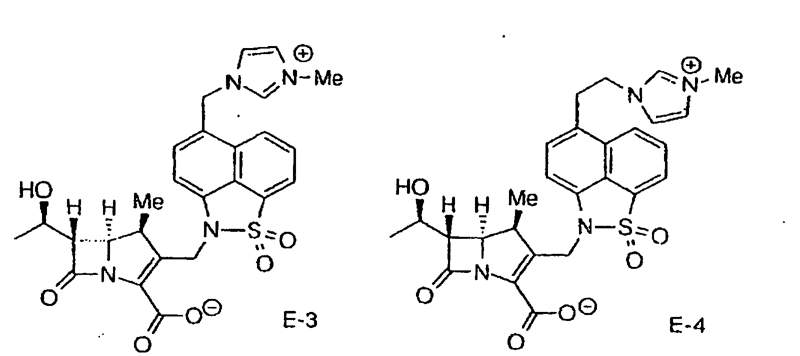 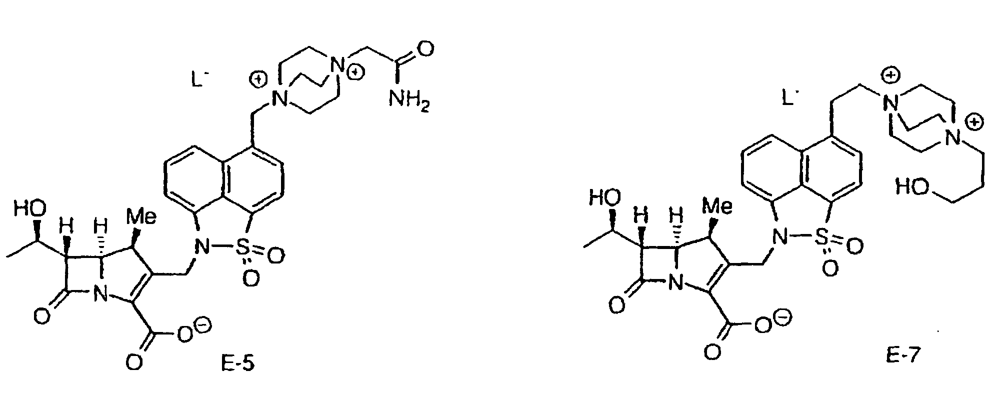 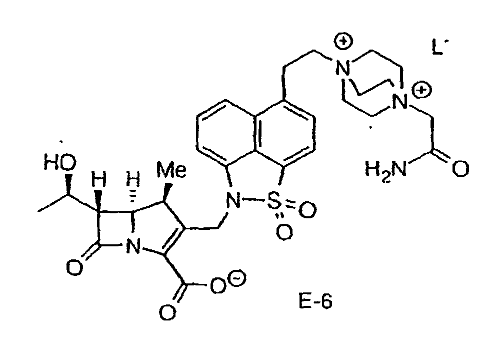 ou 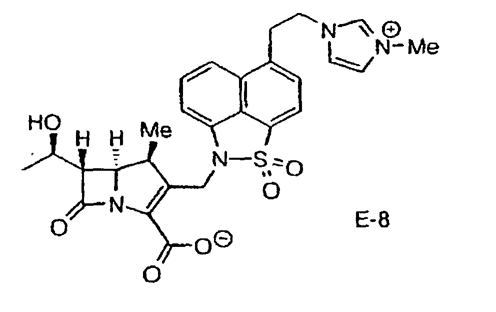 dans lesquelles L⁻ représente un contre-ion pharmaceutiquement acceptable.

22. Composé selon la revendication 1, représenté par la formule structurale 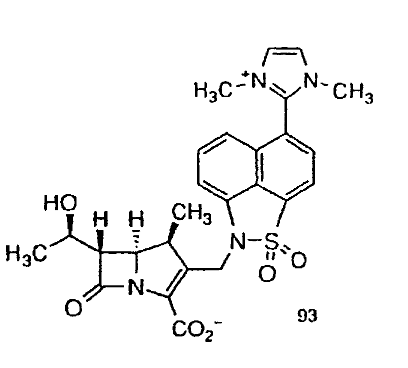 ou

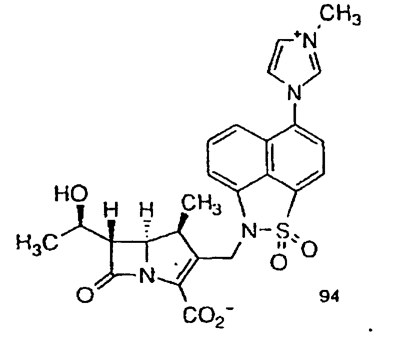

23. Composé représenté par la structure 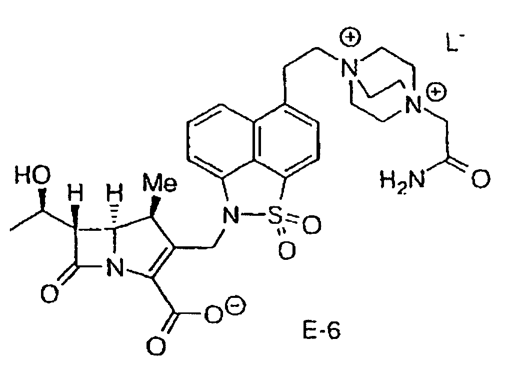 dans laquelle L⁻ représente un cation pharmaceutiquement acceptable.

24. Composé selon la revendication 1, conforme à l'un des tableaux suivants : 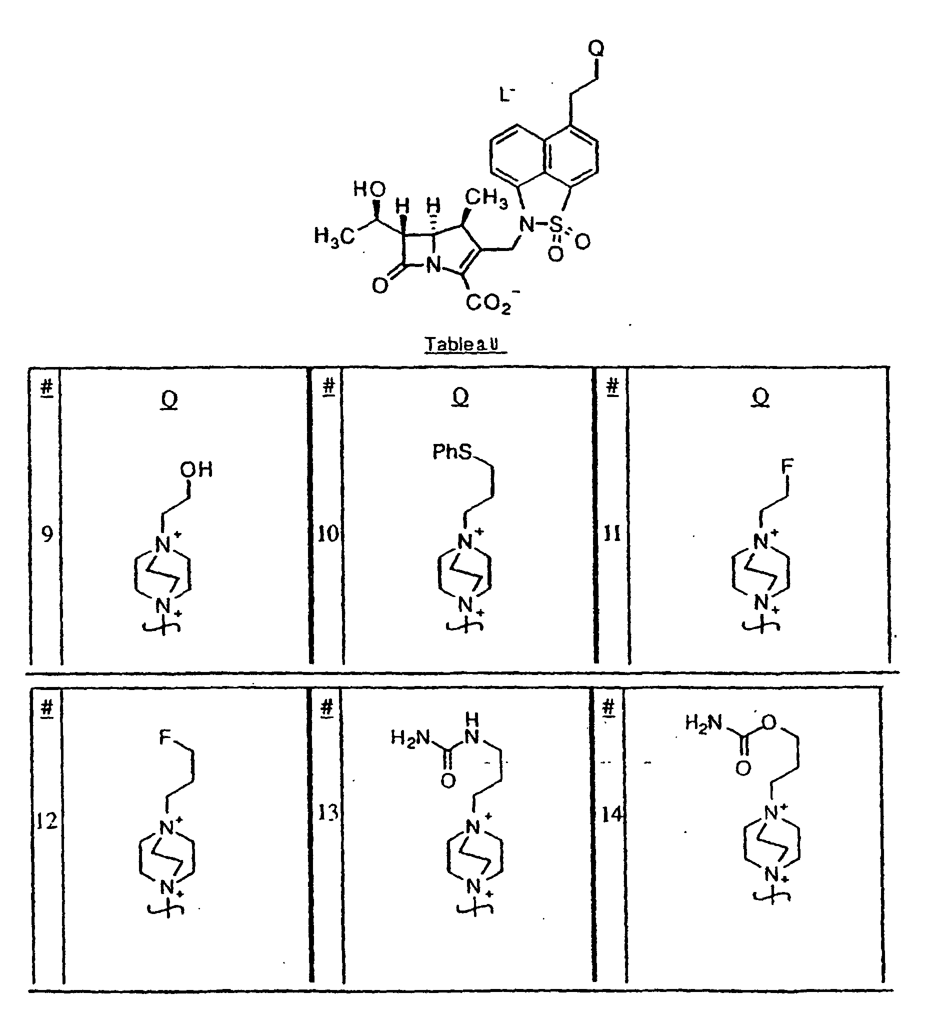 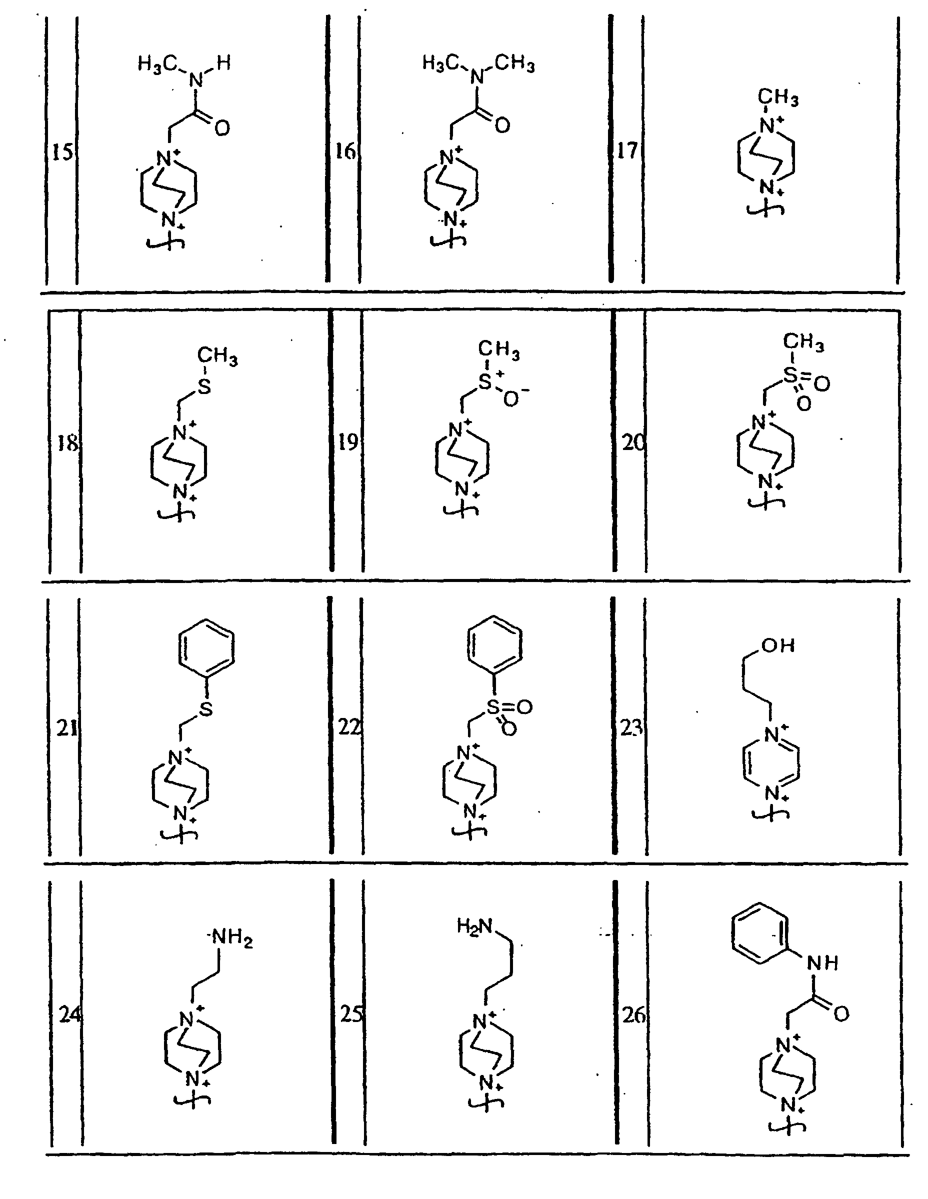 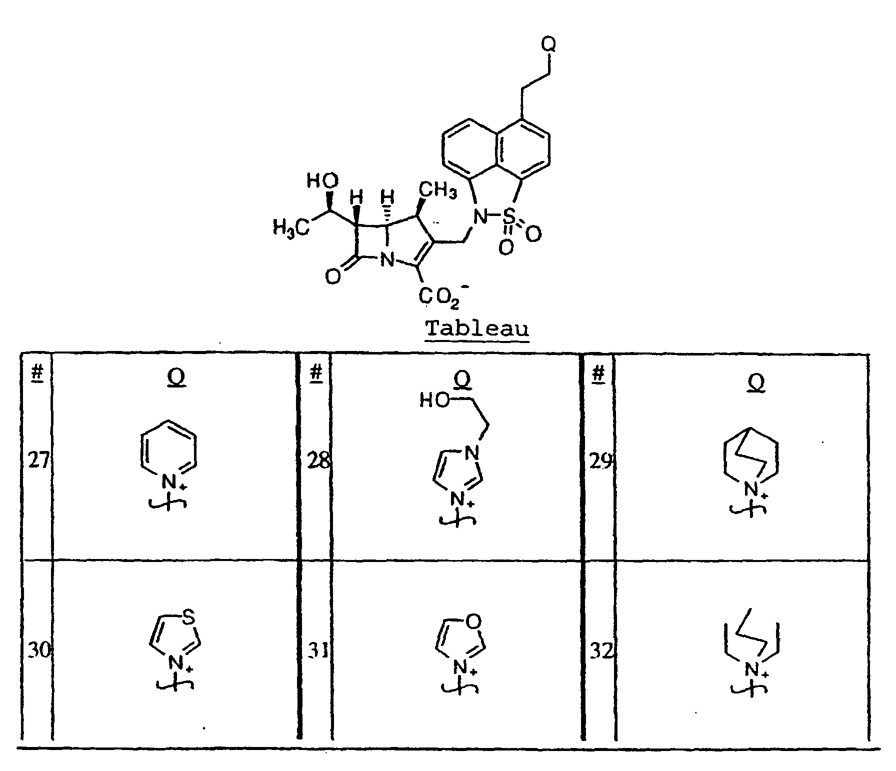 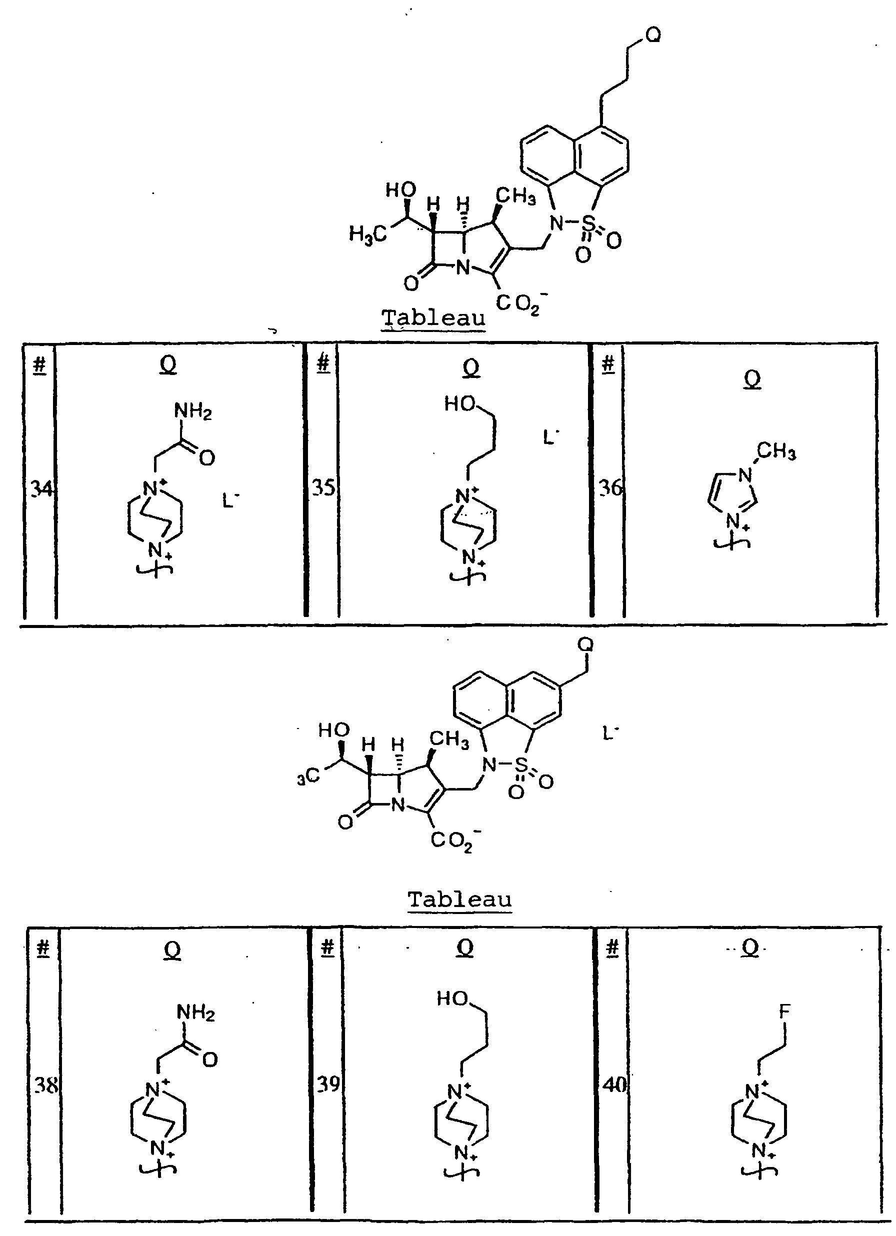 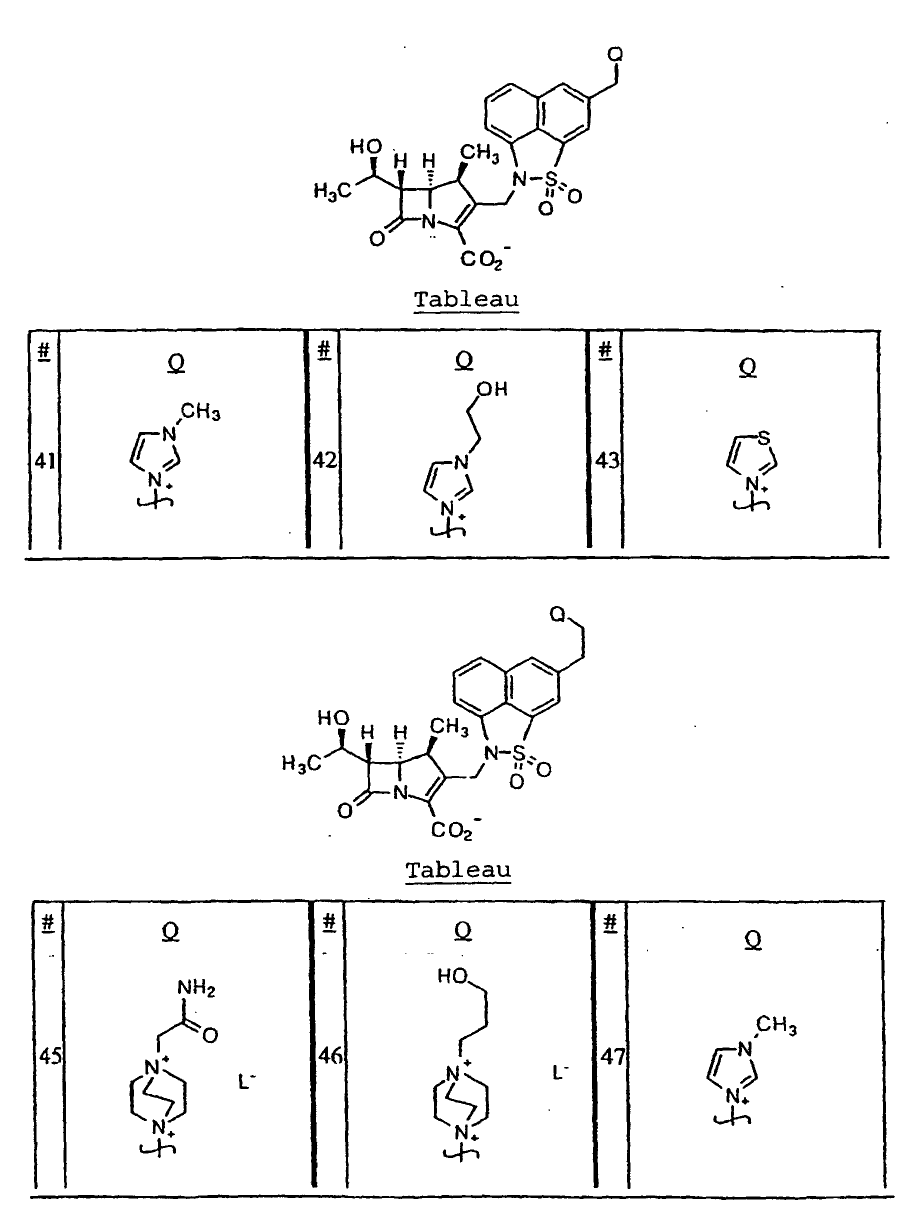 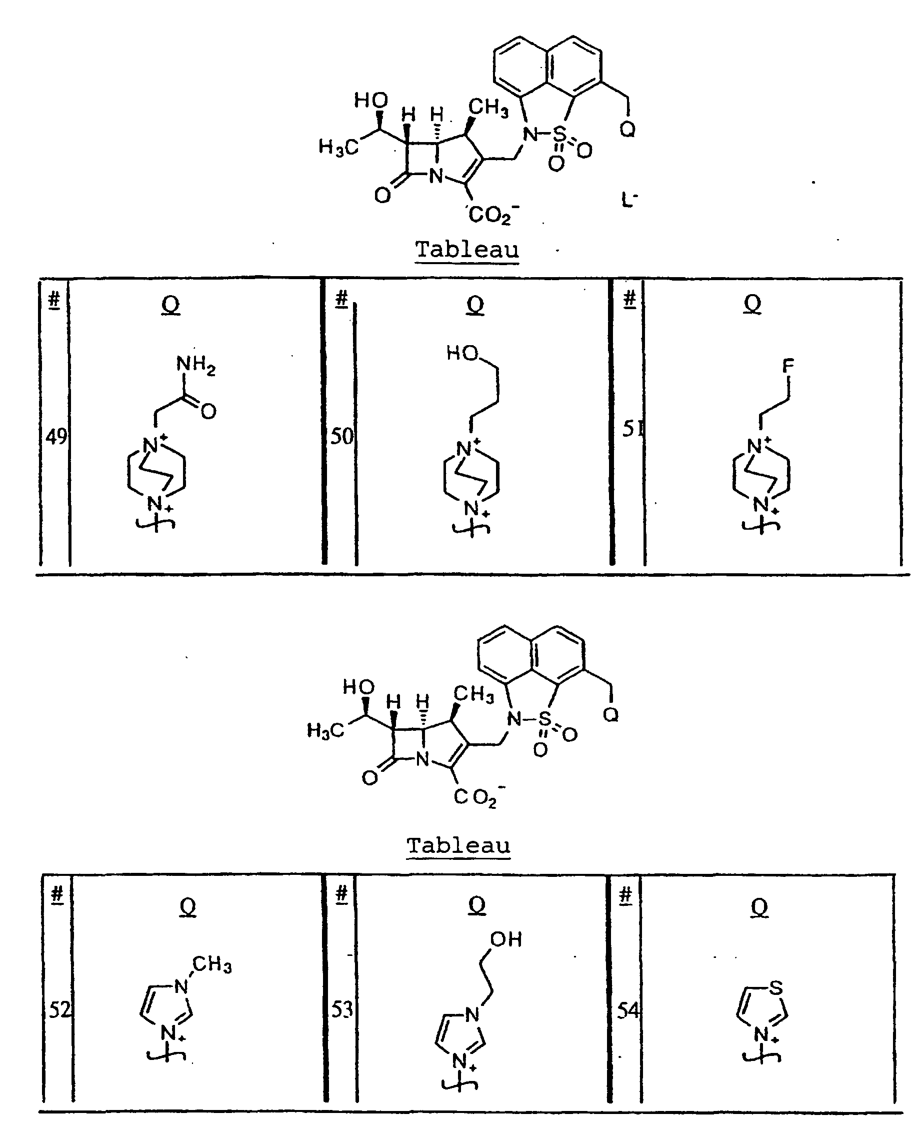 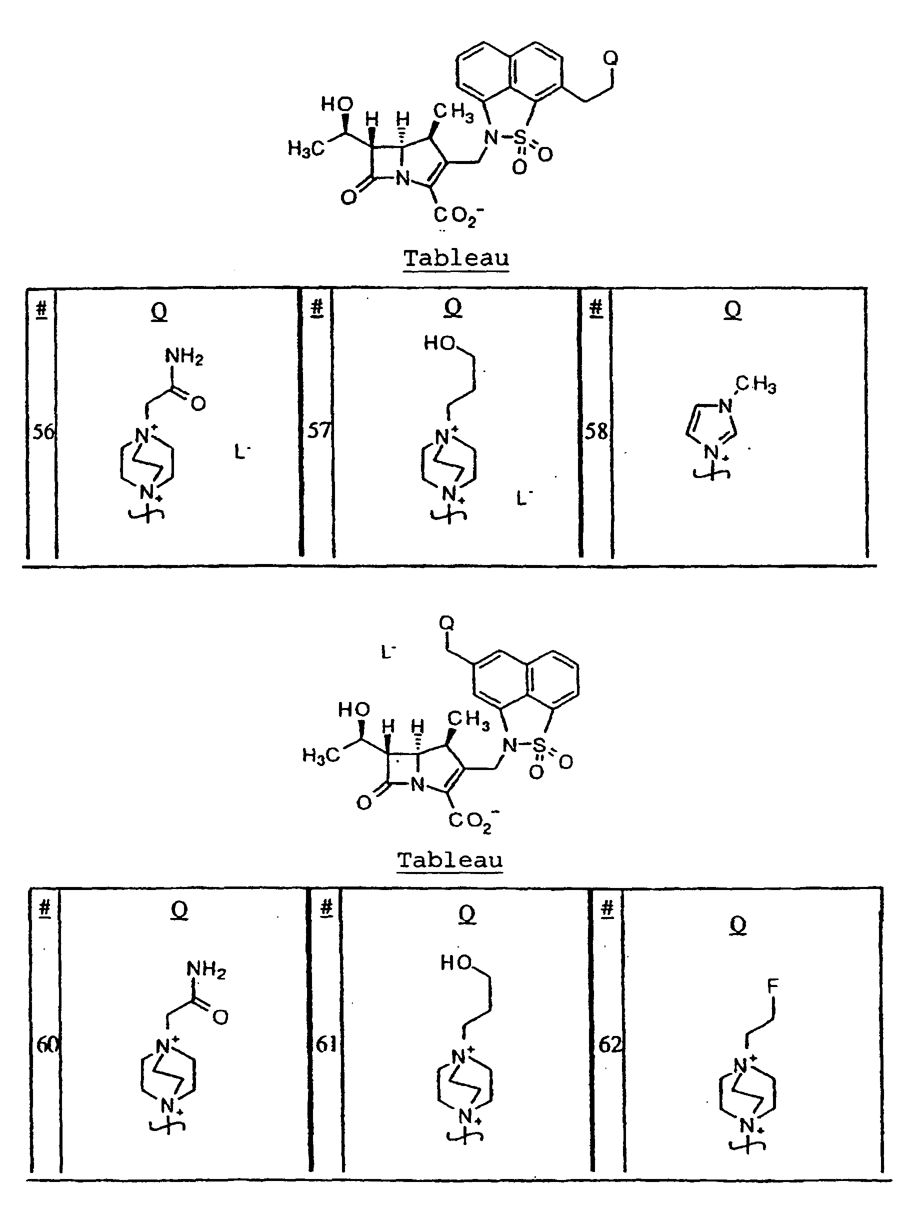 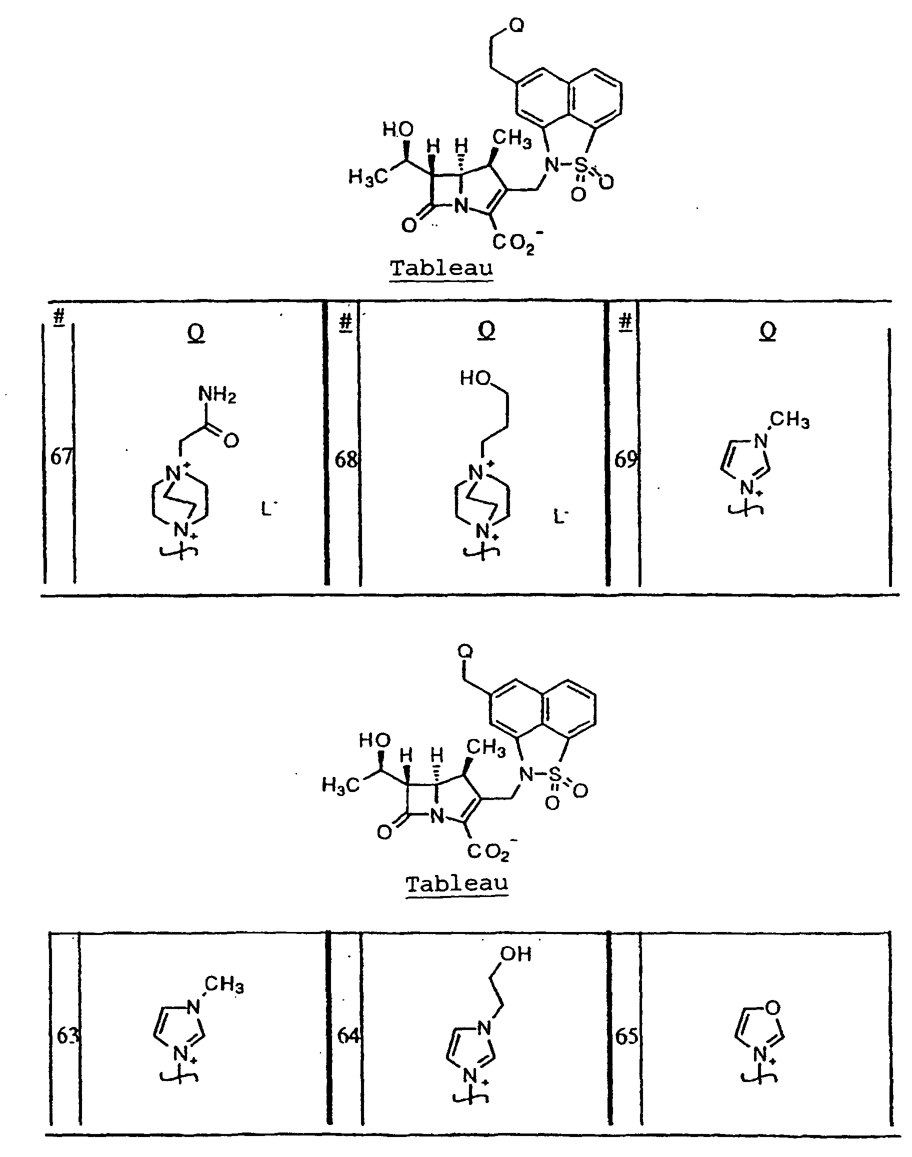 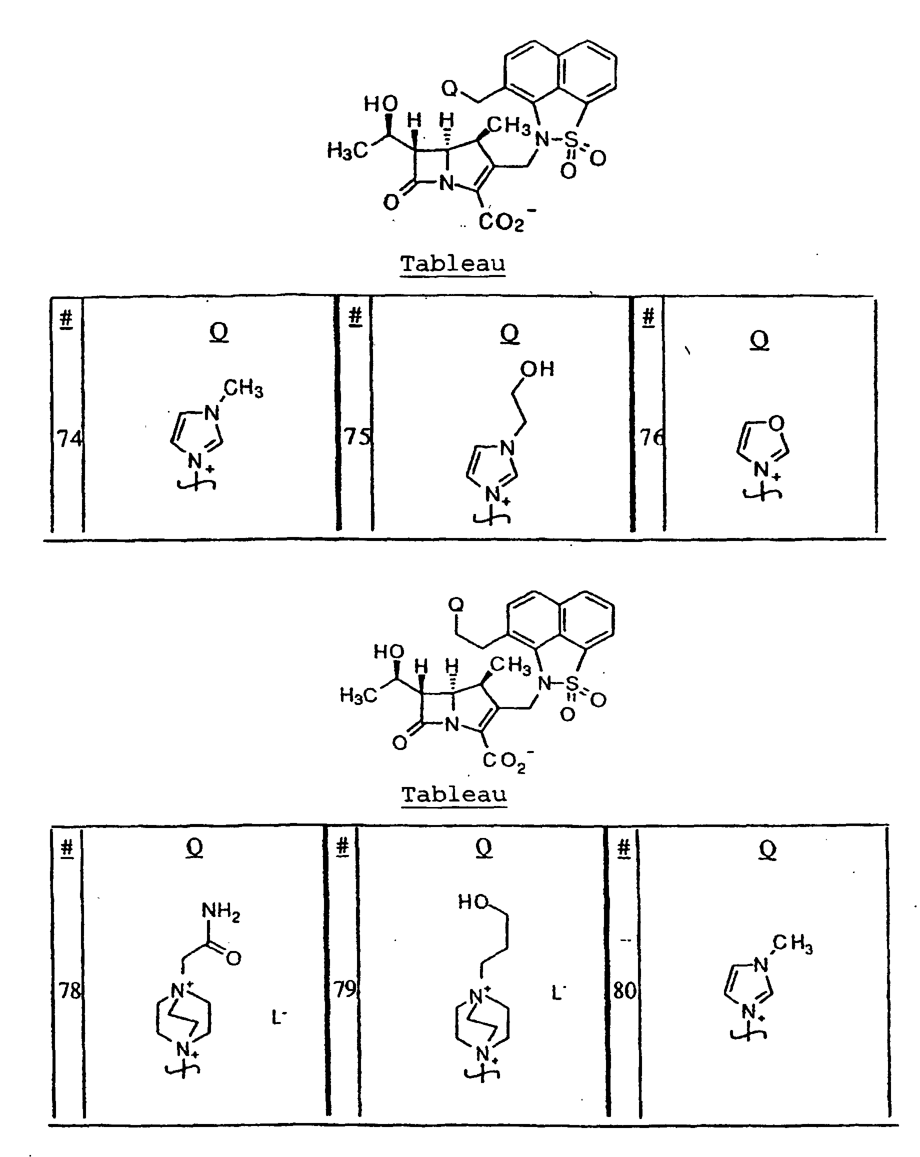 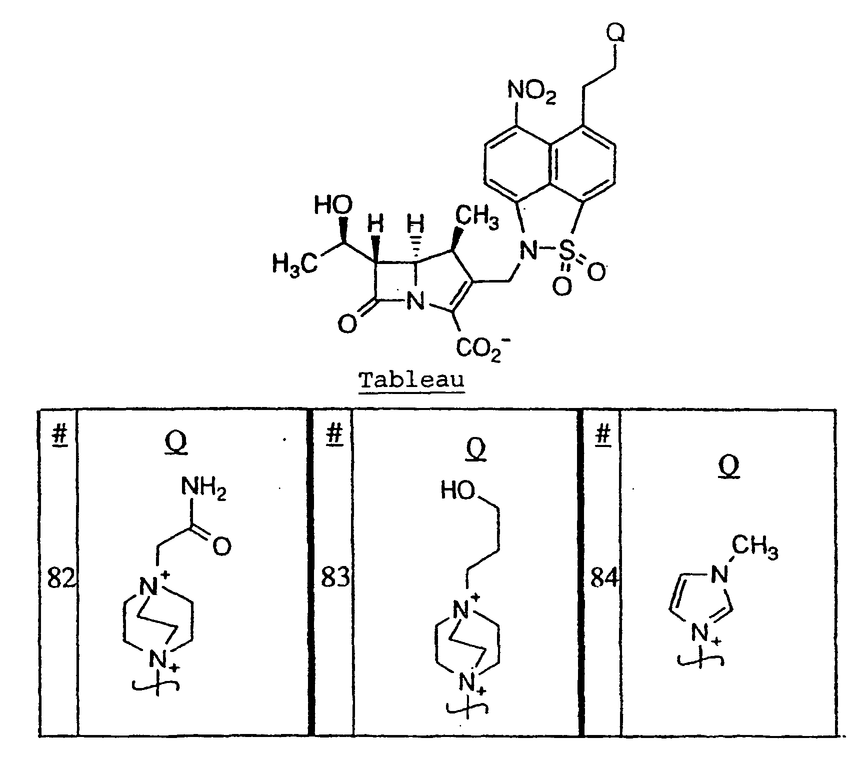 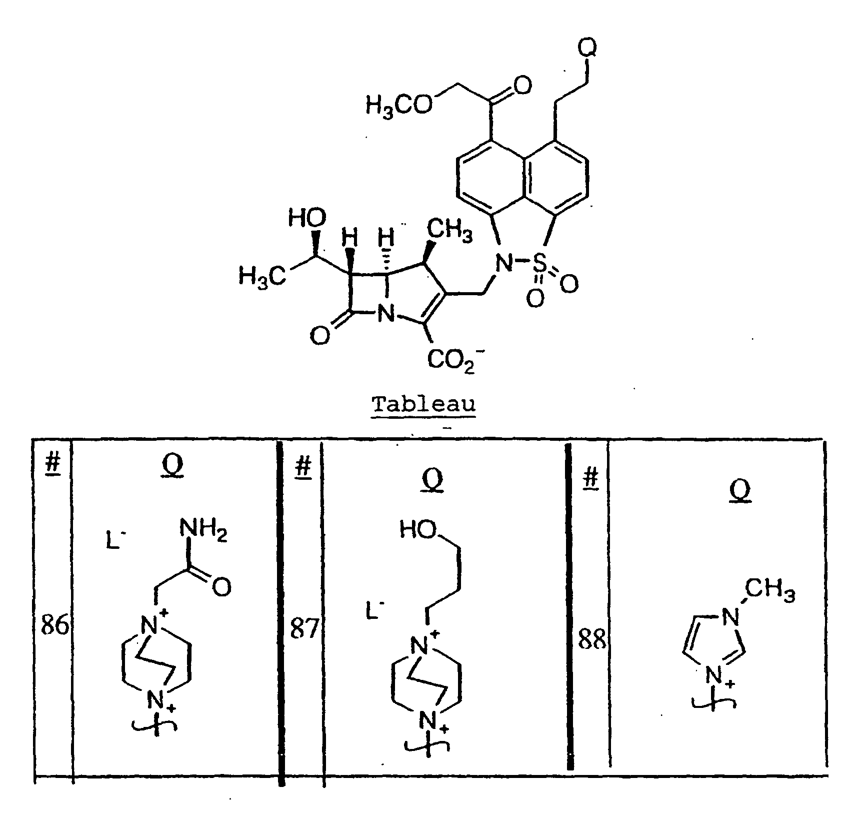 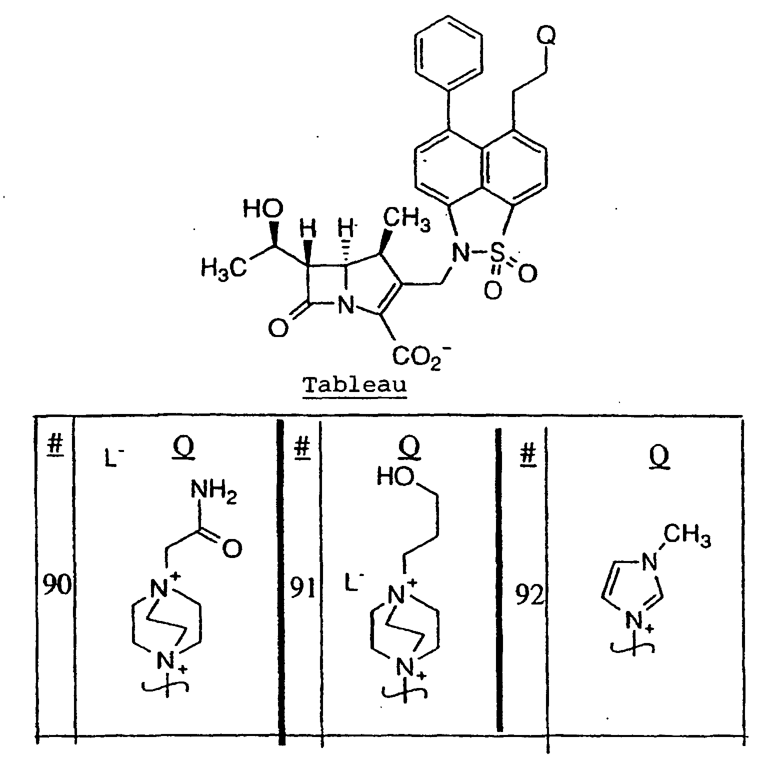 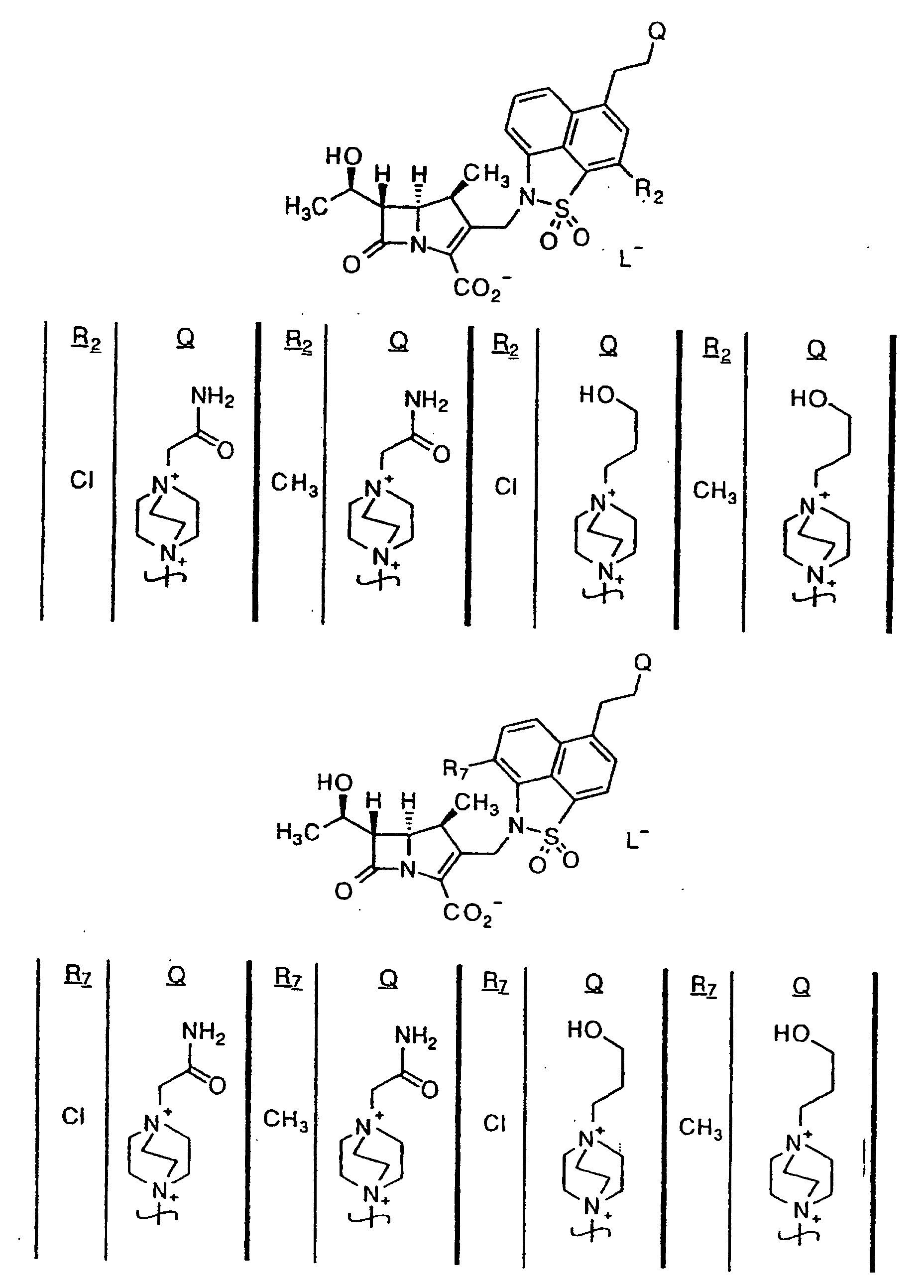 dans lesquels L⁻ représente un contre-ion pharmaceutiquement acceptable.

25. Composition pharmaceutique constituée d'un composé selon l'une quelconque des revendications 1 à 24 en combinaison avec un véhicule pharmaceutiquement acceptable.

26. Composition pharmaceutique produite en combinant un composé selon l'une quelconque des revendications 1 à 24 avec un véhicule pharmaceutiquement acceptable.

27. Utilisation d'un composé selon l'une quelconque des revendications 1 à 24 pour la fabrication d'un médicament destiné au traitement ou à la prévention d'une infection bactérienne.
